(19) Europäisches Patentamt
European Patent Office
Office européen des brevets

(11) Publication number: **0 117 014**
**B1**

(12) **EUROPEAN PATENT SPECIFICATION**

(45) Date of publication of patent specification: **06.07.88**

(21) Application number: **84300013.4**

(22) Date of filing: **03.01.84**

(51) Int. Cl.[4]: **C 07 D 239/42,**
C 07 D 239/47,
C 07 D 239/52,
C 07 D 251/46,
C 07 D 409/12, A 01 N 47/36

# (54) Herbicidal N-hydroxy-N'-sulfonylguanidines and sulfonamide inner salts.

(30) Priority: **04.01.83 US 455504**
**17.11.83 US 551381**
**18.02.83 US 467650**
**16.11.83 US 551004**

(43) Date of publication of application:
**29.08.84 Bulletin 84/35**

(45) Publication of the grant of the patent:
**06.07.88 Bulletin 88/27**

(84) Designated Contracting States:
**AT BE CH DE FR GB IT LI LU NL SE**

(56) References cited:
**EP-A-0 005 986**
**EP-A-0 048 143**
**US-A-4 127 405**
**US-A-4 169 719**
**US-A-4 301 286**
**US-A-4 310 346**
**US-A-4 394 506**

(73) Proprietor: **E.I. DU PONT DE NEMOURS AND COMPANY**
**1007 Market Street**
**Wilmington Delaware 19898 (US)**

(72) Inventor: **Shapiro, Rafael**
**1415 Fresno Road**
**Wilmington Delaware 19803 (US)**
Inventor: **Tseng, Chi-Ping**
**1103 Artwin Road**
**Wilmington Delaware 19803 (US)**

(74) Representative: **Hildyard, Edward Martin et al**
**Frank B. Dehn & Co. Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 117 014 B1

**Description**

This invention relates to novel sulfonamide inner salts, to novel N-hydroxy-N'-sulfonylguanidine compounds, to herbicidal compositions containing them and to methods of using them to control the growth of undesired vegetation.

U.S. Patent 4,169,719 discloses herbicidal sulfonamides of the general formula

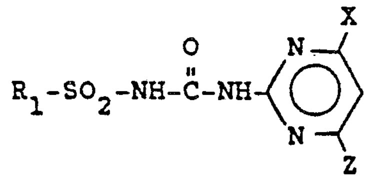

where $R_1$ can be substituted phenyl, optionally substituted thiophene or naphthalene. U.S. 4,127,405 discloses the corresponding triazine compounds. A number of additional patents or patent applications have issued or been published which disclose herbicidal sulfonamide derivatives, but no references are known which disclose the dialkylsulfonium inner salts of this invention.

U.S. Patent 4,127,405 issued November 28, 1978 discloses substituted triazinyl arylsulfonylurea compounds of the following formula:

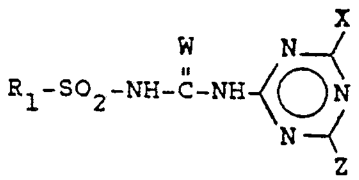

while U.S. Patent 4,169,719 issued October 2, 1979 discloses substituted pyrimidinyl arylsulfonylurea compounds of the following formula:

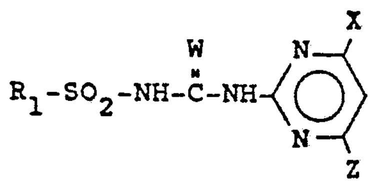

The triazine and pyrimidine compounds within the scope of the '405 and '719 patents include those wherein W can be oxygen or sulfur.

U.S. Patent 4,310,346 issued January 12, 1982 discloses sulfonylisothiourea compounds of the formula

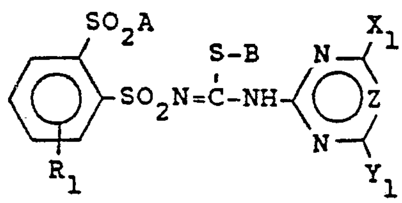

wherein

B is $C_1$—$C_6$ alkyl, $CH_2CH_2OCH_3$, $CH_2CH_2OCH_2CH_3$, $CH_2CH_2CH_2OCH_3$, $CH_2Q$,

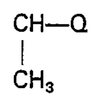

where Q is $CO_2$—$C_{1-3}$ alkyl,

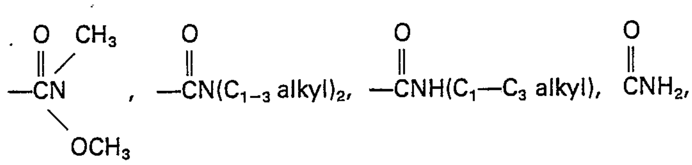

phenyl, phenyl substituted with chlorine, CN, $C_{2-4}$ alkenyl, $C_{2-4}$ alkynyl, $OR_{11}$, where $R_{11}$ is $C_1$—$C_4$ alkyl, —$CH_2OCH_2CH_2OCH_3$ or —$CH_2OCH_2CH_2OCH_2CH_3$.

South African Patent No. 79/2657, published June 30, 1980 discloses sulfonylisothiourea compounds of the formula

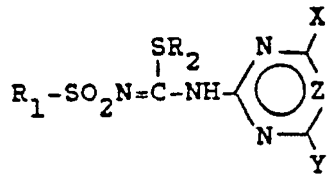

wherein

$R_2$ is $C_1$—$C_{20}$ alkyl; $CH_2CH_2OCH_3$; $CH_2CH_2OCH_2CH_3$; $CH_2CH_2CH_2OCH_3$; $CH_2OA'$ where A' is $C_1$—$C_{12}$ alkyl, —$CH_2CH_2OCH_3$, —$CH_2CH_2OCH_2CH_3$, phenyl or phenyl substituted with 1—2 $NO_2$, 1—2 Cl, or 1—2 $CH_3$; $CH_2A$,

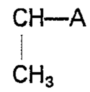

where A is $CO_2$(H, $C_1$—$C_4$ alkyl),

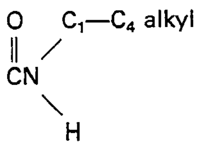 , 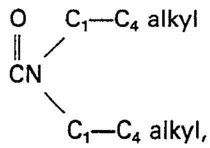 , 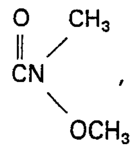 , 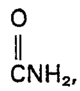

phenyl, CN, $C_2$—$C_4$ alkenyl, $C_1$—$C_4$ alkynyl, phenyl substituted with 1—2 $CH_3$, 1—2 $NO_2$, 1—2 $OCH_3$, 1—2 chlorine or phenoxy.

U.S. 4,301,286 issued November 17, 1981 discloses O-alkylsulfonylisoureas of the formula

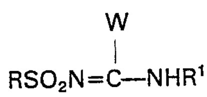

wherein

W is Cl, Br or $OR^{12}$; and

$R^{12}$ is $C_1$—$C_{12}$ alkyl, $C_3$—$C_4$ alkenyl, $CH_2CH_2OCH_3$, $CH_2CH_2OCH_2CH_3$, $(CH_2)_3OCH_3$, benzyl, $CHR^{13}CO_2R^{14}$, where $R^{13}$ is H or $CH_3$ and $R^{14}$ is $C_1$—$C_4$ alkyl.

Still further sulfonylisourea and sulfonylisothiourea are disclosed in our EP—A—30,141 and EP—A—30,142.

The compounds taught in the above seven references are useful as general or selective herbicides having both preemergent and postemergent herbicidal activity or plant growth regulant activity.

## Summary of the Invention

This invention relates to novel compounds of Formulas I and II, agriculturally suitable compositions containing them and their method-of-use as pre-emergent and/or post-emergent herbicides or plant growth regulants.

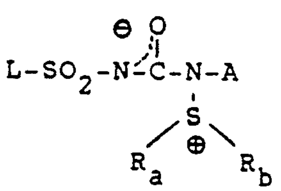 I

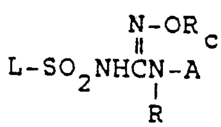 II

wherein

$R_a$ is $CH_3$ or $CH_2CH_3$;

$R_b$ is $CH_3$ or $CH_2CH_3$;

$R_c$ is H, $C(O)R_1$, $C(O)NR_2R_3$ or $CO_2R_4$;

R is H or $CH_3$;

$R_1$ is $C_1$—$C_3$ alkyl or $CF_3$;

$R_2$ is H, $CH_3$ or $C_2H_5$;

$R_3$ is $C_1$—$C_3$ alkyl;

$R_4$ is $C_1$—$C_3$ alkyl;

L is

L is 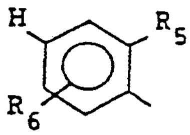 , 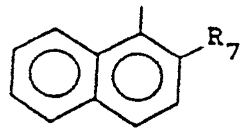 , 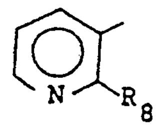 ,

L-1 L-2 L-3

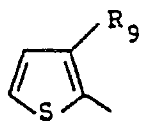 , 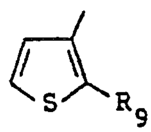 , 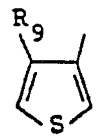 ,

L-4 L-5 L-6

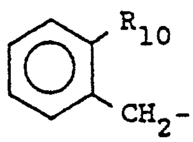 , 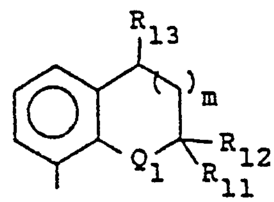 ,

L-7 L-8

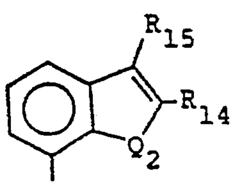 , 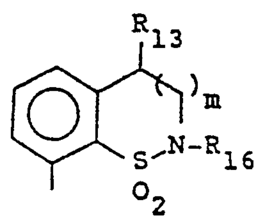 , 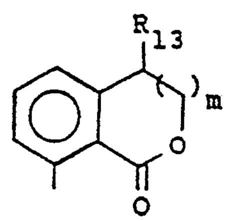 ,

L-9 L-10 L-11

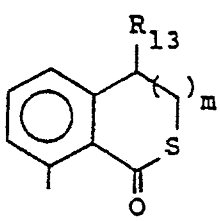 , 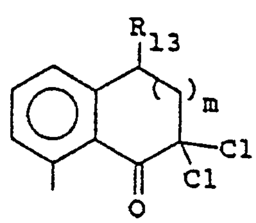 , 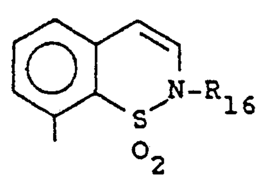 ,

L-12 L-13 L-14

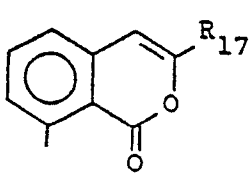 , 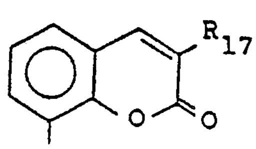 or 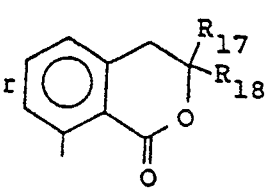 ;

L-15 L-16 L-17

$R_5$ is $C_1$—$C_4$ alkyl, $C_1$—$C_4$ alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ alkenyloxy, $C_3$—$C_4$ alkynyloxy, $C_1$—$C_2$ alkyl substituted with $OCH_3$ or

$OCH_2CH_3$, $C_6H_5$, 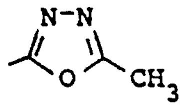 , 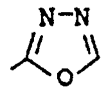 ,

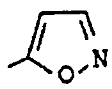 , 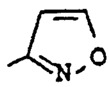 , 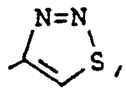 , 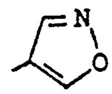 , 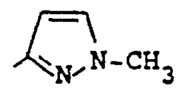 ,

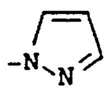 , 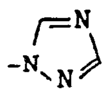 , 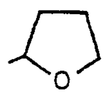 , 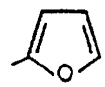 or 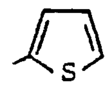 ;

$R_6$ is H, F, Cl, Br, $CF_3$, $CH_3$, $OCH_3$, $SCH_3$ or $OCF_2H$;
$R_7$ is H, $CH_3$, $OCH_3$, F, Cl, Br, $OSO_2CH_3$, $SO_2N(CH_3)_2$ or $S(O)_nCH_3$;
$R_8$ is $CH_3$, $CH_2CH_3$, $OCH_3$, $OCH_2CH_3$, F, Cl, Br, $SO_2NR_{20}R_{21}$, $SO_2N(OCH_3)CH_3$ or $S(O)_nR_{23}$;
$R_9$ is $C_1$—$C_3$ alkyl, F, Cl, Br, $NO_2$, $CO_2R_{19}$, $SO_2NR_{20}R_{21}$, $SO_2N(OCH_3)CH_3$ or $S(O)_nR_{23}$;
$R_{10}$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;
$R_{11}$ is H, $CH_3$ or $CH_2CH_3$;
$R_{12}$ is H, $CH_3$ or $CH_2CH_3$;
$R_{13}$ is H or $CH_3$;
$R_{14}$ is H or $CH_3$;
$R_{15}$ is H or $CH_3$;
$R_{16}$ is $CH_3$ or $CH_2CH_3$;
$R_{17}$ is H or $C_1$—$C_4$ alkyl;
$R_{18}$ is H or $CH_3$;
$R_{19}$ is $C_1$—$C_4$ alkyl, $CH_2CH_2OCH_3$, $CH_2CH_2Cl$ or $CH_2CH{=}CH_2$;
$R_{20}$ is $C_1$—$C_3$ alkyl;
$R_{21}$ is $C_1$—$C_3$ alkyl;
$R_{22}$ is $C_1$—$C_3$ alkyl or $N(CH_3)_2$;
$R_{23}$ is $C_1$—$C_3$ alkyl or $CH_2CH{=}CH_2$;
m is 0 or 1;
n is 0 or 2;
$Q_1$ is O, S, $SO_2$ or $NR_{17}$;
$Q_2$ is O, S or $NR_{17}$; and
W is O, S or $SO_2$;
A is

A is 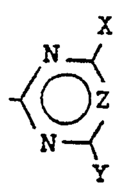 , 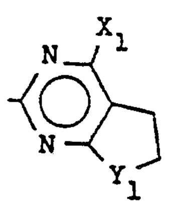 , 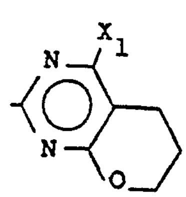 .

A-1 A-2 A-3

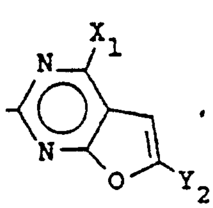 , 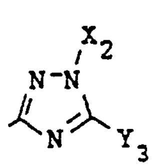 or 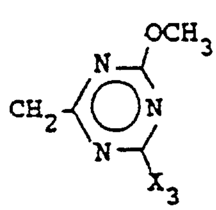 ;

A-4 A-5 A-6

X is $CH_3$, $OCH_3$, $OCH_2CH_3$, Cl, F, Br, $OCF_2H$, $CH_2F$ or $CF_3$;

Y is H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

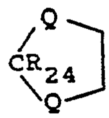,
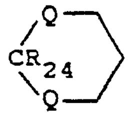,
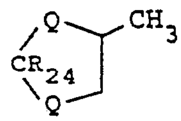
or $CR_{24}(QCH_2CH_3)_2$;

Q is O or S;

$R_{24}$ is H or $CH_3$;

Z is CH or N;

$Y_1$ is $CH_2$ or O;

$X_1$ is $CH_3$, $OCH_3$, $OCH_2CH_3$ or $OCF_2H$;

$Y_2$ is H or $CH_3$;

$X_2$ is $CH_3$, $CH_2CH_3$ or $CH_2CF_3$;

$Y_3$ is $OCH_3$, $OCH_2CH_3$, $SCH_3$, $CH_3$ or $CH_2CH_3$;

$X_3$ is $CH_3$ or $OCH_3$;

provided that

1) the total number of carbon atoms of $R_{20}$ and $R_{21}$ is less than or equal to four;

2) when m is 1, then $R_{13}$ is H;

3) when L is L—17, then $R_{17}$ and $R_{18}$ are not simultaneously H; and

4) when X is Cl, F or Br, then Z is CH and Y is $OCH_3$, $OCH_2CH_3$, $N(CH_3)_2$ or $OCF_2H$;

and also provided that for compounds of Formula I when L is L—1, $R_5$ is not

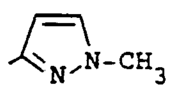,
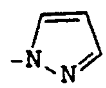
or
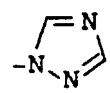.

Preferred for reasons of higher herbicidal activity, greater plant growth regulant activity or more favorable ease of or utility for synthesis are:

1) Compounds of Formula I where A is A—1.

2) Compounds of Preferred 1 where Y is $CH_3$, $OCH_3$, $OCH_2CH_3$, $CH_2OCH_3$, $OCH_2CF_3$ or $CH(OCH_3)_2$ and X is $CH_3$, $OCH_3$, Cl or $CF_3$.

3) Compounds of Preferred 2 where L is L—1, L—2, L—3, L—5, L—8, L—10, L—11, L—16 or L—17.

4) Compounds of Preferred 3 where L is L—1, $R_5$ is $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

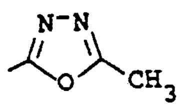,
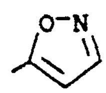,
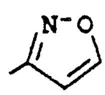
or
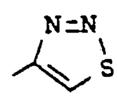;

$R_{22}$ is $C_1$—$C_3$ alkyl; $R_{23}$ is $CH_3$ and n is 2.

5) Compounds of Preferred 3 where L is L—2 and $R_7$ is Cl, $CH_3$, $OCH_3$, $SCH_3$ or Br.

6) Compounds of Preferred 3 where L is L—3 and $R_8$ is Cl, $SO_2CH_3$ or $SO_2N(CH_3)_2$.

7) Compounds of Preferred 3 where L is L—5 and $R_9$ is $CO_2CH_3$ or $CO_2CH_2CH_3$.

8) Compounds of Preferred 3 where L is L—8.

9) Compounds of Preferred 3 where L is L—10.

10) Compounds of Preferred 3 where L is L—11.

11) Compounds of Preferred 3 where L is L—16.

12) Compounds of Preferred 3 where L is L—17.

13) Compounds of Formula II where R is H, $R_c$ is H and A is A—1.

14) Compounds of Preferred 13 where Y is $CH_3$, $OCH_3$, $OCH_2CH_3$, $CH_2OCH_3$, $OCH_2CF_3$ or $CH(OCH_3)_2$ and X is $CH_3$, $OCH_3$, Cl or $CF_3$.

15) Compounds of Preferred 14 where L is L—1, L—2, L—3, L—5, L—8, L—10, L—11, L—16 or L—17.

16) Compounds of Preferred 15 where L is L—1, $R_5$ is $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

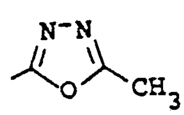,
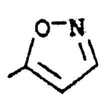,
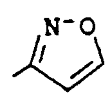
or
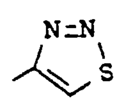;

$R_{22}$ is $C_1$—$C_3$ alkyl; $R_{23}$ is $CH_3$ and n is 2.

17) Compounds of Preferred 15 where L is L—2 and $R_7$ is Cl, $CH_3$, $OCH_3$, $SCH_3$ or Br.

18) Compounds of Preferred 15 where L is L—3 and $R_8$ is Cl, $SO_2CH_3$ or $SO_2N(CH_3)_2$.

19) Compounds of Preferred 15 where L is L—5 and $R_9$ is $CO_2CH_3$ or $CO_2CH_2CH_3$.

20) Compounds of Preferred 15 where L is L—8.

21) Compounds of Preferred 15 where L is L—10.

22) Compounds of Preferred 15 where L is L—11.

23) Compounds of Preferred 15 where L is L—16.

24) Compounds of Preferred 15 where L is L—17.

Specifically Preferred for reasons of their highest herbicidal activity or greater plant growth regulant activity or most favorable ease of synthesis are:

2-[[(4-methoxy-6-methylpyrimidin-2-yl)aminocarbonyl]aminosulfonyl]benzoic acid methyl ester, dimethylsulfonium inner salt;

2-[[(4,6-dimethoxypyrimidin-2-yl)aminocarbonyl]aminosulfonyl]benzoic acid methyl ester, dimethylsulfonium inner salt;

N-[(4,6-dimethylpyrimidin-2-yl)aminocarbonyl]-2-methylsulfonylbenzenesulfonamide, dimethylsulfonium inner salt;

2-[[(4,6-dimethylpyrimidin-2-yl)aminocarbonyl]aminosulfonyl]benzoic acid methyl ester, dimethylsulfonium inner salt;

N-[(4,6-dimethoxypyrimidin-2-yl)aminocarbonyl]-2-dimethylaminosulfonylbenzenesulfonamide, dimethylsulfonium inner salt;

2-[[1-(4,6-dimethylpyrimidin-2-yl)amino]-1-(hydroxyimino)methyl]aminosulfonyl]benzoic acid, methyl ester;

2-[[1-(4-methoxy-6-methylpyrimidin-2-yl)amino]-1-(hydroxyimino)methyl]aminosulfonyl]benzoic acid, methyl ester;

N',N'-dimethyl-N-[[1-(4,6-dimethyl-1,3,5-triazin-2-yl)amino]-1-(hydroxyimino)methyl]-1,2-benzenedisulfonamide; and

N',N'-dimethyl-N-[[1-(4-methoxy-6-methylpyrimidin-2-yl)amino]-1-(hydroxyimino)methyl]-1,2-benzenedisulfonamide.

Other groups of compounds within the scope of our invention include the compounds of formula (I) wherein

L is L—1, L—2, L—3, L—4, L—5, L—8, L—10, L—11 or L—17;

$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, $C_3$—$C_4$ alkenyloxy, $CH_2CH_2OCH_3$, $CH_2CH_2OC_2H_5$,

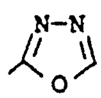 , 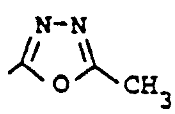 , 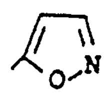 , 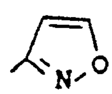 or 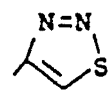 ;

$R_6$ is H, F, Cl, Br, $CF_3$, $CH_3$ or $OCH_3$;

$R_7$ is H, $CH_3$, $OCH_3$, Cl, $OSO_2CH_3$ or $S(O)_nCH_3$;

$R_8$ is $CH_3$, $OCH_3$, Cl, $SO_2NR_{20}R_{21}$ or $SO_2N(OCH_3)CH_3$;

$R_9$ is $CH_3$, Cl, Br, $NO_2$, $CO_2R_{19}$, $SO_2NR_{20}R_{21}$, $SO_2N(OCH_3)CH_3$ or $S(O)_nR_{13}$;

$R_{11}$ is H or $CH_3$;

$R_{12}$ is H or $CH_3$;

$R_{13}$ is H;

$R_{17}$ and $R_{18}$ are H;

$R_{22}$ is $C_1$—$C_3$ alkyl;

$Q_1$ is O, S or $SO_2$;

A is A—1;

X is $CH_3$ or $OCH_3$; or may be Cl when Y is $CH_3$ or $OCH_3$;

Y is H, $CH_3$, $OCH_3$, $OCH_2CH_3$, $CH_2OCH_3$, $CH_2CH_3$, $CF_3$, $OCH_2CF_3$, $CH(OCH_3)_2$;

and $R_a$, $R_b$, $R_{16}$, $R_{19}$, $R_{20}$, $R_{21}$, $R_{23}$, m, n and Z are as defined above;

and compounds of formula (II) wherein

R is H or $CH_3$;

$R_c$ is as defined above;

L is L—1;

$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, or $C_1$—$C_2$ alkyl substituted with $OCH_3$ or $OC_2H_5$;

$R_6$ is H, F, Cl, Br, $CF_3$, $CH_3$, $OCH_3$ or $SCH_3$;

$R_{19}$, $R_{20}$, $R_{21}$, $R_{23}$ and W are as defined above;

$R_{22}$ is $C_1$—$C_3$ alkyl;

A is A—1;

X is $CH_3$, $OCH_3$ or Cl;

Y is $CH_3$, $C_2H_5$, $OCH_3$, $OC_2H_5$, $CH_2OCH_3$, $CH(OCH_3)_2$ or

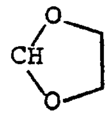

and Z is as defined above.

## Detailed Description of the Invention

### Synthesis

The compounds of Formula I can be prepared by contacting an appropriate sulfonyl isocyanate IV with an S,S-dialkyl-N-(heterocyclic)sulfilimine V as illustrated in Equation 1. (L, $R_a$, $R_b$ and A are as previously defined.)

*Equation 1*

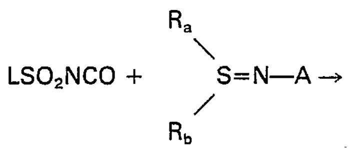

I

IV V

The reaction of Equation 1 is best carried out in an inert aprotic solvent, such as methylene chloride, tetrahydrofuran, acetonitrile, ether or chloroform, at temperatures ranging from about −20 to 50°C. In some cases, the desired product may crystallize from the reaction medium and may be filtered. Reaction products which are soluble in the reaction medium may be isolated by evaporation of the solvent, trituration of the residue with solvents such as diethyl ether, ethyl acetate, 1-chlorobutane or hexane. Chromatography (e.g., silica gel) may also be necessary for purification.

The sulfonyl isocyanates IV used as starting materials are generally known in the art and can be prepared by known methods. One method involves reacting an appropriate benzene or heterocyclic sulfonamide with phosgene in the presence of an alkyl isocyanate, such as *n*-butyl isocyanate, and a tertiary amine catalyst, such as 1,4-diazabicyclo[2.2.2.]octane, at reflux in a solvent such as xylene or chlorobenzene. See H. Ulrich and A. A. Y. Sayigh, *Newer Methods of Preparative Organic Chemistry*, Vol. VI, p. 223—241, Academic Press, New York and London, W. Foerst Ed.

The sulfonyl isocyanates IV can also be prepared from sulfonamides by a two step procedure involving (a) reacting the sulfonamides with an alkyl isocyanate in the presence of a base such as $K_2CO_3$ at reflux in an inert solvent such as 2-butanone, forming an alkylsulfonylurea, and (b) reacting this compound with phosgene and tertiary amine catalyst at reflux in xylene solvent.

The preparation of sulfonamides from ammonium hydroxide and sulfonyl chlorides is widely reported in the literature, e.g. Crossley *et al., J. Am. Chem. Soc., 60*, 2223 (1938). Certain sulfonyl chlorides are best prepared by chlorosulfonation of a substituted benzene, naphthalene, or thiophene in carbon tetrachloride according to the teaching of H. T. Clarke *et al., Org. Synth. Coll.*, Vol. 1, 2nd Ed. 1941, p. 85. Other sulfonyl chlorides can be made by diazotization of the appropriate amine with sodium nitrite in HCl, followed by reaction of the diazonium salt with sulfur dioxide and cuprous chloride in acetic acid according to the teachings of H. L. Yale and F. Sowinski, *J. Org. Chem., 25*, 1824 (1960).

Reference to the following patents and patent applications is suggested for further details regarding the preparation of the sulfonyl isocyanates IV: U.S. Patent 4,169,719, U.S. Patent 4,127,405; U.S. Patent 4,383,113; U.S. Patent 4,394,506; U.S. Patent 4,120,691; U.S. Patent 4,238,621; EP—A—30,141; EP—A—13,480; Canadian Patent 1,128,042; EP—A—64,804 and EP—A—51,466.

The sulfilimine (V) starting materials may be made in a number of ways. S,S-Dimethyl-N-(4,6-dimethylpyrimidin-2-yl)sulfilimine and S,S-dimethyl-N-(4-methylpyrimidin-2-yl)sulfilimine are taught by T. L. Gilchrist, C. J. Harris and C. W. Rees in *J.C.S. Chem. Comm.*, 486 (1974). The procedure taught in this article can be adapted to prepare other sulfilimines of Formula V as shown in Equation 2.

*Equation 2*

$$A{-}NH_2 + t\text{-}BuOCl \rightarrow \xrightarrow{SR_aR_b} \xrightarrow{NaOCH_3} V$$

VI

A heterocyclic amine VI is contacted successively with *tert*-butyloxy chloride, a dialkylmercaptide and sodium methoxide.

The reaction may be best carried out in an inert aprotic solvent, such as methylene chloride, chloroform or ethyl acetate at temperatures between −60° to 60°C for 1—48 hours with ambient pressure. The resulting solution may be washed with water. In some cases, the desired product may crystallize from the organic solvent and may be filtered. Reaction products which are soluble in the organic solvent may be isolated by evaporation of the solvent after the organic solution is dried over a drying agent such as sodium sulfate or magnesium sulfate and trituration of the residue with solvents such as diethyl ether, 1-chlorobutane or hexane. Chromatography (e.g., silica gel) may also be necessary for purification.

Sulfilimines of Formula V can also be prepared by the general procedure illustrated in Equation 3.

*Equation 3*

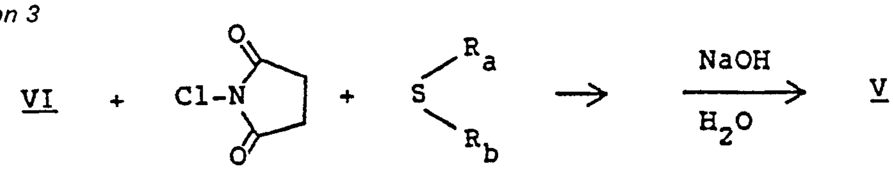


The appropriate amine VI is contacted with the appropriate dialkylmercaptide in the presence of N-chlorosuccinimide.

The reaction is best carried out in an inert aprotic solvent, such as methylene chloride, chloroform or ethyl acetate at temperatures between −30° to 60°C for 1—48 hours with ambient pressure. The resulting solution is then washed with aqueous sodium hydroxide solution and then water. The desired product can be isolated as described in the previous paragraph.

Yet another method for preparing the sulfilimines V is shown in Equation 4.

*Equation 4*

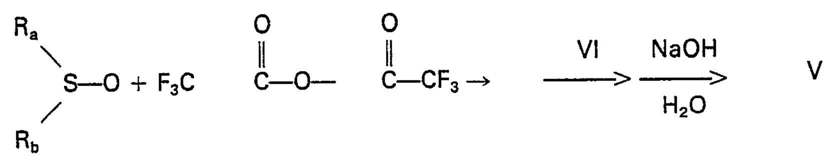


The reaction of Equation 4 may be best carried out in an inert aprotic solvent, such as methylene chloride or chloroform at temperatures between −70° to 70°C for 1—48 hours with ambient pressure. The resulting solution may be washed with sodium hydroxide aqueous solution and then water. The desired product can be isolated as described in the previous paragraph.

Heterocyclic amines of Formula VI and methods for preparing them are known in the art. The synthesis of heterocyclic amines such as those of Formula VI has been reviewed in "The Chemistry of Heterocyclic Compounds", a series published by Interscience Publ., New York and London. 2-Aminopyrimidines are described by D. J. Brown in "The Pyrimidines", Vol. XVI of this series. 2-Amino-1,3,5-triazines can be prepared according to methods described by E. M. Smolin and L. Rapaport in "*s*-Triazines and Derivatives", Vol. XIII of the same series. The synthesis of triazines is also described by F. C. Schaefer, U.S. 3,154,547 and by K. R. Huffman and F. C. Schaefer; *J. Org. Chem., 28*, 1816 (1963). See also European Patent Application Number 80300505.7, published September 17, 1980 (Pub. No. 15,683), European Patent Application Number 81303837.9, published March 3, 1982 (Pub. No. 46,677), European Patent Application Number 82306492.8, published July 27, 1983 (Pub. No. 84,224), and European Patent Application Number 82303611.6, published March 9, 1983 (Pub. No. 73,562), for description of methods for preparing heterocyclic amine derivatives.

The compounds of Formula IIa may be prepared by the reaction of hydroxylamine with an appropriately substituted S-alkyl benzenesulfonyl isothiourea of Formula III as shown in Equation 5.

*Equation 5*

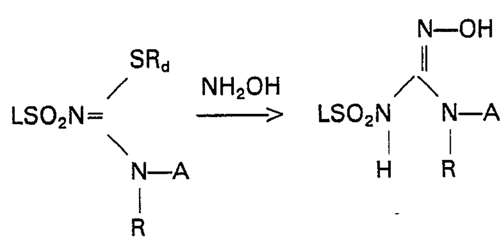


III IIa

wherein L, R and A are as previously defined, and $R_d$ is $C_1$—$C_3$ alkyl.

The reaction of Equation 5 is conducted by contacting 1—3 equivalents of hydroxylamine hydrochloride and an equivalent amount of sodium acetate with a compound of Formula III in a suitable

solvent, such as tetrahydrofuran, at 0—30° for 1—24 hours. The product may be isolated by removal of solvent and trituration of the residue with water.

The compounds of Formula III may be prepared by the methods described in U.S. 4,301,286 to which the reader is referred for further information.

Briefly, these products may be made by reacting a compound of formula

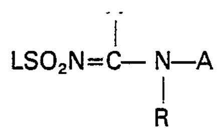

wherein X′ is Cl or Br, with $MSR_d$, where M is alkali metal i.e. Na, K or Li; or by reacting a compound of formula

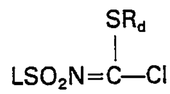

with the lithium salt of a 2-aminoheterocycle of formula

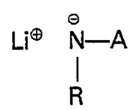

Reaction conditions and the preparation of intermediates for these reactions are described in our aforesaid U.S. Patent.

Alternatively, the compound of formula III may be prepared by the procedure outlined in Equation 6.

*Equation 6*

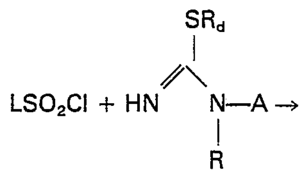

III

VIII VIII

wherein L, R, $R_d$ and A are as previously defined.

The reaction of Equation 6 may be carried out by contacting a sulfonyl chloride of Formula VII with an appropriate substituted S-alkyl isothiourea of Formula VIII in an inert solvent, such as methylene chloride or tetrahydrofuran, in the presence of an acid scavenger, such as aqueous sodium bicarbonate or a tertiary amine, such as triethylamine, for 1—3 days at 20—40°. The product is isolated by removal of solvent and trituration of the residue with water, or by extraction from water with an organic solvent, concentration, and chromatography.

Those references provided above for the preparation of the sulfonyl isocyanates IV also provide details for the preparation of the sulfonyl chlorides VII.

The preparation of the compounds of Formula VIII is shown in Equations 7, 8 and 9.

*Equation 7*

(7a)

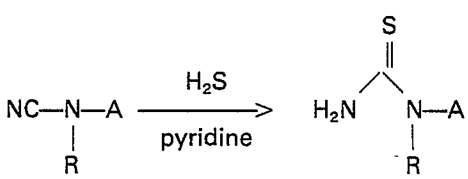


IX X

(7b)

X

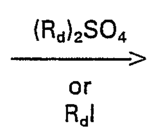


VIII

wherein R, $R_d$ and A are as previously defined.

The reaction of Equation 7a may be carried out by saturation of a solution of a compound of Formula IX in pyridine with hydrogen sulfide, allowing to react for 1 to 5 days at 25 to 40°, removal of pyridine *in vacuo*, and crystallization of the product with water or an organic solvent such as ether or methylene chloride. The resulting compound of Formula X may be reacted according to Equation 7b with one equivalent of a dialkyl sulfate or alkyl iodide at 20 to 80° for 1 to 3 days to provide the corresponding alkyl sulfate or iodide salt of VIII. The free base may be obtained by extraction from aqueous sodium bicarbonate with an organic solvent, such as ethyl acetate. An alternative synthesis of compounds of Formula X is shown in Equation 8.

*Equation 8*

(8a)

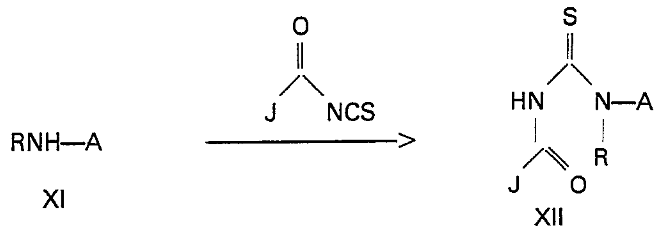

XI

XII

(8b)

XII

$H_2O$

NaOH or

MgO

X

wherein R and A are as previously defined; and J is $C_6H_5$ or $C_6H_5O$.

The reaction of Equation 8a is carried out by contacting a compound of Formula XI with 1—1.5 equivalents of benzoyl- or phenoxycarbonylisothiocyanate at 30—80° for 1—24 hours in a suitable solvent, such as acetonitrile or tetrahydrofuran. The product may be isolated by cooling or concentration and filtration. The reaction of Equation 8b may be performed by heating the compound of Formula XII in an aqueous medium, with or without a cosolvent, in the presence of 1 to 2 equivalents of an alkali metal hydroxide or an alkali earth oxide at 30 to 80° for 1—24 hours. The product is isolated by cooling, neutralization to pH 8, and filtration in the case where an alkali metal hydroxide is used, and in the case where an alkali earth oxide is used, the reaction mixture is filtered and the filtrate is concentrated, cooled, and filtered.

Heterocyclic amines of Formula XI may be prepared by the same methods cited for the synthesis of the amines of Formula VI.

A third method by which certain compounds of Formula VIIIa may be obtained is shown in Equation 9.

*Equation 9*

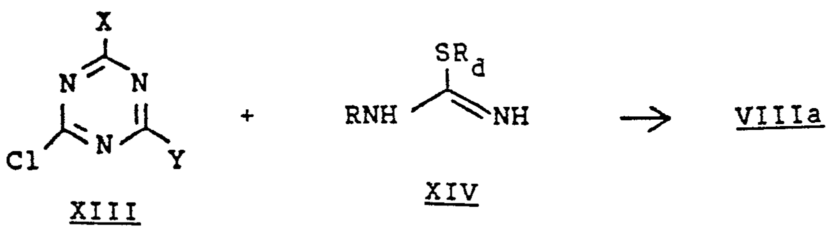

XIII + XIV VIIIa

wherein R, $R_d$, X and Y are as previously defined.

The reaction of Equation 9 may be carried out by contacting a chlorotriazine of Formula XIII with an S-alkyl isothiourea of Formula XIV in the same manner as was described in the reaction of Equation 6.

Compounds of Formulae IIb and IIc may be obtained from those of Formula IIa by the reactions of Equation 10.

*Equation 10*

(10a)

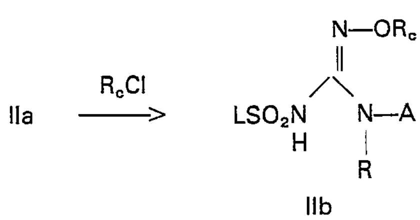


IIa

IIb

(10b)

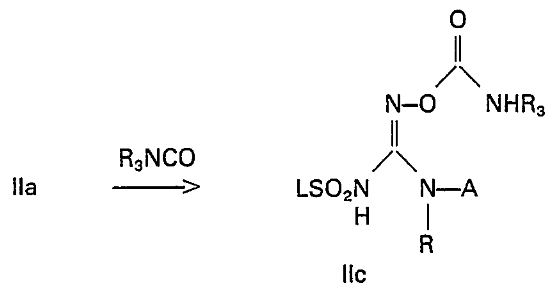


wherein $R_c$ is $C(O)R_1$, $C(O)NR_2R_3$, or $CO_2R_4$, and L, R, $R_1$, $R_2$, $R_3$, $R_4$, and A are as previously defined.

The reaction of Equation 10a is carried out by contacting IIa with 1—10 equivalents of the appropriate carboxylic acid chloride or anhydride, or alkyl chloroformate, or dialkyl carbamoyl chloride in the presence of an acid scavenger, such as pyridine or a tertiary amine, at 0 to 40° for 2 to 24 hours, adding ice-water, and filtering or extracting the product into an organic solvent.

The reaction of Equation 10b may be conducted by treating a solution of IIa in an inert solvent such as methylene chloride or acetonitrile with 1—1.5 equivalents of an alkyl isocyanate at 20—80° for 1 to 24 hours. The product is isolated by concentrating the reaction mixture and trituration with a solvent such as ether or chlorobutane.

Agriculturally suitable salts of compounds of Formula II are also useful herbicides. Salts of compounds of Formula II can be prepared in a number of ways known to the art. For example, metal salts can be made by treating compounds of Formula II with a solution of an alkali or alkaline earth metal salt having a sufficiently basic anion (e.g., hydroxide, alkoxide, carbonate or hydride). Quaternary amine salts can be made by similar techniques.

Salts of compounds of Formula II can also be prepared by exchange of one cation for another. Cationic exchange can be effected by direct treatment of an aqueous solution of a salt of a compound of Formula II (e.g., alkali metal or quaternary amine salt) with a solution containing the cation to be exchanged.

This method is most effective when the desired salt containing the exchanged cation is insoluble in water, e.g., a copper salt, and can be separated by filtration.

Exchange may also be effected by passing an aqueous solution of a salt of a compound of Formula II (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula II with a suitable acid, e.g., *p*-toluenesulfonic acid or trichloroacetic acid.

In the following examples, all parts are by weight and temperatures in °C.

Example 1

Preparation of S,S-dimethyl-N-(4,6-dimethylpyrimidin-2-yl)sulfilimine

To a solution of 12.3 g (0.10 mole) of 2-amino-4,6-dimethylpyrimidine and 8 ml (0.109 mole) of methylsulfide in 100 ml of methylene chloride at −20°C was added dropwise a solution of 13.3 g (0.10 mole) of N-chlorosuccinimide in 300 ml of methylene chloride. After addition, the resulting solution was warmed to room temperature and was kept at room temperature for one hour with stirring. The solution was then washed with 400 ml of 2.5% sodium hydroxide aqueous solution and then 400 ml of water. This solution was dried over sodium sulfate and filtered. The filtrate was washed with ethyl ether, and dried under vacuum at ambient temperature to afford 4 g of the title compound as a white solid, m.p. 117—120°C.

NMR: δ 2.23 (s, $CH_3$); δ 2.7 (s, $SCH_3$); and δ 6.23 (s, ArH).

Example 2

Preparation of S,S-dimethyl-N-(4,6-dimethoxypyrimidin-2-yl)sulfilimine

To a solution of 15.5 g (0.10 mole) of 2-amino-4,6-dimethoxypyrimidine and 8 ml (0.109 mole) of methylsulfide in 100 ml of methylene chloride at −20°C was added dropwise a solution of 13.3 g (0.10 mole) of N-chlorosuccinimide in 300 ml of methylene chloride. After addition, the resulting solution stirred at −20°C for 2 hours and then warmed up to room temperature. The solution was stirred at room temperature for another hour. The solution was washed with 400 ml of 2.5% sodium hydroxide aqueous solution and then 400 ml of water. The organic layer was separated, dried over sodium sulfate and concentrated under reduced pressure. The residue was washed with ethyl ether, and dried under vacuum at ambient temperature to afford 6 g of the title compound as a solid, m.p. 102—103°C (dec.).

NMR: δ 2.73 (s, $SCH_3$); δ 3.9 (s, $OCH_3$); and δ 5.37 (s, ArH).

Example 3

[[N-(2-Chlorophenylsulfonylaminocarbonyl)-N-(4,6-dimethylpyrimidin-2-yl)]amino]dimethylsulfonium inner salt

To a solution of 0.6 g (0.0033 mole) of S,S-dimethyl-N-(4,6-dimethylpyrimidin-2-yl)sulfilimine in 15 ml of methylene chloride was added 1.5 g (0.0069 mole) of 2-chlorophenylsulfonyl isocyanate.

The resulting solution was stirred at ambient temperature for 12 hours. The solution was then concentrated under reduced pressure. The residue was washed with 1-chlorobutane, ethyl ether and ethyl acetate. The solid was collected by filtration and dried under vacuum to afford 1.1 g of the title compound, m.p. 104—105°C (dec.).

NMR: δ 2.43 (s, $CH_3$); δ 2.87 (s, $SCH_3$); and δ 6.5—8.5 (m, ArH).

Example 4

2-Chloro-N-[[(4,6-dimethylpyrimidin-2-yl)amino]hydroxyiminomethyl]benzenesulfonamide

To a solution of 0.5 g of methyl N'-(2-chlorophenylsulfonyl)-N-(4,6-dimethylpyrimidin-2-yl)carbamimidothioate in 10 ml of tetrahydrofuran was added 0.3 g of hydroxylamine hydrochloride and 0.3 g of sodium acetate. The mixture was stirred at room temperature for 2 hours, whereupon the product was precipitated with ice-water, filtered, washed with water, and dried by suction to afford 0.3 g of the title compound, m.p. 165—167°. NMR ($CDCl_3$/DMSO-$d_6$) δ 2.6 (s, 6), 6.8 (s, 1), 7.4 (m, 3), 8.2 (m, 1), 10.2 (br, 1), 12.0 (br, 1). m/e: 355 ($M^+$).

Example 5

N-(4,6-Dimethylpyrimidin-2-yl)thiourea

A suspension of 10 g of 2-cyanoamino-4,6-dimethylpyrimidine in 50 ml of pyridine was saturated with hydrogen sulfide and stored for 1 hour at 25°. The $H_2S$ treatment was repeated twice more, and the mixture was allowed to stir at 25° for 16 hours. Methylene chloride was added, and the product was filtered and washed with methylene chloride to afford 8 g of 4,6-dimethylpyrimidin-2-yl thiourea, m.p. >260°. IR (Nujol) 3280, 3180, 3120, 1610 $cm^{-1}$.

Example 6

N-[(4-Methoxy-6-methylpyrimidin-2-yl)aminothioxomethyl]benzamide

To a hot solution of 14 g of ammonium thiocyanate in 300 ml of acetonitrile was added 24 ml of benzoyl chloride. The mixture was heated on the steam bath for 30 minutes and filtered. The filtrate was heated with 21 g of 4-methoxy-6-methylpyrimidine-2-amine for 30 minutes and cooled. The product was collected by filtration and washed with a little acetonitrile to provide 28 g of N-[[(4-methoxy-6-methylpyrimidin-2-yl)aminothiocarbonyl]amino]benzamide, m.p. 193—195°. IR (Nujol) 3290, 1720 $cm^{-1}$.

Example 7

N-(4-Methoxy-6-methylpyrimidin-2-yl)thiourea

A mixture of 25 g of the compound from Example 3 and 55 ml of 10% aqueous sodium hydroxide was heated on the steam bath for 30 minutes, cooled, neutralized with aqueous HCl to pH 8, filtered, washed with water, dried by suction, and washed with ether to afford 16 g of the title compound, m.p. 220°d.

| | | | | |
|---|---|---|---|---|
| Anal. Calc'd.: | C: 42.4, | H: 5.06, | N: 28.2, | S: 16.2 |
| Found: | C: 42.6, | H: 4.8, | N: 28.8, | S: 15.3 |

m/e 198 ($M^+$).

Example 8

Methyl 2-[[1-(4-methoxy-6-methylpyrimidin-2-ylimino)-1-(methylthio)methyl]aminosulfonyl]benzoate

To 9 g of the compound from Example 7 and 100 ml of tetrahydrofuran was added 7.6 g of dimethyl sulfate and the mixture was stirred for 2 days at room temperature. The product was filtered and washed with a little tetrahydrofuran to give 22 g of crude product. Five grams of this material was suspended in 150 ml of methylene chloride and 1 ml of water, and 5 g of sodium bicarbonate and 6 g of methyl 2-chlorosulfonyl benzoate were added. The mixture was stirred for 24 hours, washed with dilute aqueous HCl, dried with sodium sulfate, filtered, and chromatographed on silica gel to afford 2.8 g of the title compound as a crystalline solid, m.p. 143—144°.

| | | | | |
|---|---|---|---|---|
| Anal. Calc'd.: | C: 45.22, | H: 4.55, | N: 14.06, | S: 16.1 |
| Found: | C: 46.8, | H: 4.35, | N: 13.2, | S: 16 |

m/e 395 ($M^+$-$CH_3$)

NMR ($CDCl_3$) δ 2.35 (s, 3), 2.45 (s, 3), 3.90 (s, 3), 4.0 (s, 3) 6.35 (s, 1), 7.7 (m, 3), 8.2 (m, 1), 10.8 (brs, 1).

Example 9

Methyl N-(4,6-dimethoxy-1,3,5-triazin-2-yl)carbamimidothioate

A mixture of 20 g of 2-chloro-4,6-dimethoxy-*s*-triazine, 20 g of S-methyl isothiouronium sulfate, 16 g of sodium carbonate, and 50 ml of water was stirred for 72 hours, concentrated at reduced pressure, and extracted with 300 ml of methylene chloride. The organic layer was dried with $Na_2SO_4$, filtered,

concentrated to dryness, and recrystallized from chlorobutane to afford 10 g of title compound, m.p. 94—100°. NMR ($CDCl_3$) δ 2.6 (s, 3), 4.0 (s, 6), 8.2 (brs, 1).

Using the procedures and examples shown above, the compounds in Tables 1—35 can be prepared. Tables 18—22 relate to intermediates of Formula III.

## Table 1

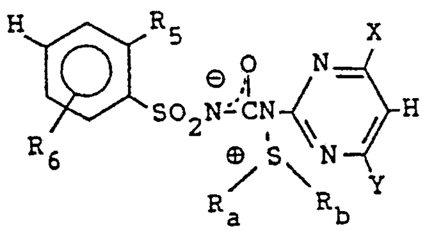

| $R_5$ | $R_a$ | $R_b$ | $R_6$ | X | Y | m.p. (°C) |
|---|---|---|---|---|---|---|
| $CO_2CH_3$ | $CH_3$ | $CH_3$ | H | $CH_3$ | $CH_3$ | 88-90° (d) |
| $CO_2CH_2CH_3$ | $CH_3$ | $CH_3$ | H | $CH_3$ | $CH_3$ | |
| $CO_2CH_3$ | $C_2H_5$ | $C_2H_5$ | H | $CH_3$ | $CH_3$ | |
| $CO_2CH_3$ | $C_2H_5$ | $CH_3$ | H | $CH_3$ | $CH_3$ | |
| $CO_2CH_2CH_2CH_2CH_3$ | $CH_3$ | $CH_3$ | H | $CH_3$ | $CH_3$ | |
| $CO_2CH_2CH{=}CH_2$ | $CH_3$ | $CH_3$ | H | $CH_3$ | $CH_3$ | |
| $CO_2CH_2CH_2OCH_3$ | $CH_3$ | $CH_3$ | H | $CH_3$ | $CH_3$ | |
| $CO_2CH_2CH_2Cl$ | $CH_3$ | $CH_3$ | H | $CH_3$ | $CH_3$ | |
| $SCH_3$ | $CH_3$ | $CH_3$ | H | $CH_3$ | $CH_3$ | |
| $SCH_2CH_3$ | $CH_3$ | $CH_3$ | H | $CH_3$ | $CH_3$ | |
| $SCH(CH_3)_2$ | $CH_3$ | $CH_3$ | H | $CH_3$ | $CH_3$ | |
| $SCH_2CH{=}CH_2$ | $CH_3$ | $CH_3$ | H | $CH_3$ | $CH_3$ | |
| $SCF_3$ | $CH_3$ | $CH_3$ | H | $CH_3$ | $CH_3$ | |
| $SCF_2H$ | $CH_3$ | $CH_3$ | H | $CH_3$ | $CH_3$ | |
| $SO_2CH_3$ | $CH_3$ | $CH_3$ | H | $CH_3$ | $CH_3$ | 90-91° (d) |
| $SO_2CH_2CH_3$ | $CH_3$ | $CH_3$ | H | $CH_3$ | $CH_3$ | |
| $SO_2CH_3$ | $C_2H_5$ | $C_2H_5$ | H | $CH_3$ | $CH_3$ | |
| $SO_2CH_3$ | $C_2H_5$ | $CH_3$ | H | $CH_3$ | $CH_3$ | |
| $SO_2CF_3$ | $CH_3$ | $CH_3$ | H | $CH_3$ | $CH_3$ | |
| $SO_2CF_2H$ | $CH_3$ | $CH_3$ | H | $CH_3$ | $CH_3$ | |
| $OSO_2CH_3$ | $CH_3$ | $CH_3$ | H | $CH_3$ | $CH_3$ | |
| $OSO_2CH_2CH_3$ | $CH_3$ | $CH_3$ | H | $CH_3$ | $CH_3$ | |
| $OSO_2CH(CH_3)_2$ | $CH_3$ | $CH_3$ | H | $CH_3$ | $CH_3$ | |
| $OCH_3$ | $CH_3$ | $CH_3$ | H | $CH_3$ | $CH_3$ | |
| $OCH_2CH_3$ | $CH_3$ | $CH_3$ | H | $CH_3$ | $CH_3$ | |

Table 1 (continued)

| $R_5$ | $R_a$ | $R_b$ | $R_6$ | X | Y | m.p.(°C) |
|---|---|---|---|---|---|---|
| $OCH_2CH_2CH_3$ | $CH_3$ | $CH_3$ | H | $CH_3$ | $CH_3$ | |
| $OCH(CH_3)CH_2CH_3$ | $CH_3$ | $CH_3$ | H | $CH_3$ | $CH_3$ | |
| $OCH_2CH=CH_2$ | $CH_3$ | $CH_3$ | H | $CH_3$ | $CH_3$ | |
| $OCH_2CH_2CH=CH_2$ | $CH_3$ | $CH_3$ | H | $CH_3$ | $CH_3$ | |
| $OCH(CH_3)CH=CH_2$ | $CH_3$ | $CH_3$ | H | $CH_3$ | $CH_3$ | |
| $SO_2N(CH_3)_2$ | $CH_3$ | $CH_3$ | H | $CH_3$ | $CH_3$ | |
| $SO_2N(CH_3)CH(CH_3)_2$ | $CH_3$ | $CH_3$ | H | $CH_3$ | $CH_3$ | |
| $SO_2N(OCH_3)CH_3$ | $CH_3$ | $CH_3$ | H | $CH_3$ | $CH_3$ | |
| $CH_2OCH_3$ | $CH_3$ | $CH_3$ | H | $CH_3$ | $CH_3$ | |
| $CH_2CH_2OCH_3$ | $CH_3$ | $CH_3$ | H | $CH_3$ | $CH_3$ | |
| F | $CH_3$ | $CH_3$ | H | $CH_3$ | $CH_3$ | |
| Cl | $CH_3$ | $CH_3$ | H | $CH_3$ | $CH_3$ | 104-105°(d) |
| Br | $CH_3$ | $CH_3$ | H | $CH_3$ | $CH_3$ | |
| $NO_2$ | $CH_3$ | $CH_3$ | H | $CH_3$ | $CH_3$ | 118-120°(d) |
| $CF_3$ | $CH_3$ | $CH_3$ | H | $CH_3$ | $CH_3$ | |
| $CH_3$ | $CH_3$ | $CH_3$ | H | $CH_3$ | $CH_3$ | |
| $CH_2CH_3$ | $CH_3$ | $CH_3$ | H | $CH_3$ | $CH_3$ | |
| $CH(CH_3)_2$ | $CH_3$ | $CH_3$ | H | $CH_3$ | $CH_3$ | |
| $CH_2CH_2CH_2CH_3$ | $CH_3$ | $CH_3$ | H | $CH_3$ | $CH_3$ | |
| $CO_2CH_2CH_2CH_3$ | $CH_3$ | $CH_3$ | H | $CH_3$ | $CH_3$ | |
| $SCH_3$ | $C_2H_5$ | $C_2H_5$ | H | $CH_3$ | $CH_3$ | |
| $SCF_3$ | $C_2H_5$ | $CH_3$ | H | $CH_3$ | $CH_3$ | |
| $SO_2CH_2CH_3$ | $C_2H_5$ | $C_2H_5$ | H | $CH_3$ | $CH_3$ | |
| $NO_2$ | $CH_3$ | $CH_3$ | H | $CH_3$ | $CH_3$ | |
| $CF_3$ | $CH_3$ | $CH_3$ | H | $CH_3$ | $CH_3$ | |
| $CH_3$ | $CH_3$ | $CH_3$ | H | $CH_3$ | $CH_3$ | |
| $NO_2$ | $CH_3$ | $CH_3$ | 5-$CF_3$ | $CH_3$ | $CH_3$ | |
| $NO_2$ | $CH_3$ | $CH_3$ | 3-Cl | $CH_3$ | $CH_3$ | |
| Cl | $CH_3$ | $CH_3$ | 6-$SCH_3$ | $CH_3$ | $CH_3$ | |
| $CF_3$ | $CH_3$ | $CH_3$ | 5-$SCH_3$ | $CH_3$ | $CH_3$ | |
| Cl | $CH_3$ | $CH_3$ | 5-Cl | $CH_3$ | $CH_3$ | |
| $NO_2$ | $CH_3$ | $CH_3$ | 5-Cl | $CH_3$ | $CH_3$ | |

Table 1 (continued)

| $R_5$ | $R_a$ | $R_b$ | $R_6$ | X | Y | m.p.(°C) |
|---|---|---|---|---|---|---|
| $SO_2N(CH_3)_2$ | $C_2H_5$ | $C_2H_5$ | 3-Cl | $CH_3$ | $CH_3$ | |
| $CO_2CH_3$ | $CH_3$ | $CH_3$ | 5-$CH_3$ | $CH_3$ | $CH_3$ | |
| Cl | $CH_3$ | $CH_3$ | 3-Br | $CH_3$ | $CH_3$ | |
| Cl | $CH_3$ | $CH_3$ | 5-Br | $CH_3$ | $CH_3$ | |
| $NO_2$ | $CH_3$ | $CH_3$ | 6-F | $CH_3$ | $CH_3$ | |
| Cl | $CH_3$ | $CH_3$ | 6-F | $CH_3$ | $CH_3$ | |
| Br | $CH_3$ | $CH_3$ | 3-F | $CH_3$ | $CH_3$ | |
| $NO_2$ | $C_2H_5$ | $C_2H_5$ | 5-$OCF_2H$ | $CH_3$ | $CH_3$ | |
| $CO_2CH_3$ | $CH_3$ | $CH_3$ | 3-$OCH_3$ | $CH_3$ | $CH_3$ | |
| $CH_3$ | $CH_3$ | $CH_3$ | 3-$OCH_3$ | $CH_3$ | $CH_3$ | |
| $CO_2CH_3$ | $CH_3$ | $CH_3$ | H | Cl | $OCH_3$ | |
| $NO_2$ | $CH_3$ | $CH_3$ | H | Cl | $OCH_3$ | |
| Cl | $CH_3$ | $CH_3$ | H | Cl | $OCH_3$ | |
| $CO_2CH_3$ | $C_2H_5$ | $CH_3$ | H | $CH_3$ | $OCH_3$ | |
| $CO_2CH_3$ | $C_2H_5$ | $C_2H_5$ | H | $CH_3$ | $OCH_3$ | |
| $NO_2$ | $C_2H_5$ | $CH_3$ | H | $CH_3$ | $OCH_3$ | |
| $SO_2N(CH_3)_2$ | $C_2H_5$ | $CH_3$ | H | $CH_3$ | $OCH_3$ | |
| $CO_2CH_3$ | $CH_3$ | $CH_3$ | H | $CH_3$ | $CH_2OCH_3$ | |
| $CO_2CH_3$ | $CH_3$ | $CH_3$ | H | $CH_3$ | $CH(OCH_3)_2$ | |
| $CO_2CH_3$ | $CH_3$ | $CH_3$ | H | $CH_3$ | $CH(OCH_2CH_2O)$ | |
| $NO_2$ | $C_2H_5$ | $C_2H_5$ | H | $CH_3$ | $OCH_3$ | |
| $SO_2N(CH_3)_2$ | $C_2H_5$ | $C_2H_5$ | H | $CH_3$ | $OCH_3$ | |
| $CO_2CH_3$ | $CH_3$ | $CH_3$ | H | $CH_3$ | $OCH_3$ | 90-91°(d) |
| $CO_2CH_2CH_3$ | $CH_3$ | $CH_3$ | H | $CH_3$ | $OCH_3$ | |
| $CO_2CH_2CH_2CH_3$ | $CH_3$ | $CH_3$ | H | $CH_3$ | $OCH_3$ | |
| $CO_2CH(CH_3)_2$ | $CH_3$ | $CH_3$ | H | $CH_3$ | $OCH_3$ | |
| $CO_2CH_2CH_2CH_2CH_3$ | $CH_3$ | $CH_3$ | H | $CH_3$ | $OCH_3$ | |
| $CO_2CH_2CH{=}CH_2$ | $CH_3$ | $CH_3$ | H | $CH_3$ | $OCH_3$ | |
| $CO_2CH_2CH_2OCH_3$ | $CH_3$ | $CH_3$ | H | $CH_3$ | $OCH_3$ | |
| $CO_2CH_2CH_2Cl$ | $CH_3$ | $CH_3$ | H | $CH_3$ | $OCH_3$ | |
| $SCH_3$ | $CH_3$ | $CH_3$ | H | $CH_3$ | $OCH_3$ | |
| $SCH_2CH_3$ | $CH_3$ | $CH_3$ | H | $CH_3$ | $OCH_3$ | |

Table 1 (continued)

| $R_5$ | $R_a$ | $R_b$ | $R_6$ | X | Y | m.p.(°C) |
|---|---|---|---|---|---|---|
| $SCH(CH_3)_2$ | $CH_3$ | $CH_3$ | H | $CH_3$ | $OCH_3$ | |
| $SCH_2CH=CH_2$ | $CH_3$ | $CH_3$ | H | $CH_3$ | $OCH_3$ | |
| $SCF_3$ | $CH_3$ | $CH_3$ | H | $CH_3$ | $OCH_3$ | |
| $SCF_2H$ | $CH_3$ | $CH_3$ | H | $CH_3$ | $OCH_3$ | |
| $SO_2CH_3$ | $CH_3$ | $CH_3$ | H | $CH_3$ | $OCH_3$ | |
| $SO_2CH_2CH_3$ | $CH_3$ | $CH_3$ | H | $CH_3$ | $OCH_3$ | |
| $SO_2CH(CH_3)_2$ | $CH_3$ | $CH_3$ | H | $CH_3$ | $OCH_3$ | |
| $SO_2CH_2CH=CH_2$ | $CH_3$ | $CH_3$ | H | $CH_3$ | $OCH_3$ | |
| $SO_2CF_3$ | $CH_3$ | $CH_3$ | H | $CH_3$ | $OCH_3$ | |
| $SO_2CF_2H$ | $CH_3$ | $CH_3$ | H | $CH_3$ | $OCH_3$ | |
| $OSO_2CH_3$ | $CH_3$ | $CH_3$ | H | $CH_3$ | $OCH_3$ | |
| $OSO_2CH_2CH_3$ | $CH_3$ | $CH_3$ | H | $CH_3$ | $OCH_3$ | |
| $OSO_2CH(CH_3)_2$ | $CH_3$ | $CH_3$ | H | $CH_3$ | $OCH_3$ | |
| $OCH_3$ | $CH_3$ | $CH_3$ | H | $CH_3$ | $OCH_3$ | |
| $OCH_2CH_3$ | $CH_3$ | $CH_3$ | H | $CH_3$ | $OCH_3$ | |
| $OCH(CH_3)_2$ | $CH_3$ | $CH_3$ | H | $CH_3$ | $OCH_3$ | |
| $OCH_2CH_2CH_2CH_3$ | $CH_3$ | $CH_3$ | H | $CH_3$ | $OCH_3$ | |
| $OCH_2CH=CH_2$ | $CH_3$ | $CH_3$ | H | $CH_3$ | $OCH_3$ | |
| $OCH_2CH_2CH=CH_2$ | $CH_3$ | $CH_3$ | H | $CH_3$ | $OCH_3$ | |
| $OCH(CH_3)CH=CH_2$ | $CH_3$ | $CH_3$ | H | $CH_3$ | $OCH_3$ | |
| $SO_2N(CH_3)_2$ | $CH_3$ | $CH_3$ | H | $CH_3$ | $OCH_3$ | 95-96°(d) |
| $SO_2N(CH_3)CH(CH_3)_2$ | $CH_3$ | $CH_3$ | H | $CH_3$ | $OCH_3$ | |
| $SO_2N(OCH_3)CH_3$ | $CH_3$ | $CH_3$ | H | $CH_3$ | $OCH_3$ | |
| $CH_2OCH_3$ | $CH_3$ | $CH_3$ | H | $CH_3$ | $OCH_3$ | |
| $CH_2CH_2OCH_3$ | $CH_3$ | $CH_3$ | H | $CH_3$ | $OCH_3$ | |
| F | $CH_3$ | $CH_3$ | H | $CH_3$ | $OCH_3$ | |
| Cl | $CH_3$ | $CH_3$ | H | $CH_3$ | $OCH_3$ | 89-90°C(d) |
| Br | $CH_3$ | $CH_3$ | H | $CH_3$ | $OCH_3$ | |
| $NO_2$ | $CH_3$ | $CH_3$ | H | $CH_3$ | $OCH_3$ | 172-173°(d) |
| $CF_3$ | $CH_3$ | $CH_3$ | H | $CH_3$ | $OCH_3$ | |
| $CH_3$ | $CH_3$ | $CH_3$ | H | $CH_3$ | $OCH_3$ | |
| $CH_2CH_3$ | $CH_3$ | $CH_3$ | H | $CH_3$ | $OCH_3$ | |

Table 1 (continued)

| $R_5$ | $R_a$ | $R_b$ | $R_6$ | X | Y | m.p.(°C) |
|---|---|---|---|---|---|---|
| $CH(CH_3)_2$ | $CH_3$ | $CH_3$ | H | $CH_3$ | $OCH_3$ | |
| $CH_2CH_2CH_2CH_3$ | $CH_3$ | $CH_3$ | H | $CH_3$ | $OCH_3$ | |
| $CO_2CH_3$ | $CH_3$ | $CH_3$ | H | Br | $OCH_3$ | |
| $SCH_3$ | $C_2H_5$ | $CH_3$ | H | $CH_3$ | $OCH_3$ | |
| $SCF_3$ | $C_2H_5$ | $CH_3$ | H | $CH_3$ | $OCH_3$ | |
| $NO_2$ | $CH_3$ | $CH_3$ | 5-$CF_3$ | $CH_3$ | $OCH_3$ | |
| $NO_2$ | $CH_3$ | $CH_3$ | 3-Cl | $CH_3$ | $OCH_3$ | |
| Cl | $CH_3$ | $CH_3$ | 6-$SCH_3$ | $CH_3$ | $OCH_3$ | |
| $CF_3$ | $CH_3$ | $CH_3$ | 5-$OCF_2H$ | $CH_3$ | $OCH_3$ | |
| Cl | $CH_3$ | $CH_3$ | 5-Cl | $CH_3$ | $OCH_3$ | |
| $NO_2$ | $CH_3$ | $CH_3$ | 5-Cl | $CH_3$ | $OCH_3$ | |
| $SO_2N(CH_3)_2$ | $CH_3$ | $CH_3$ | 3-Cl | $CH_3$ | $OCH_3$ | |
| $CO_2CH_3$ | $CH_3$ | $CH_3$ | 5-$CH_3$ | $CH_3$ | $OCH_3$ | |
| Cl | $CH_3$ | $CH_3$ | 3-Br | $CH_3$ | $OCH_3$ | |
| Cl | $CH_3$ | $CH_3$ | 5-Br | $CH_3$ | $OCH_3$ | |
| $NO_2$ | $CH_3$ | $CH_3$ | 6-F | $CH_3$ | $OCH_3$ | |
| Cl | $CH_3$ | $CH_3$ | 6-F | $CH_3$ | $OCH_3$ | |
| Br | $CH_3$ | $CH_3$ | 3-F | $CH_3$ | $OCH_3$ | |
| $NO_2$ | $CH_3$ | $CH_3$ | 5-$SCH_3$ | $CH_3$ | $OCH_3$ | |
| $CO_2CH_3$ | $CH_3$ | $CH_3$ | 3-$OCH_3$ | $CH_3$ | $OCH_3$ | |
| $CH_3$ | $CH_3$ | $CH_3$ | 3-$OCH_3$ | $CH_3$ | $OCH_3$ | |
| $CO_2CH_3$ | $C_2H_5$ | $CH_3$ | H | $OCH_3$ | $OCH_3$ | |
| $CO_2CH_3$ | $C_2H_5$ | $C_2H_5$ | H | $OCH_3$ | $OCH_3$ | |
| $NO_2$ | $C_2H_5$ | $CH_3$ | H | $OCH_3$ | $OCH_3$ | |
| $SO_2N(CH_3)_2$ | $C_2H_5$ | $CH_3$ | H | $OCH_3$ | $OCH_3$ | |
| $CO_2CH_3$ | $CH_3$ | $CH_3$ | H | $OCH_3$ | $CH_2OCH_3$ | |
| $CO_2CH_3$ | $CH_3$ | $CH_3$ | H | $OCH_3$ | $CH(OCH_3)_2$ | |
| $CO_2CH_3$ | $CH_3$ | $CH_3$ | H | $OCH_3$ | $CH(OCH_2CH_2O)$ | |
| $NO_2$ | $C_2H_5$ | $C_2H_5$ | H | $OCH_3$ | $OCH_3$ | |
| $SO_2N(CH_3)_2$ | $C_2H_5$ | $C_2H_5$ | H | $OCH_3$ | $OCH_3$ | |
| $CO_2CH_3$ | $CH_3$ | $CH_3$ | H | $OCH_3$ | $OCH_3$ | |
| $CO_2CH_2CH_3$ | $CH_3$ | $CH_3$ | H | $OCH_3$ | $OCH_3$ | |

Table 1 (continued)

| $R_5$ | $R_a$ | $R_b$ | $R_6$ | X | Y | m.p.(°C) |
|---|---|---|---|---|---|---|
| $CO_2CH_2CH_2CH_3$ | $CH_3$ | $CH_3$ | H | $OCH_3$ | $OCH_3$ | |
| $CO_2CH(CH_3)_2$ | $CH_3$ | $CH_3$ | H | $OCH_3$ | $OCH_3$ | 152-154°(d) |
| $CO_2CH_2CH_2CH_2CH_3$ | $CH_3$ | $CH_3$ | H | $OCH_3$ | $OCH_3$ | |
| $CO_2CH_2CH{=}CH_2$ | $CH_3$ | $CH_3$ | H | $OCH_3$ | $OCH_3$ | 151-153°(d) |
| $CO_2CH_2CH_2OCH_3$ | $CH_3$ | $CH_3$ | H | $OCH_3$ | $OCH_3$ | |
| $CO_2CH_2CH_2Cl$ | $CH_3$ | $CH_3$ | H | $OCH_3$ | $OCH_3$ | |
| $SCH_3$ | $CH_3$ | $CH_3$ | H | $OCH_3$ | $OCH_3$ | |
| $SCH_2CH_3$ | $CH_3$ | $CH_3$ | H | $OCH_3$ | $OCH_3$ | |
| $SCH(CH_3)_2$ | $CH_3$ | $CH_3$ | H | $OCH_3$ | $OCH_3$ | |
| $SCH_2CH{=}CH_2$ | $CH_3$ | $CH_3$ | H | $OCH_3$ | $OCH_3$ | |
| $SCF_3$ | $CH_3$ | $CH_3$ | H | $OCH_3$ | $OCH_3$ | |
| $SCF_2H$ | $CH_3$ | $CH_3$ | H | $OCH_3$ | $OCH_3$ | |
| $SO_2CH_3$ | $CH_3$ | $CH_3$ | H | $OCH_3$ | $OCH_3$ | |
| $SO_2CH_2CH_3$ | $CH_3$ | $CH_3$ | H | $OCH_3$ | $OCH_3$ | |
| $SO_2CH(CH_3)_2$ | $CH_3$ | $CH_3$ | H | $OCH_3$ | $OCH_3$ | |
| $SO_2CH_2CH{=}CH_2$ | $CH_3$ | $CH_3$ | H | $OCH_3$ | $OCH_3$ | |
| $SO_2CF_3$ | $CH_3$ | $CH_3$ | H | $OCH_3$ | $OCH_3$ | |
| $SO_2CF_2H$ | $CH_3$ | $CH_3$ | H | $OCH_3$ | $OCH_3$ | |
| $OSO_2CH_3$ | $CH_3$ | $CH_3$ | H | $OCH_3$ | $OCH_3$ | |
| $OSO_2CH_2CH_3$ | $CH_3$ | $CH_3$ | H | $OCH_3$ | $OCH_3$ | |
| $OSO_2CH(CH_3)_2$ | $CH_3$ | $CH_3$ | H | $OCH_3$ | $OCH_3$ | |
| $OCH_3$ | $CH_3$ | $CH_3$ | H | $OCH_3$ | $OCH_3$ | |
| $OCH_2CH_3$ | $CH_3$ | $CH_3$ | H | $OCH_3$ | $OCH_3$ | |
| $OCH(CH_3)_2$ | $CH_3$ | $CH_3$ | H | $OCH_3$ | $OCH_3$ | |
| $OCH_2CH_2CH_2CH_3$ | $CH_3$ | $CH_3$ | H | $OCH_3$ | $OCH_3$ | |
| $OCH_2CH{=}CH_2$ | $CH_3$ | $CH_3$ | H | $OCH_3$ | $OCH_3$ | |
| $OCH_2CH_2CH{=}CH_2$ | $CH_3$ | $CH_3$ | H | $OCH_3$ | $OCH_3$ | |
| $OCH(CH_3)CH{=}CH_2$ | $CH_3$ | $CH_3$ | H | $OCH_3$ | $OCH_3$ | |
| $SO_2N(CH_3)_2$ | $CH_3$ | $CH_3$ | H | $OCH_3$ | $OCH_3$ | 173-175°(d) |
| $SO_2N(CH_3)CH(CH_3)_2$ | $CH_3$ | $CH_3$ | H | $OCH_3$ | $OCH_3$ | |
| $SO_2N(OCH_3)CH_3$ | $CH_3$ | $CH_3$ | H | $OCH_3$ | $OCH_3$ | |
| $CH_2OCH_3$ | $CH_3$ | $CH_3$ | H | $OCH_3$ | $OCH_3$ | |

Table 1 (continued)

| $R_5$ | $R_a$ | $R_b$ | $R_6$ | X | Y | m.p.(°C) |
|---|---|---|---|---|---|---|
| $CH_2CH_2OCH_3$ | $CH_3$ | $CH_3$ | H | $OCH_3$ | $OCH_3$ | |
| F | $CH_3$ | $CH_3$ | H | $OCH_3$ | $OCH_3$ | |
| Cl | $CH_3$ | $CH_3$ | H | $OCH_3$ | $OCH_3$ | |
| Br | $CH_3$ | $CH_3$ | H | $OCH_3$ | $OCH_3$ | |
| $NO_2$ | $CH_3$ | $CH_3$ | H | $OCH_3$ | $OCH_3$ | |
| $CF_3$ | $CH_3$ | $CH_3$ | H | $OCH_3$ | $OCH_3$ | |
| $CH_3$ | $CH_3$ | $CH_3$ | H | $OCH_3$ | $OCH_3$ | |
| $CH_2CH_3$ | $CH_3$ | $CH_3$ | H | $OCH_3$ | $OCH_3$ | |
| $CH(CH_3)_2$ | $CH_3$ | $CH_3$ | H | $OCH_3$ | $OCH_3$ | |
| $CH_2CH_2CH_2CH_3$ | $CH_3$ | $CH_3$ | H | $OCH_3$ | $OCH_3$ | |
| $SO_2N(CH_3)_2$ | $CH_3$ | $CH_3$ | H | Cl | $OCH_3$ | |
| $SCH_3$ | $C_2H_5$ | $CH_3$ | H | $OCH_3$ | $OCH_3$ | |
| $SCF_3$ | $C_2H_5$ | $CH_3$ | H | $OCH_3$ | $OCH_3$ | |
| $NO_2$ | $CH_3$ | $CH_3$ | 5-$CF_3$ | $OCH_3$ | $OCH_3$ | |
| $NO_2$ | $CH_3$ | $CH_3$ | 3-Cl | $OCH_3$ | $OCH_3$ | |
| Cl | $CH_3$ | $CH_3$ | 6-$SCH_3$ | $OCH_3$ | $OCH_3$ | |
| $CF_3$ | $CH_3$ | $CH_3$ | 5-$OCF_2H$ | $OCH_3$ | $OCH_3$ | |
| Cl | $CH_3$ | $CH_3$ | 5-Cl | $OCH_3$ | $OCH_3$ | |
| $NO_2$ | $CH_3$ | $CH_3$ | 5-Cl | $OCH_3$ | $OCH_3$ | |
| $SO_2N(CH_3)_2$ | $CH_3$ | $CH_3$ | 3-Cl | $OCH_3$ | $OCH_3$ | |
| $CO_2CH_3$ | $CH_3$ | $CH_3$ | 5-$CH_3$ | $OCH_3$ | $OCH_3$ | |
| Cl | $CH_3$ | $CH_3$ | 3-Br | $OCH_3$ | $OCH_3$ | |
| Cl | $CH_3$ | $CH_3$ | 5-Br | $OCH_3$ | $OCH_3$ | |
| $NO_2$ | $CH_3$ | $CH_3$ | 6-F | $OCH_3$ | $OCH_3$ | |
| Cl | $CH_3$ | $CH_3$ | 6-F | $OCH_3$ | $OCH_3$ | |
| Br | $CH_3$ | $CH_3$ | 3-F | $OCH_3$ | $OCH_3$ | |
| $NO_2$ | $CH_3$ | $CH_3$ | 5-$SCH_3$ | $OCH_3$ | $OCH_3$ | |
| $CO_2CH_3$ | $CH_3$ | $CH_3$ | 3-$OCH_3$ | $OCH_3$ | $OCH_3$ | |
| $CH_3$ | $CH_3$ | $CH_3$ | 3-$OCH_3$ | $OCH_3$ | $OCH_3$ | |
| $CO_2CH_3$ | $CH_3$ | $CH_3$ | H | $OCF_2H$ | $CH_3$ | |
| $CO_2CH_3$ | $CH_3$ | $CH_3$ | H | $CH_2F$ | $OCH_3$ | |
| $CO_2CH_3$ | $CH_3$ | $CH_3$ | H | $CH_3$ | $C_2H_5$ | |

Table 1 (continued)

| $R_5$ | $R_a$ | $R_b$ | $R_6$ | X | Y | m.p.(°C) |
|---|---|---|---|---|---|---|
| $CO_2CH_3$ | $CH_3$ | $CH_3$ | H | $OCH_3$ | $C_2H_5$ | |
| $CO_2CH_3$ | $CH_3$ | $CH_3$ | H | $CF_3$ | $OCH_3$ | |
| $CO_2CH_3$ | $CH_3$ | $CH_3$ | H | $CF_3$ | $CH_3$ | |
| $CO_2CH_3$ | $CH_3$ | $CH_3$ | H | $OC_2H_5$ | $CH_3$ | |
| $CO_2CH_3$ | $CH_3$ | $CH_3$ | H | $OC_2H_5$ | $OCH_3$ | |
| $CO_2CH_3$ | $CH_3$ | $CH_3$ | H | $CH_3$ | $OCH_2CF_3$ | |
| $CO_2CH_3$ | $CH_3$ | $CH_3$ | H | $OCH_3$ | $OCH_2CF_3$ | |
| 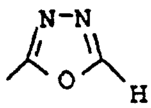 | $CH_3$ | $CH_3$ | H | $CH_3$ | $CH_3$ | |
| 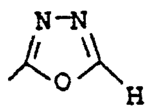 | $CH_3$ | $CH_3$ | H | $CH_3$ | $OCH_3$ | |
| 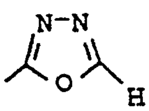 | $CH_3$ | $CH_3$ | H | $OCH_3$ | $OCH_3$ | |
| 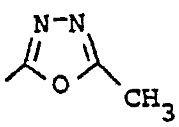 | $CH_3$ | $CH_3$ | H | $CH_3$ | $CH_3$ | |
| 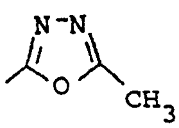 | $CH_3$ | $CH_3$ | H | $CH_3$ | $OCH_3$ | |
| 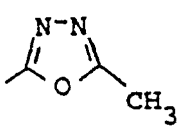 | $CH_3$ | $CH_3$ | H | $OCH_3$ | $OCH_3$ | |
| 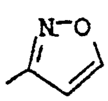 | $CH_3$ | $CH_3$ | H | $CH_3$ | $CH_3$ | |
| 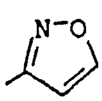 | $CH_3$ | $CH_3$ | H | $CH_3$ | $OCH_3$ | |

Table 1 (continued)

| $R_5$ | $R_a$ | $R_b$ | $R_6$ | X | Y | m.p.(°C) |
|---|---|---|---|---|---|---|
| 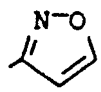 | $CH_3$ | $CH_3$ | H | $OCH_3$ | $OCH_3$ | |
| 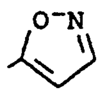 | $CH_3$ | $CH_3$ | H | $CH_3$ | $CH_3$ | |
| 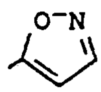 | $CH_3$ | $CH_3$ | H | $CH_3$ | $OCH_3$ | |
| 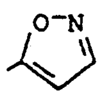 | $CH_3$ | $CH_3$ | H | $OCH_3$ | $OCH_3$ | |
| 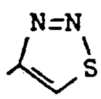 | $CH_3$ | $CH_3$ | H | $CH_3$ | $CH_3$ | |
| 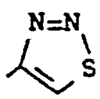 | $CH_3$ | $CH_3$ | H | $CH_3$ | $OCH_3$ | |
| 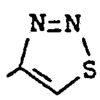 | $CH_3$ | $CH_3$ | H | $OCH_3$ | $OCH_3$ | |
| $CO_2CH_3$ | $CH_3$ | $CH_3$ | H | $OCH_3$ | $N(CH_3)_2$ | |
| $CO_2CH_3$ | $CH_3$ | $CH_3$ | H | $OCH_3$ | $SCH_3$ | |
| $CO_2CH_3$ | $CH_3$ | $CH_3$ | H | $OCH_3$ | $OCH_2CH=CH_2$ | |
| $CO_2CH_3$ | $CH_3$ | $CH_3$ | H | $OCH_3$ | $OCH_2C\equiv CH$ | |
| $CO_2CH_3$ | $CH_3$ | $CH_3$ | H | $OCH_3$ | $CH_2SCH_3$ | |
| $CO_2CH_3$ | $CH_3$ | $CH_3$ | H | $OCH_3$ | $SCF_2H$ | |

Table 2

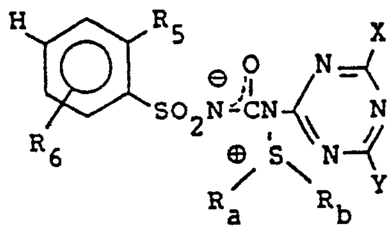

| $R_5$ | $R_a$ | $R_b$ | $R_6$ | X | Y | m.p.(°C) |
|---|---|---|---|---|---|---|
| $CO_2CH_3$ | $CH_3$ | $CH_3$ | H | $CH_3$ | $CH_3$ | |
| $CO_2CH_2CH_3$ | $CH_3$ | $CH_3$ | H | $CH_3$ | $CH_3$ | |
| $CO_2CH_2CH_2CH_3$ | $CH_3$ | $CH_3$ | H | $CH_3$ | $CH_3$ | |
| $CO_2CH_3$ | $C_2H_5$ | $C_2H_5$ | H | $CH_3$ | $CH_3$ | |
| $CO_2CH_3$ | $C_2H_5$ | $CH_3$ | H | $CH_3$ | $CH_3$ | |
| $CO_2CH_2CH{=}CH_2$ | $CH_3$ | $CH_3$ | H | $CH_3$ | $CH_3$ | |
| $CO_2CH_2CH_2OCH_3$ | $CH_3$ | $CH_3$ | H | $CH_3$ | $CH_3$ | |
| $CO_2CH_2CH_2Cl$ | $CH_3$ | $CH_3$ | H | $CH_3$ | $CH_3$ | |
| $SCH_3$ | $CH_3$ | $CH_3$ | H | $CH_3$ | $CH_3$ | |
| $SCH_2CH_3$ | $CH_3$ | $CH_3$ | H | $CH_3$ | $CH_3$ | |
| $SCH(CH_3)_2$ | $CH_3$ | $CH_3$ | H | $CH_3$ | $CH_3$ | |
| $SCH_2CH{=}CH_2$ | $CH_3$ | $CH_3$ | H | $CH_3$ | $CH_3$ | |
| $SCF_3$ | $CH_3$ | $CH_3$ | H | $CH_3$ | $CH_3$ | |
| $SCF_2H$ | $CH_3$ | $CH_3$ | H | $CH_3$ | $CH_3$ | |
| $SO_2CH_3$ | $CH_3$ | $CH_3$ | H | $CH_3$ | $CH_3$ | |
| $SO_2CH_3$ | $C_2H_5$ | $C_2H_5$ | H | $CH_3$ | $CH_3$ | |
| $SO_2CH_3$ | $C_2H_5$ | $CH_3$ | H | $CH_3$ | $CH_3$ | |
| $SO_2CH_2CH{=}CH_2$ | $CH_3$ | $CH_3$ | H | $CH_3$ | $CH_3$ | |
| $SO_2CF_3$ | $CH_3$ | $CH_3$ | H | $CH_3$ | $CH_3$ | |
| $SO_2CF_2H$ | $CH_3$ | $CH_3$ | H | $CH_3$ | $CH_3$ | |
| $OSO_2CH_3$ | $CH_3$ | $CH_3$ | H | $CH_3$ | $CH_3$ | |
| $OSO_2CH_2CH_3$ | $CH_3$ | $CH_3$ | H | $CH_3$ | $CH_3$ | |
| $OSO_2CH(CH_3)_2$ | $CH_3$ | $CH_3$ | H | $CH_3$ | $CH_3$ | |
| $OCH_3$ | $CH_3$ | $CH_3$ | H | $CH_3$ | $CH_3$ | |
| $OCH_2CH_3$ | $CH_3$ | $CH_3$ | H | $CH_3$ | $CH_3$ | |

Table 2 (continued)

| $R_5$ | $R_a$ | $R_b$ | $R_6$ | X | Y | m.p.(°C) |
|---|---|---|---|---|---|---|
| $OCH_2CH_2CH_3$ | $CH_3$ | $CH_3$ | H | $CH_3$ | $CH_3$ | |
| $OCH(CH_3)CH_2CH_3$ | $CH_3$ | $CH_3$ | H | $CH_3$ | $CH_3$ | |
| $OCH_2CH=CH_2$ | $CH_3$ | $CH_3$ | H | $CH_3$ | $CH_3$ | |
| $OCH_2CH_2CH=CH_2$ | $CH_3$ | $CH_3$ | H | $CH_3$ | $CH_3$ | |
| $OCH(CH_3)CH=CH_2$ | $CH_3$ | $CH_3$ | H | $CH_3$ | $CH_3$ | |
| $SO_2N(CH_3)_2$ | $CH_3$ | $CH_3$ | H | $CH_3$ | $CH_3$ | |
| $SO_2N(CH_3)CH(CH_3)_2$ | $CH_3$ | $CH_3$ | H | $CH_3$ | $CH_3$ | |
| $SO_2N(OCH_3)CH_3$ | $CH_3$ | $CH_3$ | H | $CH_3$ | $CH_3$ | |
| $CH_2OCH_3$ | $CH_3$ | $CH_3$ | H | $CH_3$ | $CH_3$ | |
| $CH_2CH_2OCH_3$ | $CH_3$ | $CH_3$ | H | $CH_3$ | $CH_3$ | |
| F | $CH_3$ | $CH_3$ | H | $CH_3$ | $CH_3$ | |
| Cl | $CH_3$ | $CH_3$ | H | $CH_3$ | $CH_3$ | |
| Br | $CH_3$ | $CH_3$ | H | $CH_3$ | $CH_3$ | |
| $NO_2$ | $CH_3$ | $CH_3$ | H | $CH_3$ | $CH_3$ | |
| $CF_3$ | $CH_3$ | $CH_3$ | H | $CH_3$ | $CH_3$ | |
| $CH_3$ | $CH_3$ | $CH_3$ | H | $CH_3$ | $CH_3$ | |
| $CH_2CH_3$ | $CH_3$ | $CH_3$ | H | $CH_3$ | $CH_3$ | |
| $CH(CH_3)_2$ | $CH_3$ | $CH_3$ | H | $CH_3$ | $CH_3$ | |
| $CH_2CH_2CH_2CH_3$ | $CH_3$ | $CH_3$ | H | $CH_3$ | $CH_3$ | |
| $CO_2CH_2CH_2CH_3$ | $CH_3$ | $CH_3$ | H | $CH_3$ | $CH_3$ | |
| $SO_2CH_2CH_3$ | $C_2H_5$ | $C_2H_5$ | H | $CH_3$ | $CH_3$ | |
| $CH_2OCH_3$ | $CH_3$ | $CH_3$ | H | $CH_3$ | $CH_3$ | |
| $CH_2CH_2OCH_3$ | $CH_3$ | $CH_3$ | H | $CH_3$ | $CH_3$ | |
| $NO_2$ | $CH_3$ | $CH_3$ | $5-CF_3$ | $CH_3$ | $CH_3$ | |
| $NO_2$ | $CH_3$ | $CH_3$ | 3-Cl | $CH_3$ | $CH_3$ | |
| Cl | $CH_3$ | $CH_3$ | $6-SCH_3$ | $CH_3$ | $CH_3$ | |
| $CF_3$ | $CH_3$ | $CH_3$ | $5-OCF_2H$ | $CH_3$ | $CH_3$ | |
| Cl | $CH_3$ | $CH_3$ | 5-Cl | $CH_3$ | $CH_3$ | |
| $NO_2$ | $CH_3$ | $CH_3$ | 5-Cl | $CH_3$ | $CH_3$ | |
| $SO_2N(CH_3)_2$ | $C_2H_5$ | $C_2H_5$ | 3-Cl | $CH_3$ | $CH_3$ | |
| $CO_2CH_3$ | $CH_3$ | $CH_3$ | $5-CH_3$ | $CH_3$ | $CH_3$ | |
| Cl | $CH_3$ | $CH_3$ | 3-Br | $CH_3$ | $CH_3$ | |

Table 2 (continued)

| $R_5$ | $R_a$ | $R_b$ | $R_6$ | X | Y | m.p.(°C) |
|---|---|---|---|---|---|---|
| Cl | $CH_3$ | $CH_3$ | 5-Br | $CH_3$ | $CH_3$ | |
| $NO_2$ | $CH_3$ | $CH_3$ | 6-F | $CH_3$ | $CH_3$ | |
| Cl | $CH_3$ | $CH_3$ | 6-F | $CH_3$ | $CH_3$ | |
| Br | $CH_3$ | $CH_3$ | 3-F | $CH_3$ | $CH_3$ | |
| $NO_2$ | $C_2H_5$ | $C_2H_5$ | 5-$SCH_3$ | $CH_3$ | $CH_3$ | |
| $CO_2CH_3$ | $CH_3$ | $CH_3$ | 3-$OCH_3$ | $CH_3$ | $CH_3$ | |
| $CH_3$ | $CH_3$ | $CH_3$ | 3-$OCH_3$ | $CH_3$ | $CH_3$ | |
| $CO_2CH_3$ | $C_2H_5$ | $CH_3$ | H | $CH_3$ | $OCH_3$ | |
| $CO_2CH_3$ | $C_2H_5$ | $C_2H_5$ | H | $CH_3$ | $OCH_3$ | |
| $NO_2$ | $C_2H_5$ | $CH_3$ | H | $CH_3$ | $OCH_3$ | |
| $SO_2N(CH_3)_2$ | $C_2H_5$ | $CH_3$ | H | $CH_3$ | $OCH_3$ | |
| $CO_2CH_3$ | $CH_3$ | $CH_3$ | H | $CH_3$ | $CH_2OCH_3$ | |
| $CO_2CH_3$ | $CH_3$ | $CH_3$ | H | $CH_3$ | $CH(OCH_3)_2$ | |
| $CO_2CH_3$ | $CH_3$ | $CH_3$ | H | $CH_3$ | $CH(OCH_2CH_2O)$ | |
| $NO_2$ | $C_2H_5$ | $C_2H_5$ | H | $CH_3$ | $OCH_3$ | |
| $SO_2N(CH_3)_2$ | $C_2H_5$ | $C_2H_5$ | H | $CH_3$ | $OCH_3$ | |
| $CO_2CH_3$ | $CH_3$ | $CH_3$ | H | $CH_3$ | $OCH_3$ | |
| $CO_2CH_2CH_3$ | $CH_3$ | $CH_3$ | H | $CH_3$ | $OCH_3$ | |
| $CO_2CH_2CH_2CH_3$ | $CH_3$ | $CH_3$ | H | $CH_3$ | $OCH_3$ | |
| $CO_2CH(CH_3)_2$ | $CH_3$ | $CH_3$ | H | $CH_3$ | $OCH_3$ | 89-91°(d) |
| $CO_2CH_2CH_2CH_2CH_3$ | $CH_3$ | $CH_3$ | H | $CH_3$ | $OCH_3$ | |
| $CO_2CH_2CH{=}CH_2$ | $CH_3$ | $CH_3$ | H | $CH_3$ | $OCH_3$ | |
| $CO_2CH_2CH_2OCH_3$ | $CH_3$ | $CH_3$ | H | $CH_3$ | $OCH_3$ | |
| $CO_2CH_2CH_2Cl$ | $CH_3$ | $CH_3$ | H | $CH_3$ | $OCH_3$ | |
| $SCH_3$ | $CH_3$ | $CH_3$ | H | $CH_3$ | $OCH_3$ | |
| $SCH_2CH_3$ | $CH_3$ | $CH_3$ | H | $CH_3$ | $OCH_3$ | |
| $SCH(CH_3)_2$ | $CH_3$ | $CH_3$ | H | $CH_3$ | $OCH_3$ | |
| $SCH_2CH{=}CH_2$ | $CH_3$ | $CH_3$ | H | $CH_3$ | $OCH_3$ | |
| $SCF_3$ | $CH_3$ | $CH_3$ | H | $CH_3$ | $OCH_3$ | |
| $SCF_2H$ | $CH_3$ | $CH_3$ | H | $CH_3$ | $OCH_3$ | |
| $SO_2CH_3$ | $CH_3$ | $CH_3$ | H | $CH_3$ | $OCH_3$ | |
| $SO_2CH_2CH_3$ | $CH_3$ | $CH_3$ | H | $CH_3$ | $OCH_3$ | |

Table 2 (continued)

| $R_5$ | $R_a$ | $R_b$ | $R_6$ | X | Y | m.p.(°C) |
|---|---|---|---|---|---|---|
| $SO_2CH(CH_3)_2$ | $CH_3$ | $CH_3$ | H | $CH_3$ | $OCH_3$ | |
| $SO_2CH_2CH=CH_2$ | $CH_3$ | $CH_3$ | H | $CH_3$ | $OCH_3$ | |
| $SO_2CF_3$ | $CH_3$ | $CH_3$ | H | $CH_3$ | $OCH_3$ | |
| $SO_2CF_2H$ | $CH_3$ | $CH_3$ | H | $CH_3$ | $OCH_3$ | |
| $OSO_2CH_3$ | $CH_3$ | $CH_3$ | H | $CH_3$ | $OCH_3$ | |
| $OSO_2CH_2CH_3$ | $CH_3$ | $CH_3$ | H | $CH_3$ | $OCH_3$ | |
| $OSO_2CH(CH_3)_2$ | $CH_3$ | $CH_3$ | H | $CH_3$ | $OCH_3$ | |
| $OCH_3$ | $CH_3$ | $CH_3$ | H | $CH_3$ | $OCH_3$ | |
| $OCH_2CH_3$ | $CH_3$ | $CH_3$ | H | $CH_3$ | $OCH_3$ | |
| $OCH(CH_3)_2$ | $CH_3$ | $CH_3$ | H | $CH_3$ | $OCH_3$ | |
| $OCH_2CH_2CH_2CH_3$ | $CH_3$ | $CH_3$ | H | $CH_3$ | $OCH_3$ | |
| $OCH_2CH=CH_2$ | $CH_3$ | $CH_3$ | H | $CH_3$ | $OCH_3$ | |
| $OCH_2CH_2CH=CH_2$ | $CH_3$ | $CH_3$ | H | $CH_3$ | $OCH_3$ | |
| $OCH(CH_3)CH=CH_2$ | $CH_3$ | $CH_3$ | H | $CH_3$ | $OCH_3$ | |
| $SO_2N(CH_3)_2$ | $CH_3$ | $CH_3$ | H | $CH_3$ | $OCH_3$ | |
| $SO_2N(CH_3)CH(CH_3)_2$ | $CH_3$ | $CH_3$ | H | $CH_3$ | $OCH_3$ | |
| $SO_2N(OCH_3)CH_3$ | $CH_3$ | $CH_3$ | H | $CH_3$ | $OCH_3$ | |
| $CH_2OCH_3$ | $CH_3$ | $CH_3$ | H | $CH_3$ | $OCH_3$ | |
| $CH_2CH_2OCH_3$ | $CH_3$ | $CH_3$ | H | $CH_3$ | $OCH_3$ | |
| F | $CH_3$ | $CH_3$ | H | $CH_3$ | $OCH_3$ | |
| Cl | $CH_3$ | $CH_3$ | H | $CH_3$ | $OCH_3$ | |
| Br | $CH_3$ | $CH_3$ | H | $CH_3$ | $OCH_3$ | |
| $NO_2$ | $CH_3$ | $CH_3$ | H | $CH_3$ | $OCH_3$ | |
| $CF_3$ | $CH_3$ | $CH_3$ | H | $CH_3$ | $OCH_3$ | |
| $CH_3$ | $CH_3$ | $CH_3$ | H | $CH_3$ | $OCH_3$ | |
| $CH_2CH_3$ | $CH_3$ | $CH_3$ | H | $CH_3$ | $OCH_3$ | |
| $CH(CH_3)_2$ | $CH_3$ | $CH_3$ | H | $CH_3$ | $OCH_3$ | |
| $CH_2CH_2CH_2CH_3$ | $CH_3$ | $CH_3$ | H | $CH_3$ | $OCH_3$ | |
| $SCH_3$ | $C_2H_5$ | $CH_3$ | H | $CH_3$ | $OCH_3$ | |
| $SCF_3$ | $C_2H_5$ | $CH_3$ | H | $CH_3$ | $OCH_3$ | |
| $NO_2$ | $CH_3$ | $CH_3$ | 5-$CF_3$ | $CH_3$ | $OCH_3$ | |
| $NO_2$ | $CH_3$ | $CH_3$ | 3-Cl | $CH_3$ | $OCH_3$ | |

Table 2 (continued)

| $R_5$ | $R_a$ | $R_b$ | $R_6$ | X | Y | m.p.(°C) |
|---|---|---|---|---|---|---|
| Cl | $CH_3$ | $CH_3$ | 6-$SCH_3$ | $CH_3$ | $OCH_3$ | |
| $CF_3$ | $CH_3$ | $CH_3$ | 5-$OCF_2H$ | $CH_3$ | $OCH_3$ | |
| Cl | $CH_3$ | $CH_3$ | 5-Cl | $CH_3$ | $OCH_3$ | |
| $NO_2$ | $CH_3$ | $CH_3$ | 5-Cl | $CH_3$ | $OCH_3$ | |
| $SO_2N(CH_3)_2$ | $CH_3$ | $CH_3$ | 3-Cl | $CH_3$ | $OCH_3$ | |
| $CO_2CH_3$ | $CH_3$ | $CH_3$ | 5-$CH_3$ | $CH_3$ | $OCH_3$ | |
| Cl | $CH_3$ | $CH_3$ | 3-Br | $CH_3$ | $OCH_3$ | |
| Cl | $CH_3$ | $CH_3$ | 5-Br | $CH_3$ | $OCH_3$ | |
| $NO_2$ | $CH_3$ | $CH_3$ | 6-F | $CH_3$ | $OCH_3$ | |
| Cl | $CH_3$ | $CH_3$ | 6-F | $CH_3$ | $OCH_3$ | |
| Br | $CH_3$ | $CH_3$ | 3-F | $CH_3$ | $OCH_3$ | |
| $NO_2$ | $CH_3$ | $CH_3$ | 5-$SCH_3$ | $CH_3$ | $OCH_3$ | |
| $CO_2CH_3$ | $CH_3$ | $CH_3$ | 3-$OCH_3$ | $CH_3$ | $OCH_3$ | |
| $CH_3$ | $CH_3$ | $CH_3$ | 3-$OCH_3$ | $CH_3$ | $OCH_3$ | |
| $CO_2CH_3$ | $C_2H_5$ | $CH_3$ | H | $OCH_3$ | $OCH_3$ | |
| $CO_2CH_3$ | $C_2H_5$ | $C_2H_5$ | H | $OCH_3$ | $OCH_3$ | |
| $NO_2$ | $C_2H_5$ | $CH_3$ | H | $OCH_3$ | $OCH_3$ | |
| $SO_2N(CH_3)_2$ | $C_2H_5$ | $CH_3$ | H | $OCH_3$ | $OCH_3$ | |
| $CO_2CH_3$ | $CH_3$ | $CH_3$ | H | $OCF_2H$ | $OCH_3$ | |
| $CO_2CH_3$ | $CH_3$ | $CH_3$ | H | $CF_3$ | $OCH_3$ | |
| $CO_2CH_3$ | $CH_3$ | $CH_3$ | H | $CH_2F$ | $OCH_3$ | |
| $NO_2$ | $C_2H_5$ | $C_2H_5$ | H | $OCH_3$ | $OCH_3$ | |
| $SO_2N(CH_3)_2$ | $C_2H_5$ | $C_2H_5$ | H | $OCH_3$ | $OCH_3$ | |
| $CO_2CH_3$ | $CH_3$ | $CH_3$ | H | $OCH_3$ | $OCH_3$ | 88-90°(d) |
| $CO_2CH_2CH_3$ | $CH_3$ | $CH_3$ | H | $OCH_3$ | $OCH_3$ | |
| $CO_2CH_2CH_2CH_3$ | $CH_3$ | $CH_3$ | H | $OCH_3$ | $OCH_3$ | |
| $CO_2CH(CH_3)_2$ | $CH_3$ | $CH_3$ | H | $OCH_3$ | $OCH_3$ | |
| $CO_2CH_2CH_2CH_2CH_3$ | $CH_3$ | $CH_3$ | H | $OCH_3$ | $OCH_3$ | |
| $CO_2CH_2CH{=}CH_2$ | $CH_3$ | $CH_3$ | H | $OCH_3$ | $OCH_3$ | |
| $CO_2CH_2CH_2OCH_3$ | $CH_3$ | $CH_3$ | H | $OCH_3$ | $OCH_3$ | |
| $CO_2CH_2CH_2Cl$ | $CH_3$ | $CH_3$ | H | $OCH_3$ | $OCH_3$ | |
| $SCH_3$ | $CH_3$ | $CH_3$ | H | $OCH_3$ | $OCH_3$ | |

Table 2 (continued)

| $R_5$ | $R_a$ | $R_b$ | $R_6$ | X | Y | m.p.(°C) |
|---|---|---|---|---|---|---|
| $SCH_2CH_3$ | $CH_3$ | $CH_3$ | H | $OCH_3$ | $OCH_3$ | |
| $SCH(CH_3)_2$ | $CH_3$ | $CH_3$ | H | $OCH_3$ | $OCH_3$ | |
| $SCH_2CH=CH_2$ | $CH_3$ | $CH_3$ | H | $OCH_3$ | $OCH_3$ | |
| $SCF_3$ | $CH_3$ | $CH_3$ | H | $OCH_3$ | $OCH_3$ | |
| $SCF_2H$ | $CH_3$ | $CH_3$ | H | $OCH_3$ | $OCH_3$ | |
| $SO_2CH_3$ | $CH_3$ | $CH_3$ | H | $OCH_3$ | $OCH_3$ | |
| $SO_2CH_2CH_3$ | $CH_3$ | $CH_3$ | H | $OCH_3$ | $OCH_3$ | |
| $SO_2CH(CH_3)_2$ | $CH_3$ | $CH_3$ | H | $OCH_3$ | $OCH_3$ | |
| $SO_2CH_2CH=CH_2$ | $CH_3$ | $CH_3$ | H | $OCH_3$ | $OCH_3$ | |
| $SO_2CF_3$ | $CH_3$ | $CH_3$ | H | $OCH_3$ | $OCH_3$ | |
| $SO_2CF_2H$ | $CH_3$ | $CH_3$ | H | $OCH_3$ | $OCH_3$ | |
| $OSO_2CH_3$ | $CH_3$ | $CH_3$ | H | $OCH_3$ | $OCH_3$ | |
| $OSO_2CH_2CH_3$ | $CH_3$ | $CH_3$ | H | $OCH_3$ | $OCH_3$ | |
| $OSO_2CH(CH_3)_2$ | $CH_3$ | $CH_3$ | H | $OCH_3$ | $OCH_3$ | |
| $OCH_3$ | $CH_3$ | $CH_3$ | H | $OCH_3$ | $OCH_3$ | |
| $OCH_2CH_3$ | $CH_3$ | $CH_3$ | H | $OCH_3$ | $OCH_3$ | |
| $OCH(CH_3)_2$ | $CH_3$ | $CH_3$ | H | $OCH_3$ | $OCH_3$ | |
| $OCH_2CH_2CH_2CH_3$ | $CH_3$ | $CH_3$ | H | $OCH_3$ | $OCH_3$ | |
| $OCH_2CH=CH_2$ | $CH_3$ | $CH_3$ | H | $OCH_3$ | $OCH_3$ | |
| $OCH_2CH_2CH=CH_2$ | $CH_3$ | $CH_3$ | H | $OCH_3$ | $OCH_3$ | |
| $OCH(CH_3)CH=CH_2$ | $CH_3$ | $CH_3$ | H | $OCH_3$ | $OCH_3$ | |
| $SO_2N(CH_3)_2$ | $CH_3$ | $CH_3$ | H | $OCH_3$ | $OCH_3$ | |
| $SO_2N(CH_3)CH(CH_3)_2$ | $CH_3$ | $CH_3$ | H | $OCH_3$ | $OCH_3$ | |
| $SO_2N(OCH_3)CH_3$ | $CH_3$ | $CH_3$ | H | $OCH_3$ | $OCH_3$ | |
| $CH_2OCH_3$ | $CH_3$ | $CH_3$ | H | $OCH_3$ | $OCH_3$ | |
| $CH_2CH_2OCH_3$ | $CH_3$ | $CH_3$ | H | $OCH_3$ | $OCH_3$ | |
| F | $CH_3$ | $CH_3$ | H | $OCH_3$ | $OCH_3$ | |
| Cl | $CH_3$ | $CH_3$ | H | $OCH_3$ | $OCH_3$ | 98-99°(d) |
| Br | $CH_3$ | $CH_3$ | H | $OCH_3$ | $OCH_3$ | |
| $NO_2$ | $CH_3$ | $CH_3$ | H | $OCH_3$ | $OCH_3$ | 125-127°(d) |
| $CF_3$ | $CH_3$ | $CH_3$ | H | $OCH_3$ | $OCH_3$ | |
| $CH_3$ | $CH_3$ | $CH_3$ | H | $OCH_3$ | $OCH_3$ | |

Table 2 (continued)

| $R_5$ | $R_a$ | $R_b$ | $R_6$ | X | Y | m.p.(°C) |
|---|---|---|---|---|---|---|
| $CH_2CH_3$ | $CH_3$ | $CH_3$ | H | $OCH_3$ | $OCH_3$ | |
| $CH(CH_3)_2$ | $CH_3$ | $CH_3$ | H | $OCH_3$ | $OCH_3$ | |
| $CH_2CH_2CH_2CH_3$ | $CH_3$ | $CH_3$ | H | $OCH_3$ | $OCH_3$ | |
| $SCH_3$ | $C_2H_5$ | $CH_3$ | H | $OCH_3$ | $OCH_3$ | |
| $SCF_3$ | $C_2H_5$ | $CH_3$ | H | $OCH_3$ | $OCH_3$ | |
| $NO_2$ | $CH_3$ | $CH_3$ | 5-$CF_3$ | $OCH_3$ | $OCH_3$ | |
| $NO_2$ | $CH_3$ | $CH_3$ | 3-Cl | $OCH_3$ | $OCH_3$ | |
| Cl | $CH_3$ | $CH_3$ | 6-$SCH_3$ | $OCH_3$ | $OCH_3$ | |
| $CF_3$ | $CH_3$ | $CH_3$ | 5-$OCF_2H$ | $OCH_3$ | $OCH_3$ | |
| Cl | $CH_3$ | $CH_3$ | 5-Cl | $OCH_3$ | $OCH_3$ | |
| $NO_2$ | $CH_3$ | $CH_3$ | 5-Cl | $OCH_3$ | $OCH_3$ | |
| $SO_2N(CH_3)_2$ | $CH_3$ | $CH_3$ | 3-Cl | $OCH_3$ | $OCH_3$ | |
| $CO_2CH_3$ | $CH_3$ | $CH_3$ | 5-$CH_3$ | $OCH_3$ | $OCH_3$ | |
| Cl | $CH_3$ | $CH_3$ | 3-Br | $OCH_3$ | $OCH_3$ | |
| Cl | $CH_3$ | $CH_3$ | 5-Br | $OCH_3$ | $OCH_3$ | |
| $NO_2$ | $CH_3$ | $CH_3$ | 6-F | $OCH_3$ | $OCH_3$ | |
| Cl | $CH_3$ | $CH_3$ | 6-F | $OCH_3$ | $OCH_3$ | |
| Br | $CH_3$ | $CH_3$ | 3-F | $OCH_3$ | $OCH_3$ | |
| $NO_2$ | $CH_3$ | $CH_3$ | 5-$SCH_3$ | $OCH_3$ | $OCH_3$ | |
| $CO_2CH_3$ | $CH_3$ | $CH_3$ | 3-$OCH_3$ | $OCH_3$ | $OCH_3$ | |
| $CH_3$ | $CH_3$ | $CH_3$ | 3-$OCH_3$ | $OCH_3$ | $OCH_3$ | |
| $CO_2CH_3$ | $CH_3$ | $CH_3$ | H | $CH_3$ | H | |
| $CO_2CH_3$ | $CH_3$ | $CH_3$ | H | $OCH_3$ | H | |
| $CO_2CH_3$ | $CH_3$ | $CH_3$ | H | $CH_3$ | $C_2H_5$ | |
| $CO_2CH_3$ | $CH_3$ | $CH_3$ | H | $OCH_3$ | $C_2H_5$ | |
| $CO_2CH_3$ | $CH_3$ | $CH_3$ | H | $CF_3$ | $OCH_3$ | |
| $CO_2CH_3$ | $CH_3$ | $CH_3$ | H | $CF_3$ | $CH_3$ | |
| $CO_2CH_3$ | $CH_3$ | $CH_3$ | H | $OC_2H_5$ | $N(CH_3)_2$ | |
| $CO_2CH_3$ | $CH_3$ | $CH_3$ | H | $OC_2H_5$ | $CH_3$ | |
| $CO_2CH_3$ | $CH_3$ | $CH_3$ | H | $CH_3$ | $OCH_2CF_3$ | |
| $CO_2CH_3$ | $CH_3$ | $CH_3$ | H | $OCH_3$ | $OCH_2CF_3$ | |

Table 2 (continued)

| $R_5$ | $R_a$ | $R_b$ | $R_6$ | X | Y | m.p.(°C) |
|---|---|---|---|---|---|---|
| 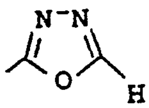 | $CH_3$ | $CH_3$ | H | $CH_3$ | $CH_3$ | |
| 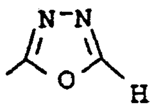 | $CH_3$ | $CH_3$ | H | $CH_3$ | $OCH_3$ | |
| 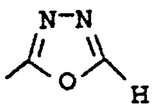 | $CH_3$ | $CH_3$ | H | $OCH_3$ | $OCH_3$ | |
| 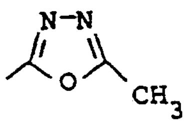 | $CH_3$ | $CH_3$ | H | $CH_3$ | $CH_3$ | |
| 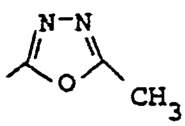 | $CH_3$ | $CH_3$ | H | $CH_3$ | $OCH_3$ | |
| 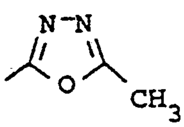 | $CH_3$ | $CH_3$ | H | $OCH_3$ | $OCH_3$ | |
| 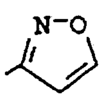 | $CH_3$ | $CH_3$ | H | $CH_3$ | $CH_3$ | |
| 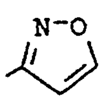 | $CH_3$ | $CH_3$ | H | $CH_3$ | $OCH_3$ | |
| 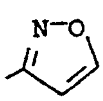 | $CH_3$ | $CH_3$ | H | $OCH_3$ | $OCH_3$ | |
| 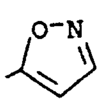 | $CH_3$ | $CH_3$ | H | $CH_3$ | $CH_3$ | |
| 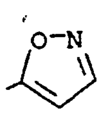 | $CH_3$ | $CH_3$ | H | $CH_3$ | $OCH_3$ | |

Table 2 (continued)

| $R_5$ | $R_a$ | $R_b$ | $R_6$ | X | Y | m.p.(°C) |
|---|---|---|---|---|---|---|
| 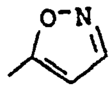 | $CH_3$ | $CH_3$ | H | $OCH_3$ | $OCH_3$ | |
| 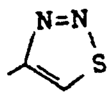 | $CH_3$ | $CH_3$ | H | $CH_3$ | $CH_3$ | |
| 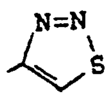 | $CH_3$ | $CH_3$ | H | $CH_3$ | $OCH_3$ | |
| 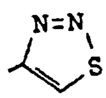 | $CH_3$ | $CH_3$ | H | $OCH_3$ | $OCH_3$ | |
| $CO_2CH_3$ | $CH_3$ | $CH_3$ | H | $CH_3$ | $OCH_2CH=CH_2$ | |
| $CO_2CH_3$ | $CH_3$ | $CH_3$ | H | $CH_3$ | $SCH_3$ | |
| $CO_2CH_3$ | $CH_3$ | $CH_3$ | H | $CH_3$ | $OCH_2C\equiv CH$ | |
| $CO_2CH_3$ | $CH_3$ | $CH_3$ | H | $CH_3$ | $CH_2SCH_3$ | |
| $CO_2CH_3$ | $CH_3$ | $CH_3$ | H | $CH_3$ | $SCF_2H$ | |

Table 3

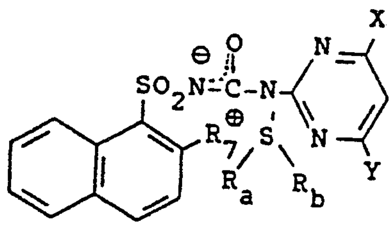

| $R_7$ | $R_a$ | $R_b$ | X | Y | m.p.(°C) |
|---|---|---|---|---|---|
| H | $CH_3$ | $CH_3$ | $CH_3$ | $CH_3$ | |
| $CH_3$ | $CH_3$ | $CH_3$ | $CH_3$ | $CH_3$ | |
| F | $CH_3$ | $CH_3$ | $CH_3$ | $CH_3$ | |
| Br | $CH_3$ | $CH_3$ | $CH_3$ | $CH_3$ | |
| $OCH_3$ | $CH_3$ | $CH_3$ | $CH_3$ | $CH_3$ | |
| $SCH_3$ | $CH_3$ | $CH_3$ | $CH_3$ | $CH_3$ | |
| Cl | $CH_3$ | $CH_3$ | $CH_3$ | $CH_3$ | |
| $OSO_2CH_3$ | $CH_3$ | $CH_3$ | $CH_3$ | $CH_3$ | |
| $SO_2CH_3$ | $CH_3$ | $CH_3$ | $CH_3$ | $CH_3$ | |
| H | $CH_3$ | $CH_3$ | $CH_3$ | $OCH_3$ | |
| $CH_3$ | $CH_3$ | $CH_3$ | $CH_3$ | $OCH_3$ | |
| F | $CH_3$ | $CH_3$ | $CH_3$ | $OCH_3$ | |
| Br | $CH_3$ | $CH_3$ | $CH_3$ | $OCH_3$ | |
| $OCH_3$ | $CH_3$ | $CH_3$ | $CH_3$ | $OCH_3$ | |
| $SCH_3$ | $CH_3$ | $CH_3$ | $CH_3$ | $OCH_3$ | |
| Cl | $CH_3$ | $CH_3$ | $CH_3$ | $OCH_3$ | |
| $OSO_2CH_3$ | $CH_3$ | $CH_3$ | $CH_3$ | $OCH_3$ | |
| $SO_2CH_3$ | $CH_3$ | $CH_3$ | $CH_3$ | $OCH_3$ | |
| $OCH_3$ | $C_2H_5$ | $C_2H_5$ | $CH_3$ | $CH_3$ | |
| $CH_3$ | $C_2H_5$ | $CH_3$ | $CH_3$ | $CH_3$ | |
| F | $C_2H_5$ | $C_2H_5$ | $CH_3$ | $OCH_3$ | |
| Br | $C_2H_5$ | $CH_3$ | $CH_3$ | $CH_3$ | |
| $OCH_3$ | $C_2H_5$ | $CH_3$ | $OCH_3$ | $OCH_3$ | |
| SMe | $C_2H_5$ | $C_2H_5$ | $CH_3$ | $OCH_3$ | |
| Cl | $C_2H_5$ | $C_2H_5$ | $CH_3$ | $CH_3$ | |

Table 3 (continued)

| $R_7$ | $R_a$ | $R_b$ | X | Y | m.p.(°C) |
|---|---|---|---|---|---|
| $OSO_2CH_3$ | $C_2H_5$ | $C_2H_5$ | $CH_3$ | $CH_3$ | |
| $SO_2CH_3$ | $C_2H_5$ | $C_2H_5$ | $CH_3$ | $OCH_3$ | |
| H | $CH_3$ | $CH_3$ | $OCH_3$ | $OCH_3$ | |
| $CH_3$ | $CH_3$ | $CH_3$ | $OCH_3$ | $OCH_3$ | |
| F | $CH_3$ | $CH_3$ | $OCH_3$ | $OCH_3$ | |
| Br | $CH_3$ | $CH_3$ | $OCH_3$ | $OCH_3$ | |
| $OCH_3$ | $CH_3$ | $CH_3$ | $OCH_3$ | $OCH_3$ | |
| $SCH_3$ | $CH_3$ | $CH_3$ | $OCH_3$ | $OCH_3$ | |
| Cl | $CH_3$ | $CH_3$ | $OCH_3$ | $OCH_3$ | |
| $OSO_2CH_3$ | $CH_3$ | $CH_3$ | $OCH_3$ | $OCH_3$ | |
| $SO_2CH_3$ | $CH_3$ | $CH_3$ | $OCH_3$ | $OCH_3$ | |
| Cl | $CH_3$ | $CH_3$ | Cl | $OCH_3$ | |
| Cl | $CH_3$ | $CH_3$ | Cl | $OC_2H_5$ | |
| Cl | $CH_3$ | $CH_3$ | F | $OCH_3$ | |
| Cl | $CH_3$ | $CH_3$ | Br | $OCH_3$ | |
| Cl | $CH_3$ | $CH_3$ | $OCF_2H$ | $CH_3$ | |
| Cl | $CH_3$ | $CH_3$ | $CH_2F$ | $OCH_3$ | |
| Cl | $CH_3$ | $CH_3$ | $CH_3$ | $CH_2OCH_3$ | |
| Cl | $CH_3$ | $CH_3$ | $OCH_3$ | $CH_2OCH_3$ | |
| Cl | $CH_3$ | $CH_3$ | $CH_3$ | $CH(OCH_3)_2$ | |
| Cl | $CH_3$ | $CH_3$ | $OCH_3$ | $CH(OCH_3)_2$ | |
| Cl | $CH_3$ | $CH_3$ | $CH_3$ | $CH(OCH_2CH_2O)$ | |
| Cl | $CH_3$ | $CH_3$ | $OCH_3$ | $CH(OCH_2CH_2O)$ | |
| Cl | $CH_3$ | $CH_3$ | $CF_3$ | $CH_3$ | |
| Cl | $CH_3$ | $CH_3$ | $CF_3$ | $OCH_3$ | |
| Cl | $CH_3$ | $CH_3$ | $OC_2H_5$ | $CH_3$ | |
| Cl | $CH_3$ | $CH_3$ | $OC_2H_5$ | $OCH_3$ | |
| Cl | $CH_3$ | $CH_3$ | $CH_3$ | $N(CH_3)_2$ | |
| Cl | $CH_3$ | $CH_3$ | $OCH_3$ | $SCH_3$ | |

Table 4

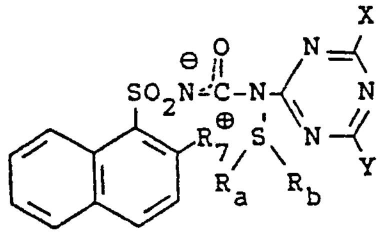

| $R_7$ | $R_a$ | $R_b$ | X | Y | m.p.(°C) |
|---|---|---|---|---|---|
| H | $CH_3$ | $CH_3$ | $CH_3$ | $CH_3$ | |
| $CH_3$ | $CH_3$ | $CH_3$ | $CH_3$ | $CH_3$ | |
| F | $CH_3$ | $CH_3$ | $CH_3$ | $CH_3$ | |
| Br | $CH_3$ | $CH_3$ | $CH_3$ | $CH_3$ | |
| $OCH_3$ | $CH_3$ | $CH_3$ | $CH_3$ | $CH_3$ | |
| $SCH_3$ | $CH_3$ | $CH_3$ | $CH_3$ | $CH_3$ | |
| Cl | $CH_3$ | $CH_3$ | $CH_3$ | $CH_3$ | |
| $OSO_2CH_3$ | $CH_3$ | $CH_3$ | $CH_3$ | $CH_3$ | |
| $SO_2CH_3$ | $CH_3$ | $CH_3$ | $CH_3$ | $CH_3$ | |
| H | $CH_3$ | $CH_3$ | $CH_3$ | $OCH_3$ | |
| $CH_3$ | $CH_3$ | $CH_3$ | $CH_3$ | $OCH_3$ | |
| F | $CH_3$ | $CH_3$ | $CH_3$ | $OCH_3$ | |
| Br | $CH_3$ | $CH_3$ | $CH_3$ | $OCH_3$ | |
| $OCH_3$ | $CH_3$ | $CH_3$ | $CH_3$ | $OCH_3$ | |
| $SCH_3$ | $CH_3$ | $CH_3$ | $CH_3$ | $OCH_3$ | |
| Cl | $CH_3$ | $CH_3$ | $CH_3$ | $OCH_3$ | |
| $OSO_2CH_3$ | $CH_3$ | $CH_3$ | $CH_3$ | $OCH_3$ | |
| $SO_2CH_3$ | $CH_3$ | $CH_3$ | $CH_3$ | $OCH_3$ | |
| $OCH_3$ | $C_2H_5$ | $C_2H_5$ | $CH_3$ | $CH_3$ | |
| $CH_3$ | $C_2H_5$ | $CH_3$ | $CH_3$ | $CH_3$ | |
| F | $C_2H_5$ | $C_2H_5$ | $CH_3$ | $OCH_3$ | |
| Br | $C_2H_5$ | $CH_3$ | $CH_3$ | $CH_3$ | |
| $OCH_3$ | $C_2H_5$ | $CH_3$ | $OCH_3$ | $OCH_3$ | |
| $SCH_3$ | $C_2H_5$ | $C_2H_5$ | $CH_3$ | $OCH_3$ | |
| Cl | $C_2H_5$ | $C_2H_5$ | $CH_3$ | $CH_3$ | |

Table 4 (continued)

| $R_7$ | $R_a$ | $R_b$ | X | Y | m.p.(°C) |
|---|---|---|---|---|---|
| $OSO_2CH_3$ | $C_2H_5$ | $C_2H_5$ | $CH_3$ | $CH_3$ | |
| $SO_2CH_3$ | $C_2H_5$ | $C_2H_5$ | $CH_3$ | $OCH_3$ | |
| H | $CH_3$ | $CH_3$ | $OCH_3$ | $OCH_3$ | |
| $CH_3$ | $CH_3$ | $CH_3$ | $OCH_3$ | $OCH_3$ | |
| F | $CH_3$ | $CH_3$ | $OCH_3$ | $OCH_3$ | |
| Br | $CH_3$ | $CH_3$ | $OCH_3$ | $OCH_3$ | |
| $OCH_3$ | $CH_3$ | $CH_3$ | $OCH_3$ | $OCH_3$ | |
| $SCH_3$ | $CH_3$ | $CH_3$ | $OCH_3$ | $OCH_3$ | |
| Cl | $CH_3$ | $CH_3$ | $OCH_3$ | $OCH_3$ | |
| $OSO_2CH_3$ | $CH_3$ | $CH_3$ | $OCH_3$ | $OCH_3$ | |
| $SO_2CH_3$ | $CH_3$ | $CH_3$ | $OCH_3$ | $OCH_3$ | |
| Cl | $CH_3$ | $CH_3$ | $CH_3$ | H | |
| Cl | $CH_3$ | $CH_3$ | $OCH_3$ | H | |
| Cl | $CH_3$ | $CH_3$ | $CH_3$ | $C_2H_5$ | |
| Cl | $CH_3$ | $CH_3$ | $OCH_3$ | $C_2H_5$ | |
| Cl | $CH_3$ | $CH_3$ | $CH_3$ | $CH_2OCH_3$ | |
| Cl | $CH_3$ | $CH_3$ | $OCH_3$ | $CH_2OCH_3$ | |
| Cl | $CH_3$ | $CH_3$ | $CH_3$ | $CH(OCH_3)_2$ | |
| Cl | $CH_3$ | $CH_3$ | $OCH_3$ | $CH(OCH_3)_2$ | |
| Cl | $CH_3$ | $CH_3$ | $CH_3$ | $CH(OCH_2CH_2O)$ | |
| Cl | $CH_3$ | $CH_3$ | $OCH_3$ | $CH(OCH_2CH_2O)$ | |
| Cl | $CH_3$ | $CH_3$ | $CF_3$ | $CH_3$ | |
| Cl | $CH_3$ | $CH_3$ | $CF_3$ | $OCH_3$ | |
| Cl | $CH_3$ | $CH_3$ | $OC_2H_5$ | $CH_3$ | |
| Cl | $CH_3$ | $CH_3$ | $OC_2H_5$ | $OCH_3$ | |
| Cl | $CH_3$ | $CH_3$ | $CH_3$ | $OCH_2CF_3$ | |
| Cl | $CH_3$ | $CH_3$ | $OCH_3$ | $OCH_2CF_3$ | |

Table 5

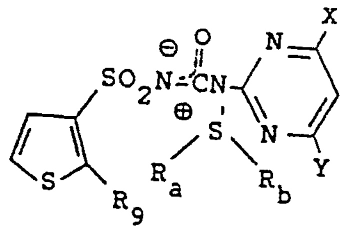

| $R_9$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| Cl | $CH_3$ | $CH_3$ | $CH_3$ | $CH_3$ | |
| Br | $CH_3$ | $CH_3$ | $CH_3$ | $CH_3$ | |
| $NO_2$ | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| $CO_2CH_3$ | $CH_3$ | $CH_3$ | $CH_3$ | $CH_3$ | |
| F | $CH_3$ | $CH_3$ | $CH_3$ | $OCH_3$ | |
| $CH_3$ | $CH_3$ | $CH_3$ | $CH_3$ | $OCH_3$ | |
| Cl | $CH_3$ | $CH_3$ | $CH_3$ | $OCH_3$ | |
| Br | $CH_3$ | $CH_3$ | $CH_3$ | $OCH_3$ | |
| $NO_2$ | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $CO_2CH_3$ | $CH_3$ | $CH_3$ | $CH_3$ | $OCH_3$ | |
| $CO_2CH_3$ | $C_2H_5$ | $C_2H_5$ | $OCH_3$ | $OCH_3$ | |
| $CO_2CH_3$ | $C_2H_5$ | $CH_3$ | $CH_3$ | $CH_3$ | |
| Cl | $C_2H_5$ | $CH_3$ | $OCH_3$ | $OCH_3$ | |
| Br | $C_2H_5$ | $C_2H_5$ | $OCH_3$ | $OCH_3$ | |
| $NO_2$ | $C_2H_5$ | $C_2H_5$ | $CH_3$ | $OCH_3$ | |
| $SO_2CH_3$ | $C_2H_5$ | $C_2H_5$ | $CH_3$ | $CH_3$ | |
| $SO_2N(CH_3)_2$ | $C_2H_5$ | $C_2H_5$ | $CH_3$ | $CH_3$ | |
| $CO_2CH_3$ | $C_2H_5$ | $C_2H_5$ | $CH_3$ | $CH_3$ | |

Table 5 (continued)

| $R_9$ | $R_a$ | $R_b$ | X | Y | m.p.(°C) |
|---|---|---|---|---|---|
| F | $CH_3$ | $CH_3$ | $OCH_3$ | $OCH_3$ | |
| $CH_3$ | $CH_3$ | $CH_3$ | $OCH_3$ | $OCH_3$ | |
| Cl | $CH_3$ | $CH_3$ | $OCH_3$ | $OCH_3$ | |
| Br | $CH_3$ | $CH_3$ | $OCH_3$ | $OCH_3$ | |
| $NO_2$ | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |
| $CO_2CH_3$ | $CH_3$ | $CH_3$ | $OCH_3$ | $OCH_3$ | 125-127°(d) |
| $CO_2CH_3$ | $CH_3$ | $CH_3$ | Cl | $OCH_3$ | |
| $CO_2CH_3$ | $CH_3$ | $CH_3$ | F | $OCH_3$ | |
| $CO_2CH_3$ | $CH_3$ | $CH_3$ | $OCF_2H$ | $CH_3$ | |
| $CO_2CH_3$ | $CH_3$ | $CH_3$ | Br | $OCH_3$ | |
| $CO_2CH_3$ | $CH_3$ | $CH_3$ | $CH_3$ | $C_2H_5$ | |
| $CO_2CH_3$ | $CH_3$ | $CH_3$ | $OCH_3$ | $C_2H_5$ | |
| $CO_2CH_3$ | $CH_3$ | $CH_3$ | $CH_3$ | $CH_2OCH_3$ | |
| $CO_2CH_3$ | $CH_3$ | $CH_3$ | $OCH_3$ | $CH_2OCH_3$ | |
| $CO_2CH_3$ | $CH_3$ | $CH_3$ | $CH_3$ | $CH(OCH_3)_2$ | |
| $CO_2CH_3$ | $CH_3$ | $CH_3$ | $OCH_3$ | $CH(OCH_3)_2$ | |
| $CO_2CH_3$ | $CH_3$ | $CH_3$ | $CH_3$ | $CH(OCH_2CH_2O)$ | |
| $CO_2CH_3$ | $CH_3$ | $CH_3$ | $OCH_3$ | $CH(OCH_2CH_2O)$ | |
| $CO_2CH_3$ | $CH_3$ | $CH_3$ | $CF_3$ | $CH_3$ | |
| $CO_2CH_3$ | $CH_3$ | $CH_3$ | $CF_3$ | $OCH_3$ | |
| $CO_2CH_3$ | $CH_3$ | $CH_3$ | $OC_2H_5$ | $CH_3$ | |
| $CO_2CH_3$ | $CH_3$ | $CH_3$ | $OC_2H_5$ | $OCH_3$ | |
| $CO_2CH_3$ | $CH_3$ | $CH_3$ | $CH_3$ | $OCH_2CF_3$ | |
| $CO_2CH_3$ | $CH_3$ | $CH_3$ | $OCH_3$ | $OCH_2CF_3$ | |

Table 6

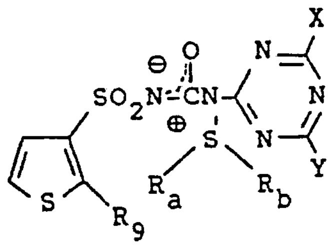

| $R_9$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| Cl | $CH_3$ | $CH_3$ | $CH_3$ | $CH_3$ | |
| Br | $CH_3$ | $CH_3$ | $CH_3$ | $CH_3$ | |
| $NO_2$ | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| $CO_2CH_3$ | $CH_3$ | $CH_3$ | $CH_3$ | $CH_3$ | 110-115°(d) |
| F | $CH_3$ | $CH_3$ | $CH_3$ | $OCH_3$ | |
| $CH_3$ | $CH_3$ | $CH_3$ | $CH_3$ | $OCH_3$ | |
| Cl | $CH_3$ | $CH_3$ | $CH_3$ | $OCH_3$ | |
| Br | $CH_3$ | $CH_3$ | $CH_3$ | $OCH_3$ | |
| $NO_2$ | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $CO_2CH_3$ | $CH_3$ | $CH_3$ | $CH_3$ | $OCH_3$ | 98-100°C |
| $CO_2CH_3$ | $C_2H_5$ | $C_2H_5$ | $OCH_3$ | $OCH_3$ | |
| $CO_2CH_3$ | $C_2H_5$ | $CH_3$ | $CH_3$ | $CH_3$ | |
| Cl | $C_2H_5$ | $CH_3$ | $OCH_3$ | $OCH_3$ | |
| Br | $C_2H_5$ | $C_2H_5$ | $OCH_3$ | $OCH_3$ | |
| $NO_2$ | $C_2H_5$ | $C_2H_5$ | $CH_3$ | $OCH_3$ | |
| $SO_2CH_3$ | $C_2H_5$ | $C_2H_5$ | $CH_3$ | $CH_3$ | |
| $SO_2N(CH_3)_2$ | $C_2H_5$ | $C_2H_5$ | $CH_3$ | $CH_3$ | |
| $CO_2CH_3$ | $C_2H_5$ | $C_2H_5$ | $CH_3$ | $CH_3$ | |

Table 6 (continued)

| $R_9$ | $R_a$ | $R_b$ | X | Y | m.p.(°C) |
|---|---|---|---|---|---|
| F | $CH_3$ | $CH_3$ | $OCH_3$ | $OCH_3$ | |
| $CH_3$ | $CH_3$ | $CH_3$ | $OCH_3$ | $OCH_3$ | |
| Cl | $CH_3$ | $CH_3$ | $OCH_3$ | $OCH_3$ | |
| Br | $CH_3$ | $CH_3$ | $OCH_3$ | $OCH_3$ | |
| $NO_2$ | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |
| $CO_2CH_3$ | $CH_3$ | $CH_3$ | $OCH_3$ | $OCH_3$ | |
| $CO_2CH_3$ | $CH_3$ | $CH_3$ | $CH_2F$ | $CH_3$ | |
| $CO_2CH_3$ | $CH_3$ | $CH_3$ | $OCH_2F$ | $OCH_3$ | |
| $CO_2CH_3$ | $CH_3$ | $CH_3$ | $CH_3$ | $C_2H_5$ | |
| $CO_2CH_3$ | $CH_3$ | $CH_3$ | $OCH_3$ | $C_2H_5$ | |
| $CO_2CH_3$ | $CH_3$ | $CH_3$ | $CH_3$ | $CH_2OCH_3$ | |
| $CO_2CH_3$ | $CH_3$ | $CH_3$ | $OCH_3$ | $CH_2OCH_3$ | |
| $CO_2CH_3$ | $CH_3$ | $CH_3$ | $CH_3$ | $CH(OCH_3)_2$ | |
| $CO_2CH_3$ | $CH_3$ | $CH_3$ | $OCH_3$ | $CH(OCH_3)_2$ | |
| $CO_2CH_3$ | $CH_3$ | $CH_3$ | $CH_3$ | $CH(OCH_2CH_2O)$ | |
| $CO_2CH_3$ | $CH_3$ | $CH_3$ | $OCH_3$ | $CH(OCH_2CH_2O)$ | |
| $CO_2CH_3$ | $CH_3$ | $CH_3$ | $CF_3$ | $CH_3$ | |
| $CO_2CH_3$ | $CH_3$ | $CH_3$ | $CF_3$ | $OCH_3$ | |
| $CO_2CH_3$ | $CH_3$ | $CH_3$ | $OC_2H_5$ | $N(CH_3)_2$ | |
| $CO_2CH_3$ | $CH_3$ | $CH_3$ | $OC_2H_5$ | $SCH_3$ | |
| $CO_2CH_3$ | $CH_3$ | $CH_3$ | $CH_3$ | $OCH_2CF_3$ | |
| $CO_2CH_3$ | $CH_3$ | $CH_3$ | $OCH_3$ | $OCH_2CF_3$ | |

Table 7

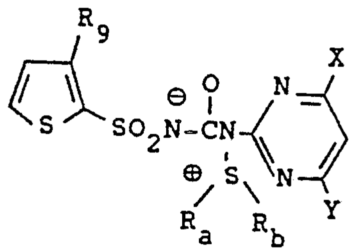

| $R_9$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| Cl | $CH_3$ | $CH_3$ | $CH_3$ | $CH_3$ | |
| Br | $CH_3$ | $CH_3$ | $CH_3$ | $CH_3$ | |
| $NO_2$ | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| $CO_2CH_3$ | $CH_3$ | $CH_3$ | $CH_3$ | $CH_3$ | |
| F | $CH_3$ | $CH_3$ | $CH_3$ | $OCH_3$ | |
| $CH_3$ | $CH_3$ | $CH_3$ | $CH_3$ | $OCH_3$ | |
| Cl | $CH_3$ | $CH_3$ | $CH_3$ | $OCH_3$ | |
| Br | $CH_3$ | $CH_3$ | $CH_3$ | $OCH_3$ | |
| $NO_2$ | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $CO_2CH_3$ | $CH_3$ | $CH_3$ | $CH_3$ | $OCH_3$ | |
| $CO_2CH_3$ | $C_2H_5$ | $C_2H_5$ | $OCH_3$ | $OCH_3$ | |
| $CO_2CH_3$ | $C_2H_5$ | $CH_3$ | $CH_3$ | $CH_3$ | |
| Cl | $C_2H_5$ | $CH_3$ | $OCH_3$ | $OCH_3$ | |
| Br | $C_2H_5$ | $C_2H_5$ | $OCH_3$ | $OCH_3$ | |
| $NO_2$ | $C_2H_5$ | $C_2H_5$ | $CH_3$ | $OCH_3$ | |
| $SO_2CH_3$ | $C_2H_5$ | $C_2H_5$ | $CH_3$ | $CH_3$ | |
| $SO_2N(CH_3)_2$ | $C_2H_5$ | $C_2H_5$ | $CH_3$ | $CH_3$ | |
| $CO_2CH_3$ | $C_2H_5$ | $C_2H_5$ | $CH_3$ | $CH_3$ | |

Table 7 (continued)

| $R_9$ | $R_a$ | $R_b$ | X | Y | m.p.(°C) |
|---|---|---|---|---|---|
| F | $CH_3$ | $CH_3$ | $OCH_3$ | $OCH_3$ | |
| $CH_3$ | $CH_3$ | $CH_3$ | $OCH_3$ | $OCH_3$ | |
| Cl | $CH_3$ | $CH_3$ | $OCH_3$ | $OCH_3$ | |
| Br | $CH_3$ | $CH_3$ | $OCH_3$ | $OCH_3$ | |
| $NO_2$ | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |
| $CO_2CH_3$ | $CH_3$ | $CH_3$ | $OCH_3$ | $OCH_3$ | |
| $CO_2CH_3$ | $CH_3$ | $CH_3$ | Cl | $OCH_3$ | |
| $CO_2CH_3$ | $CH_3$ | $CH_3$ | Cl | $OC_2H_5$ | |
| $CO_2CH_3$ | $CH_3$ | $CH_3$ | Br | $OCH_3$ | |
| $CO_2CH_3$ | $CH_3$ | $CH_3$ | F | $OCH_3$ | |
| $CO_2CH_3$ | $CH_3$ | $CH_3$ | $CH_3$ | $C_2H_5$ | |
| $CO_2CH_3$ | $CH_3$ | $CH_3$ | $OCH_3$ | $C_2H_5$ | |
| $CO_2CH_3$ | $CH_3$ | $CH_3$ | $CH_3$ | $CH_2OCH_3$ | |
| $CO_2CH_3$ | $CH_3$ | $CH_3$ | $OCH_3$ | $CH_2OCH_3$ | |
| $CO_2CH_3$ | $CH_3$ | $CH_3$ | $CH_3$ | $CH(OCH_3)_2$ | |
| $CO_2CH_3$ | $CH_3$ | $CH_3$ | $OCH_3$ | $CH(OCH_3)_2$ | |
| $CO_2CH_3$ | $CH_3$ | $CH_3$ | $CH_3$ | $CH(OCH_2CH_2O)$ | |
| $CO_2CH_3$ | $CH_3$ | $CH_3$ | $OCH_3$ | $CH(OCH_2CH_2O)$ | |
| $CO_2CH_3$ | $CH_3$ | $CH_3$ | $CF_3$ | $CH_3$ | |
| $CO_2CH_3$ | $CH_3$ | $CH_3$ | $CF_3$ | $OCH_3$ | |
| $CO_2CH_3$ | $CH_3$ | $CH_3$ | $OC_2H_5$ | $N(CH_3)_2$ | |
| $CO_2CH_3$ | $CH_3$ | $CH_3$ | $OC_2H_5$ | $CH_2SCH_3$ | |
| $CO_2CH_3$ | $CH_3$ | $CH_3$ | $CH_3$ | $OCH_2CF_3$ | |
| $CO_2CH_3$ | $CH_3$ | $CH_3$ | $OCH_3$ | $OCH_2CF_3$ | |

Table 8

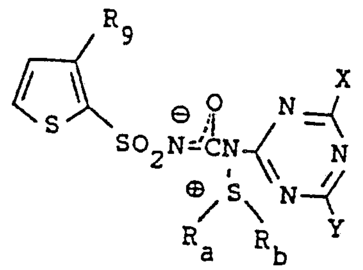

| $R_9$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| Cl | $CH_3$ | $CH_3$ | $CH_3$ | $CH_3$ | |
| Br | $CH_3$ | $CH_3$ | $CH_3$ | $CH_3$ | |
| $NO_2$ | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| $CO_2CH_3$ | $CH_3$ | $CH_3$ | $CH_3$ | $CH_3$ | |
| F | $CH_3$ | $CH_3$ | $CH_3$ | $OCH_3$ | |
| $CH_3$ | $CH_3$ | $CH_3$ | $CH_3$ | $OCH_3$ | |
| Cl | $CH_3$ | $CH_3$ | $CH_3$ | $OCH_3$ | |
| Br | $CH_3$ | $CH_3$ | $CH_3$ | $OCH_3$ | |
| $NO_2$ | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $CO_2CH_3$ | $CH_3$ | $CH_3$ | $CH_3$ | $OCH_3$ | |
| $CO_2CH_3$ | $C_2H_5$ | $C_2H_5$ | $OCH_3$ | $OCH_3$ | |
| $CO_2CH_3$ | $C_2H_5$ | $CH_3$ | $CH_3$ | $CH_3$ | |
| Cl | $C_2H_5$ | $CH_3$ | $OCH_3$ | $OCH_3$ | |
| Br | $C_2H_5$ | $C_2H_5$ | $OCH_3$ | $OCH_3$ | |
| $NO_2$ | $C_2H_5$ | $C_2H_5$ | $CH_3$ | $OCH_3$ | |
| $SO_2CH_3$ | $C_2H_5$ | $C_2H_5$ | $CH_3$ | $CH_3$ | |
| $SO_2N(CH_3)_2$ | $C_2H_5$ | $C_2H_5$ | $CH_3$ | $CH_3$ | |
| $CO_2CH_3$ | $C_2H_5$ | $C_2H_5$ | $CH_3$ | $CH_3$ | |

Table 8 (continued)

| $R_9$ | $R_a$ | $R_b$ | X | Y | m.p.(°C) |
|---|---|---|---|---|---|
| F | $CH_3$ | $CH_3$ | $OCH_3$ | $OCH_3$ | |
| $CH_3$ | $CH_3$ | $CH_3$ | $OCH_3$ | $OCH_3$ | |
| Cl | $CH_3$ | $CH_3$ | $OCH_3$ | $OCH_3$ | |
| Br | $CH_3$ | $CH_3$ | $OCH_3$ | $OCH_3$ | |
| $NO_2$ | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |
| $CO_2CH_3$ | $CH_3$ | $CH_3$ | $OCH_3$ | $OCH_3$ | |
| $CO_2CH_3$ | $CH_3$ | $CH_3$ | $CH_2F$ | $CH_3$ | |
| $CO_2CH_3$ | $CH_3$ | $CH_3$ | $OCF_2H$ | $OCH_3$ | |
| $CO_2CH_3$ | $CH_3$ | $CH_3$ | $CH_3$ | $C_2H_5$ | |
| $CO_2CH_3$ | $CH_3$ | $CH_3$ | $OCH_3$ | $C_2H_5$ | |
| $CO_2CH_3$ | $CH_3$ | $CH_3$ | $CH_3$ | $CH_2OCH_3$ | |
| $CO_2CH_3$ | $CH_3$ | $CH_3$ | $OCH_3$ | $CH_2OCH_3$ | |
| $CO_2CH_3$ | $CH_3$ | $CH_3$ | $CH_3$ | $CH(OCH_3)_2$ | |
| $CO_2CH_3$ | $CH_3$ | $CH_3$ | $OCH_3$ | $CH(OCH_3)_2$ | |
| $CO_2CH_3$ | $CH_3$ | $CH_3$ | $CH_3$ | $CH(OCH_2CH_2O)$ | |
| $CO_2CH_3$ | $CH_3$ | $CH_3$ | $OCH_3$ | $CH(OCH_2CH_2O)$ | |
| $CO_2CH_3$ | $CH_3$ | $CH_3$ | $CF_3$ | $CH_3$ | |
| $CO_2CH_3$ | $CH_3$ | $CH_3$ | $CF_3$ | $OCH_3$ | |
| $CO_2CH_3$ | $CH_3$ | $CH_3$ | $OC_2H_5$ | $CH_3$ | |
| $CO_2CH_3$ | $CH_3$ | $CH_3$ | $OC_2H_5$ | $OCH_3$ | |
| $CO_2CH_3$ | $CH_3$ | $CH_3$ | $CH_3$ | $OCH_2CF_3$ | |
| $CO_2CH_3$ | $CH_3$ | $CH_3$ | $OCH_3$ | $OCH_2CF_3$ | |

Table 9

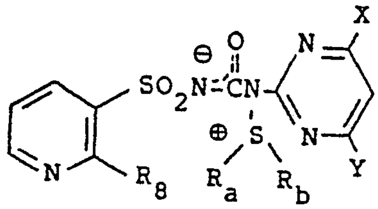

| $R_8$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| $OCH_3$ | $CH_3$ | $CH_3$ | $CH_3$ | $CH_3$ | |
| Cl | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| Br | $CH_3$ | $CH_3$ | $CH_3$ | $OCH_3$ | |
| $CH_3$ | $CH_3$ | $CH_3$ | $CH_3$ | $OCH_3$ | |
| $OCH_3$ | $CH_3$ | $CH_3$ | $CH_3$ | $OCH_3$ | |
| Cl | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $SO_2CH_3$ | $C_2H_5$ | $C_2H_5$ | $CH_3$ | $CH_3$ | |
| $CH_3$ | $C_2H_5$ | $CH_3$ | $CH_3$ | $CH_3$ | |
| $OCH_3$ | $C_2H_5$ | $CH_3$ | $OCH_3$ | $OCH_3$ | |
| Cl | $C_2H_5$ | $C_2H_5$ | $OCH_3$ | $OCH_3$ | |
| $SO_2CH_3$ | $C_2H_5$ | $CH_3$ | $CH_3$ | $CH_3$ | |
| $SO_2N(CH_3)_2$ | $C_2H_5$ | $C_2H_5$ | $CH_3$ | $CH_3$ | |
| F | $CH_3$ | $CH_3$ | $OCH_3$ | $OCH_3$ | |
| $CH_3$ | $CH_3$ | $CH_3$ | $OCH_3$ | $OCH_3$ | |
| $OCH_3$ | $CH_3$ | $CH_3$ | $OCH_3$ | $OCH_3$ | |
| Cl | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |

Table 9 (continued)

| $R_8$ | $R_a$ | $R_b$ | X | Y | m.p.(°C) |
|---|---|---|---|---|---|
| $SO_2CH_3$ | $CH_3$ | $CH_3$ | Cl | $OCH_3$ | |
| $SO_2CH_3$ | $CH_3$ | $CH_3$ | Cl | $OC_2H_5$ | |
| $SO_2CH_3$ | $CH_3$ | $CH_3$ | F | $OCH_3$ | |
| $SO_2CH_3$ | $CH_3$ | $CH_3$ | Br | $OCH_3$ | |
| $SO_2CH_3$ | $CH_3$ | $CH_3$ | $CH_3$ | $C_2H_5$ | |
| $SO_2CH_3$ | $CH_3$ | $CH_3$ | $OCH_3$ | $C_2H_5$ | |
| $SO_2CH_3$ | $CH_3$ | $CH_3$ | $CH_3$ | $CH_2OCH_3$ | |
| $SO_2CH_3$ | $CH_3$ | $CH_3$ | $OCH_3$ | $CH_2OCH_3$ | |
| $SO_2CH_3$ | $CH_3$ | $CH_3$ | $CH_3$ | $CH(OCH_3)_2$ | |
| $SO_2CH_3$ | $CH_3$ | $CH_3$ | $OCH_3$ | $CH(OCH_3)_2$ | |
| $SO_2CH_3$ | $CH_3$ | $CH_3$ | $CH_3$ | $CH(OCH_2CH_2O)$ | |
| $SO_2CH_3$ | $CH_3$ | $CH_3$ | $OCH_3$ | $CH(OCH_2CH_2O)$ | |
| $SO_2CH_3$ | $CH_3$ | $CH_3$ | $CF_3$ | $CH_3$ | |
| $SO_2CH_3$ | $CH_3$ | $CH_3$ | $CF_3$ | $OCH_3$ | |
| $SO_2CH_3$ | $CH_3$ | $CH_3$ | $OC_2H_5$ | $CH_3$ | |
| $SO_2CH_3$ | $CH_3$ | $CH_3$ | $OC_2H_5$ | $OCH_3$ | |
| $SO_2CH_3$ | $CH_3$ | $CH_3$ | $CH_3$ | $OCH_2CF_3$ | |
| $SO_2CH_3$ | $CH_3$ | $CH_3$ | $OCH_3$ | $OCH_2CF_3$ | |

Table 10

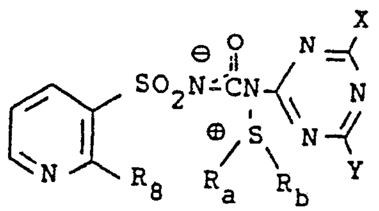

| $R_8$ | $R_a$ | $R_b$ | X | Y | m.p.(°C) |
|---|---|---|---|---|---|
| F | $CH_3$ | $CH_3$ | $CH_3$ | $CH_3$ | |
| $CH_3$ | $CH_3$ | $CH_3$ | $CH_3$ | $CH_3$ | |
| $OCH_3$ | $CH_3$ | $CH_3$ | $CH_3$ | $CH_3$ | |
| Cl | $CH_3$ | $CH_3$ | $CH_3$ | $CH_3$ | |
| $SO_2CH_3$ | $CH_3$ | $CH_3$ | $CH_3$ | $CH_3$ | |
| $SO_2N(CH_3)_2$ | $CH_3$ | $CH_3$ | $CH_3$ | $CH_3$ | |
| Br | $CH_3$ | $CH_3$ | $CH_3$ | $OCH_3$ | |
| $CH_3$ | $CH_3$ | $CH_3$ | $CH_3$ | $OCH_3$ | |
| $OCH_3$ | $CH_3$ | $CH_3$ | $CH_3$ | $OCH_3$ | |
| Cl | $CH_3$ | $CH_3$ | $CH_3$ | $OCH_3$ | |
| $SO_2CH_3$ | $CH_3$ | $CH_3$ | $CH_3$ | $OCH_3$ | |
| $SO_2N(CH_3)_2$ | $CH_3$ | $CH_3$ | $CH_3$ | $OCH_3$ | |
| $SO_2CH_3$ | $C_2H_5$ | $C_2H_5$ | $CH_3$ | $CH_3$ | |
| $CH_3$ | $C_2H_5$ | $CH_3$ | $CH_3$ | $CH_3$ | |
| $OCH_3$ | $C_2H_5$ | $CH_3$ | $OCH_3$ | $OCH_3$ | |
| Cl | $C_2H_5$ | $C_2H_5$ | $OCH_3$ | $OCH_3$ | |
| $SO_2CH_3$ | $C_2H_5$ | $CH_3$ | $CH_3$ | $CH_3$ | |
| $SO_2N(CH_3)_2$ | $C_2H_5$ | $C_2H_5$ | $CH_3$ | $CH_3$ | |
| F | $CH_3$ | $CH_3$ | $OCH_3$ | $OCH_3$ | |
| $CH_3$ | $CH_3$ | $CH_3$ | $OCH_3$ | $OCH_3$ | |
| $OCH_3$ | $CH_3$ | $CH_3$ | $OCH_3$ | $OCH_3$ | |
| Cl | $CH_3$ | $CH_3$ | $OCH_3$ | $OCH_3$ | |
| $SO_2CH_3$ | $CH_3$ | $CH_3$ | $OCH_3$ | $OCH_3$ | |
| $SO_2N(CH_3)_2$ | $CH_3$ | $CH_3$ | $OCH_3$ | $OCH_3$ | |

Table 10 (continued)

| $R_8$ | $R_a$ | $R_b$ | X | Y | m.p.(°C) |
|---|---|---|---|---|---|
| $SO_2CH_3$ | $CH_3$ | $CH_3$ | $OCF_2H$ | $CH_3$ | |
| $SO_2CH_3$ | $CH_3$ | $CH_3$ | $OCH_2F$ | $OCH_3$ | |
| $SO_2CH_3$ | $CH_3$ | $CH_3$ | $CH_3$ | $C_2H_5$ | |
| $SO_2CH_3$ | $CH_3$ | $CH_3$ | $OCH_3$ | $C_2H_5$ | |
| $SO_2CH_3$ | $CH_3$ | $CH_3$ | $CH_3$ | $CH_2OCH_3$ | |
| $SO_2CH_3$ | $CH_3$ | $CH_3$ | $OCH_3$ | $CH_2OCH_3$ | |
| $SO_2CH_3$ | $CH_3$ | $CH_3$ | $CH_3$ | $CH(OCH_3)_2$ | |
| $SO_2CH_3$ | $CH_3$ | $CH_3$ | $OCH_3$ | $CH(OCH_3)_2$ | |
| $SO_2CH_3$ | $CH_3$ | $CH_3$ | $CH_3$ | $CH(OCH_2CH_2O)$ | |
| $SO_2CH_3$ | $CH_3$ | $CH_3$ | $OCH_3$ | $CH(OCH_2CH_2O)$ | |
| $SO_2CH_3$ | $CH_3$ | $CH_3$ | $CF_3$ | $CH_3$ | |
| $SO_2CH_3$ | $CH_3$ | $CH_3$ | $CF_3$ | $OCH_3$ | |
| $SO_2CH_3$ | $CH_3$ | $CH_3$ | $OC_2H_5$ | $CH_3$ | |
| $SO_2CH_3$ | $CH_3$ | $CH_3$ | $OC_2H_5$ | $OCH_3$ | |
| $SO_2CH_3$ | $CH_3$ | $CH_3$ | $CH_3$ | $OCH_2CF_3$ | |
| $SO_2CH_3$ | $CH_3$ | $CH_3$ | $OCH_3$ | $OCH_2CF_3$ | |

Table 11

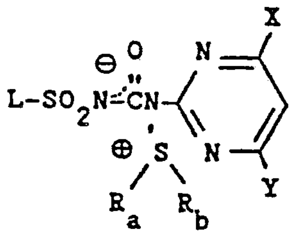

| L | $R_a$ | $R_b$ | X | Y | m.p.(°C) |
|---|---|---|---|---|---|
| L-6: $R_9=CO_2CH_3$ | $CH_3$ | $CH_3$ | $CH_3$ | $CH_3$ | |
| L-6: $R_9=SO_2N(CH_3)_2$ | $CH_3$ | $CH_3$ | $CH_3$ | $CH_3$ | |
| L-6: $R_9=SO_2CH_3$ | $CH_3$ | $CH_3$ | $CH_3$ | $CH_3$ | |
| L-7: $R_{10}=CO_2CH_3$ | $CH_3$ | $CH_3$ | $CH_3$ | $CH_3$ | |
| L-9: $Q_2=O$; $R_{14}=CH_3$; $R_{15}=H$ | $CH_3$ | $CH_3$ | $CH_3$ | $CH_3$ | |
| L-9: $Q_2=S$; $R_{14}=CH_3$; $R_{15}=H$ | $CH_3$ | $CH_3$ | $CH_3$ | $CH_3$ | |
| L-6: $R_9=CO_2CH_3$ | $CH_3$ | $CH_3$ | $CH_3$ | $OCH_3$ | |
| L-6: $R_9=SO_2N(CH_3)_2$ | $CH_3$ | $CH_3$ | $CH_3$ | $OCH_3$ | |
| L-6: $R_9=SO_2CH_3$ | $CH_3$ | $CH_3$ | $CH_3$ | $OCH_3$ | |
| L-7: $R_{10}=CO_2CH_3$ | $CH_3$ | $CH_3$ | $CH_3$ | $OCH_3$ | |
| L-9: $Q_2=O$; $R_{14}=CH_3$; $R_{15}=H$ | $CH_3$ | $CH_3$ | $CH_3$ | $OCH_3$ | |
| L-9: $Q_2=S$; $R_{14}=CH_3$; $R_{15}=H$ | $CH_3$ | $CH_3$ | $CH_3$ | $OCH_3$ | |
| L-6: $R_9=CO_2CH_3$ | $C_2H_5$ | $C_2H_5$ | $CH_3$ | $CH_3$ | |
| L-6: $R_9=SO_2N(CH_3)_2$ | $C_2H_5$ | $CH_3$ | $CH_3$ | $CH_3$ | |
| L-6: $R_9=SO_2CH_3$ | $C_2H_5$ | $CH_3$ | $OCH_3$ | $OCH_3$ | |
| L-6: $R_9=CO_2CH_3$ | $C_2H_5$ | $C_2H_5$ | $OCH_3$ | $OCH_3$ | |
| L-16: $R_{17}=H$ | $C_2H_5$ | $CH_3$ | $CH_3$ | $CH_3$ | |
| L-17: $R_{17}=CH_3$; $R_{18}=H$ | $C_2H_5$ | $C_2H_5$ | $CH_3$ | $CH_3$ | |
| L-6: $R_9=CO_2CH_3$ | $CH_3$ | $CH_3$ | $OCH_3$ | $OCH_3$ | |
| L-6: $R_9=SO_2N(CH_3)_2$ | $CH_3$ | $CH_3$ | $OCH_3$ | $OCH_3$ | |
| L-6: $R_9=SO_2CH_3$ | $CH_3$ | $CH_3$ | $OCH_3$ | $OCH_3$ | |
| L-7: $R_{10}=CO_2CH_3$ | $CH_3$ | $CH_3$ | $OCH_3$ | $OCH_3$ | |
| L-9: $Q_2=O$; $R_{14}=CH_3$; $R_{15}=H$ | $CH_3$ | $CH_3$ | $OCH_3$ | $OCH_3$ | |
| L-9: $Q_2=S$; $R_{14}=CH_3$; $R_{15}=H$ | $CH_3$ | $CH_3$ | $OCH_3$ | $OCH_3$ | |
| L-8: $Q_1=O$; $R_{11}=R_{12}=CH_3$; $R_{13}=H$; m=0 | $CH_3$ | $CH_3$ | $OCH_3$ | $OCH_3$ | |

Table 11 (continued)

| L | $R_a$ | $R_b$ | X | Y | m.p. (°C) |
|---|---|---|---|---|---|
| L-8: $Q_1$=S; $R_{11}$=H; $R_{12}$=$CH_3$; $R_{13}$=H, m=0 | $CH_3$ | $C_2H_5$ | $CH_3$ | $OCH_3$ | |
| L-8: $Q_1$=$SO_2$; $R_{11}$=$CH_3$; $R_{12}$=H; $R_{13}$=H; m=0 | $CH_3$ | $CH_3$ | $OCH_3$ | $OCH_3$ | |
| L-8: $Q_1$=$SO_2$; $R_{11}$=$R_{12}$=$CH_3$; $R_{13}$=H; m=1 | $CH_3$ | $CH_3$ | $CH_3$ | $CH_3$ | |
| L-8: $Q_1$=$SO_2$; $R_{11}$=H; $R_{12}$=$CH_3$, $R_{13}$=H; m=0 | $CH_3$ | $CH_3$ | $OCHF_2$ | $OCH_3$ | |
| L-12: m=0; $R_{13}$=H | $CH_3$ | $CH_3$ | $CH_3$ | $CH_3$ | |
| L-13: m=1; $R_{13}$=H | $CH_3$ | $CH_3$ | $CH_3$ | $CH_3$ | |
| L-14: $R_{16}$=$CH_3$ | $CH_3$ | $CH_3$ | $CH_3$ | $CH_3$ | |
| L-15: $R_{17}$=H | $CH_3$ | $CH_3$ | $CH_3$ | $CH_3$ | |
| L-16: $R_{17}$=H | $CH_3$ | $CH_3$ | $CH_3$ | $CH_3$ | |
| L-17: $R_{17}$=$CH_3$; $R_{18}$=H | $CH_3$ | $CH_3$ | $CH_3$ | $CH_3$ | |
| L-12: m=0; $R_{13}$=H | $CH_3$ | $CH_3$ | $CH_3$ | $OCH_3$ | |
| L-13: m=1; $R_{13}$=H | $CH_3$ | $CH_3$ | $CH_3$ | $OCH_3$ | |
| L-14: $R_{16}$=$CH_3$ | $CH_3$ | $CH_3$ | $CH_3$ | $OCH_3$ | |
| L-15: $R_{17}$=H | $CH_3$ | $CH_3$ | $CH_3$ | $OCH_3$ | |
| L-16: $R_{17}$=H | $CH_3$ | $CH_3$ | $CH_3$ | $OCH_3$ | |
| L-17: $R_{17}$=$CH_3$; $R_{18}$=H | $CH_3$ | $CH_3$ | $CH_3$ | $OCH_3$ | |
| L-7: $R_{10}$=$CO_2CH_3$ | $C_2H_5$ | $C_2H_5$ | $CH_3$ | $CH_3$ | |
| L-9: $Q_2$=O; $R_{14}$=$CH_3$; $R_{15}$=H | $C_2H_5$ | $CH_3$ | $CH_3$ | $CH_3$ | |
| L-12: m=0; $R_{13}$=H | $C_2H_5$ | $CH_3$ | $OCH_3$ | $OCH_3$ | |
| L-13: m=1; $R_{13}$=H | $C_2H_5$ | $C_2H_5$ | $OCH_3$ | $OCH_3$ | |
| L-14: $R_{16}$=$CH_3$ | $C_2H_5$ | $CH_3$ | $CH_3$ | $CH_3$ | |
| L-15: $R_{17}$=H | $C_2H_5$ | $C_2H_5$ | $CH_3$ | $CH_3$ | |
| L-12: m=0; $R_{13}$=H | $CH_3$ | $CH_3$ | $OCH_3$ | $OCH_3$ | |
| L-13: m=1; $R_{13}$=H | $CH_3$ | $CH_3$ | $OCH_3$ | $OCH_3$ | |
| L-14: $R_{16}$=$CH_3$ | $CH_3$ | $CH_3$ | $OCH_3$ | $OCH_3$ | |
| L-15: $R_{17}$=H | $CH_3$ | $CH_3$ | $OCH_3$ | $OCH_3$ | |
| L-16: $R_{17}$=H | $CH_3$ | $CH_3$ | $OCH_3$ | $OCH_3$ | |
| L-17: $R_{17}$=$CH_3$; $R_{18}$=$CH_3$ | $CH_3$ | $CH_3$ | $OCH_3$ | $OCH_3$ | |
| L-10: $R_{13}$=H; $R_{16}$=$CH_3$; m=0 | $CH_3$ | $CH_3$ | $OCH_3$ | $OCH_3$ | |
| L-10: $R_{13}$=H; $R_{16}$=$C_2H_5$; m=0 | $CH_3$ | $C_2H_5$ | $CH_3$ | $OCH_3$ | |
| L-10: $R_{13}$=$CH_3$; $R_{16}$=$CH_3$; m=0 | $C_2H_5$ | $C_2H_5$ | $CH_3$ | $CH_3$ | |

Table 11 (continued)

| L | $R_a$ | $R_b$ | X | Y | m.p. (°C) |
|---|---|---|---|---|---|
| L-11: $R_{13}$=H; m=0 | $CH_3$ | $CH_3$ | $OCH_3$ | $OCH_3$ | |
| L-11: $R_{13}$=H; m=1 | $CH_3$ | $CH_3$ | Cl | $OCH_3$ | |
| L-11: $R_{13}$=$CH_3$; m=0 | $C_2H_5$ | $CH_3$ | $CH_3$ | $CH(OCH_3)_2$ | |

Table 12

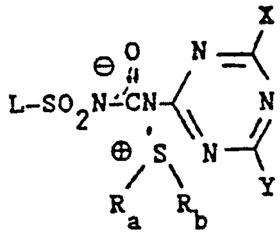

| L | $R_a$ | $R_b$ | X | Y | m.p.(°C) |
|---|---|---|---|---|---|
| L-6: $R_9=CO_2CH_3$ | $CH_3$ | $CH_3$ | $CH_3$ | $CH_3$ | |
| L-6: $R_9=SO_2N(CH_3)_2$ | $CH_3$ | $CH_3$ | $CH_3$ | $CH_3$ | |
| L-6: $R_9=SO_2CH_3$ | $CH_3$ | $CH_3$ | $CH_3$ | $CH_3$ | |
| L-7: $R_{10}=CO_2CH_3$ | $CH_3$ | $CH_3$ | $CH_3$ | $CH_3$ | |
| L-9: $Q_2=O$; $R_{14}=CH_3$; $R_{15}=H$ | $CH_3$ | $CH_3$ | $CH_3$ | $CH_3$ | |
| L-9: $Q_2=S$; $R_{14}=CH_3$; $R_{15}=H$ | $CH_3$ | $CH_3$ | $CH_3$ | $CH_3$ | |
| L-6: $R_9=CO_2CH_3$ | $CH_3$ | $CH_3$ | $CH_3$ | $OCH_3$ | |
| L-6: $R_9=SO_2N(CH_3)_2$ | $CH_3$ | $CH_3$ | $CH_3$ | $OCH_3$ | |
| L-6: $R_9=SO_2CH_3$ | $CH_3$ | $CH_3$ | $CH_3$ | $OCH_3$ | |
| L-7: $R_{10}=CO_2CH_3$ | $CH_3$ | $CH_3$ | $CH_3$ | $OCH_3$ | |
| L-9: $Q_2=O$; $R_{14}=CH_3$; $R_{15}=H$ | $CH_3$ | $CH_3$ | $CH_3$ | $OCH_3$ | |
| L-9: $Q_2=S$; $R_{14}=CH_3$; $R_{15}=H$ | $CH_3$ | $CH_3$ | $CH_3$ | $OCH_3$ | |
| L-6: $R_9=CO_2CH_3$ | $C_2H_5$ | $C_2H_5$ | $CH_3$ | $CH_3$ | |
| L-6: $R_9=SO_2N(CH_3)_2$ | $C_2H_5$ | $CH_3$ | $CH_3$ | $CH_3$ | |
| L-6: $R_9=SO_2CH_3$ | $C_2H_5$ | $CH_3$ | $OCH_3$ | $OCH_3$ | |
| L-6: $R_9=CO_2CH_3$ | $C_2H_5$ | $C_2H_5$ | $OCH_3$ | $OCH_3$ | |
| L-16: $R_{17}=H$ | $C_2H_5$ | $CH_3$ | $CH_3$ | $CH_3$ | |
| L-17: $R_{17}=CH_3$; $R_{18}=H$ | $C_2H_5$ | $C_2H_5$ | $CH_3$ | $CH_3$ | |
| L-6: $R_9=CO_2CH_3$ | $CH_3$ | $CH_3$ | $OCH_3$ | $OCH_3$ | |
| L-6: $R_9=SO_2N(CH_3)_2$ | $CH_3$ | $CH_3$ | $OCH_3$ | $OCH_3$ | |
| L-6: $R_9=SO_2CH_3$ | $CH_3$ | $CH_3$ | $OCH_3$ | $OCH_3$ | |
| L-7: $R_{10}=CO_2CH_3$ | $CH_3$ | $CH_3$ | $OCH_3$ | $OCH_3$ | |
| L-9: $Q_2=O$; $R_{14}=CH_3$; $R_{15}=H$ | $CH_3$ | $CH_3$ | $OCH_3$ | $OCH_3$ | |
| L-9: $Q_2=S$; $R_{14}=CH_3$; $R_{15}=H$ | $CH_3$ | $CH_3$ | $OCH_3$ | $OCH_3$ | |
| L-8: $Q_1=O$; $R_{11}=R_{12}=CH_3$; $R_{13}=H$; m=0 | $CH_3$ | $CH_3$ | $OCH_3$ | $OCH_3$ | |

Table 12 (continued)

| L | $R_a$ | $R_b$ | X | Y | m.p. (°C) |
|---|---|---|---|---|---|
| L-8: $Q_1$=S; $R_{11}$=H; $R_{12}$=$CH_3$; $R_{13}$=H, m=0 | $CH_3$ | $C_2H_5$ | $CH_3$ | $OCH_3$ | |
| L-8: $Q_1$=$SO_2$; $R_{11}$=$CH_3$; $R_{12}$=H; $R_{13}$=H; m=0 | $CH_3$ | $CH_3$ | $OCH_3$ | $OCH_3$ | |
| L-8: $Q_1$=$SO_2$; $R_{11}$=$R_{12}$=$CH_3$; $R_{13}$=H; m=1 | $CH_3$ | $CH_3$ | $CH_3$ | $CH_3$ | |
| L-8: $Q_1$=$SO_2$; $R_{11}$=H; $R_{12}$=$CH_3$, $R_{13}$=H; m=0 | $CH_3$ | $CH_3$ | $OCHF_2$ | $OCH_3$ | |
| L-12: m=0; $R_{13}$=H | $CH_3$ | $CH_3$ | $CH_3$ | $CH_3$ | |
| L-13: m=1; $R_{13}$=H | $CH_3$ | $CH_3$ | $CH_3$ | $CH_3$ | |
| L-14: $R_{16}$=$CH_3$ | $CH_3$ | $CH_3$ | $CH_3$ | $CH_3$ | |
| L-15: $R_{17}$=H | $CH_3$ | $CH_3$ | $CH_3$ | $CH_3$ | |
| L-16: $R_{17}$=H | $CH_3$ | $CH_3$ | $CH_3$ | $CH_3$ | |
| L-17: $R_{17}$=$CH_3$; $R_{18}$=H | $CH_3$ | $CH_3$ | $CH_3$ | $CH_3$ | |
| L-12: m=0; $R_{13}$=H | $CH_3$ | $CH_3$ | $CH_3$ | $OCH_3$ | |
| L-13: m=1; $R_{13}$=H | $CH_3$ | $CH_3$ | $CH_3$ | $OCH_3$ | |
| L-14: $R_{16}$=$CH_3$ | $CH_3$ | $CH_3$ | $CH_3$ | $OCH_3$ | |
| L-15: $R_{17}$=H | $CH_3$ | $CH_3$ | $CH_3$ | $OCH_3$ | |
| L-16: $R_{17}$=H | $CH_3$ | $CH_3$ | $CH_3$ | $OCH_3$ | |
| L-17: $R_{17}$=$CH_3$; $R_{18}$=H | $CH_3$ | $CH_3$ | $CH_3$ | $OCH_3$ | |
| L-7: $R_{10}$=$CO_2CH_3$ | $C_2H_5$ | $C_2H_5$ | $CH_3$ | $CH_3$ | |
| L-9: $Q_2$=O; $R_{14}$=$CH_3$; $R_{15}$=H | $C_2H_5$ | $CH_3$ | $CH_3$ | $CH_3$ | |
| L-12: m=0; $R_{13}$=H | $C_2H_5$ | $CH_3$ | $OCH_3$ | $OCH_3$ | |
| L-13: m=1; $R_{13}$=H | $C_2H_5$ | $C_2H_5$ | $OCH_3$ | $OCH_3$ | |
| L-14: $R_{16}$=$CH_3$ | $C_2H_5$ | $CH_3$ | $CH_3$ | $CH_3$ | |
| L-15: $R_{17}$=H | $C_2H_5$ | $C_2H_5$ | $CH_3$ | $CH_3$ | |
| L-12: m=0; $R_{13}$=H | $CH_3$ | $CH_3$ | $OCH_3$ | $OCH_3$ | |
| L-13: m=1; $R_{13}$=H | $CH_3$ | $CH_3$ | $OCH_3$ | $OCH_3$ | |
| L-14: $R_{16}$=$CH_3$ | $CH_3$ | $CH_3$ | $OCH_3$ | $OCH_3$ | |
| L-15: $R_{17}$=H | $CH_3$ | $CH_3$ | $OCH_3$ | $OCH_3$ | |
| L-16: $R_{17}$=H | $CH_3$ | $CH_3$ | $OCH_3$ | $OCH_3$ | |
| L-17: $R_{17}$=$CH_3$; $R_{18}$=$CH_3$ | $CH_3$ | $CH_3$ | $OCH_3$ | $OCH_3$ | |
| L-10: $R_{13}$=H; $R_{16}$=$CH_3$; m=0 | $CH_3$ | $CH_3$ | $OCH_3$ | $OCH_3$ | |
| L-10: $R_{13}$=H; $R_{16}$=$C_2H_5$; m=0 | $CH_3$ | $C_2H_5$ | $CH_3$ | $OCH_3$ | |
| L-10: $R_{13}$=$CH_3$; $R_{16}$=$CH_3$; m=0 | $C_2H_5$ | $C_2H_5$ | $CH_3$ | $CH_3$ | |

Table 12 (continued)

| L | $R_a$ | $R_b$ | X | Y | m.p. (°C) |
|---|---|---|---|---|---|
| L-11: $R_{13}$=H; m=0 | $CH_3$ | $CH_3$ | $OCH_3$ | $OCHF_2$ | |
| L-11: $R_{13}$=H; m=1 | $CH_3$ | $CH_3$ | $CF_3$ | $OCH_3$ | |
| L-11: $R_{13}$=$CH_3$; m=0 | $C_2H_5$ | $CH_3$ | $CH_3$ | $CH(OCH_3)_2$ | |

Table 13

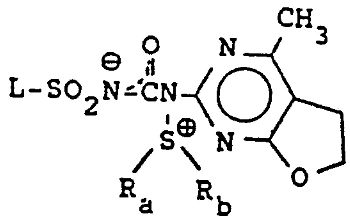

| L | $R_a$ | $R_b$ |
|---|---|---|
| L-1: $R_5=CO_2CH_3$; $R_6=H$ | $C_2H_5$ | $C_2H_5$ |
| L-1: $R_5=CO_2CH_3$; $R_6=H$ | $CH_3$ | $C_2H_5$ |
| L-1: $R_5=CO_2CH_3$; $R_6=H$ | $CH_3$ | $CH_3$ |
| L-1: $R_5=CO_2CH_3$; $R_6=5\text{-}OCH_3$ | $CH_3$ | $CH_3$ |
| L-1: $R_5=SO_2N(CH_3)_2$; $R_6=H$ | $CH_3$ | $CH_3$ |
| L-1: $R_5=SO_2CH_3$; $R_6=H$ | $CH_3$ | $CH_3$ |
| L-1: $R_5=Cl$; $R_6=H$ | $CH_3$ | $CH_3$ |
| L-1: $R_5=SO_2CH_2CH_2CH_3$; $R_6=H$ | $CH_3$ | $CH_3$ |
| L-2: $R_7=SO_2CH_3$ | $CH_3$ | $CH_3$ |
| L-3: $R_8=SO_2N(CH_3)_2$ | $CH_3$ | $CH_3$ |
| L-4: $R_9=CO_2CH_3$ | $CH_3$ | $CH_3$ |
| L-5: $R_9=CO_2CH_3$ | $CH_3$ | $CH_3$ |
| L-6: $R_9=CO_2CH_3$ | $CH_3$ | $CH_3$ |
| L-7: $R_{10}=CO_2CH_3$ | $CH_3$ | $CH_3$ |
| L-8: $Q_1=O$; $m=0$; $R_{11}=CH_3$; $R_{12}=CH_3$; $R_{13}=H$ | $CH_3$ | $CH_3$ |
| L-9: $Q_2=O$; $R_{14}=CH_3$; $R_{15}=H$ | $CH_3$ | $CH_3$ |
| L-10: $m=0$; $R_{16}=CH_3$; $R_{13}=H$ | $CH_3$ | $CH_3$ |
| L-11: $m=1$; $R_{13}=H$ | $CH_3$ | $CH_3$ |
| L-11: $m=0$; $R_{13}=H$ | $CH_3$ | $CH_3$ |
| L-12: $m=0$; $R_{13}=H$ | $CH_3$ | $CH_3$ |
| L-13: $m=1$; $R_{13}=H$ | $CH_3$ | $CH_3$ |
| L-14: $R_{16}=CH_3$ | $CH_3$ | $CH_3$ |
| L-15: $R_{17}=H$ | $CH_3$ | $CH_3$ |
| L-16: $R_{17}=H$ | $CH_3$ | $CH_3$ |
| L-17: $R_{17}=CH_3$; $R_{18}=H$ | $CH_3$ | $CH_3$ |

Table 14

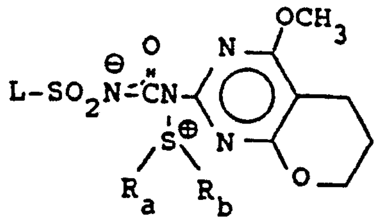

| L | $R_a$ | $R_b$ |
|---|---|---|
| L-1: $R_5=CO_2CH_3$; $R_6=H$ | $C_2H_5$ | $C_2H_5$ |
| L-1: $R_5=CO_2CH_3$; $R_6=H$ | $CH_3$ | $C_2H_5$ |
| L-1: $R_5=CO_2CH_3$; $R_6=H$ | $CH_3$ | $CH_3$ |
| L-1: $R_5=CO_2CH_3$; $R_6=5\text{-}OCH_3$ | $CH_3$ | $CH_3$ |
| L-1: $R_5=SO_2N(CH_3)_2$; $R_6=H$ | $CH_3$ | $CH_3$ |
| L-1: $R_5=SO_2CH_3$; $R_6=H$ | $CH_3$ | $CH_3$ |
| L-1: $R_5=Cl$; $R_6=H$ | $CH_3$ | $CH_3$ |
| L-1: $R_5=SO_2CH_2CH_2CH_3$; $R_6=H$ | $CH_3$ | $CH_3$ |
| L-2: $R_7=SO_2CH_3$ | $CH_3$ | $CH_3$ |
| L-3: $R_8=SO_2N(CH_3)_2$ | $CH_3$ | $CH_3$ |
| L-4: $R_9=CO_2CH_3$ | $CH_3$ | $CH_3$ |
| L-5: $R_9=CO_2CH_3$ | $CH_3$ | $CH_3$ |
| L-6: $R_9=CO_2CH_3$ | $CH_3$ | $CH_3$ |
| L-7: $R_{10}=CO_2CH_3$ | $CH_3$ | $CH_3$ |
| L-8: $Q_1=O$; m=0; $R_{11}=CH_3$; $R_{12}=CH_3$; $R_{13}=H$ | $CH_3$ | $CH_3$ |
| L-9: $Q_2=O$; $R_{14}=CH_3$; $R_{15}=H$ | $CH_3$ | $CH_3$ |
| L-10: m=0; $R_{16}=CH_3$; $R_{13}=H$ | $CH_3$ | $CH_3$ |
| L-11: m=1; $R_{13}=H$ | $CH_3$ | $CH_3$ |
| L-11: m=0; $R_{13}=H$ | $CH_3$ | $CH_3$ |
| L-12: m=0; $R_{13}=H$ | $CH_3$ | $CH_3$ |
| L-13: m=1; $R_{13}=H$ | $CH_3$ | $CH_3$ |
| L-14: $R_{16}=CH_3$ | $CH_3$ | $CH_3$ |
| L-15: $R_{17}=H$ | $CH_3$ | $CH_3$ |
| L-16: $R_{17}=H$ | $CH_3$ | $CH_3$ |
| L-17: $R_{17}=CH_3$; $R_{18}=H$ | $CH_3$ | $CH_3$ |

Table 15

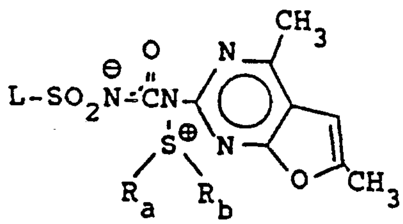

| $\underline{L}$ | $\underline{R_a}$ | $\underline{R_b}$ |
|---|---|---|
| L-1: $R_5=CO_2CH_3$; $R_6=H$ | $C_2H_5$ | $C_2H_5$ |
| L-1: $R_5=CO_2CH_3$; $R_6=H$ | $CH_3$ | $C_2H_5$ |
| L-1: $R_5=CO_2CH_3$; $R_6=H$ | $CH_3$ | $CH_3$ |
| L-1: $R_5=CO_2CH_3$; $R_6=5\text{-}OCH_3$ | $CH_3$ | $CH_3$ |
| L-1: $R_5=SO_2N(CH_3)_2$; $R_6=H$ | $CH_3$ | $CH_3$ |
| L-1: $R_5=SO_2CH_3$; $R_6=H$ | $CH_3$ | $CH_3$ |
| L-1: $R_5=Cl$; $R_6=H$ | $CH_3$ | $CH_3$ |
| L-1: $R_5=SO_2CH_2CH_2CH_3$; $R_6=H$ | $CH_3$ | $CH_3$ |
| L-2: $R_7=SO_2CH_3$ | $CH_3$ | $CH_3$ |
| L-3: $R_8=SO_2N(CH_3)_2$ | $CH_3$ | $CH_3$ |
| L-4: $R_9=CO_2CH_3$ | $CH_3$ | $CH_3$ |
| L-5: $R_9=CO_2CH_3$ | $CH_3$ | $CH_3$ |
| L-6: $R_9=CO_2CH_3$ | $CH_3$ | $CH_3$ |
| L-7: $R_{10}=CO_2CH_3$ | $CH_3$ | $CH_3$ |
| L-8: $Q_1=O$; $m=0$; $R_{11}=CH_3$; $R_{12}=CH_3$; $R_{13}=H$ | $CH_3$ | $CH_3$ |
| L-9: $Q_2=O$; $R_{14}=CH_3$; $R_{15}=H$ | $CH_3$ | $CH_3$ |
| L-10: $m=0$; $R_{16}=CH_3$; $R_{13}=H$ | $CH_3$ | $CH_3$ |
| L-11: $m=1$; $R_{13}=H$ | $CH_3$ | $CH_3$ |
| L-11: $m=0$; $R_{13}=H$ | $CH_3$ | $CH_3$ |
| L-12: $m=0$; $R_{13}=H$ | $CH_3$ | $CH_3$ |
| L-13: $m=1$; $R_{13}=H$ | $CH_3$ | $CH_3$ |
| L-14: $R_{16}=CH_3$ | $CH_3$ | $CH_3$ |
| L-15: $R_{17}=H$ | $CH_3$ | $CH_3$ |
| L-16: $R_{17}=H$ | $CH_3$ | $CH_3$ |
| L-17: $R_{17}=CH_3$; $R_{18}=H$ | $CH_3$ | $CH_3$ |

Table 16

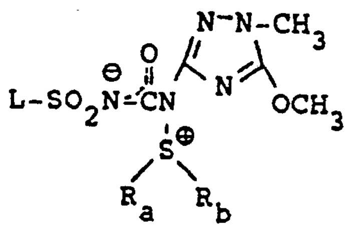

| L | $R_a$ | $R_b$ |
|---|---|---|
| L-1: $R_5=CO_2CH_3$; $R_6=H$ | $C_2H_5$ | $C_2H_5$ |
| L-1: $R_5=CO_2CH_3$; $R_6=H$ | $CH_3$ | $C_2H_5$ |
| L-1: $R_5=CO_2CH_3$; $R_6=H$ | $CH_3$ | $CH_3$ |
| L-1: $R_5=CO_2CH_3$; $R_6=5\text{-}OCH_3$ | $CH_3$ | $CH_3$ |
| L-1: $R_5=SO_2N(CH_3)_2$; $R_6=H$ | $CH_3$ | $CH_3$ |
| L-1: $R_5=SO_2CH_3$; $R_6=H$ | $CH_3$ | $CH_3$ |
| L-1: $R_5=Cl$; $R_6=H$ | $CH_3$ | $CH_3$ |
| L-1: $R_5=SO_2CH_2CH_2CH_3$; $R_6=H$ | $CH_3$ | $CH_3$ |
| L-2: $R_7=SO_2CH_3$ | $CH_3$ | $CH_3$ |
| L-3: $R_8=SO_2N(CH_3)_2$ | $CH_3$ | $CH_3$ |
| L-4: $R_9=CO_2CH_3$ | $CH_3$ | $CH_3$ |
| L-5: $R_9=CO_2CH_3$ | $CH_3$ | $CH_3$ |
| L-6: $R_9=CO_2CH_3$ | $CH_3$ | $CH_3$ |
| L-7: $R_{10}=CO_2CH_3$ | $CH_3$ | $CH_3$ |
| L-8: $Q_1=O$; $m=0$; $R_{11}=CH_3$; $R_{12}=CH_3$; $R_{13}=H$ | $CH_3$ | $CH_3$ |
| L-9: $Q_2=O$; $R_{14}=CH_3$; $R_{15}=H$ | $CH_3$ | $CH_3$ |
| L-10: $m=0$; $R_{16}=CH_3$; $R_{13}=H$ | $CH_3$ | $CH_3$ |
| L-11: $m=1$; $R_{13}=H$ | $CH_3$ | $CH_3$ |
| L-11: $m=0$; $R_{13}=H$ | $CH_3$ | $CH_3$ |
| L-12: $m=0$; $R_{13}=H$ | $CH_3$ | $CH_3$ |
| L-13: $m=1$; $R_{13}=H$ | $CH_3$ | $CH_3$ |
| L-14: $R_{16}=CH_3$ | $CH_3$ | $CH_3$ |
| L-15: $R_{17}=H$ | $CH_3$ | $CH_3$ |
| L-16: $R_{17}=H$ | $CH_3$ | $CH_3$ |
| L-17: $R_{17}=CH_3$; $R_{18}=H$ | $CH_3$ | $CH_3$ |

Table 17

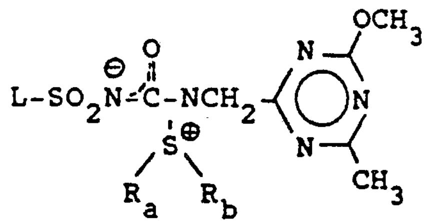

| L | $R_a$ | $R_b$ |
|---|---|---|
| L-1: $R_5=CO_2CH_3$; $R_6=H$ | $C_2H_5$ | $C_2H_5$ |
| L-1: $R_5=CO_2CH_3$; $R_6=H$ | $CH_3$ | $C_2H_5$ |
| L-1: $R_5=CO_2CH_3$; $R_6=H$ | $CH_3$ | $CH_3$ |
| L-1: $R_5=CO_2CH_3$; $R_6=5\text{-}OCH_3$ | $CH_3$ | $CH_3$ |
| L-1: $R_5=SO_2N(CH_3)_2$; $R_6=H$ | $CH_3$ | $CH_3$ |
| L-1: $R_5=SO_2CH_3$; $R_6=H$ | $CH_3$ | $CH_3$ |
| L-1: $R_5=Cl$; $R_6=H$ | $CH_3$ | $CH_3$ |
| L-1: $R_5=SO_2CH_2CH_2CH_3$; $R_6=H$ | $CH_3$ | $CH_3$ |
| L-2: $R_7=SO_2CH_3$ | $CH_3$ | $CH_3$ |
| L-3: $R_8=SO_2N(CH_3)_2$ | $CH_3$ | $CH_3$ |
| L-4: $R_9=CO_2CH_3$ | $CH_3$ | $CH_3$ |
| L-5: $R_9=CO_2CH_3$ | $CH_3$ | $CH_3$ |
| L-6: $R_9=CO_2CH_3$ | $CH_3$ | $CH_3$ |
| L-7: $R_{10}=CO_2CH_3$ | $CH_3$ | $CH_3$ |
| L-8: $Q_1=O$; m=0; $R_{11}=CH_3$; $R_{12}=CH_3$; $R_{13}=H$ | $CH_3$ | $CH_3$ |
| L-9: $Q_2=O$; $R_{14}=CH_3$; $R_{15}=H$ | $CH_3$ | $CH_3$ |
| L-10: m=0; $R_{16}=CH_3$; $R_{13}=H$ | $CH_3$ | $CH_3$ |
| L-11: m=1; $R_{13}=H$ | $CH_3$ | $CH_3$ |
| L-11: m=0; $R_{13}=H$ | $CH_3$ | $CH_3$ |
| L-12: m=0; $R_{13}=H$ | $CH_3$ | $CH_3$ |
| L-13: m=1; $R_{13}=H$ | $CH_3$ | $CH_3$ |
| L-14: $R_{16}=CH_3$ | $CH_3$ | $CH_3$ |
| L-15: $R_{17}=H$ | $CH_3$ | $CH_3$ |
| L-16: $R_{17}=H$ | $CH_3$ | $CH_3$ |
| L-17: $R_{17}=CH_3$; $R_{18}=H$ | $CH_3$ | $CH_3$ |

Table 18

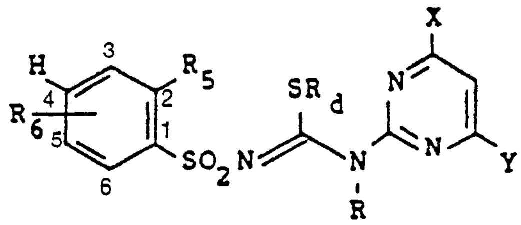

| $R_5$ | $R_6$ | $R_d$ | R | X | Y | m.p.(°C) |
|---|---|---|---|---|---|---|
| $SCH_2CH{=}CH_2$ | 3-Cl | $CH_3$ | H | $OCH_3$ | $OCH_3$ | |
| $SCH_2CH{=}CH_2$ | H | $C_2H_5$ | H | $OCH_3$ | $OC_2H_5$ | |
| $OSO_2$-*n*-$C_3H_7$ | H | $CH_3$ | $CH_3$ | $CH_3$ | $CH_2OCH_3$ | |
| $OSO_2$-*n*-$C_3H_7$ | H | $CH_3$ | H | $CH_3$ | $SCH_3$ | |
| $OSO_2CH_3$ | H | $CH_3$ | H | $OCH_3$ | $CH_3$ | |
| $OSO_2CH_3$ | H | $CH_3$ | H | $OCH_3$ | $OCH_3$ | |
| $CH_2CH_3$ | 5-Br | $CH_3$ | H | $CH_3$ | $OCH_3$ | |
| $CH_2CH_3$ | H | $CH_3$ | H | $CH_3$ | $CH_3$ | |
| *n*-$C_4H_9$ | H | n-$C_3H_7$ | H | $OCH_3$ | $OCH_3$ | |
| *i*-$C_3H_7$ | H | $CH_3$ | $CH_3$ | $OCH_3$ | $CH_3$ | |
| $OSO_2N(CH_3)_2$ | H | $CH_3$ | H | $CH_3$ | $CH(OCH_2CH_2O)$ | |
| $OCH_2CH_3$ | H | $CH_3$ | H | $CH_3$ | $CH_3$ | |
| $OCH_2CH_3$ | H | $CH_3$ | H | $CH_3$ | $OCH_3$ | |
| O-*i*-$C_3H_7$ | H | $CH_3$ | H | $OCH_3$ | $OCH_3$ | |
| O-*i*-$C_3H_7$ | H | $CH_3$ | H | $OCH_3$ | $C_2H_5$ | |
| O-*n*-$C_4H_9$ | H | $CH_3$ | H | $CH_3$ | $CH(OCH_3)_2$ | |
| O-*n*-$C_4H_9$ | H | $CH_3$ | H | $CH_3$ | $OC_2H_5$ | |
| $SO_2CF_2H$ | 5-$CH_3$ | $CH_3$ | H | $OCH_3$ | $OCH_3$ | |
| $SO_2CF_2H$ | H | $C_2H_5$ | H | $OCH_3$ | $OC_2H_5$ | |
| $SCF_2H$ | 6-$SCH_3$ | $CH_3$ | $CH_3$ | $CH_3$ | $CH_2OCH_3$ | |
| $SCF_2H$ | H | $CH_3$ | H | $CH_3$ | $SCH_3$ | |
| $OCF_3$ | H | $CH_3$ | H | $OCH_3$ | $CH_3$ | |
| $OCF_3$ | H | $CH_3$ | H | $OCH_3$ | $OCH_3$ | |
| $CH_2OCH_3$ | H | n-$C_3H_7$ | H | $OCH_3$ | $OCH_3$ | |

Table 18 (continued)

| $R_5$ | $R_6$ | $R_d$ | R | X | Y | m.p.(°C) |
|---|---|---|---|---|---|---|
| $CH_2OCH_3$ | H | $CH_3$ | $CH_3$ | $OCH_3$ | $CH_3$ | |
| $CH_2OCH_2CH_3$ | H | $CH_3$ | H | Cl | $OCH_3$ | |
| $OCF_2H$ | H | $CH_3$ | H | $OCH_3$ | $CH_3$ | |
| $OCF_2H$ | H | $CH_3$ | H | $OCH_3$ | $OCH_3$ | |
| $OCF_2H$ | H | $CH_3$ | H | $CH_3$ | $CH(OCH_2CH_2O)$ | |

Table 19

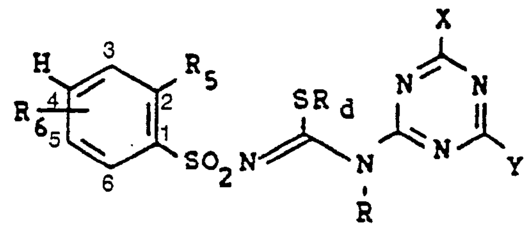

| $R_5$ | $R_6$ | $R_d$ | R | X | Y | m.p.(°C) |
|---|---|---|---|---|---|---|
| $SO_2CH_2CH=CH_2$ | H | $CH_3$ | H | $OCH_3$ | $OCH_3$ | |
| $SO_2CH_2CH=CH_2$ | H | $CH_3$ | H | $OCH_3$ | $C_2H_5$ | |
| $OSO_2$-$n$-$C_3H_7$ | H | $CH_3$ | $CH_3$ | $CH_3$ | $CH_2OCH_3$ | |
| $OSO_2$-$n$-$C_3H_7$ | H | $CH_3$ | H | $CH_3$ | $SCH_3$ | |
| $OSO_2CH_3$ | H | $CH_3$ | H | $OCH_3$ | $CH_3$ | |
| $OSO_2CH_3$ | H | $CH_3$ | H | $OCH_3$ | $OCH_3$ | |
| $CH_2CH_3$ | 3-Br | $CH_3$ | H | $CH_3$ | $OCH_3$ | |
| $CH_2CH_3$ | H | $CH_3$ | H | $CH_3$ | $CH_3$ | |
| $n$-$C_4H_9$ | H | $n$-$C_3H_7$ | H | $OCH_3$ | $OCH_3$ | |
| $i$-$C_3H_7$ | H | $CH_3$ | $CH_3$ | $OCH_3$ | $CH_3$ | |
| $OSO_2N(CH_3)_2$ | H | $CH_3$ | H | $CH_3$ | $CH(OCH_2CH_2O)$ | |
| $OCH_2CH_3$ | H | $CH_3$ | H | $CH_3$ | $CH_3$ | |
| $OCH_2CH_3$ | H | $CH_3$ | H | $CH_3$ | $OCH_3$ | |
| O-$i$-$C_3H_7$ | H | $CH_3$ | H | $OCH_3$ | $OCH_3$ | |
| O-$i$-$C_3H_7$ | H | $CH_3$ | H | $OCH_3$ | $C_2H_5$ | |
| O-$n$-$C_4H_9$ | H | $CH_3$ | H | $CH_3$ | $CH(OCH_3)_2$ | |
| O-$n$-$C_4H_9$ | H | $CH_3$ | H | $CH_3$ | $OC_2H_5$ | |
| $SO_2CF_2H$ | 6-$SCH_3$ | $CH_3$ | H | $OCH_3$ | $OCH_3$ | |
| $SO_2CF_2H$ | H | $C_2H_5$ | H | $OCH_3$ | $OC_2H_5$ | |
| $SCF_2H$ | H | $CH_3$ | $CH_3$ | $CH_3$ | $CH_2OCH_3$ | |
| $SCF_2H$ | H | $CH_3$ | H | $CH_3$ | $SCH_3$ | |
| $OCF_3$ | H | $CH_3$ | H | $OCH_3$ | $CH_3$ | |
| $OCF_3$ | H | $CH_3$ | H | $OCH_3$ | $OCH_3$ | |
| $CH_2OCH_3$ | H | $n$-$C_3H_7$ | H | $OCH_3$ | $OCH_3$ | |

Table 19 (continued)

| $R_5$ | $R_6$ | $R_d$ | R | X | Y | m.p.(°C) |
|---|---|---|---|---|---|---|
| $CH_2OCH_3$ | H | $CH_3$ | $CH_3$ | $OCH_3$ | $CH_3$ | |
| $OCF_2H$ | H | $CH_3$ | H | $OCH_3$ | $CH_3$ | |
| $OCF_2H$ | H | $CH_3$ | H | $OCH_3$ | $OCH_3$ | |
| $OCF_2H$ | H | $CH_3$ | H | $CH_3$ | $CH_3$ | |
| $CH_2CH_2OC_2H_5$ | H | $CH_3$ | H | $OCH_3$ | $CH_3$ | |

Table 20

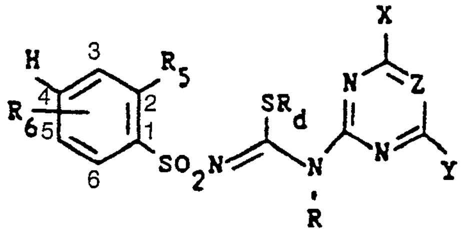

| $R_d$ | R | $R_5$ | $R_6$ | X | Y | Z | m.p. (°C) |
|---|---|---|---|---|---|---|---|
| $CH_3$ | H | $OCH_2CH_2OCH_3$ | H | $CH_3$ | H | CH | |
| $CH_3$ | H | $OCH_2CH{=}CH_2$ | H | $CH_3$ | $CH_3$ | N | |
| $CH_3$ | H | $OCH_2C{\equiv}CH$ | 6-$OCH_3$ | $CH_3$ | $CH_3$ | CH | |
| $CH_3$ | H | $C_6H_5$ | H | $CH_3$ | $CH_3$ | N | |
| $CH_3$ | H | 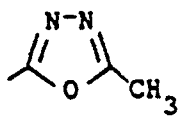 | H | $CH_3$ | $OCH_3$ | CH | |
| $CH_3$ | H | 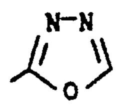 | H | $OCH_3$ | $OCH_3$ | N | |
| $CH_3$ | H | 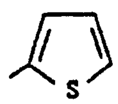 | H | $OCH_3$ | $OCH_2C{\equiv}CH$ | N | |
| $CH_3$ | H | 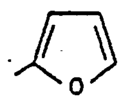 | H | $OCH_3$ | $OCH_2CH_2OCH_3$ | N | |
| $CH_3$ | H | $C_2H_5$ | H | $OCH_3$ | $OCF_2H$ | CH | |
| $CH_3$ | H | $OCF_3$ | H | $OCH_3$ | $SCF_2H$ | CH | |
| $CH_3$ | H | $SO_2CH_3$ | 3-$OCF_2H$ | $OCH_3$ | $C(CH_3)(OCH_3)_2$ | CH | |
| $CH_3$ | H | $OCH_2CH_3$ | H | $OCH_3$ | $CF_3$ | N | |
| $CH_3$ | H | $SO_2CH_2CH_3$ | H | $OCH_3$ | $N(CH_3)_2$ | CH | |
| $CH_3$ | $CH_3$ | $SO_2CH_2CH_3$ | H | $OCH_3$ | $C(CH_3)(SCH_3)_2$ | N | |
| $CH_3$ | H | $SO_2CH_2CH_3$ | H | $OCF_2H$ | $CH(SCH_2CH_2S)$ | CH | |
| $CH_3$ | H | $SO_2CH_2CH_3$ | H | $CF_3$ | $CH_2CH_3$ | CH | |
| $CH_3$ | H | $SO_2CH_2CH_3$ | H | F | $OCH_3$ | CH | |
| $CH_3$ | H | $SO_2CH_2CH_3$ | H | $OC_2H_5$ | $C(CH_3)(OCH_2CH_2O)$ | CH | |

Table 21

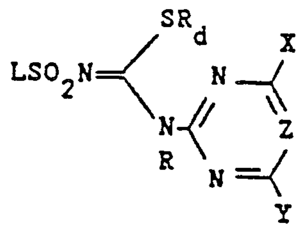

| $R_d$ | R | L | X | Y | Z | m.p. (°C) |
|---|---|---|---|---|---|---|
| $CH_3$ | H | $L_3:R_8=SO_2N(CH_3)_2$ | $CH_3$ | $CH_3$ | CH | |
| $CH_3$ | H | $L_3:R_8=SO_2CH_3$ | $CH_3$ | $OCH_3$ | CH | |
| $CH_3$ | $CH_3$ | $L_3:R_8=SO_2CH_2CH_3$ | Cl | $OCH_3$ | CH | |
| $i$-$C_3H_7$ | H | $L_4:R_9=Br$ | $OCH_3$ | $OCH_3$ | CH | |
| $CH_3$ | H | $L_5:R_9=C_2H_5$ | $OCH_3$ | $OCH_2CH=CH_2$ | CH | |
| $CH_3$ | H | $L_6:R_9=Cl$ | $OCH_3$ | $OCH_2C\equiv CH$ | N | |
| $CH_3$ | H | $L_7:R_{10}=CO_2CH_3$ | $OCH_3$ | $OCH_2CF_3$ | N | |
| $CH_3$ | H | $L_8:Q_1=O$, m=0, $R_{11}=R_{12}=R_{13}=CH_3$ | $OCH_3$ | $CH_2SCH_3$ | CH | |
| $CH_3$ | H | $L_9:Q_2=S$, $R_{14}=R_{15}=H$ | $OCH_3$ | $OCF_2H$ | CH | |
| $CH_3$ | H | $L_{10}$:m=0, $R_{13}=H$, $R_{16}=CH_3$ | $OCH_3$ | $SCF_2H$ | CH | |
| $CH_3$ | H | $L_{11}$:m=0, $R_{13}=H$ | $OCH_3$ | $C(CH_3)(OCH_3)_2$ | CH | |
| $CH_3$ | H | $L_{12}$:m=1, $R_{13}=CH_3$ | $OCH_3$ | $CF_3$ | CH | |
| $CH_3$ | H | $L_{13}$:m=0, $R_{13}=H$ | $OCH_3$ | $N(CH_3)_2$ | CH | |
| $CH_3$ | $CH_3$ | $L_{14}:R_{16}=CH_2CH_3$ | $OCH_3$ | $C(CH_3)(SCH_3)_2$ | CH | |
| $CH_3$ | H | $L_{15}:R_{17}=CH_3$ | $OCF_2H$ | $CH(SCH_2CH_2S)$ | CH | |
| $CH_3$ | H | $L_{16}:R_{17}=H$ | $CF_3$ | $CH_2CH_3$ | CH | |
| $CH_3$ | H | $L_{17}:R_{17}=R_{18}=CH_3$ | F | $OCH_3$ | CH | |
| $CH_3$ | H | $L_{17}:R_{17}=R_{18}=CH_3$ | $OC_2H_5$ | $C(CH_3)(OCH_2CH_2O)$ | CH | |

Table 22

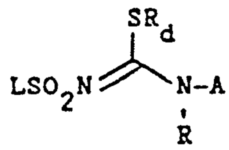

| $\underline{L}$ | $\underline{R_d}$ | $\underline{R}$ | $\underline{A}$ | m.p. (°C) |
|---|---|---|---|---|
| $L_1:R_5=CO_2CH_3$, $R_6=H$ | $CH_3$ | H | $A_4:X_1=CH_3$, $Y_2=H$ | |
| $L_1:R_5=SO_2CH_3$, $R_6=H$ | $CH_3$ | H | $A_5:X_2=CH_2CH_3$, $Y_3=SCH_3$ | |
| $L_1:R_5=NO_2$, $R_6=H$ | $CH_3$ | H | $A_6:X_3=OCH_3$ | |
| $L_1:R_5=Br$, $R_6=H$ | $CH_3$ | H | $A_3:X_1=OCH_3$ | |
| $L_1:R_5=Cl$, $R_6=H$ | $CH_3$ | H | $A_5:X_2=CH_3$, $Y_3=OC_2H_5$ | |
| $L_1:R_5=C_2H_5$, $R_6=H$ | $CH_3$ | H | $A_2:X_1=OCH_3$, $Y_1=O$ | |
| $L_1:R_5=SCF_3$, $R_6=H$ | $C_2H_5$ | H | $A_3:X_1=OCF_2H$ | |
| $L_{17}:R_{17}=H$, $R_{18}=CH_3$ | $CH_3$ | H | $A_2:X_1=OCH_3$, $Y_1=CH_2$ | |
| $L_{16}:R_{17}=CH_3$ | $CH_3$ | H | $A_3:X_1=OCF_2H$ | |
| $L_3:R_8=F$ | $CH_3$ | H | $A_5:X_2=CH_2CH_3$, $Y_3=OCH_3$ | |
| $L_4:R_9=\underline{i}-C_3H_7$ | $CH_3$ | H | $A_5:X_2=CH_3$, $Y_3=CH_2CH_3$ | |
| $L_5:R_9=SO_2CH_3$ | $CH_3$ | H | $A_6:X_3=OCH_3$ | |
| $L_6:R_9=SO_2CH_3$ | $CH_3$ | H | $A_2:X_1=OCH_3$, $Y_1=O$ | |
| $L_7:R_{10}=OCH_2CH_3$ | $CH_3$ | H | $A_3:X_1=CH_3$ | |
| $L_8:Q_1=S$, $R_{11}=R_{12}=CH_3$, m=1, $R_{13}=H$ | $CH_3$ | H | $A_6:X_3=CH_3$ | |
| $L_9:Q_2=O$, $R_{14}=R_{15}=CH_3$ | $CH_3$ | H | $A_4:X_1=OCH_3$, $Y_2=CH_3$ | |
| $L_{10}:R_{16}=C_2H_5$, $R_{13}=CH_3$, m=0 | $CH_3$ | H | $A_5:X_2=CH_3$, $Y_3=SCH_3$ | |
| $L_{11}$:m=1, $R_{13}=H$ | $CH_3$ | H | $A_2:X_1=CH_3$, $Y_1=CH_2$ | |
| $L_{12}$:m=0, $R_{13}=CH_3$ | $CH_3$ | H | $A_2:X_1=OCH_3$, $Y_1=CH_2$ | |
| $L_{13}$:m=1, $R_{13}=H$ | $CH_3$ | H | $A_5:X_2=CH_2CF_3$, $Y_3=CH_2CH_3$ | |
| $L_{14}:R_{16}=CH_3$ | $CH_3$ | H | $A_4:X_1=OCH_3$, $Y_2=H$ | |
| $L_{15}:R_{17}=CH_3$ | $CH_3$ | H | $A_4:X_1=OCH_3$, $Y_2=H$ | |

Table 23

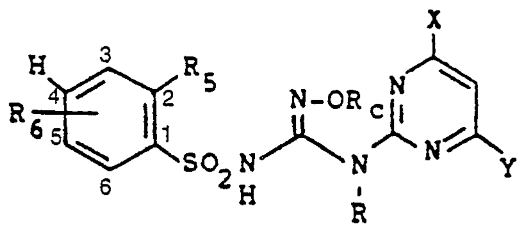

| $R_5$ | $R_6$ | R | $R_c$ | X | Y | m.p.(°C) |
|---|---|---|---|---|---|---|
| Cl | H | H | H | $CH_3$ | $CH_3$ | 165-167° |
| Cl | H | H | H | $CH_3$ | $OCH_3$ | 121-128° |
| Cl | H | H | H | $OCH_3$ | $OC_2H_5$ | |
| Cl | H | H | H | $OCH_3$ | $OCH_3$ | 142-144° |
| F | H | H | H | $CH_3$ | $CH_3$ | |
| F | H | H | H | $CH_3$ | $OCH_3$ | |
| Br | H | H | H | $OCH_3$ | $CH_3$ | |
| Br | H | H | H | $OCH_3$ | $OCH_3$ | |
| $NO_2$ | H | H | H | $CH_3$ | $CH(OCH_2CH_2O)$ | |
| $NO_2$ | H | $CH_3$ | H | $CH_3$ | $C_2H_5$ | |
| $CF_3$ | H | H | H | $OCH_3$ | $OC_2H_5$ | |
| $CF_3$ | H | H | H | $OCH_3$ | $CH_3$ | |
| $OCH_3$ | H | H | H | $CH_3$ | $OCH_3$ | |
| $OCH_3$ | H | H | H | $CH_3$ | $CH(OCH_3)_2$ | |
| $OCH_3$ | H | H | H | Cl | $OCH_3$ | |
| $OC_2H_5$ | H | H | H | $CH_3$ | $CH_2OCH_3$ | |
| $OC_2H_5$ | H | H | H | $CH_3$ | $CH_3$ | |
| $OC_2H_5$ | H | $CH_3$ | H | $OCH_3$ | $OCH_3$ | |
| O-*i*-$C_3H_7$ | H | H | H | $OCH_3$ | $CH_3$ | |
| O-*n*-$C_3H_7$ | H | H | H | $CH_3$ | $OCH_3$ | |
| O-*n*-$C_3H_7$ | H | H | H | $CH_3$ | $CH_3$ | |
| O-*i*-$C_4H_9$ | H | H | H | $OCH_3$ | $OCH_3$ | |
| O-*i*-$C_4H_9$ | H | H | H | $OCH_3$ | $CH_3$ | |
| $CO_2CH_3$ | H | H | H | $CH_3$ | $CH_3$ | $M^+$ = 379* |
| $CO_2CH_3$ | H | H | H | $CH_3$ | $OCH_3$ | $M^+$ = 395* |

Table 23 (continued)

| $R_5$ | $R_6$ | R | $R_c$ | X | Y | m.p.(°C) |
|---|---|---|---|---|---|---|
| $CO_2CH_3$ | H | H | H | $OCH_3$ | $OC_2H_5$ | |
| $SO_2N(CH_3)_2$ | H | H | H | $OCH_3$ | $OCH_3$ | |
| $SO_2N(CH_3)_2$ | H | H | H | $CH_3$ | $CH_3$ | |
| $SO_2N(CH_3)_2$ | H | H | H | $CH_3$ | $OCH_3$ | 166-168° |
| $SO_2N(CH_3)_2$ | H | H | H | $OCH_3$ | $OCH_2CH_3$ | |
| $SO_2N(CH_3)_2$ | H | H | H | $CH_3$ | $CH(OCH_2CH_2O)$ | |
| $SO_2N(CH_2CH_3)_2$ | H | $CH_3$ | H | $CH_3$ | $C_2H_5$ | |
| $SO_2N(CH_2CH_3)_2$ | H | H | H | $OCH_3$ | $OC_2H_5$ | |
| $SO_2N(CH_2CH_3)_2$ | H | H | H | $OCH_3$ | $CH_3$ | |
| $CO_2CH_2CH_3$ | H | H | H | $CH_3$ | $OCH_3$ | |
| $CO_2CH_2CH_3$ | H | H | H | $CH_3$ | $CH(OCH_3)_2$ | |
| $CO_2CH_3$ | H | H | H | Cl | $OCH_3$ | |
| $CO_2CH_3$ | H | H | H | $CH_3$ | $CH_2OCH_3$ | |
| $CO_2$-*i*-$C_3H_7$ | H | H | H | $CH_3$ | $CH_3$ | |
| $CO_2$-*i*-$C_3H_7$ | H | $CH_3$ | H | $OCH_3$ | $OCH_3$ | |
| $SO_2N(OCH_3)CH_3$ | H | H | H | $OCH_3$ | $CH_3$ | |
| $CO_2CH_2CH_2OCH_3$ | H | H | H | $CH_3$ | $OCH_3$ | |
| $CO_2CH_2CH{=}CH_2$ | H | H | H | $CH_3$ | $CH_3$ | |
| $CO_2CH_2CH_2Cl$ | H | H | H | $OCH_3$ | $OCH_3$ | |
| $CO_2$-*n*-$C_4H_9$ | H | H | H | $OCH_3$ | $CH_3$ | |
| $SO_2CH_3$ | H | H | H | $CH_3$ | $CH_3$ | |
| $SO_2CH_3$ | H | H | H | $CH_3$ | $OCH_3$ | |
| $SO_2CH_3$ | H | H | H | $OCH_3$ | $OC_2H_5$ | |
| $SO_2CH_3$ | H | H | H | $OCH_3$ | $OCH_3$ | |
| $SO_2CH_2CH{=}CH_2$ | H | H | H | $CH_3$ | $CH_3$ | |
| $SO_2CH_2CH{=}CH_2$ | H | H | H | $CH_3$ | $OCH_3$ | |
| $SO_2CH_2CH_2CH_3$ | H | H | H | $OCH_3$ | $CH_3$ | |
| $SCH_2CH_3$ | H | H | H | $OCH_3$ | $OCH_3$ | |
| $SCF_3$ | H | H | H | $CH_3$ | $CH(OCH_2CH_2O)$ | |
| $SO_2CHF_2$ | H | $CH_3$ | H | $CH_3$ | $C_2H_5$ | |
| $CH_3$ | H | H | H | $OCH_3$ | $OC_2H_5$ | |

Table 23 (continued)

| $R_5$ | $R_6$ | R | $R_d$ | X | Y | m.p.(°C) |
|---|---|---|---|---|---|---|
| $i$-$C_3H_7$ | H | H | H | $OCH_3$ | $CH_3$ | |
| $n$-$C_4H_9$ | H | H | H | $CH_3$ | $OCH_3$ | |
| $OSO_2N(CH_3)_2$ | H | H | H | $CH_3$ | $CH(OCH_3)_2$ | |
| $OSO_2CH(CH_3)_2$ | H | H | H | Cl | $OCH_3$ | |
| $OSO_2C_2H_5$ | H | H | H | $CH_3$ | $CH_2OCH_3$ | |
| $OSO_2CH_3$ | H | $CH_3$ | H | $OCH_3$ | $OCH_3$ | |
| $OSO_2CH_3$ | H | H | H | $OCH_3$ | $CH_3$ | |
| $CH_2OCH_3$ | H | H | H | $CH_3$ | $OCH_3$ | |
| $CH_2CH_2OCH_3$ | H | H | H | $CH_3$ | $CH_3$ | |
| $OCF_3$ | H | H | H | $OCH_3$ | $OCH_3$ | |
| $SO_2$-$i$-$C_3H_7$ | H | H | H | $OCH_3$ | $CH_3$ | |
| Cl | H | H | $CH_3CO$ | $CH_3$ | $CH_3$ | 131-136°d |
| Cl | 5-$CF_3$ | H | H | $CH_3$ | $OCH_3$ | |
| Cl | H | H | $CH_3OCO$ | $OCH_3$ | $OC_2H_5$ | |
| Cl | 6-$SCH_3$ | H | H | $OCH_3$ | $OCH_3$ | |
| F | 5-$SCH_3$ | H | $CH_3CO$ | $CH_3$ | $CH_3$ | |
| F | 6-$OCH_3$ | H | H | $CH_3$ | $OCH_3$ | |
| Br | H | H | $(CH_3)_2NCO$ | $OCH_3$ | $CH_3$ | |
| Br | 3-$OCH_3$ | H | H | $OCH_3$ | $OCH_3$ | |
| $NO_2$ | H | H | $CH_3NHCO$ | $CH_3$ | $CH(OCH_2CH_2O)$ | |
| $NO_2$ | 6-$CH_3$ | $CH_3$ | H | $CH_3$ | $C_2H_5$ | |
| $CF_3$ | 5-$CH_3$ | H | $CF_3CO$ | $OCH_3$ | $OC_2H_5$ | |
| $CF_3$ | 6-Cl | H | $C_2H_5CO$ | $OCH_3$ | $CH_3$ | |
| $OCH_3$ | H | H | $C_2H_5OCO$ | $CH_3$ | $OCH_3$ | |
| $OCH_3$ | 5-Cl | H | H | $CH_3$ | $CH(OCH_3)_2$ | |
| $OCH_3$ | H | H | $CH_3CO$ | Cl | $OCH_3$ | |
| $OC_2H_5$ | 5-F | H | H | $CH_3$ | $CH_2OCH_3$ | |
| $OC_2H_5$ | 5-F | H | $Et_2NCO$ | $CH_3$ | $CH_3$ | |
| $OC_2H_5$ | 3-Br | $CH_3$ | H | $OCH_3$ | $OCH_3$ | |
| O-$i$-$C_3H_7$ | H | H | EtOCO | $OCH_3$ | $CH_3$ | |

Table 23 (continued)

| $R_5$ | $R_6$ | R | $R_c$ | X | Y | m.p.(°C) |
|---|---|---|---|---|---|---|
| O-*n*-$C_3H_7$ | H | H | *n*-$C_3H_7CO$ | $CH_3$ | $OCH_3$ | |
| O-*n*-$C_3H_7$ | 6-$CH_3$ | H | H | $CH_3$ | $CH_3$ | |
| O-*i*-$C_4H_9$ | 3-$CF_3$ | H | $CH_3OCO$ | $OCH_3$ | $OCH_3$ | |
| O-*i*-$C_4H_9$ | 6-$CF_3$ | H | H | $OCH_3$ | $CH_3$ | |
| $CO_2CH_3$ | 5-$CF_3$ | H | H | $CH_3$ | $CH_3$ | |
| $CO_2CH_3$ | 6-$CF_3$ | H | H | $CH_3$ | $OCH_3$ | |
| $CO_2CH_3$ | H | H | $CH_3OCO$ | $OCH_3$ | $OC_2H_5$ | |
| $SO_2N(CH_3)_2$ | 6-$SCH_3$ | H | H | $OCH_3$ | $OCH_3$ | |
| $SO_2N(CH_3)_2$ | 5-$SCH_3$ | H | $CH_3CO$ | $CH_3$ | $CH_3$ | |
| $SO_2N(CH_3)_2$ | 6-$OCH_3$ | H | H | $CH_3$ | $OCH_3$ | |
| $SO_2N(CH_3)_2$ | H | H | $(CH_3)_2NCO$ | $OCH_3$ | $OCH_3$ | |
| $SO_2N(CH_3)_2$ | 3-$OCH_3$ | H | H | $OCH_3$ | $OCH_2CH_3$ | |
| $SO_2N(CH_3)_2$ | H | H | $CH_3NHCO$ | $CH_3$ | $CH(OCH_2CH_2O)$ | |
| $SO_2N(CH_2CH_3)_2$ | 3-$CH_3$ | $CH_3$ | H | $CH_3$ | $C_2H_5$ | |
| $SO_2N(CH_2CH_3)_2$ | 5-$CH_3$ | H | $CF_3CO$ | $OCH_3$ | $OC_2H_5$ | |
| $SO_2N(CH_2CH_3)_2$ | 6-Cl | H | $C_2H_5CO$ | $OCH_3$ | $CH_3$ | |
| $CO_2CH_2CH_3$ | H | H | $C_2H_5CO$ | $CH_3$ | $OCH_3$ | |
| $CO_2CH_2CH_3$ | 5-Cl | H | H | $CH_3$ | $CH(OCH_3)_2$ | |
| $CO_2CH_3$ | H | H | $CH_3OCO$ | Cl | $OCH_3$ | |
| $CO_2CH_3$ | 5-F | H | H | $CH_3$ | $CH_2OCH_3$ | |
| $CO_2$-*i*-$C_3H_7$ | 5-F | H | $(C_2H_5)_2NCO$ | $CH_3$ | $CH_3$ | |
| $CO_2$-*i*-$C_3H_7$ | 3-Br | $CH_3$ | H | $OCH_3$ | $OCH_3$ | |
| $SO_2N(OCH_3)CH_3$ | H | H | *n*-$C_3H_7CO$ | $OCH_3$ | $CH_3$ | |
| $CO_2CH_2CH_2OCH_3$ | H | H | H | $CH_3$ | $OCH_3$ | |
| $CO_2CH_2CH{=}CH_2$ | 6-$CH_3$ | H | H | $CH_3$ | $CH_3$ | |
| $CO_2CH_2CH_2Cl$ | 3-$CF_3$ | H | H | $OCH_3$ | $OCH_3$ | |
| $CO_2$-*n*-$C_4H_9$ | 6-$CF_3$ | H | H | $OCH_3$ | $CH_3$ | |
| $SO_2CH_3$ | 5-$CF_3$ | H | H | $CH_3$ | $CH_3$ | |
| $SO_2CH_3$ | 3-$CF_3$ | H | H | $CH_3$ | $OCH_3$ | |
| $SO_2CH_3$ | H | H | $CH_3OCO$ | $OCH_3$ | $OC_2H_5$ | |
| $SO_2CH_3$ | 6-$SCH_3$ | H | H | $OCH_3$ | $OCH_3$ | |

Table 23 (continued)

| $R_5$ | $R_6$ | R | $R_c$ | X | Y | m.p.(°C) |
|---|---|---|---|---|---|---|
| $SO_2CH_2CH=CH_2$ | 5-$SCH_3$ | H | $CH_3CO$ | $CH_3$ | $CH_3$ | |
| $SO_2CH_2CH=CH_2$ | 6-$OCH_3$ | H | H | $CH_3$ | $OCH_3$ | |
| $SO_2CH_2CH_2CH_3$ | H | H | $(CH_3)_2NCO$ | $OCH_3$ | $CH_3$ | |
| $SCH_2CH_3$ | 3-$OCH_3$ | H | H | $OCH_3$ | $OCH_3$ | |
| $SCF_3$ | H | H | $CH_3NHCO$ | $CH_3$ | $CH(OCH_2CH_2O)$ | |
| $SO_2CHF_2$ | 3-$CH_3$ | $CH_3$ | H | $CH_3$ | $C_2H_5$ | |
| $CH_3$ | 5-$CH_3$ | H | $CF_3CO$ | $OCH_3$ | $OC_2H_5$ | |
| *i*-$C_3H_7$ | 6-Cl | H | H | $OCH_3$ | $CH_3$ | |
| *n*-$C_4H_9$ | H | H | $C_2H_5CO$ | $CH_3$ | $OCH_3$ | |
| $OSO_2N(CH_3)_2$ | H | H | $CH_3OCO$ | Cl | $OCH_3$ | |
| $OSO_2CH(CH_3)_2$ | 5-F | H | $CH_3CO$ | $CH_3$ | $CH_2OCH_3$ | |
| $OSO_2CH_3$ | 3-Br | $CH_3$ | H | $OCH_3$ | $OCH_3$ | |
| $OSO_2CH_3$ | H | H | *n*-$C_3H_7OCO$ | $OCH_3$ | $CH_3$ | |
| $CH_2OCH_3$ | H | H | $C_3H_7CO$ | $CH_3$ | $OCH_3$ | |
| $CH_2CH_2OCH_3$ | 6-$CH_3$ | H | H | $CH_3$ | $CH_3$ | |
| $OCF_3$ | 3-$CF_3$ | H | H | $OCH_3$ | $OCH_3$ | |
| $SO_2$-*i*-$C_3H_7$ | 5-$CF_3$ | H | H | $OCH_3$ | $CH_3$ | |
| $CH_2CH_2OC_2H_5$ | H | H | H | $OCH_3$ | $CH_3$ | |

* Data refer to molecular ion in electron-impact mass spectrum.

Table 24

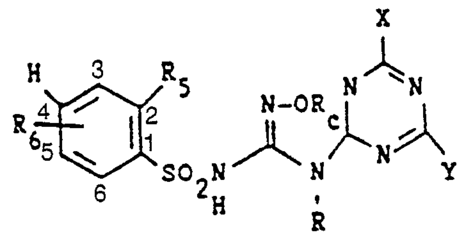

| $R_5$ | $R_6$ | R | $R_c$ | X | Y | m.p.(°C) |
|---|---|---|---|---|---|---|
| Cl | H | H | H | $CH_3$ | $CH_3$ | |
| Cl | H | H | H | $CH_3$ | $OCH_3$ | |
| Cl | H | H | H | $OCH_3$ | $OC_2H_5$ | |
| Cl | H | H | H | $OCH_3$ | $OCH_3$ | 137-140° |
| F | H | H | H | $CH_3$ | $CH_3$ | |
| F | H | H | H | $CH_3$ | $OCH_3$ | |
| Br | H | H | H | $OCH_3$ | $CH_3$ | |
| Br | H | H | H | $OCH_3$ | $OCH_3$ | |
| $NO_2$ | H | H | H | $CH_3$ | $CH(OCH_2CH_2O)$ | |
| $NO_2$ | H | $CH_3$ | H | $CH_3$ | $C_2H_5$ | |
| $CF_3$ | H | H | H | $OCH_3$ | $OC_2H_5$ | |
| $CF_3$ | H | H | H | $OCH_3$ | $CH_3$ | |
| $OCH_3$ | H | H | H | $CH_3$ | $OCH_3$ | |
| $OCH_3$ | H | H | H | $CH_3$ | $CH(OCH_3)_2$ | |
| $OCH_3$ | H | H | H | $CH_3$ | $CH_3$ | |
| $OCH_3$ | H | H | H | $OCH_3$ | $OCH_3$ | |
| $OC_2H_5$ | H | H | H | $CH_3$ | $CH_2OCH_3$ | |
| $OC_2H_5$ | H | H | H | $CH_3$ | $CH_3$ | |
| $OC_2H_5$ | H | $CH_3$ | H | $OCH_3$ | $OCH_3$ | |
| O-*i*-$C_3H_7$ | H | H | H | $OCH_3$ | $CH_3$ | |
| O-*n*-$C_3H_7$ | H | H | H | $CH_3$ | $OCH_3$ | |
| O-*n*-$C_3H_7$ | H | H | H | $CH_3$ | $CH_3$ | |
| O-*i*-$C_4H_9$ | H | H | H | $OCH_3$ | $OCH_3$ | |
| O-*i*-$C_4H_9$ | H | H | H | $OCH_3$ | $CH_3$ | |
| $CO_2CH_3$ | H | H | H | $CH_3$ | $CH_3$ | |

Table 24 (continued)

| $R_5$ | $R_6$ | R | $R_c$ | X | Y | m.p.(°C) |
|---|---|---|---|---|---|---|
| $CO_2CH_3$ | H | H | H | $CH_3$ | $OCH_3$ | |
| $CO_2CH_3$ | H | H | H | $OCH_3$ | $OC_2H_5$ | |
| $SO_2N(CH_3)_2$ | H | H | H | $OCH_3$ | $OCH_3$ | δ 3.0 and 4.0 (1:1)** |
| $SO_2N(CH_3)_2$ | H | H | H | $CH_3$ | $CH_3$ | |
| $SO_2N(CH_3)_2$ | H | H | H | $CH_3$ | $OCH_3$ | |
| $SO_2N(CH_3)_2$ | H | H | H | $OCH_3$ | $OCH_2CH_3$ | |
| $SO_2N(CH_3)_2$ | H | H | H | $CH_3$ | $CH(OCH_2CH_2O)$ | |
| $SO_2N(CH_2CH_3)_2$ | H | $CH_3$ | H | $CH_3$ | $C_2H_5$ | |
| $SO_2N(CH_2CH_3)_2$ | H | H | H | $OCH_3$ | $OC_2H_5$ | |
| $SO_2N(CH_2CH_3)_2$ | H | H | H | $OCH_3$ | $CH_3$ | |
| $CO_2CH_2CH_3$ | H | H | H | $CH_3$ | $OCH_3$ | |
| $CO_2CH_2CH_3$ | H | H | H | $CH_3$ | $CH(OCH_3)_2$ | |
| $CO_2CH_3$ | H | H | H | $CH_3$ | $CH_3$ | |
| $CO_2CH_3$ | H | H | H | $CH_3$ | $OCH_3$ | |
| $CO_2CH_3$ | H | H | H | $CH_3$ | $CH_2OCH_3$ | |
| $CO_2$-*i*-$C_3H_7$ | H | H | H | $CH_3$ | $CH_3$ | |
| $CO_2$-*i*-$C_3H_7$ | H | $CH_3$ | H | $OCH_3$ | $OCH_3$ | |
| $SO_2N(OCH_3)CH_3$ | H | H | H | $OCH_3$ | $CH_3$ | |
| $CO_2CH_2CH_2OCH_3$ | H | H | H | $CH_3$ | $OCH_3$ | |
| $CO_2CH_2CH=CH_2$ | H | H | H | $CH_3$ | $CH_3$ | |
| $CO_2CH_2CH_2Cl$ | H | H | H | $OCH_3$ | $OCH_3$ | |
| $CO_2$-*n*-$C_4H_9$ | H | H | H | $OCH_3$ | $CH_3$ | |
| $SO_2CH_3$ | H | H | H | $CH_3$ | $CH_3$ | |
| $SO_2CH_3$ | H | H | H | $CH_3$ | $OCH_3$ | |
| $SO_2CH_3$ | H | H | H | $OCH_3$ | $OC_2H_5$ | |
| $SO_2CH_3$ | H | H | H | $OCH_3$ | $OCH_3$ | |
| $SO_2CH_2CH=CH_2$ | H | H | H | $CH_3$ | $CH_3$ | |
| $SO_2CH_2CH=CH_2$ | H | H | H | $CH_3$ | $OCH_3$ | |
| $SO_2CH_2CH_2CH_3$ | H | H | H | $OCH_3$ | $CH_3$ | |
| $SCH_2CH_3$ | H | H | H | $OCH_3$ | $OCH_3$ | |
| $SCF_3$ | H | H | H | $CH_3$ | $CH(OCH_2CH_2O)$ | |
| $SO_2CHF_2$ | H | $CH_3$ | H | $CH_3$ | $C_2H_5$ | |

Table 24 (continued)

| $R_5$ | $R_6$ | R | $R_c$ | X | Y | m.p.(°C) |
|---|---|---|---|---|---|---|
| $CH_3$ | H | H | H | $OCH_3$ | $OC_2H_5$ | |
| $i$-$C_3H_7$ | H | H | H | $OCH_3$ | $CH_3$ | |
| $n$-$C_4H_9$ | H | H | H | $CH_3$ | $OCH_3$ | |
| $OSO_2N(CH_3)_2$ | H | H | H | $CH_3$ | $CH(OCH_3)_2$ | |
| $OSO_2N(CH_3)_2$ | H | H | H | $CH_3$ | $CH_3$ | |
| $OSO_2CH(CH_3)_2$ | H | H | H | $CH_3$ | $OCH_3$ | |
| $OSO_2C_2H_5$ | H | H | H | $CH_3$ | $CH_2OCH_3$ | |
| $OSO_2CH_3$ | H | $CH_3$ | H | $OCH_3$ | $OCH_3$ | |
| $OSO_2CH_3$ | H | H | H | $OCH_3$ | $CH_3$ | |
| $CH_2OCH_3$ | H | H | H | $CH_3$ | $OCH_3$ | |
| $CH_2CH_2OCH_3$ | H | H | H | $CH_3$ | $CH_3$ | |
| $OCF_3$ | H | H | H | $OCH_3$ | $OCH_3$ | |
| $SO_2$-$i$-$C_3H_7$ | H | H | H | $OCH_3$ | $CH_3$ | |
| Cl | 3-$CF_3$ | H | H | $CH_3$ | $CH_3$ | |
| Cl | 5-$CF_3$ | H | H | $CH_3$ | $OCH_3$ | |
| Cl | H | H | $CH_3OCO$ | $OCH_3$ | $OC_2H_5$ | |
| Cl | 6-$SCH_3$ | H | H | $OCH_3$ | $OCH_3$ | |
| F | 5-$SCH_3$ | H | $CH_3CO$ | $CH_3$ | $CH_3$ | |
| F | 6-$OCH_3$ | H | H | $CH_3$ | $OCH_3$ | |
| Br | H | H | $(CH_3)_2NCO$ | $OCH_3$ | $CH_3$ | |
| Br | 3-$OCH_3$ | H | H | $OCH_3$ | $OCH_3$ | |
| $NO_2$ | H | H | $CH_3NHCO$ | $CH_3$ | $CH(OCH_2CH_2O)$ | |
| $NO_2$ | 3-$CH_3$ | $CH_3$ | H | $CH_3$ | $C_2H_5$ | |
| $CF_3$ | 5-$CH_3$ | H | $CF_3CO$ | $OCH_3$ | $OC_2H_5$ | |
| $CF_3$ | 6-Cl | H | $C_2H_5CO$ | $OCH_3$ | $CH_3$ | |
| $OCH_3$ | H | H | $C_2H_5OCO$ | $CH_3$ | $OCH_3$ | |
| $OCH_3$ | 5-Cl | H | H | $CH_3$ | $CH(OCH_3)_2$ | |
| $OCH_3$ | 6-Cl | H | H | $CH_3$ | $CH_3$ | |
| $OCH_3$ | H | H | $n$-$C_3H_7CO$ | $CH_3$ | $OCH_3$ | |
| $OC_2H_5$ | 5-F | H | H | $CH_3$ | $CH_2OCH_3$ | |
| $OC_2H_5$ | 5-F | H | $Et_2NCO$ | $CH_3$ | $CH_3$ | |
| $OC_2H_5$ | 3-Br | $CH_3$ | H | $OCH_3$ | $OCH_3$ | |

Table 24 (continued)

| $R_5$ | $R_6$ | R | $R_c$ | X | Y | m.p.(°C) |
|---|---|---|---|---|---|---|
| O-*i*-$C_3H_7$ | H | H | EtOCO | $OCH_3$ | $CH_3$ | |
| O-*n*-$C_3H_7$ | H | H | *n*-$C_3H_7CO$ | $CH_3$ | $OCH_3$ | |
| O-*n*-$C_3H_7$ | 6-$CH_3$ | H | H | $CH_3$ | $CH_3$ | |
| O-*i*-$C_4H_9$ | 3-$CF_3$ | H | $CH_3OCO$ | $OCH_3$ | $OCH_3$ | |
| O-*i*-$C_4H_9$ | 5-$CF_3$ | H | H | $OCH_3$ | $CH_3$ | |
| $CO_2CH_3$ | 3-$CF_3$ | H | H | $CH_3$ | $CH_3$ | |
| $CO_2CH_3$ | 5-$CF_3$ | H | H | $CH_3$ | $OCH_3$ | |
| $CO_2CH_3$ | H | H | $CH_3OCO$ | $OCH_3$ | $OC_2H_5$ | |
| $SO_2N(CH_3)_2$ | 6-$SCH_3$ | H | H | $OCH_3$ | $OCH_3$ | |
| $SO_2N(CH_3)_2$ | 5-$SCH_3$ | H | $CH_3CO$ | $CH_3$ | $CH_3$ | |
| $SO_2N(CH_3)_2$ | 6-$OCH_3$ | H | H | $CH_3$ | $OCH_3$ | |
| $SO_2N(CH_3)_2$ | H | H | $(CH_3)_2NCO$ | $OCH_3$ | $OCH_3$ | |
| $SO_2N(CH_3)_2$ | 3-$OCH_3$ | H | H | $OCH_3$ | $OCH_2CH_3$ | |
| $SO_2N(CH_3)_2$ | H | H | $CH_3NHCO$ | $CH_3$ | $CH(OCH_2CH_2O)$ | |
| $SO_2N(CH_2CH_3)_2$ | 3-$CH_3$ | $CH_3$ | H | $CH_3$ | $C_2H_5$ | |
| $SO_2N(CH_2CH_3)_2$ | 5-$CH_3$ | H | *n*-$C_3H_7CO$ | $OCH_3$ | $OC_2H_5$ | |
| $SO_2N(CH_2CH_3)_2$ | 6-Cl | H | H | $OCH_3$ | $CH_3$ | |
| $CO_2CH_2CH_3$ | H | H | $CF_3CO$ | $CH_3$ | $OCH_3$ | |
| $CO_2CH_2CH_3$ | 5-Cl | H | $C_2H_5OCO$ | $CH_3$ | $CH(OCH_3)_2$ | |
| $CO_2CH_3$ | 6-Cl | H | H | $CH_3$ | $CH_3$ | |
| $CO_2CH_3$ | H | H | $C_2H_5CO$ | $CH_3$ | $OCH_3$ | |
| $CO_2CH_3$ | 5-F | H | H | $CH_3$ | $CH_2OCH_3$ | |
| $CO_2$-*i*-$C_3H_7$ | 5-F | H | H | $CH_3$ | $CH_3$ | |
| $CO_2$-*i*-$C_3H_7$ | 3-Br | $CH_3$ | H | $OCH_3$ | $OCH_3$ | |
| $SO_2N(OCH_3)CH_3$ | H | H | $Et_2NCO$ | $OCH_3$ | $CH_3$ | |
| $CO_2CH_2CH_2OCH_3$ | H | H | *n*-$C_3H_7OCO$ | $CH_3$ | $OCH_3$ | |
| $CO_2CH_2CH=CH_2$ | 6-$CH_3$ | H | H | $CH_3$ | $CH_3$ | |
| $CO_2CH_2CH_2Cl$ | H | H | $CH_3OCO$ | $OCH_3$ | $OCH_3$ | |
| $CO_2$-*n*-$C_4H_9$ | H | H | $C_2H_5CO$ | $OCH_3$ | $CH_3$ | |
| $SO_2CH_3$ | 3-$CF_3$ | H | H | $CH_3$ | $CH_3$ | |
| $SO_2CH_3$ | 5-$CF_3$ | H | H | $CH_3$ | $OCH_3$ | |
| $SO_2CH_3$ | H | H | $CH_3OCO$ | $OCH_3$ | $OC_2H_5$ | |

Table 24 (continued)

| $R_5$ | $R_6$ | R | $R_c$ | X | Y | m.p.(°C) |
|---|---|---|---|---|---|---|
| $SO_2CH_3$ | 6-$SCH_3$ | H | H | $OCH_3$ | $OCH_3$ | |
| $SO_2CH_2CH{=}CH_2$ | 5-$SCH_3$ | H | H | $CH_3$ | $CH_3$ | |
| $SO_2CH_2CH{=}CH_2$ | 6-$OCH_3$ | H | $(CH_3)_2NCO$ | $CH_3$ | $OCH_3$ | |
| $SO_2CH_2CH_2CH_3$ | H | H | H | $OCH_3$ | $CH_3$ | |
| $SCH_2CH_3$ | 3-$OCH_3$ | H | H | $OCH_3$ | $OCH_3$ | |
| $SCF_3$ | H | H | $CH_3NHCO$ | $CH_3$ | $CH(OCH_2CH_2O)$ | |
| $SO_2CHF_2$ | 3-$CH_3$ | $CH_3$ | H | $CH_3$ | $C_2H_5$ | |
| $CH_3$ | 5-$CH_3$ | H | $C_2H_5OCO$ | $OCH_3$ | $OC_2H_5$ | |
| *i*-$C_3H_7$ | 6-Cl | H | H | $OCH_3$ | $CH_3$ | |
| *n*-$C_4H_9$ | H | H | n-$C_3H_7OCO$ | $CH_3$ | $OCH_3$ | |
| $OSO_2N(CH_3)_2$ | 6-Cl | H | $C_2H_5CO$ | $CH_3$ | $CH_3$ | |
| $OSO_2N(CH_3)_2$ | H | H | *n*-$C_3H_7CO$ | $CH_3$ | $OCH_3$ | |
| $OSO_2CH(CH_3)_2$ | 5-F | H | H | $CH_3$ | $CH_2OCH_3$ | |
| $OSO_2CH_3$ | 3-Br | $CH_3$ | $C_2H_5NHCO$ | $OCH_3$ | $OCH_3$ | |
| $OSO_2CH_3$ | H | H | $(C_2H_5)_2NCO$ | $OCH_3$ | $CH_3$ | |
| $CH_2OCH_3$ | H | H | $CH_3CO$ | $CH_3$ | $OCH_3$ | |
| $CH_2CH_2OCH_3$ | 6-$CH_3$ | H | H | $CH_3$ | $CH_3$ | |
| $OCF_3$ | H | H | $C_2H_5OCO$ | $OCH_3$ | $OCH_3$ | |
| $SO_2$-*i*-$C_3H_7$ | H | H | *n*-$C_3H_7NHCO$ | $OCH_3$ | $CH_3$ | |
| $CH_2CH_2OC_2H_5$ | H | H | H | $OCH_3$ | $CH_3$ | |

**Data refer to characteristic signals in NMR spectrum ($CDCl_3$) in ppm downfield from tetramethylsilane.

Table 25

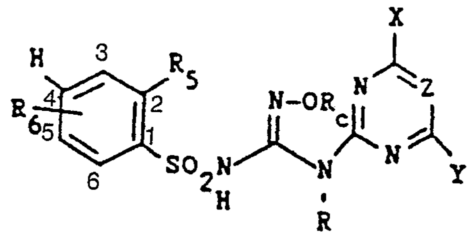

| $R_c$ | R | $R_5$ | $R_6$ | X | Y | Z | m.p. (°C) |
|---|---|---|---|---|---|---|---|
| H | H | $OCH_2CH_2OCH_3$ | H | $CH_3$ | H | CH | |
| H | H | $OCH_2CH{=}CH_2$ | H | $CH_3$ | $CH_3$ | N | |
| H | H | $OCH_2CH{\equiv}CH$ | 6-$OCH_3$ | $CH_3$ | $CH_3$ | CH | |
| H | H | $C_6H_5$ | H | $CH_3$ | $CH_3$ | N | |
| H | H | 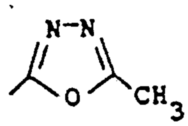 | H | $CH_3$ | $OCH_3$ | CH | |
| H | H | 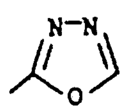 | H | $OCH_3$ | $OCH_3$ | N | |
| H | H | Cl | H | $OCH_3$ | $OCH_2C{\equiv}CH$ | N | |
| $CONHCH_3$ | H | Cl | H | $OCH_3$ | $OCH_2CF_3$ | CH | |
| H | H | Cl | H | $OCH_3$ | $OCH_2CH_2OCH_3$ | N | |
| H | H | Cl | H | $OCH_3$ | $CH_2SCH_3$ | CH | |
| H | H | F | H | $OCH_3$ | $OCF_2H$ | CH | |
| H | H | F | H | $OCH_3$ | $SCF_2H$ | CH | |
| H | H | F | 3-$OCF_2H$ | $OCH_3$ | $C(CH_3)(OCH_3)_2$ | CH | |
| H | H | F | H | $OCH_3$ | $CF_3$ | N | |
| H | H | Br | H | $OCH_3$ | $N(CH_3)_2$ | CH | |
| H | $CH_3$ | Br | H | $OCH_3$ | $C(CH_3)(SCH_3)_2$ | N | |
| H | $CH_3$ | Br | H | $OCF_2H$ | $CH(SCH_2CH_2S)$ | CH | |
| H | $CH_3$ | Br | H | $CF_3$ | $CH_2CH_3$ | CH | |
| H | $CH_3$ | Br | H | F | $OCH_3$ | CH | |
| H | $CH_3$ | Br | H | $OC_2H_5$ | $C(CH_3)(OCH_2CH_2O)$ | CH | |

Table 25 (continued)

| $R_c$ | R | $R_5$ | $R_6$ | X | Y | Z | m.p.(°C) |
|---|---|---|---|---|---|---|---|
| H | H | 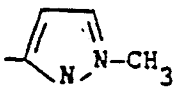 | H | $CH_3$ | H | CH | |
| H | H | 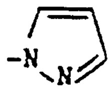 | H | $CH_3$ | $CH_3$ | CH | |
| H | H | 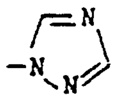 | H | $CH_3$ | $OCH_3$ | CH | |
| $COCF_3$ | H | O-*n*-$C_3H_7$ | H | $OCH_3$ | $OCH_3$ | CH | |
| H | H | O-*n*-$C_3H_7$ | H | $OCH_3$ | $OCH_2CH{=}CH_2$ | CH | |
| H | H | O-*n*-$C_3H_7$ | H | $OCH_3$ | $OCH_2C{\equiv}CH$ | N | |
| H | H | $OCH_3$ | 5-Br | $OCH_3$ | $OCH_2CF_3$ | N | |
| H | H | $OCH_3$ | H | $OCH_3$ | $OCH_2CH_2OCH_3$ | N | |
| H | H | $OCH_3$ | H | $OCH_3$ | $CH_2SCH_3$ | CH | |
| H | H | $NO_2$ | H | $OCH_3$ | $OCF_2H$ | CH | |
| H | H | $NO_2$ | H | $OCH_3$ | $SCF_2H$ | CH | |
| H | H | $NO_2$ | H | $OCH_3$ | $C(CH_3)(OCH_3)_2$ | CH | |
| H | H | $CF_3$ | H | $OCH_3$ | $CF_3$ | CH | |
| H | H | $CF_3$ | H | $OCH_3$ | $N(CH_3)_2$ | CH | |
| H | $CH_3$ | $CF_3$ | H | $OCH_3$ | $C(CH_3)(SCH_3)_2$ | CH | |
| H | $CH_3$ | $CO_2CH_3$ | H | $OCF_2H$ | $CH(SCH_2CH_2S)$ | CH | |
| H | $CH_3$ | $CO_2CH_3$ | H | $CF_3$ | $CH_2CH_3$ | CH | |
| H | $CH_3$ | $CO_2CH_3$ | H | F | $OCH_3$ | CH | |
| H | $CH_3$ | $CO_2CH_3$ | H | $OC_2H_5$ | $C(CH_3)(OCH_2CH_2O)$ | CH | |
| H | H | 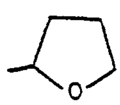 | H | $CH_3$ | H | N | |
| H | H | 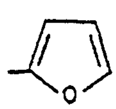 | H | $CH_3$ | $CH_3$ | N | |
| H | H | 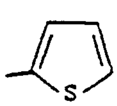 | H | $CH_3$ | $OCH_3$ | CH | |
| H | H | $CO_2CH_2CH{=}CH_2$ | H | $OCH_3$ | $OCH_3$ | CH | |
| H | H | $CO_2CH_2CH{=}CH_2$ | H | $OCH_3$ | $OCH_2CH{=}CH_2$ | CH | |

Table 25 (continued)

| $R_c$ | R | $R_5$ | $R_6$ | X | Y | Z | m.p.(°C) |
|---|---|---|---|---|---|---|---|
| H | H | $CO_2CH_2CH=CH_2$ | H | $OCH_3$ | $OCH_2C{\equiv}CH$ | N | |
| H | H | $SO_2N(CH_3)_2$ | H | $OCH_3$ | $OCH_2CF_3$ | N | |
| H | H | $SO_2N(CH_3)_2$ | H | $OCH_3$ | $OCH_2CH_2OCH_3$ | N | |
| H | H | $SO_2N(CH_3)_2$ | H | $OCH_3$ | $CH_2SCH_3$ | N | |
| H | H | $SO_2N(OCH_3)CH_3$ | H | $OCH_3$ | $OCF_2H$ | CH | |
| H | H | $SO_2N(CH_3)C_2H_5$ | H | $OCH_3$ | $SCF_2H$ | CH | |
| H | H | $SCF_3$ | H | $OCH_3$ | $C(CH_3)(OCH_3)_2$ | CH | |
| H | H | $SO_2CHF_2$ | H | $OCH_3$ | $CF_3$ | CH | |
| H | H | $CO_2CH_2CH_2Cl$ | H | $OCH_3$ | $N(CH_3)_2$ | CH | |
| H | $CH_3$ | $CO_2CH_2CH_3$ | H | $OCH_3$ | $C(CH_3)(SCH_3)_2$ | CH | |
| H | H | $CO_2CH_2CH_3$ | H | $OCF_2H$ | $CH(SCH_2CH_2S)$ | CH | |
| H | H | $CO_2CH_2CH_3$ | H | $CF_3$ | $CH_2CH_3$ | CH | |
| H | H | $SO_2CH_3$ | H | F | $OCH_3$ | CH | |
| CO-*i*-$C_3H_7$ | H | $SO_2CH_3$ | H | $OC_2H_5$ | $C(CH_3)(OCH_2CH_2O)$ | CH | |
| H | H | 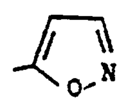 | H | $CH_3$ | H | N | |
| H | H | 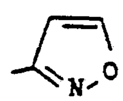 | H | $CH_3$ | $CH_3$ | N | |
| H | H | 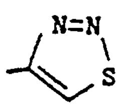 | 6-$OCF_2H$ | $CH_3$ | $OCH_3$ | CH | |
| H | H | 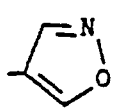 | H | $OCH_3$ | $OCH_3$ | CH | |
| H | H | $OCH_3$ | H | $OCH_3$ | $OCH_2C{\equiv}CH$ | CH | |
| H | H | $OCH_3$ | H | $OCH_3$ | $OCH_2CF_3$ | CH | |
| H | H | $OCH_3$ | H | $OCH_3$ | $OCH_2CH_2OCH_3$ | CH | |
| H | H | $OC_2H_5$ | H | $OCH_3$ | $CH_2SCH_3$ | CH | |
| H | H | $OC_2H_5$ | 3-F | $OCH_3$ | $OCF_2H$ | CH | |
| $CO_2CH_2CH_3$ | H | $OC_2H_5$ | H | $OCH_3$ | $SCF_2H$ | CH | |
| H | H | $CH_3$ | H | $OCH_3$ | $C(CH_3)(OCH_3)_2$ | N | |
| H | H | $CH_3$ | H | $OCH_3$ | $CF_3$ | N | |

Table 25 (continued)

| $R_c$ | R | $R_5$ | $R_6$ | X | Y | Z | m.p.(°C) |
|---|---|---|---|---|---|---|---|
| H | H | $CH_3$ | H | $OCH_3$ | $N(CH_3)_2$ | N | |
| H | $CH_3$ | *i*-$C_3H_7$ | H | $OCH_3$ | $C(CH_3)(SCH_3)_2$ | CH | |
| H | H | *i*-$C_3H_7$ | H | $OCF_2H$ | $CH(SCH_2CH_2S)$ | CH | |
| H | H | *i*-$C_3H_7$ | H | $CF_3$ | $CH_2CH_3$ | CH | |
| H | H | *i*-$C_3H_7$ | H | F | $OCH_3$ | CH | |
| H | H | *n*-$C_4H_9$ | H | $OC_2H_5$ | $C(CH_3)(OCH_2CH_2O)$ | N | |

Table 26

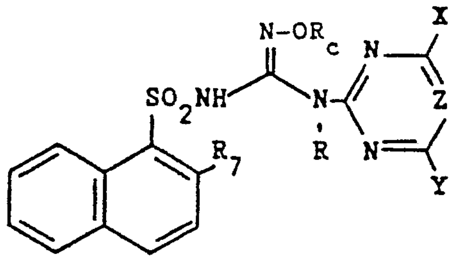

| $R_7$ | R | $R_c$ | X | Y | Z | m.p.(°C) |
|---|---|---|---|---|---|---|
| H | H | H | $CH_3$ | $CH_3$ | CH | |
| $CH_3$ | H | $CON(CH_3)_2$ | $OCH_3$ | $CH_3$ | N | |
| F | H | H | $CH_3$ | $CH_3$ | CH | |
| Cl | H | H | $OCH_3$ | $OCH_3$ | CH | |
| Br | H | H | F | $OCH_3$ | CH | |
| $SO_2N(CH_3)_2$ | H | H | $CH_3$ | $OCH_3$ | CH | |
| $OSO_2CH_3$ | H | H | $OCF_2H$ | $OCH_3$ | CH | |
| $SO_2CH_3$ | H | H | $CH_2F$ | $OC_2H_5$ | CH | |
| H | $CH_3$ | H | $OCH_2CH_3$ | $CH_2OCH_3$ | N | |
| $CH_3$ | H | H | $CF_3$ | $N(CH_3)_2$ | CH | |
| $OCH_3$ | H | H | $OCH_3$ | $CF_3$ | CH | |
| F | H | $CO_2CH_2CH_3$ | $CH_3$ | $CH(OCH_3)_2$ | CH | |
| Cl | H | H | $OCH_3$ | $OCF_2H$ | N | |
| Br | H | H | $OCH_3$ | $OCH_2CH_2OCH_3$ | N | |
| $SO_2N(CH_3)_2$ | H | H | $OCH_3$ | $CH_2SCH_3$ | N | |
| $OSO_2CH_3$ | H | H | $OCH_3$ | $OCH_2C{\equiv}CH$ | CH | |
| $SCH_3$ | H | H | $OCH_2CH_3$ | 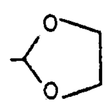 | CH | |
| $SCH_3$ | H | H | $CH_3$ | | CH | |
| $CH_3$ | H | H | $CH_3$ | $OCH_3$ | N | |
| H | H | H | $CH_3$ | $OCH_3$ | N | |

Table 27

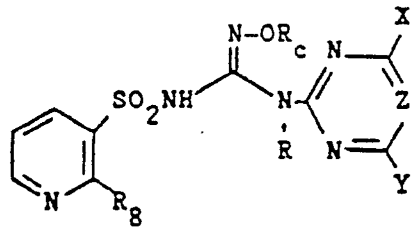

| $R_8$ | R | $R_c$ | X | Y | Z | m.p. (°C) |
|---|---|---|---|---|---|---|
| $CH_3$ | H | $CON(CH_3)_2$ | $OCH_3$ | 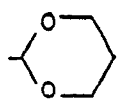 | N | |
| $CH_2CH_3$ | H | H | Cl | $OCH_3$ | CH | |
| $OCH_3$ | H | H | $CH_3$ | $CH_2OC_2H_5$ | CH | |
| $OCH_2CH_3$ | H | H | $OCH_3$ | $CH_3$ | CH | |
| Cl | H | H | $CH_3$ | $CH_3$ | CH | |
| Br | H | H | $OCF_2H$ | $CH_3$ | CH | |
| $SO_2N(CH_2CH_3)_2$ | H | H | $CH_2F$ | $OCH_3$ | CH | |
| $SO_2N(CH_3)_2$ | $CH_3$ | H | $OCH_2CH_3$ | $OCH_3$ | N | |
| $SO_2N(OCH_3)CH_3$ | H | H | $CF_3$ | $OCH_3$ | CH | |
| $SCH_2CH{=}CH_2$ | H | H | $OCH_3$ | $OCH_3$ | CH | |
| $SO_2C_2H_5$ | H | $CO_2CH_2CH_3$ | $CH_3$ | $OCH_3$ | CH | |
| S-*n*-$C_3H_7$ | H | H | $OCH_3$ | $C_2H_5$ | N | |
| $SO_2CH_3$ | H | H | $OCH_3$ | $OC_2H_5$ | N | |
| $SCH_3$ | H | H | $OCH_3$ | $SCF_2H$ | N | |
| $CH_3$ | H | H | $OCH_3$ | $C(CH_3)(SCH_3)_2$ | CH | |
| F | H | H | $OCH_2CH_3$ | $OCF_2H$ | CH | |
| Cl | H | H | $CH_3$ | $CH(OC_2H_5)_2$ | CH | |
| Br | H | H | $CH_3$ | $CH_3$ | N | |
| $OCH_3$ | H | H | $CH_3$ | $CH_3$ | N | |

Table 28

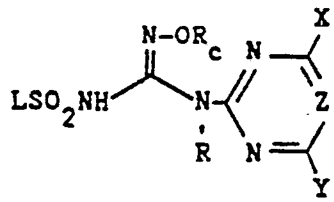

| L | $R_9$ | R | $R_c$ | X | Y | Z | m.p. (°C) |
|---|---|---|---|---|---|---|---|
| $L_4$ | $CH_3$ | H | H | $CH_3$ | $OCH_3$ | CH | |
| $L_4$ | F | H | $CON(CH_3)_2$ | $OCH_3$ | $OCH_3$ | N | |
| $L_4$ | $NO_2$ | H | H | Cl | $OCH_3$ | CH | |
| $L_4$ | $CO_2C_2H_5$ | H | H | $CH_3$ | $OCH_3$ | CH | |
| $L_4$ | $SO_2N(CH_3)_2$ | H | H | $OCH_3$ | $CH_2SCH_3$ | CH | |
| $L_5$ | $n$-$C_3H_7$ | H | H | $CH_3$ | $N(CH_3)_2$ | CH | |
| $L_5$ | Cl | H | H | $OCF_2H$ | $CH_2OCH_3$ | CH | |
| $L_5$ | Br | H | H | $CH_2F$ | $CH_2OCH_3$ | CH | |
| $L_5$ | $CO_2CH_3$ | $CH_3$ | H | $OCH_2CH_3$ | $CH_2OCH_3$ | N | |
| $L_5$ | $SO_2N(C_2H_5)_2$ | H | H | $CF_3$ | $CH_3$ | CH | |
| $L_5$ | $SO_2N(OCH_3)CH_3$ | H | H | $OCH_3$ | $CH_3$ | CH | |
| $L_5$ | $SO_2C_2H_5$ | H | $CO_2CH_2CH_3$ | $CH_3$ | $CH_3$ | CH | |
| $L_5$ | $SCH_2CH=CH_2$ | H | H | $OCH_3$ | $CH_3$ | N | |
| $L_5$ | $SO_2CH_3$ | H | H | $OCH_3$ | 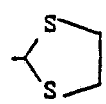 | N | |
| $L_6$ | F | H | H | $OCH_3$ | $CH(OCH_3)_2$ | CH | |
| $L_6$ | $CO_2$-$i$-$C_3H_7$ | H | H | $OCH_2CH_3$ | $OCH_3$ | CH | |
| $L_6$ | $SO_2CH_3$ | H | H | $CH_3$ | $OCH_3$ | CH | |
| $L_6$ | $SO_2N(CH_3)_2$ | H | H | $CH_3$ | $OCH_3$ | N | |
| $L_6$ | Br | H | H | $CH_3$ | $OCH_3$ | N | |

Table 29

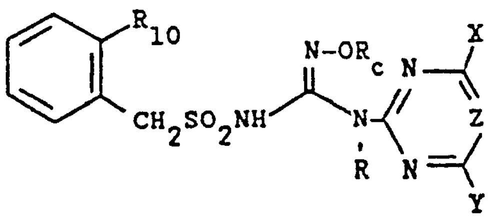

| $R_{10}$ | R | $R_c$ | X | Y | Z | m.p. (°C) |
|---|---|---|---|---|---|---|
| Cl | H | H | $CH_3$ | $CH_3$ | CH | |
| $NO_2$ | H | $CON(CH_3)_2$ | $OCH_3$ | $CH_3$ | N | |
| $CO_2CH_2CH_3$ | H | H | $CH_3$ | $CH_3$ | CH | |
| $SO_2N(CH_3)_2$ | H | H | $OCH_3$ | $CH_3$ | CH | |
| $OSO_2CH_3$ | H | H | F | $OCH_3$ | CH | |
| $SO_2CH_3$ | H | H | $CH_3$ | $OCH_3$ | CH | |
| $SO_2CH_2CH_3$ | H | H | $OCF_2H$ | $OCH_3$ | CH | |
| $OCH_3$ | H | H | $CH_2F$ | $OCH_3$ | CH | |
| $OCH_2CH_3$ | $CH_3$ | H | $OCH_2CH_3$ | $OCH_3$ | N | |
| $OCH_2CH_3$ | H | H | $CF_3$ | $CH_2SCH_3$ | CH | |
| $OCH_2CH_3$ | H | H | $OCH_3$ | $CF_3$ | CH | |
| $OCH_3$ | H | $CO_2CH_2CH_3$ | $CH_3$ | $CH_2CH_3$ | CH | |
| $SO_2CH_2CH_3$ | H | H | $OCH_3$ | $OCH_3$ | N | |
| $SO_2CH_3$ | H | H | $OCH_3$ | $SCF_2H$ | N | |
| $OSO_2CH_3$ | H | H | $OCH_3$ | $CH_3$ | N | |
| $CO_2CH_2CH_3$ | H | H | $OCH_3$ | $CH_3$ | CH | |
| $CO_2CH_3$ | H | H | $OCH_2CH_3$ | $CH_3$ | CH | |
| $CO_2CH_3$ | H | H | $CH_3$ | $CH_3$ | CH | |
| $CO_2CH_3$ | H | H | $CH_3$ | $OCH_3$ | N | |
| $NO_2$ | H | H | $CH_3$ | $OCH_3$ | N | |

Table 30

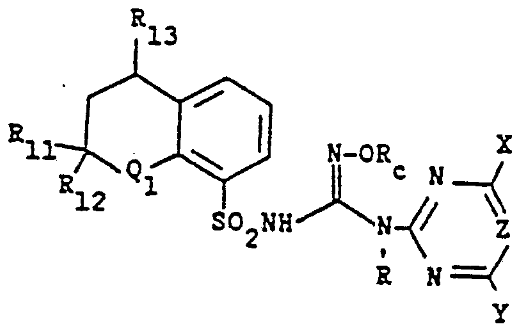

| $Q_1$ | $R_{11}$ | $R_{12}$ | $R_{13}$ | R | $R_c$ | X | Y | Z | m.p. (°C) |
|---|---|---|---|---|---|---|---|---|---|
| O | H | H | H | H | H | $CH_3$ | $OCH_3$ | CH | |
| S | H | H | H | H | $CON(CH_3)_2$ | $OCH_3$ | $OCH_3$ | N | |
| $SO_2$ | H | H | H | H | H | Cl | $OCH_3$ | CH | |
| NH | H | H | H | H | H | $CH_3$ | $OCH_3$ | CH | |
| $NCH_3$ | H | H | H | H | H | $OCH_3$ | $CH_3$ | CH | |
| S | $CH_3$ | $CH_3$ | H | H | H | $CH_3$ | $CH_3$ | CH | |
| $SO_2$ | $CH_3$ | $CH_3$ | H | H | H | $OCF_2H$ | $CH_3$ | CH | |
| NH | $CH_3$ | $CH_3$ | H | H | H | $CH_2F$ | $OCH_2CH_3$ | CH | |
| O | $CH_2CH_3$ | $CH_3$ | H | H | H | $CF_3$ | $OCH_2CH_3$ | CH | |
| S | $CH_2CH_3$ | $CH_3$ | H | H | H | $OCH_3$ | $CF_3$ | CH | |
| $SO_2$ | $CH_2CH_3$ | $CH_2CH_3$ | H | H | $CO_2CH_2CH_3$ | $CH_3$ | $OCF_2H$ | CH | |
| NH | $CH_2CH_3$ | $CH_3$ | H | H | H | $OCH_3$ | $OCH_3$ | N | |
| $NCH_3$ | $CH_2CH_3$ | $CH_3$ | H | H | H | $OCH_3$ | $OCH_3$ | N | |
| O | H | $CH_3$ | H | H | H | $OCH_3$ | $OCH_3$ | N | |
| S | H | $CH_3$ | H | H | H | $OCH_3$ | $OCH_3$ | CH | |
| $SO_2$ | H | $CH_3$ | H | H | H | $OCH_2CH_3$ | $OCH_3$ | CH | |
| NH | H | $CH_3$ | H | H | H | $CH_3$ | $C(CH_3)(OCH_3)_2$ | CH | |
| $NCH_3$ | H | $CH_3$ | H | H | H | $CH_3$ | $CH_3$ | N | |
| O | $CH_3$ | $CH_3$ | H | H | H | $CH_3$ | $CH_3$ | N | |

Table 31

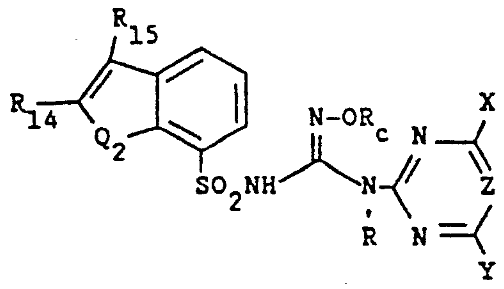

| $Q_2$ | $R_{14}$ | $R_{15}$ | R | $R_c$ | X | Y | Z | m.p. (°C) |
|---|---|---|---|---|---|---|---|---|
| O | H | H | H | H | $CH_3$ | $CH_3$ | CH | |
| O | H | $CH_3$ | H | $CON(CH_3)_2$ | $OCH_3$ | $CH_3$ | N | |
| O | H | $CH_3$ | H | H | $CH_3$ | $CH_3$ | CH | |
| O | $CH_3$ | H | H | H | $OCH_3$ | $CH(OCH_3)_2$ | CH | |
| O | $CH_3$ | $CH_3$ | H | H | F | $OCH_3$ | CH | |
| O | $CH_3$ | H | H | H | $CH_3$ | $OCH_3$ | CH | |
| S | H | $CH_3$ | H | H | $OCF_2H$ | $OCH_3$ | CH | |
| S | H | H | H | H | $CH_2F$ | $OCH_3$ | CH | |
| S | H | $CH_3$ | $CH_3$ | H | $OCH_2CH_3$ | $CH_2SCH_3$ | N | |
| S | H | H | H | H | $CF_3$ | $OCH_2CH_3$ | CH | |
| S | $CH_3$ | $CH_3$ | H | H | $OCH_3$ | $OCH_3$ | CH | |
| S | $CH_3$ | H | H | $CO_2CH_2CH_3$ | $CH_3$ | $OCH_3$ | CH | |
| S | $CH_3$ | $CH_3$ | H | H | $OCH_3$ | $OCH_3$ | N | |
| NH | H | H | H | H | $OCH_3$ | $OCH_3$ | N | |
| NH | $CH_3$ | $CH_3$ | H | H | $OCH_3$ | $OCH_3$ | N | |
| NH | H | H | H | H | $OCH_3$ | $OCH_3$ | CH | |
| $NCH_3$ | H | $CH_3$ | H | H | $OCH_2CH_3$ | $CH_3$ | CH | |
| $NCH_3$ | $CH_3$ | H | H | H | $CH_3$ | $CH_3$ | CH | |
| $NCH_3$ | $CH_3$ | $CH_3$ | H | H | $CH_3$ | $CH_3$ | N | |
| $NCH_3$ | $CH_3$ | H | H | H | $CH_3$ | $CH_3$ | N | |

Table 32

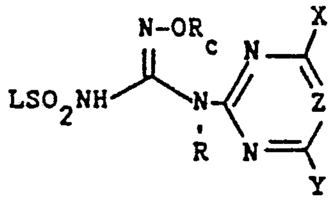

| L | m | $R_{13}$ | $R_{16}$ | R | $R_c$ | X | Y | Z | m.p. (°C) |
|---|---|---|---|---|---|---|---|---|---|
| L-10 | 0 | H | $CH_3$ | H | H | $CH_3$ | $CH_3$ | CH | |
| L-10 | 1 | H | $CH_3$ | H | $CON(CH_3)_2$ | $OCH_3$ | $CH_3$ | N | |
| L-11 | 0 | $CH_3$ | - | H | H | $CH_3$ | $CH_3$ | CH | |
| L-11 | 0 | $CH_3$ | - | H | H | $OCH_3$ | $CH(OCH_3)_2$ | CH | |
| L-11 | 0 | $CH_3$ | - | H | H | F | $OCH_3$ | CH | |
| L-11 | 0 | H | - | H | H | $CH_3$ | $OCH_3$ | CH | |
| L-12 | 1 | H | - | H | H | $OCF_2H$ | $OCH_3$ | CH | |
| L-12 | 0 | H | - | H | H | $CH_2F$ | $OCH_3$ | CH | |
| L-12 | 1 | H | - | $CH_3$ | H | $OCH_2CH_3$ | $CH_2SCH_3$ | N | |
| L-13 | 0 | $CH_3$ | - | H | H | $CF_3$ | $OCH_2CH_3$ | CH | |
| L-13 | 1 | H | - | H | H | $OCH_3$ | $OCH_3$ | CH | |
| L-13 | 0 | H | - | H | $CO_2CH_2CH_3$ | $CH_3$ | $OCH_3$ | CH | |
| L-13 | 1 | H | - | H | H | $OCH_3$ | $OCH_3$ | N | |
| L-14 | 0 | H | - | H | H | $OCH_3$ | $OCH_3$ | N | |
| L-14 | 1 | H | - | H | H | $OCH_3$ | $OCH_3$ | N | |
| L-14 | 0 | $CH_3$ | - | H | H | $OCH_3$ | $OCH_3$ | CH | |
| L-10 | 1 | H | $CH_3$ | H | H | $OCH_2CH_3$ | $CH_3$ | CH | |
| L-10 | 0 | H | $C_2H_5$ | H | H | $CH_3$ | $CH_3$ | CH | |
| L-10 | 1 | H | $C_2H_5$ | H | H | $CH_3$ | $CH_3$ | N | |
| L-10 | 0 | H | $C_2H_5$ | H | H | $CH_3$ | $CH_3$ | N | |

Table 33

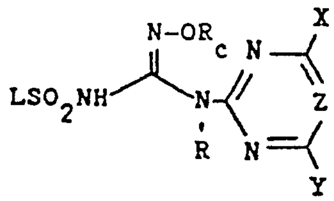

| $\underline{L}$ | $\underline{R_{17}}$ | $\underline{R_{18}}$ | $\underline{R}$ | $\underline{R_c}$ | $\underline{X}$ | $\underline{Y}$ | $\underline{Z}$ | m.p. (°C) |
|---|---|---|---|---|---|---|---|---|
| $L_{15}$ | H | – | H | H | $CH_3$ | $CH_3$ | CH | |
| $L_{15}$ | H | – | H | $CON(CH_3)_2$ | $OCH_3$ | $CH_3$ | N | |
| $L_{15}$ | H | – | H | H | $CH_3$ | $CH_3$ | CH | |
| $L_{15}$ | H | – | H | H | $OCH_3$ | $CH(OCH_3)_2$ | CH | |
| $L_{15}$ | H | – | H | H | F | $OCH_3$ | CH | |
| $L_{15}$ | H | – | H | H | $CH_3$ | $OCH_3$ | CH | |
| $L_{15}$ | $CH_3$ | – | H | H | $OCF_2H$ | $OCH_3$ | CH | |
| $L_{15}$ | $CH_3$ | – | H | H | $CH_2F$ | $OCH_3$ | CH | |
| $L_{15}$ | $CH_3$ | – | $CH_3$ | H | $OCH_2CH_3$ | $CH_2SCH_3$ | N | |
| $L_{16}$ | $CH_3$ | – | H | H | $CF_3$ | $OCH_2CH_3$ | CH | |
| $L_{16}$ | $CH_3$ | – | H | H | $OCH_3$ | $OCH_3$ | CH | |
| $L_{16}$ | $CH_3$ | – | H | $CO_2CH_2CH_3$ | $CH_3$ | $OCH_3$ | CH | |
| $L_{16}$ | $CH_3$ | – | H | H | $OCH_3$ | $OCH_3$ | N | |
| $L_{16}$ | H | – | H | H | $OCH_3$ | $OCH_3$ | N | |
| $L_{16}$ | H | – | H | H | $OCH_3$ | $OCH_3$ | N | |
| $L_{16}$ | H | – | H | H | $OCH_3$ | $OCH_3$ | CH | |
| $L_{17}$ | $CH_3$ | $CH_3$ | H | H | $OCH_2CH_3$ | $CH_3$ | CH | |
| $L_{17}$ | $CH_3$ | H | H | H | $CH_3$ | $CH_3$ | CH | |
| $L_{17}$ | $CH_3$ | $CH_3$ | H | H | $CH_3$ | $CH_3$ | N | |
| $L_{17}$ | $CH_3$ | $CH_3$ | H | H | $CH_3$ | $CH_3$ | N | |

Table 34

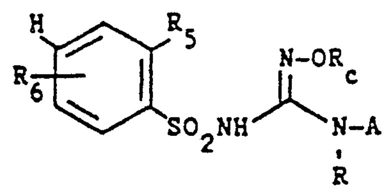

| $R_5$ | $R_6$ | R | $R_c$ | A |
|---|---|---|---|---|
| $CH_3$ | H | H | H | $A_2$: $Y_1=CH_2$; $X_1=CH_3$ |
| $i-C_3H_7$ | H | H | H | $A_2$: $Y_1=O$; $X_1=OCH_3$ |
| $OC_2H_5$ | H | H | H | $A_2$: $Y_1=CH_2$; $X_1=OCF_2H$ |
| $O-n-C_4H_9$ | H | H | H | $A_3$: $X_1=CH_3$ |
| F | $3-CF_3$ | H | $CO_2CH_3$ | $A_3$: $X_1=OCH_2CH_3$ |
| Cl | H | H | $CO_2CH_3$ | $A_4$: $X_1=OCH_3$; $Y_2=H$ |
| $NO_2$ | H | $CH_3$ | $CO_2CH_3$ | $A_4$: $X_1=CH_3$; $Y_2=CH_3$ |
| $CF_3$ | H | H | $CO_2CH_3$ | $A_5$: $X_2=CH_3$; $Y_3=OCH_3$ |
| $CO_2CH_3$ | H | H | $CO_2CH_3$ | $A_5$: $X_2=CH_3$; $Y_3=SCH_3$ |
| $CO_2CH_2CH_3$ | $3-CH_3$ | H | $CO_2CH_3$ | $A_5$: $X_2=CH_3$; $Y_3=C_2H_5$ |
| $SO_2N(CH_3)_2$ | H | H | $CO_2CH_3$ | $A_6$: $X_3=CH_3$ |
| $SO_2N(C_2H_5)_2$ | H | H | $CO_2CH_3$ | $A_6$: $X_3=OCH_3$ |
| $OSO_2CH_2CH_3$ | H | $CH_3$ | $CO_2CH_3$ | $A_2$: $Y_1=O$; $X_1=OCH_2CH_3$ |
| $SCH_3$ | 6-Cl | H | $CO_2CH_3$ | $A_2$: $Y_1=CH_2$; $X_1=OCH_3$ |
| $SO_2CF_3$ | H | H | H | $A_2$: $Y_1=O$; $X_1=CH_3$ |
| $OCHF_2$ | H | H | $COCH_3$ | $A_3$: $X_1=OCF_2H$ |
| $OCH_2CH=CH_2$ | H | H | H | $A_3$: $X_1=OCH_3$ |
| $C_6H_5$ | H | H | H | $A_4$: $X_1=OCH_3$; $Y_2=CH_3$ |
| 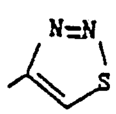 | H | H | H | $A_4$: $X_1=OCF_2H$; $Y_2=H$ |
| 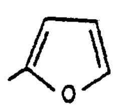 | H | H | H | $A_5$: $X_2=CH_2CF_3$; $Y_3=CH_3$ |
| $CH_2OCH_3$ | H | H | H | $A_6$: $X_3=OCH_3$ |
| $CH_2OCH_3$ | H | H | H | $A_6$: $X_3=CH_3$ |

Table 35

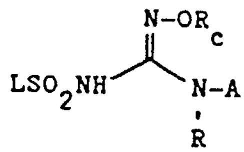

| $\underline{L}$ | $\underline{R}$ | $\underline{R_c}$ | $\underline{A}$ |
|---|---|---|---|
| L-2: $R_7=SO_2N(CH_3)_2$ | H | H | $A_2$: $Y_1=CH_2$; $X_1=CH_3$ |
| L-2: $R_7=OSO_2CH_3$ | H | H | $A_2$: $Y_1=O$; $X_1=OCH_3$ |
| L-2: $R_7=Cl$ | H | H | $A_2$: $Y_1=CH_2$; $X_1=OCF_2H$ |
| L-2: $R_7=Br$ | H | H | $A_3$: $X_1=CH_3$ |
| L-3: $R_8=CH_3$ | H | $CO_2CH_3$ | $A_3$: $X_1=OCH_2CH_3$ |
| L-3: $R_8=OCH_3$ | H | $CO_2CH_3$ | $A_4$: $X_1=OCH_3$; $Y_2=H$ |
| L-3: $R_8=SO_2CH_2CH_3$ | $CH_3$ | $CO_2CH_3$ | $A_4$: $X_1=CH_3$; $Y_2=CH_3$ |
| L-4: $R_9=CO_2CH_3$ | H | $CO_2CH_3$ | $A_5$: $X_2=CH_3$; $Y_3=OCH_3$ |
| L-5: $R_9=CO_2$-$\underline{i}$-$C_3H_7$ | H | $CO_2CH_3$ | $A_5$: $X_2=CH_3$; $Y_3=SCH_3$ |
| L-6: $R_9=Cl$ | H | $CO_2CH_3$ | $A_5$: $X_2=CH_3$; $Y_3=C_2H_5$ |
| L-4: $R_9=NO_2$ | H | $CO_2CH_3$ | $A_6$: $X_3=CH_3$ |
| L-5: $R_9=SO_2N(CH_3)_2$ | H | $CO_2CH_3$ | $A_6$: $X_3=OCH_3$ |
| L-6: $R_9=C_2H_5$ | $CH_3$ | $CO_2CH_3$ | $A_2$: $Y_1=O$; $X_1=OCH_2CH_3$ |
| L-7: $R_{10}=CO_2CH_3$ | H | $CO_2CH_3$ | $A_2$: $Y_1=CH_2$; $X_1=OCH_3$ |
| L-7: $R_{10}=SO_2N(CH_3)_2$ | H | H | $A_2$: $Y_1=O$; $X_1=CH_3$ |
| L-7: $R_{10}=OSO_2CH_3$ | H | $COCH_3$ | $A_3$: $X_1=OCF_2H$ |
| L-8: $R_{11}=R_{12}=R_{13}=CH_3$; m=0; $Q_1=S$ | H | H | $A_3$: $X_1=OCH_3$ |
| L-9: $R_{14}=CH_3$; $R_{15}=H$; $Q_2=NCH_3$ | H | H | $A_4$: $X_1=OCH_3$; $Y_2=CH_3$ |
| L-10: $R_{16}=C_2H_5$; $R_{13}=H$; m=1 | H | H | $A_4$: $X_1=OCF_2H$; $Y_2=H$ |
| L-11: $R_{13}=H$; m=0 | H | H | $A_5$: $X_2=C_2H_5$; $Y_3=OC_2H_5$ |
| L-12: $R_{13}=CH_3$; m=0 | H | H | $A_5$: $X_2=CH_2CF_3$; $Y_3=CH_3$ |
| L-13: $R_{13}=CH_3$; m=0 | H | H | $A_5$: $X_2=CH_3$; $Y_3=C_2H_5$ |
| L-14: $R_{16}=CH_3$ | H | H | $A_6$: $X_3=OCH_3$ |
| L-15: $R_{17}=H$ | H | H | $A_2$: $Y_1=CH_2$; $X_1=CH_3$ |
| L-15: $R_{17}=H$ | H | H | $A_2$: $Y_1=O$; $X_1=OCH_3$ |
| L-15: $R_{17}=H$ | H | H | $A_2$: $Y_1=CH_2$, $X_1=OCF_2H$ |

Table 35 (continued)

| L | R | R' | A |
|---|---|---|---|
| L-16: $R_{17}=CH_3$ | H | H | $A_3$: $X_1=CH_3$ |
| L-16: $R_{17}=CH_3$ | H | $CO_2CH_3$ | $A_3$: $X_1=OCH_2CH_3$ |
| L-16: $R_{17}=CH_3$ | H | $CO_2CH_3$ | $A_4$: $X_1=OCH_3$; $Y_2=H$ |
| L-17: $R_{17}=H$; $R_{18}=CH_3$ | $CH_3$ | $CO_2CH_3$ | $A_4$: $X_1=CH_3$; $Y_2=CH_3$ |
| L-17: $R_{17}=H$; $R_{18}=CH_3$ | H | $CO_2CH_3$ | $A_5$: $X_2=CH_3$; $Y_3=OCH_3$ |
| L-17: $R_{17}=H$; $R_{18}=CH_3$ | H | $CO_2CH_3$ | $A_5$: $X_2=CH_3$; $Y_3=SCH_3$ |
| L-8: $R_{11}=R_{12}=R_{13}=H$; m=1; $Q_1=SO_2$ | H | $CO_2CH_3$ | $A_5$: $X_2=CH_3$; $Y_3=C_2H_5$ |
| L-8: $R_{11}=R_{12}=R_{13}=H$; m=1; $Q_1=SO_2$ | H | $CO_2CH_3$ | $A_6$: $X_3=CH_3$ |
| L-8: $R_{11}=R_{12}=R_{13}=H$; m=1; $Q_1=SO_2$ | H | $CO_2CH_3$ | $A_6$: $X_3=OCH_3$ |
| L-9: $R_{14}=R_{15}=H$; $Q_2=S$ | $CH_3$ | $CO_2CH_3$ | $A_2$: $Y_1=O$; $X_1=OCH_2CH_3$ |
| L-9: $R_{14}=R_{15}=H$; $Q_2=S$ | H | $CO_2CH_3$ | $A_2$: $Y_1=CH_2$; $X_1=OCH_3$ |
| L-9: $R_{14}=R_{15}=H$; $Q_2=S$ | H | H | $A_2$: $Y_1=O$; $X_1=CH_3$ |
| L-10: $R_{13}=R_{16}=CH_3$; m=0 | H | $COCH_3$ | $A_3$: $X_1=OCF_2H$ |
| L-10: $R_{13}=R_{16}=CH_3$; m=0 | H | H | $A_3$: $X_1=OCH_3$ |
| L-10: $R_{13}=R_{16}=CH_3$; m=0 | H | H | $A_4$: $X_1=OCH_3$; $Y_2=CH_3$ |
| L-11: $R_{13}=CH_3$; m=0 | H | H | $A_4$: $X_1=OCF_2H$; $Y_2=H$ |
| L-11: $R_{13}=CH_3$; m=0 | H | H | $A_5$: $X_2=C_2H_5$; $Y_3=OC_2H_5$ |
| L-11: $R_{13}=CH_3$; m=0 | H | H | $A_5$: $X_2=CH_2CF_3$; $Y_3=CH_3$ |
| L-12: $R_{13}=H$; m=1 | H | H | $A_5$: $X_2=CH_3$; $Y_3=C_2H_5$ |
| L-13: $R_{13}=H$; m=1 | H | H | $A_6$: $X_3=OCH_3$ |
| L-14: $R_{16}=H$ | H | H | $A_6$: $X_3=CH_3$ |

Formulations

Useful formulations of the compounds of Formulas I and II can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

TABLE 36

| | Active Ingredient | Weight Percent* Diluent(s) | Surfactant(s) |
|---|---|---|---|
| Wettable Powders | 20—90 | 0—74 | 1—10 |
| Oil Suspensions, Emulsions, Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins. *et al.,* "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley and Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 10

*Wettable Powder*

| | |
|---|---|
| N-[(4,6-dimethoxypyrimidin-2-yl)aminocarbonyl]-2-dimethylamino-sulfonylbenzenesulfonamide, dimethylsulfonium inner salt | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 11

*Wettable Powder*

| | |
|---|---|
| N-[(4,6-dimethoxypyrimidin-2-yl)aminocarbonyl]-2-dimethylamino-sulfonylbenzenesulfonamide, dimethylsulfonium inner salt | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 12

*Granule*

| | |
|---|---|
| Wettable Powder of Example 11 | 5% |
| attapulgite granules | 95% |

(U.S.S. 20—40 mesh; 0.84—0.42 mm)

A slurry of wettable powder containing 25% solids is sprayed on the surface of attapulgite granules in a double-cone blender. The granules are dried and packaged.

Example 13

*Extruded Pellet*

| | |
|---|---|
| 2-[[(4,6-dimethoxypyrimidin-2-yl)aminocarbonyl]-2-dimethylamino-sulfonyl]benzoic acid methyl ester, dimethylsulfonium inner salt | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 14

*Oil Suspension*

| | |
|---|---|
| N-[(4,6-dimethoxypyrimidin-2-yl)aminocarbonyl]-2-dimethylamino-sulfonylbenzenesulfonamide, dimethylsulfonium inner salt | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 15

*Wettable Powder*

| | |
|---|---|
| 2-[[(4,6-dimethoxypyrimidin-2-yl)aminocarbonyl]-2-dimethylamino-sulfonyl]benzoic acid methyl ester, dimethylsulfonium inner salt | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 16

*Low Strength Granule*

| | |
|---|---|
| N-[(4,6-dimethylpyrimidin-2-yl)aminocarbonyl]-2-dimethylamino-sulfonylbenzenesulfonamide, dimethylsulfonium inner salt | 1% |
| N,N-dimethylformamide | 9% |
| attapulgite granules | 90% |
| (U.S.S. 20—40 sieve) | |

The active ingrdient is dissolved in the solvent and the solution is sprayed upon dedusted granules in a double cone blender. After spraying of the solution has been completed, the blender is allowed to run for a short period and then the granules are packaged.

Example 17

*Aqueous Suspension*

| | |
|---|---|
| N-[(4,6-dimethylpyrimidin-2-yl)aminocarbonyl]-2-dimethylamino-sulfonylbenzenesulfonamide, dimethylsulfonium inner salt | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 18

*High Strength Concentrate*

| | |
|---|---|
| N′,N′-dimethyl-N-[[1-(4-methoxy-6-methylpyrimidin-2-yl)amino]-1-(hydroxyimino)methyl]-1,2-benzenedisulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 19

*Wettable Powder*

| | |
|---|---|
| N′,N′-dimethyl-N-[[1-(4,6-dimethyl-1,3,5-triazin-2-yl)amino]-1-(hydroxyimino)methyl]-1,2-benzenedisulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 20

*Wettable Powder*

| | |
|---|---|
| 2-[[1-(4,6-dimethylpyrimidin-2-yl)amino]-1-(hydroxyimino)-methyl]-aminosulfonyl]benzoic acid, methyl ester | 40% |
| sodium ligninsulfonate | 20% |
| montmorillonite clay | 40% |

The ingredients are thoroughly blended, coarsely hammer-milled and then air-milled to produce particles esentially all below 10 microns in size. The material is reblended and then packaged.

Example 21

*Oil Suspension*

| | |
|---|---|
| N′,N′-dimethyl-N-[[1-(dimethyl-1,3,5-triazin-2-yl)amino]-1-(hydroxyimino)methyl]-1,2-benzenedisulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 22

*Dust*

| | |
|---|---|
| 2-[[1-(4-methoxy-6-methylpyrimidin-2-yl)amino]-1-(hydroxyimino)methyl]aminosulfonyl]benzoic acid, methyl ester | 10% |
| attapulgite | 10% |
| Pyrophyllite | 80% |

The active ingredient is blended with attapulgite and then passed through a hammer-mill to produce particles substantially all below 200 microns.. The ground concentrate is then blended with powdered pyrophyllite until homogeneous.

Example 23

*Emulsifiable Concentrate*

| | |
|---|---|
| N',N'-dimethyl-N-[[1-(4-methoxy-6-methylpyrimidin-2-yl)-amino]-1-(hydroxyimino)methyl]-1,2-benzenedisulfonamide | 20% |
| chlorobenzene | 74% |
| sorbitan monostearate and polyoxyethylene condensates thereof | 6% |

The ingredients are combined and stirred to produce a solution which can be emulsified in water for application.

*Utility*

The compounds of the present invention are active herbicides. They have utility for broad-spectrum pre-and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for the selective pre- or post-emergence weed control in crops, such as wheat, rice, and barley.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.03 to 10 kg/ha, the lower rates being suggested for use on lighter soils and/or those having a low organic matter content, for selective weed control or for situations where only short-term persistence is required. For plant growth regulant use, the compounds should be applied to the crop in an effective but substantially non-phytotoxic amount.

The compounds of the invention may be used in combination with any other commercial herbicide examples of which are those of the triazine, triazole, uracil, urea, amide, diphenylether, carbamate and bipyridylium types.

The herbicidal properties of the subject compounds were discovered in a number of greenhouse tests. The test procedures and results follow.

*Test A*

Seeds of crabgrass *(Digitaria* spp.*)*, barnyardgrass *(Echinochloa crusgalli)*, wild oats *(Avena fatua)*, sicklepod *(Cassia obtusifolia)*, morningglory *(Ipomoea* spp.*)*, cocklebur *(Xanthium pensylvanicum)*, sorghum, corn, soybean, sugar beet, rice, wheat, and purple nutsedge *(Cyperus rotundus)* tubers were planted and treated preemergence with the test chemicals dissolved in a non-phytotoxic solvent. At the same time, these crop and weed species, along with cotton and bush bean, were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis;
E = emergence inhibition;
G = growth retardation;
H = formative effect;
U = unusual pigmentation; and
6Y = abscised buds or flowers.

Note that compounds tested are highly active herbicides at the low rates of application selected for this test.

Compounds

Compound 1

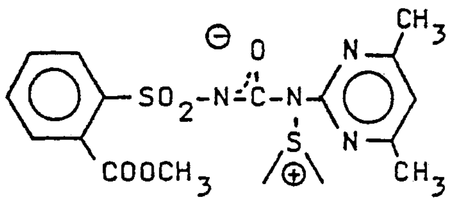

Compound 2

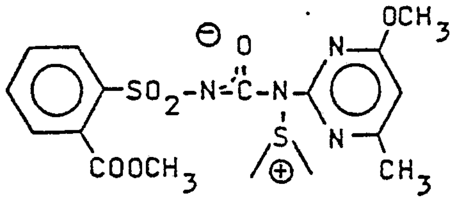

Compound 3

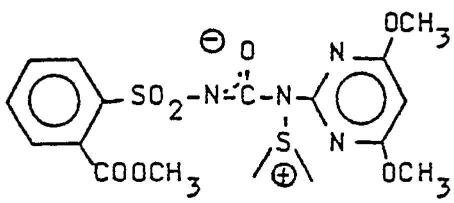

Compound 4

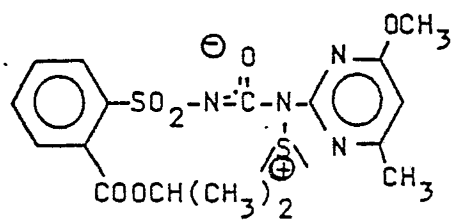

Compound 5

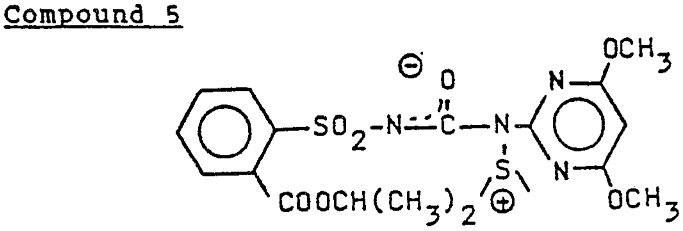

Compounds (continued)

Compound 6

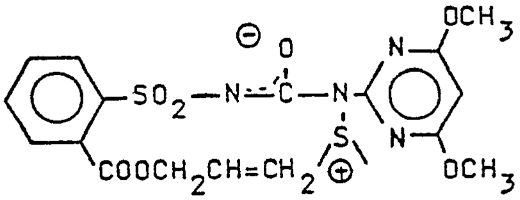

Compound 7

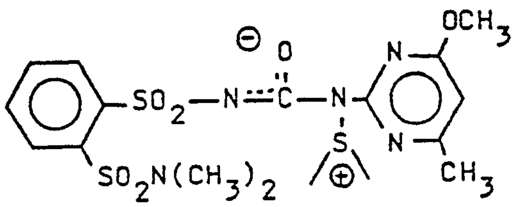

Compound 8

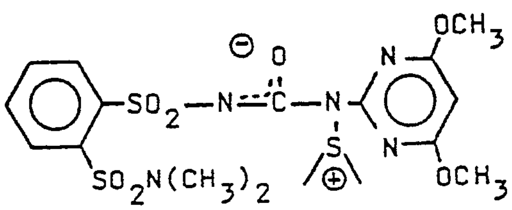

Compound 9.

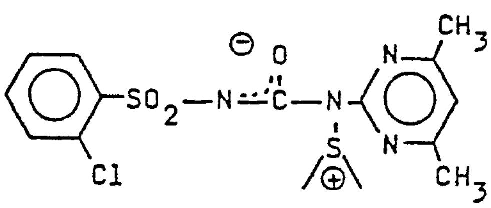

Compound 10

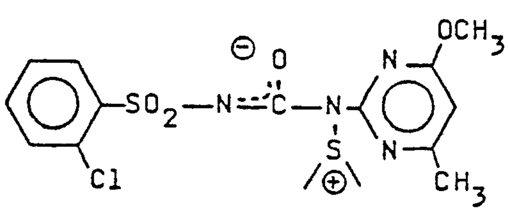

Compounds (continued)

Compound 11

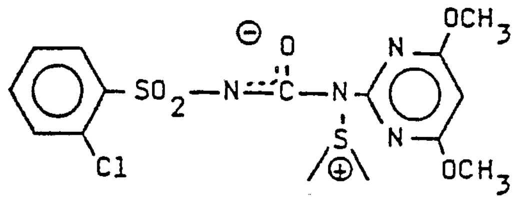

Compound 12

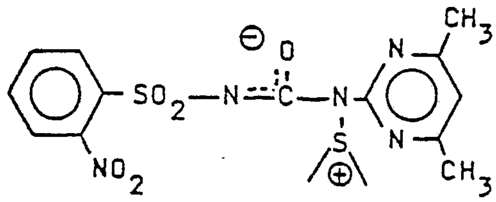

Compound 13

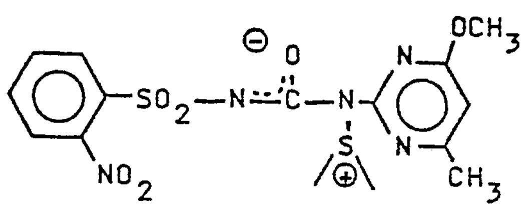

Compound 14

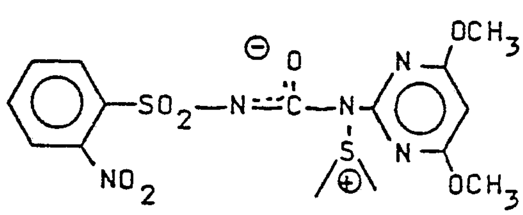

Compound 15

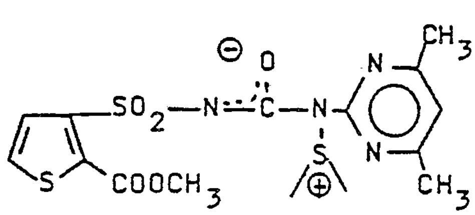

Compounds (continued)

Compound 16

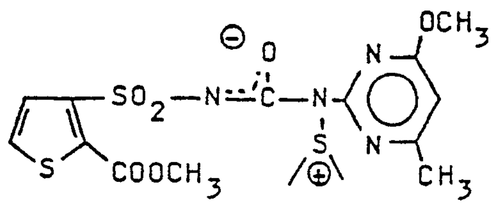

Compound 17

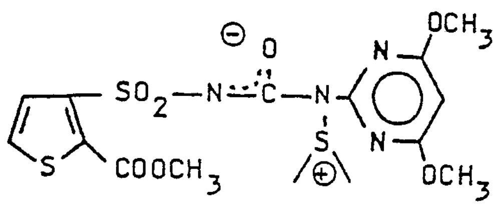

Compound 18

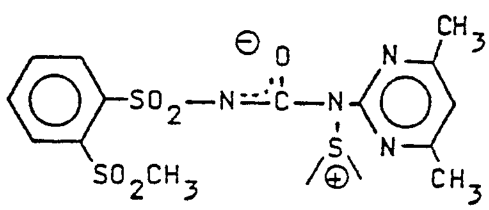

Compound 19

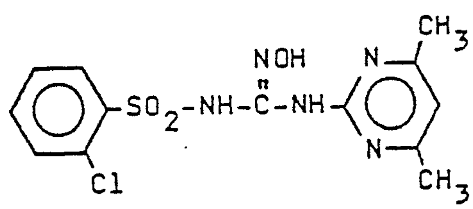

Compound 20

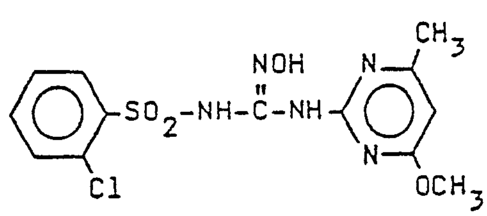

Compound 21

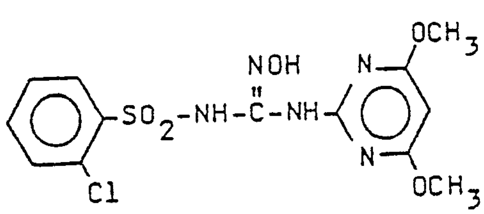

Compounds (continued)

Compound 22

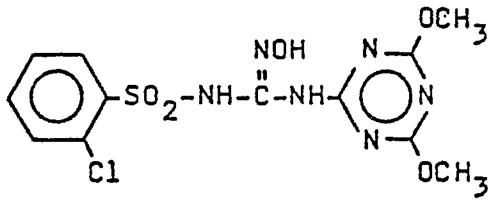

Compound 23

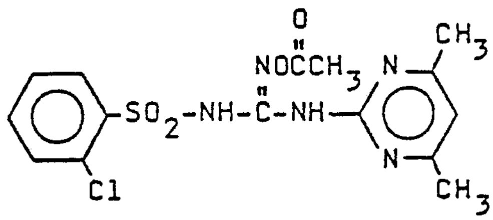

Compound 24

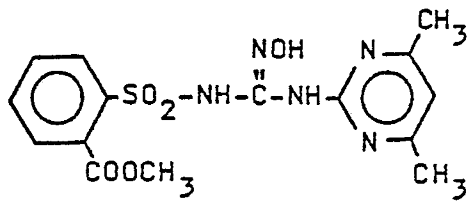

Compound 25

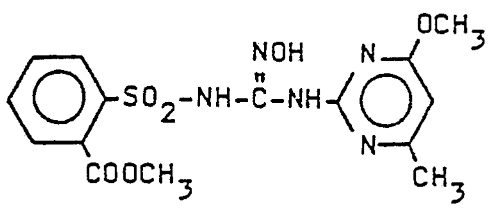

Compound 26

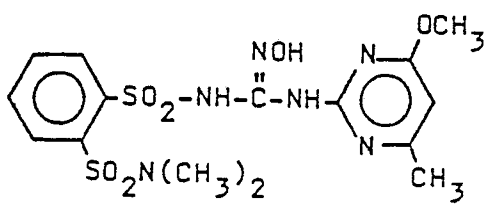

Compound 27

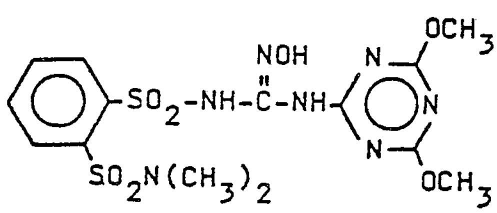

Table A

| | Cmpd. 1 | Cmpd. 2 | Cmpd. 3 | Cmpd. 4 |
|---|---|---|---|---|
| Rate kg/ha | 0.4 | 0.4 | 0.4 | 0.4 |
| POST-EMERGENCE | | | | |
| Bush bean | 9C | 9C | 9C | 7C,9G,6Y |
| Cotton | 7C,9G | 9C | 5C,9G | 5C,9G |
| Morningglory | 10C | 10C | 9C | 10C |
| Cocklebur | 10C | 9C | 2C,7G | 5C,9G |
| Sicklepod | 9C | 10C | 10C | 6C,9G |
| Nutsedge | 9C | 9C | 10C | 1C,9G |
| Crabgrass | 9C | 9C | 4C,7G | 1C,7G |
| Barnyardgrass | 9C | 9C | 9C | 9C |
| Wild Oats | 9C | 5C,9G | 1C,2G | 1C,9G |
| Wheat | 6C,9G | 5U,9G | 1C,3G | 2C,9G |
| Corn | 10C | 9C | 7U,10C | 2C,9G |
| Soybean | 9C | 9C | 9C | 4C,9G |
| Rice | 9C | 9C | 6C,9G | 5C,9G |
| Sorghum | 10C | 10C | 9C | 3C,9G |
| Sugar beet | - | - | - | - |
| PRE-EMERGENCE | | | | |
| Morningglory | 2C,9G | 9C | 8G | 9G |
| Cocklebur | 9H | 9H | 9H | 9H |
| Sicklepod | 2C,9G | 9G | 2C,8G | 9G |
| Nutsedge | 10E | 10E | 8G | 10E |
| Crabgrass | 6C,9G | 3C,8G | 2C,5G | 1C,4G |
| Barnyardgrass | 6C,9H | 5C,9H | 6C,9H | 4C,9H |
| Wild Oats | 4C,9H | 5C,9H | 2C,8G | 2C,9G |
| Wheat | 10E | 6C,9H | 2C,9G | 1C,9H |
| Corn | 10E | 9H | 4C,8G | 2C,9G |
| Soybean | 9H | 9H | 1C,4H | 9H |
| Rice | 10E | 10E | 10E | 9H |
| Sorghum | 10H | 5C,9H | 3C,8G | 5C,9H |
| Sugar beet | - | - | - | - |

Table A (continued)

| | Cmpd. 5 | Cmpd. 6 | Cmpd. 7 | Cmpd. 8 |
|---|---|---|---|---|
| Rate kg/ha | 0.4 | 0.4 | 0.4 | 0.4 |
| POST-EMERGENCE | | | | |
| Bush bean | 6C,9G,6Y | 9D,9G,6Y | 9C | 8D,8G,6Y |
| Cotton | 4C,9G | 1C,5G | 9C | 6C,9G |
| Morningglory | 1C,4G | 1C,5G | 9C | 3C,7G |
| Cocklebur | 3C,8H | 2C,9H | 10C | 3C,9H |
| Sicklepod | 3C,9G | 2C,6G | 9C | 6C,9G |
| Nutsedge | 7C,9G | 3C,8G | 5C,9G | 10C |
| Crabgrass | 2G | 3H | 6C,9G | 5C,9G |
| Barnyardgrass | 2C,7H | 3C,9H | 9C | 6C,9H |
| Wild Oats | 0 | 0 | 9C | 1C |
| Wheat | 0 | 0 | 5C,9G | 1C,9H |
| Corn | 2C,7H | 2H,9G | 5U,9C | 3U,9G |
| Soybean | 3H | 2C,2H | 9C | 9C |
| Rice | 7G | 2C,9G | 6C,9G | 6C,9G |
| Sorghum | 3C,9G | 2C,9H | 10C | 6C,9G |
| Sugar beet | - | - | - | - |
| PRE-EMERGENCE | | | | |
| Morningglory | 8G | 2C,5H | 9C | 7G |
| Cocklebur | - | 9H | 9H | 9H |
| Sicklepod | 2C,9G | 1C,4G | 9G | 1C,8G |
| Nutsedge | 1C,5G | 2C,8G | 10E | 1C,7G |
| Crabgrass | 1C | 1C | 3C,9G | 3C,4G |
| Barnyardgrass | 3C,5H | 2C | 5C,9H | 6C,9H |
| Wild Oats | 1C | 0 | 5C,9G | 3C,9G |
| Wheat | 0 | 0 | 5C,9H | 2C,9G |
| Corn | 3C,7G | 2C,7G | 9H | 5C,8G |
| Soybean | 1C | 0 | 9H | 2C,6G |
| Rice | 3C,6G | 3C,8G | 10E | 10E |
| Sorghum | 1C,3G | 1C | 8C,9H | 2C,9H |
| Sugar beet | - | - | - | - |

Table A (continued)

| | Cmpd. 9 | Cmpd. 10 | Cmpd. 11 | Cmpd. 12 |
|---|---|---|---|---|
| Rate kg/ha | 0.4 | 0.4 | 0.4 | 0.4 |
| POST-EMERGENCE | | | | |
| Bush bean | 6C,9G,6Y | 9C | 4C,8G,6Y | 3C,7G,6Y |
| Cotton | 6C,9G | 9C | 3C,3H,9G | 2C,8G |
| Morningglory | 9C | 9C | 5C,8G | 2C,7G |
| Cocklebur | 10C | 4C,9G | 4G | 7G |
| Sicklepod | 9C | 9C | 3C,9G | 2C |
| Nutsedge | 10C | 3C,8G | 5C,9G | 10C |
| Crabgrass | 7C,9G | 2C,8G | 0 | 5C,8G |
| Barnyardgrass | 9C | 9C | 0 | 4C,6H |
| Wild Oats | 3C,9G | 2C,9G | 0 | 0 |
| Wheat | 1C,9G | 4C,9G | 0 | 0 |
| Corn | 3U,9C | 4U,9C | 2C,5G | 1U,8H |
| Soybean | 4C,9G | 5C,9G | 5C,9G | 1C,7H |
| Rice | 6C,9G | 5C,9G | 2G | 2C,9G |
| Sorghum | 9C | 4C,9G | 2G | 1C,6H |
| Sugar beet | - | - | - | - |
| PRE-EMERGENCE | | | | |
| Morningglory | 9G | 9G | 1C,7G | 1C,7G |
| Cocklebur | 9H | 9H | 5C,9H | 2C,9H |
| Sicklepod | 2C,9G | 9G | 3C,9G | 2C,8G |
| Nutsedge | 10E | 5G | 10E | 8G |
| Crabgrass | 2C,7G | 1C,4G | 3C | 1C,4G |
| Barnyardgrass | 1C,9H | 9H | 5C,6H | 6C,7H |
| Wild Oats | 2C,9G | 9G | 0 | 7G |
| Wheat | 2C,9G | 9G | 0 | 4G |
| Corn | 10E | 4C,9G | 3C,5G | 2C,7G |
| Soybean | 3C,9H | 3C,8H | 2C,3H | 2C,4H |
| Rice | 10E | 10E | 5C,8H | 3C,9H |
| Sorghum | 7C,9H | 3C,9H | 0 | 2C,5G |
| Sugar beet | - | - | - | - |

Table A (continued)

| | Cmpd. 13 | Cmpd. 14 | Cmpd. 15 | Cmpd. 16 |
|---|---|---|---|---|
| Rate kg/ha | 0.4 | 0.4 | 0.4 | 0.4 |
| POST-EMERGENCE | | | | |
| Bush bean | 4C,8G,6Y | 2C,6G,6Y | 7C,9G,6Y | 7C,9G,6Y |
| Cotton | 3C,7G | 5C,9G | 6C,9G | 9C |
| Morningglory | 2C,8G | 2C,8G | 2C,7G | 5C,9G |
| Cocklebur | 5G | 2C | 5C,9G | 6C,9G |
| Sicklepod | 3G | 6C,9G | 1C | 1C,1H |
| Nutsedge | 5G | 5C,9G | 9C | 2C,9G |
| Crabgrass | 3G | 0 | 2C,9H | 5C,9G |
| Barnyardgrass | 7H | 0 | 5C,9H | 6C,9H |
| Wild Oats | 2G | 0 | 1C | 5C,9H |
| Wheat | 3G | 0 | 2C,9G | 10C |
| Corn | 2C,9H | 3C,8H | 1U,9H | 3C,9G |
| Soybean | 2H,6G | 3C,8H | 3G | 1H,3G |
| Rice | 9G | 0 | 3C,9G | 5C,9G |
| Sorghum | 2C,9H | 0 | 4C,9H | 2C,9G |
| Sugar beet | - | - | - | - |
| PRE-EMERGENCE | | | | |
| Morningglory | 9G | 9G | 8G | 5C,9G |
| Cocklebur | 9H | 9H | 9H | 9H |
| Sicklepod | 9G | 9G | 3G | 5G |
| Nutsedge | 8G | 8G | 2C,8G | 9G |
| Crabgrass | 2G | 2C | 2C,7G | 2C,8G |
| Barnyardgrass | 5C,9G | 2C,3H | 9H | 3C,9H |
| Wild Oats | 2C,7G | 0 | 2C,6G | 8G |
| Wheat | 4G | 0 | 9H | 3C,9H |
| Corn | 2C,8H | 2C,6G | 9H | 2C,9H |
| Soybean | 2C,6G | 2C | 2G | 1C,6H |
| Rice | 8H | 4G | 10E | 10E |
| Sorghum | 2C,5H | 1C | 3C,9H | 2C,9H |
| Sugar beet | - | - | - | - |

Table A (continued)

| | Cmpd. 17 | Cmpd. 18 |
|---|---|---|
| Rate kg/ha | 0.4 | 0.4 |
| POST-EMERGENCE | | |
| Bush bean | 1C,1H | 9C |
| Cotton | 2C,3H,8G | 7C,9G |
| Morningglory | 1C,3G | 9C |
| Cocklebur | 4G | 6C,9G |
| Sicklepod | 2C | 9C |
| Nutsedge | 2C,4G | 10C |
| Crabgrass | 0 | 9C |
| Barnyardgrass | 2C,7H | 9C |
| Wild Oats | 0 | 9C |
| Wheat | 0 | 5C,9G |
| Corn | 2C,7H | 5U,9G |
| Soybean | 4H | 5C,9G |
| Rice | 6G | 6C,9G |
| Sorghum | 2C,9G | 10C |
| Sugar beet | - | - |
| PRE-EMERGENCE | | |
| Morningglory | 2C,5G | 3C,9G |
| Cocklebur | 9H | 2C,9H |
| Sicklepod | 2C | 5C,9H |
| Nutsedge | 2C,8G | 10E |
| Crabgrass | 2C | 2C,9G |
| Barnyardgrass | 5C,7H | 2C,9H |
| Wild Oats | 0 | 5C,9H |
| Wheat | 2C | 5C,9H |
| Corn | 2C,7G | 2C,9H |
| Soybean | 0 | 3C,9H |
| Rice | 10E | 10E |
| Sorghum | 6G | 6C,9H |
| Sugar beet | - | |

Table A (continued)

| | Cmpd. 19 | Cmpd. 19 | Cmpd. 20 | Cmpd. 21 |
|---|---|---|---|---|
| Rate kg/ha | .05 | 0.4 | .05 | .05 |
| POST-EMERGENCE | | | | |
| Bush bean | 3C,7H,6Y | 9C | 9C | 9C |
| Cotton | 5C,7H | 6C,9G | 4C,9G | 3C,9G |
| Morningglory | 3C | 4C,8G | 2C,9G | 3C,8H |
| Cocklebur | 3G | 8G | 3C,9G | 3C,8H |
| Sicklepod | 2C | 3C,8G | 2C,8G | 4C,9G |
| Nutsedge | 2C,6G | 10C | 4C,9G | 9C |
| Crabgrass | 0 | 2C,8G | 1C,3H | 2C,3G |
| Barnyardgrass | 2C,6H | 9C | 5C,9H | 2C,9H |
| Wild Oats | 1C | 1C,7G | 0 | 2C,4G |
| Wheat | 1C | 1C,9G | 0 | 3G |
| Corn | 2U,8H | 6U,9G | 3U,9G | 2U,9G |
| Soybean | 1C,3H | 4C,9G | 2C,9H | 3C,9H |
| Rice | 2C,8H | 5C,9G | 3C,9G | 4C,9G |
| Sorghum | 1U,9G | 5U,9G | 2C,9G | 2C,9G |
| Sugar beet | - | - | - | - |
| PRE-EMERGENCE | | | | |
| Morningglory | 3C | 8G | 9G | 9G |
| Cocklebur | 2C,7H | 8H | 9H | - |
| Sicklepod | 2C,5G | 3C,9G | 2C,8G | 9G |
| Nutsedge | 5G | 3C,9G | 2C,5G | 10E |
| Crabgrass | 0 | 3C | 1C,3G | 1C,5G |
| Barnyardgrass | 3C,6H | 3C,9H | 5C,9H | 9H |
| Wild Oats | 0 | 2C,7G | 5C,9G | 8G |
| Wheat | 3G | 2C,8G | 4C,9G | 2C,8G |
| Corn | 2C,6G | 1C,9G | 2C,9G | 2C,9H |
| Soybean | 2C | 3C,6G | 3C,8H | 7H |
| Rice | 4C,6G | 10E | 5C,9G | 10E |
| Sorghum | 2C,7H | 3C,9H | 4C,9G | 1C,9H |
| Sugar beet | - | - | - | - |

Table A (continued)

| | Cmpd. 22 | Cmpd. 23 | Cmpd. 24 | Cmpd. 25 |
|---|---|---|---|---|
| Rate kg/ha | .05 | .05 | .05 | .05 |
| POST-EMERGENCE | | | | |
| Bush bean | 1C | 5C,9G,6Y | 3C,6H,6Y | 9C |
| Cotton | 4C,8G | 4C,8H | 2C,5G | 6C,9G |
| Morningglory | 3C,8G | 3C,5G | 3G | 2C,7G |
| Cocklebur | 2C | 5G | 4G | 4C,9H |
| Sicklepod | 5C,9G | 1C | 3G | 9C |
| Nutsedge | 0 | 3C,8G | 7C,9G | 10C |
| Crabgrass | 3C | 2H | 10C | 4C,9G |
| Barnyardgrass | 2C,5H | 3C,8H | 4H | 9C |
| Wild Oats | 0 | 2C | 0 | 5C,9H |
| Wheat | 0 | 1C | 0 | 5C,9G |
| Corn | 2G | 3C,8H | 2C,8H | 5C,9G |
| Soybean | 3C,8G | 2C,7H | 2C,2H | 5C,9G |
| Rice | 3G | 3C,9H | 2C,8H | 5C,9G |
| Sorghum | 3G | 3C,9G | 2C,9H | 4U,9G |
| Sugar beet | - | - | 2C | 10C |
| PRE-EMERGENCE | | | | |
| Morningglory | 9G | 3C | 2C,4G | 8H |
| Cocklebur | 9H | 9H | 9H | 8H |
| Sicklepod | 9G | 3C,8H | 2C,5G | 2C,9G |
| Nutsedge | 9G | 3C,8G | 2C,5G | 10E |
| Crabgrass | 3G | 2C | 1C | 2C |
| Barnyardgrass | 2C,5G | 5C,9H | 2H | 4C,9H |
| Wild Oats | 1C | 2C,8G | 2C,8G | 3C,9G |
| Wheat | 1C | 3C,9G | 9G | 2C,9H |
| Corn | 2C,8G | 4C,9H | 9G | 3C,9H |
| Soybean | 8H | 3C,5H | 4C,7G | 3C,7H |
| Rice | 6G | 5C,9H | 3C,8H | 10E |
| Sorghum | 2C,5G | 4C,9H | 2C,9H | 2C,9G |
| Sugar beet | - | - | 2C,9G | 9C |

Table A (continued)

| | Cmpd. 26 | Cmpd. 27 |
|---|---|---|
| Rate kg/ha | .05 | .05 |
| POST-EMERGENCE | | |
| Bush bean | 5C,9G,6Y | 5C,9G,6Y |
| Cotton | 9C | - |
| Morningglory | 5C,9G | 5C,9G |
| Cocklebur | 4C,9G | 4C,9G |
| Sicklepod | 3C,8G | 4C,3H |
| Nutsedge | 2C,7G | 5G |
| Crabgrass | 2C,9G | 2C,8G |
| Barnyardgrass | 2C,9H | 5C,9H |
| Wild Oats | 2C,8G | 0 |
| Wheat | 5C,9G | 0 |
| Corn | 3U,9H | 9C |
| Soybean | 2C,9H | 9C |
| Rice | 5C,9G | 5C,9G |
| Sorghum | 2C,9G | 9C |
| Sugar beet | 3C,9G | 9C |
| PRE-EMERGENCE | | |
| Morningglory | 9C | 9C |
| Cocklebur | 9H | - |
| Sicklepod | 5C,9G | 2C,9G |
| Nutsedge | 10E | 6G |
| Crabgrass | 3C,9G | 2C,6G |
| Barnyardgrass | 4C,9H | 3C,9H |
| Wild Oats | 3C,9G | 3C,9G |
| Wheat | 10C | 1C,6G |
| Corn | 9G | 5C,9H |
| Soybean | 3C,9H | 9H |
| Rice | 10E | 10E |
| Sorghum | 5C,9H | 5C,9H |
| Sugar beet | 5C,9G | 9C |

Several of the compounds of this invention were further tested in a pre-emergence herbicide screen as described in Test B.

*Test B*

Two plastic bulb pans werre filled with fertilized and limed Woodstown sandy loam. One pan was planted with corn, sorghum, Kentucky bluegrass and several grass weeds. The other pan was planted with cotton, soybeans, purple nutsedge *(Cyperus rotundus)*, and several broadleaf weeds. The following grass and broadleaf weeds were planted: crabgrass *(Digitaria sanquinalis)*, barnyardgrass *(Echinochloa crusgalli)*, wild oats *(Avena fatua)*, johnsongrass *(Sorghum halepense)*, dallisgrass *(Paspalum dilatatum)*, giant foxtail *(Setaria faberii)*, cheatgrass *(Bromus secalinus)*, mustard *(Brassica arvensis)*, cocklebur *(Xanthium pensylvanicum)*, pigweed *(Amaranthus retroflexus)*, morningglory *(Ipomoea hederacea)*, sicklepod *(Cassia obtusifolia)*, teaweed *(Sida spinosa)*, velvetleaf *(Abutilon theophrasti)*, and jimsonweed *(Datura stramonium)*. A 12.5 cm diameter plastic pot was also filled with prepared soil and planted with rice and wheat. Another 12.5 cm pot was planted with sugar beets. The above four containers were treated pre-emergence with several test compounds within the scope of the invention.

Twenty-eight days after treatment, the plants were evaluated and visually rated for response to the chemical treatments utilizing the rating system described previously for Test A. The data is summarized in Table B.

Table B

PRE-EMERGENCE ON
WOODSTOWN SANDY LOAM

| | Compound 1 | | |
|---|---|---|---|
| Rate kg/ha | 0.03 | 0.12 | 0.008 |
| Crabgrass | 7G | 9G,5H | 2G |
| Barnyardgrass | 8G,9C | 9G,9C | 6G,3C |
| Sorghum | - | - | 9G |
| Wild Oats | 8G,8C | 10C | 7G |
| Johnsongrass | 9G,8C | 10C | 7G |
| Dallisgrass | 9G,3H | 9G,3H | 6G |
| Giant foxtail | 8G,5H | 9G,9C | 2G |
| Ky. bluegrass | 10E | 10E | 10C |
| Cheatgrass | 10C | 10C | 9G |
| Sugar beets | 7G,7C | 10C | 8G |
| Corn | 10C | 10C | 10C |
| Mustard | 9G,9C | 10C | 8G |
| Cocklebur | 7G,3H | 8G,5H | 6G |
| Pigweed | 10E | 10E | - |
| Nutsedge | 10E | 10E | 10C |
| Cotton | 9G,5H | 9G,5H | 6G |
| Morningglory | 7G | 9G,8C | 5G |
| Sicklepod | 6G | 8G,5C | 4G |
| Teaweed | 7G,3C | 8G,5C | 8G |
| Velvetleaf | 6G,3H | 9G,9C | 9G |
| Jimsonweed | 6G | 10C | 5G |
| Soybean | 7G,5H | 9G,8C | 6G |
| Rice | 10E | 10E | 10C |
| Wheat | 8G,8C | 10E | 3G |

Table B (continued)

PRE-EMERGENCE ON
WOODSTOWN SANDY LOAM

| | Compound 19 | | Compound 20 | |
|---|---|---|---|---|
| Rate kg/ha | 0.120 | 0.500 | 0.030 | 0.120 |
| Crabgrass | 2G | 5G | 2G | 4G |
| Barnyardgrass | 8G,3H | 10C | 7G,3H | 9G,5H |
| Sorghum | - | - | 7G,5H | 9G,5H |
| Wild Oats | 2G | 5G | 4G | 5G |
| Johnsongrass | 5G,3H | 8G,3H | 4G,3H | 6G,2H |
| Dallisgrass | 6G | 8G,5H | 2G | 4G |
| Giant foxtail | 6G,5H | 8G,5H | 4G | 6G,3H |
| Ky. bluegrass | 8G,8C | 10C | 6G | 7G |
| Cheatgrass | 6G,3C | 10C | 8G,8C | 8G,9C |
| Sugar beets | 8G,8C | 10C | 8G,8C | 10C |
| Corn | 7G,5H | 9G,8C | 6G,3H | 7G,3H |
| Mustard | 9G,9C | 10C | 9G,8C | 9G,9C |
| Cocklebur | 6G | 7G,3H | 6G | 5G |
| Pigweed | 8G | 10C | - | - |
| Nutsedge | 7G | 10E | 7G | 7G |
| Cotton | 3G | 8G | 3G | 5G,3H |
| Morningglory | 0 | 6G,5H | 4G | 9G,5C |
| Sicklepod | 6G | 7G | 7G | 8G,3H |
| Teaweed | 5G | 8G,3H | 5G | 10C |
| Velvetleaf | 6G,5H | 9G,9C | 5G,5H | 8G,9C |
| Jimsonweed | 6G | 7G | 7G | 8G,8C |
| Soybean | 3G | 6G,5H | 3G,3H | 6G,5H |
| Rice | 9G,9C | 10C | 7G,8C | 10C |
| Wheat | 4G | 6G | 0 | 4G |

Table B (continued)

PRE-EMERGENCE ON

WOODSTOWN SANDY LOAM

| | Compound 21 | | Compound 22 | |
|---|---|---|---|---|
| Rate kg/ha | 0.030 | 0.120 | 0.007 | 0.030 |
| Crabgrass | 0 | 6G | 0 | 0 |
| Barnyardgrass | 6G,2H | 8G,5H | 0 | 3G |
| Sorghum | 6G,3H | 9G,5H | 0 | 0 |
| Wild Oats | 3G | 6G | 0 | 0 |
| Johnsongrass | 4G,2H | 7G,5H | 0 | 0 |
| Dallisgrass | 0 | 5G | 0 | 0 |
| Giant foxtail | 3G | 7G,3H | 0 | 0 |
| Ky. bluegrass | 5G,3H | 7G,3H | 4G | 0 |
| Cheatgrass | 8G,8C | 8G,9C | 0 | 0 |
| Sugar beets | 10C | 10C | 6G | 8G |
| Corn | 5G,3H | 7G,3H | 2G | 3G |
| Mustard | 9G,5H | 9G,9C | 9G | 9G,9C |
| Cocklebur | 0 | 3G | 0 | 2G |
| Pigweed | 10E | 10E | - | - |
| Nutsedge | 9G,9E | 10E | 0 | 5G |
| Cotton | 2G | 4G | 2G | 5G |
| Morningglory | 3G | 3G | 0 | 7G |
| Sicklepod | 8G | 8G | 5G | 6G |
| Teaweed | 7G | 8G | 6G | 9G |
| Velvetleaf | 7G,5H | 9G,8C | 5G,5H | 8G |
| Jimsonweed | 5G | 6G | 0 | 4G |
| Soybean | 3G | 6G,5H | 5G | 8G |
| Rice | 8G,8C | 10E | 0 | 5G |
| Wheat | 2G | 4G | 0 | 0 |

Table B (continued)

PRE-EMERGENCE ON
WOODSTOWN SANDY LOAM

| | Compound 23 | |
|---|---|---|
| Rate kg/ha | 0.030 | 0.120 |
| Crabgrass | 0 | 0 |
| Barnyardgrass | 2G | 3H,8G |
| Sorghum | 2G | 3H,8G |
| Wild Oats | 0 | 2G |
| Johnsongrass | 0 | 2H,4G |
| Dallisgrass | 4G | 8G |
| Giant foxtail | 3H,4G | 5H,8G |
| Ky. bluegrass | 6G | 10E |
| Cheatgrass | 5G | 8G |
| Sugar beets | 5G | 8G |
| Corn | 3G | 3H,7G |
| Mustard | 9G,9C | 9G,9C |
| Cocklebur | 2G | 2G |
| Pigweed | - | - |
| Nutsedge | 2G | 6G |
| Cotton | 3G | 5G |
| Morningglory | 2G | 2G |
| Sicklepod | 2G | 5G |
| Teaweed | 0 | 0 |
| Velvetleaf | 0 | 5G |
| Jimsonweed | 0 | 4G |
| Soybean | 3G | 3G |
| Rice | 8G | 9G,9C |
| Wheat | 2G | 3G |

A number of compounds were also tested to more accurately define their utility in post-emergence applications.

*Test C*

The test chemicals, dissolved in a non-phytotoxic solvent, were applied in an overall spray to the foliage and surrounding soil of selected plant species. One day after treatment, plants were observed for rapid burn injury. Approximately fourteen days after treatment, all species were visually compared to untreated controls and rated for response to treatment. The rating system was as described previously for Test A. The data are presented in Table C.

All plant species were seeded in Woodstown sandy loam soil and grown in a greenhouse. The following species were grown in soil contained in plastic pots (25 cm diameter by 13 cm deep): soybeans, cotton, alfalfa, corn, rice, wheat, sorghum, velvetleaf *(Abutilon theophrasti)*, sesbania *(Sesbania exaltata)*. Sicklepod *(Cassia obtusifolia)*,, morningglory *(Ipomoea hederacea)*, jimsonweed *(Datura stramonium)*, cocklebur *(Xanthium pensylvanicum)*, crabgrass *(Digitaria* sp.*)*, nutsedge *(Cyperus rotundus)*, barnyardgrass *(Echinochloa crusgalli)*, *giant foxtail (Setaria faberii)* and wild oats *(Avena fatua)*. The following species were grown in soil in a paper cup (12 cm diameter by 13 cm deep): sunflower, sugar beets, and mustard. All plants werre sprayed approximately 14 days after planting. Additional plant species, such as johnsongrass and field bindweed, are sometimes added to this standard test in order to evaluate unusual selectivity.

Again, Compound 1 appeared to be one of the most active compounds when applied post-emergence. All of the compounds tested, however, were shown to be highly active post-emergence herbicides. Some of the compounds, for example. Compounds 3, 4, 6 and 15, show potential utility for selective post-emergence weed control in wheat.

Table C

Over-the-Top Soil/Foliage Treatment

Compound 1

| Rate kg/ha | 0.25 | 0.06 | 0.015 | 0.004 |
|---|---|---|---|---|
| Soybeans | 10C | 10C | 10C | 8G |
| Velvetleaf | 10C | 10C | 7G,8C | 2G |
| Sesbania | 10C | 10C | 10C | 9G |
| Sicklepod | 10C | 10C | 9G | 8G |
| Cotton | 10C | 10C | - | - |
| Morningglory | 10C | 10C | 1G | 1G |
| Alfalfa | 10C | 10C | 4G,4C | 5G,4C |
| Jimsonweed | 10C | 9G | - | 9G |
| Cocklebur | 10C | 9G | 8G | 7G |
| Sunflower | 10C | 9G,8C | 4G | 2G |
| Mustard | 9G,8C | - | 7G,1C | 4G |
| Sugar beets | 10C | 10C | 4C,7G | 5G,3C |
| Corn | 10C | 10C | 10C | 10C |
| Crabgrass | 8G | 9G | 10C | 5G,4C |
| Rice | 9G,6C | 9G,4C | 3G,2C | 4G |
| Nutsedge | 10C | 9G | 5G,4C | 3G |
| Barnyardgrass | 10C | 9G,8C | 8G | 7G |
| Wheat | 8G,6C | 7G | 7G,4C | 8G,7C |
| Giant Foxtail | 9G,6C | 9G,5C | - | 4G |
| Wild Oats | 7G,6C | 8G,6C | 4G | 4G |
| Sorghum | 8G,8C | 8G,8C | 8G,9C | 8G |
| Johnsongrass | 10C | 10C | 10C | 7C |
| Field Bindweed | 9G,4C | 9G,5C | 2C | 4G |

Table C (continued)

Over-the-Top Soil/Foliage Treatment

| | Compound 20 | | Compound 23 | |
|---|---|---|---|---|
| Rate, kg/ha | 0.015 | 0.004 | 0.06 | 0.015 |
| Soybeans | 8G,6C | 6G | 3G | 0 |
| Velvetleaf | 0 | 0 | 4G | 0 |
| Sesbania | 0 | - | 0 | 0 |
| Sicklepod | 0 | 0 | 0 | 0 |
| Cotton | 3G | 3G | 3G | 3G |
| Morningglory | 2G | 0 | 4G | 3G |
| Alfalfa | 7G | 5G | 4G | 3G |
| Jimsonweed | 3G | 0 | 0 | 0 |
| Cocklebur | 0 | - | 2G | 3G |
| Sunflower | 2G | 0 | 3G | 1G |
| Rape | 2G | 0 | 5G | 0 |
| Sugar beets | 2G | 0 | - | - |
| Corn | 2G | 0 | 1C | 0 |
| Crabgrass | 4G | 0 | 0 | 0 |
| Rice | 6G | 2G | 6G | 6G |
| Nutsedge | 0 | 0 | 0 | 0 |
| Barnyardgrass | 0 | 3G | 0 | 0 |
| Wheat | 0 | 0 | 0 | 0 |
| Giant Foxtail | 2G | 4G | 0 | 0 |
| Wild Oats | 0 | 0 | 0 | 0 |
| Sorghum | 3G | 5G | 8G | 6G |
| Johnsongrass | 0 | 0 | 0 | 0 |
| Field Bindweed | 3G | 0 | 0 | 0 |

*Test D*

Two ten-inch in diameter plastic pans lined with polyethylene liners were filled with prepared Woodstown sandy loam soil. One pan was planted with seeds of wheat *(Triticum aestivum)*, barley *(Hordeum vulgare)*, wild oats *(Avena fatua)*, downy brome *(Bromus tectorum)*, cheatgrass *(Bromus secalinus)*, blackgrass *(Alopecurus myosuroides)*, annual bluegrass *(Poa annua)*, green foxtail *(Setaria viridis)*, quackgrass *(Agropyron repens)*, Italian ryegrass *(Lolium multiflorum)* and ripgut brome *(Bromus rigidus)*. The other pan was planted with seeds of Russian thistle *(Salsola kali)*, tansy mustard *(Descuraina pinnata)*, cleavers *(Galium aparine)*, tumble mustard *(Sisymbrium altissium)*, kochia *(Kochia scoparia)*, shepherd's purse *(Capsella bursa-pastoris)*, *Matricaria inodora*, black nightshade *(Solanum nigrum)*, yellow rocket *(Barbarea vulgaris)*, rapeseed *(Brassica napus)*, and wild buckwheat *(Polygonum convolvulus)*. The above two pans were treated preemergence. At the same time, two pans in which the above plant species were growing were treated postemergence. Plant height at the time of treatment ranged from 1—15 cm depending on plant species.

The compounds applied were diluted with a non-phytotoxic solvent and sprayed over the top of the pans. An untreated control and a solvent-alone control were included for comparison. All treatments were maintained in the greenhouse for 19—21 days at which time the treatments were compared to the controls and the effects visually rated. The recorded data are presented in Table D. Some of the compounds tested have utility for selective weed control in cereal crops such as wheat and barley.

Table D

Compound 3

| | Pre-Emergence | Post-Emergence |
|---|---|---|
| Rate kg/ha | 0.12 | |
| wheat | 0 | 0 |
| barley | 0 | 0 |
| wild oats | 0 | 0 |
| downy brome | 0 | 2G |
| cheatgrass | 2G | 2C,5G |
| blackgrass | 4G | 1C,4G |
| annual bluegrass | 4G | 4G |
| green foxtail | 0 | 5G |
| quackgrass | 2G | 7G |
| Italian ryegrass | 3G | 3G |
| ripgut brome | 3G | 3G |
| Russian thistle | 0 | 0 |
| tansy mustard | 9G | 2C,4G |
| *Galium aparine* | 2G | 0 |
| tumble mustard | 6G | 7C,9G |
| kochia | 0 | 3G |
| shepherd's purse | 8G | 3C,8G |
| *Matricaria inodora* | 7G | 3C,8G |
| black nightshade | 5G | 4G |
| yellow rocket | 5G | 2C,6G |
| wild mustard | 4G | 10C |
| wild buckwheat | 3G | 3G |

Table D (continued)

Compound 20

| | Pre-Emergence | | Post-Emergence | |
|---|---|---|---|---|
| Rate kg/ha | 0.060 | 0.015 | 0.060 | 0.015 |
| wheat | 7G | 2G | 6G | 3G |
| barley | 1C,7G | 2G | 6G | 3G |
| wild oats | 1C,7G | 5G | 5G | 2G |
| downy brome | 2C,9G | 8G | 8G | 7G |
| cheatgrass | 1C;9G | 8G | 8G | 7G |
| blackgrass | 2C,8G | 7G | 5G | 3G |
| annual bluegrass | 8G | 7G | 7G | 3G |
| green foxtail | 2C,8G | 2C,4G | 2C,8G | 4G |
| quackgrass | 9G | 8G | 8G | 6G |
| Italian ryegrass | 1C,8G | 7G | 4C,8G | 7G |
| ripgut brome | 8G | 8G | 2C,8G | 7G |
| Russian thistle | 2C,3G | 2G | 8C,8G | 4C,5G |
| tansy mustard | 2C,9G | 9G | 8C,8G | 5C,7G |
| Galium aparine | 6G | 8G | 7G | 4G |
| tumble mustard | 1C,8G | 9G | 10C | 7G |
| kochia | 7G | 5G | 3C,8G | 4G |
| shepherd's purse | 2C,9G | 9G | 4C,9G | 7G |
| *Matricaria inodora* | 7G | 7G | 8C,8G | 2C,7G |
| black nightshade | 8G | 5G | 6G | 6G |
| yellow rocket | 9G | 8G | 7G | 5G |
| rapeseed | 2C,9G | 9G | 10C | 2C,8G |
| wild buckwheat | 7G | 6G | 7G | 4G |

Table D (continued)

Compound 21

| | Pre-Emergence | | Post-Emergence | |
|---|---|---|---|---|
| Rate kg/ha | 0.060 | 0.015 | 0.060 | 0.015 |
| wheat | 3G | 0 | 3G | 0 |
| barley | 3G | 0 | 5G | 0 |
| wild oats | 5G | 0 | 4G | 0 |
| downy brome | 9G | 8G | 9G | 7G |
| cheatgrass | 2C,9G | 7G | 8G | 6G |
| blackgrass | 7G | 6G | 6G | 3G |
| annual bluegrass | 7G | 6G | 2C,8G | 3G |
| green foxtail | 2C,6G | 3G | 2C,6G | 6G |
| quackgrass | 9G | 8G | 8G | 7G |
| Italian ryegrass | 8G | 8G | 2C,8G | 6G |
| ripgut brome | 8G | 6G | 7G | 6G |
| Russian thistle | 1C,2G | 0 | 8C,8G | 0 |
| tansy mustard | 9G | 9G | 10C | 9G |
| Galium aparine | 10E | 8G | 6G | 5G |
| tumble mustard | 2C,9G | 9G | 10C | 9G |
| kochia | 8G | 2C,5G | 2C,8G | 5G |
| shepherd's purse | 9C,9G | 9C,9G | 10C | 2C,8G |
| *Matricaria inodora* | 8G | 9G | 8C,8G | 8C,8G |
| black nightshade | 6G | 3G | 7G | 6G |
| yellow rocket | 9G | 7G | 8C,8G | 8C,8G |
| rapeseed | 3C,9G | 9G | 10C | 10C |
| wild buckwheat | 6G | 4G | 2C,8G | 7G |

Table D (continued)

Compound 23

| | Pre-Emergence | | Post-Emergence | |
|---|---|---|---|---|
| Rate kg/ha | 0.060 | 0.250 | 0.060 | 0.250 |
| wheat | 1C,2G | 5G | 2G | 7G |
| barley | 6G | 2C,8G | 3G | 7G |
| wild oats | 5G | 7G | 3G | 5G |
| downy brome | 5G | 8G | 5G | 6G |
| cheatgrass | 6G | 8G | 5G | 7G |
| blackgrass | 5G | 1C,7G | 6G | 2C,7G |
| annual bluegrass | 6G | 1C,8G | 4G | 3C,8G |
| green foxtail | 0 | 2C,6G | 0 | 2C,5G |
| quackgrass | 4G | 8G | 2G | 6G |
| Italian ryegrass | 8G | 2C,9G | 2C,6G | 2C,8G |
| ripgut brome | 6G | 1C,8G | 5G | 7G |
| Russian thistle | 0 | 2C,5G | 0 | 10C |
| tansy mustard | 8G | 2C,9G | 5G | 8G |
| Galium aparine | 9G | 10E | 0 | 3G |
| tumble mustard | 7G | 9G | 0 | 6G |
| kochia | - | - | - | 0 |
| shepherd's purse | 2C,9G | 10C | 5G | 10C |
| *Matricaria inodora* | 8G | 9G | 3G | 9G |
| black nightshade | 0 | 5G | 0 | 4G |
| yellow rocket | 8G | 9G | 0 | 6G |
| rapeseed | 2C,8G | 9G | 7G | 9C,9G |
| wild buckwheat | 0 | 6G | 0 | 3G |

A final test was designed to evaluate the potential utility of compounds from within the scope of the invention for selective weed control in rice.

*Test E*

The crop was transplanted into simulated paddies containing soil and propagules of barnyardgrass *(Echinochloa* sp.*)*, water chestnut *(Eleocharis* sp.*)*, and arrowhead *(Sagittaria* sp.*)*. Three days after transplanting, the test chemicals were applied to the paddy water in a non-phytotoxic solvent at the rates shown in Table E. The paddies were maintained in a greenhouse, and plant response ratings were taken several weeks after application utilizing the rating system as described for Test A. Compounds 11 and 14 appear to have utility for weed control in transplanted rice.

Table E

| | Compound 11 |
|---|---|
| Rate, grams/hectare | 100 |
| Rice | 1G |
| Barnyardgrass | 9C |
| Water Chestnut | 10C |
| Arrowhead | 6G,5H |

| | Compound 14 | |
|---|---|---|
| Rate, grams/hectare | 25 | 100 |
| Rice | 0 | 0 |
| Barnyardgrass | 7C | 8C |
| Water Chestnut | 0 | 0 |
| Arrowhead | 0 | 0 |

**Claims for the Contracting States: BE CH DE FR GB IT LI LU NL SE**

1. A compound of the formula:

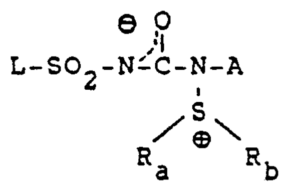

I

wherein
$R_a$ is $CH_3$ or $CH_2CH_3$;
$R_b$ is $CH_3$ or $CH_2CH_3$;
L is

L is 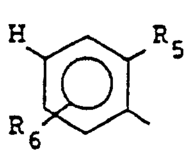 , 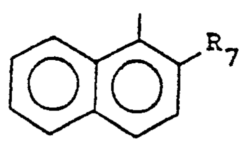 , 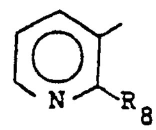 ,

L-1 L-2 L-3

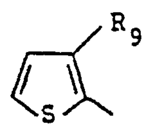 , 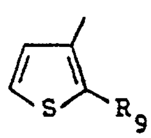 , 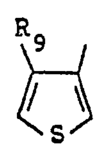 ,

L-4 L-5 L-6

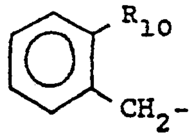 , 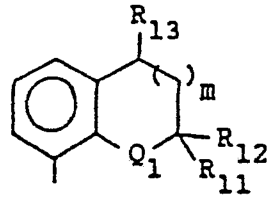 ,

L-7 L-8

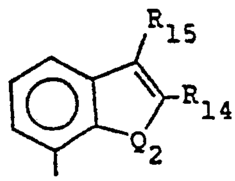 , 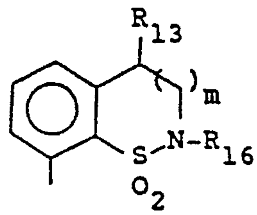 , 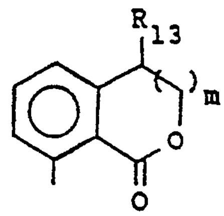 ,

L-9 L-10 L-11

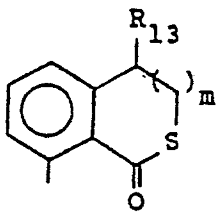 , 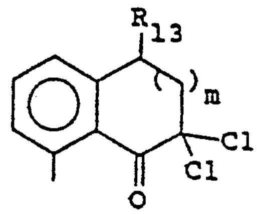 , 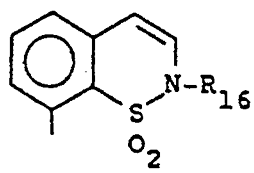 ,

L-12 L-13 L-14

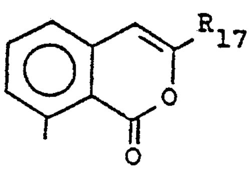 , 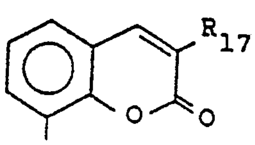 or 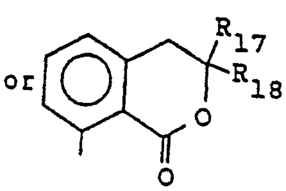 ;

L-15 L-16 L-17

$R_5$ is $C_1$—$C_4$ alkyl, $C_1$—$C_4$ alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

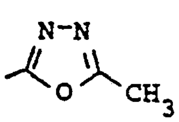 , 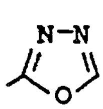 , 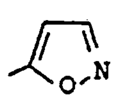 , 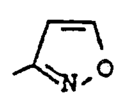 , 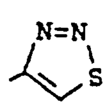 , 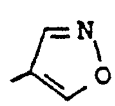 ,

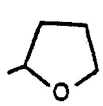 , 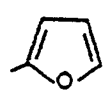 or 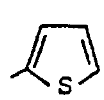 ;

$R_6$ is H, F, Cl, Br, $CF_3$, $CH_3$, $OCH_3$, $SCH_3$ or $OCF_2H$;
$R_7$ is H, $CH_3$, $OCH_3$, F, Cl, Br, $OSO_2CH_3$, $SO_2N(CH_3)_2$ or $S(O)_nCH_3$;
$R_8$ is $CH_3$, $CH_2CH_3$, $OCH_3$, $OCH_2CH_3$, F, Cl, Br, $SO_2NR_{20}R_{21}$, $SO_2N(OCH_3)CH_3$ or $S(O)_nR_{23}$;
$R_9$ is $C_1$—$C_3$ alkyl, F, Cl, Br, $NO_2$, $CO_2R_{19}$, $SO_2NR_{20}R_{21}$, $SO_2N(OCH_3)CH_3$ or $S(O)_nR_{23}$;
$R_{10}$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;
$R_{11}$ is H, $CH_3$ or $CH_2CH_3$;
$R_{12}$ is H, $CH_3$ or $CH_2CH_3$;
$R_{13}$ is H or $CH_3$;
$R_{14}$ is H or $CH_3$;
$R_{15}$ is H or $CH_3$;
$R_{16}$ is $CH_3$ or $CH_2CH_3$;
$R_{17}$ is H or $C_1$—$C_4$ alkyl;
$R_{18}$ is H or $CH_3$;
$R_{19}$ is $C_1$—$C_4$ alkyl, $CH_2CH_2OCH_3$, $CH_2CH_2Cl$ or $CH_2CH{=}CH_2$;
$R_{20}$ is $C_1$—$C_3$ alkyl;
$R_{21}$ is $C_1$—$C_3$ alkyl;
$R_{22}$ is $C_1$—$C_3$ alkyl or $N(CH_3)_2$;
$R_{23}$ is $C_1$—$C_3$ alkyl or $CH_2CH{=}CH_2$;
m is 0 or 1;
n is 0 or 2;
$Q_1$ is O, S, $SO_2$ or $NR_{17}$;
$Q_2$ is O, S or $NR_{17}$; and
W is O, S or $SO_2$;
A is

A is 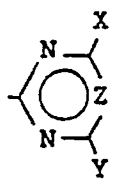 , 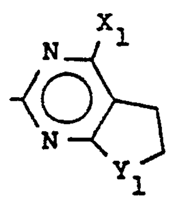 , 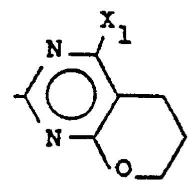

A-1 A-2 A-3

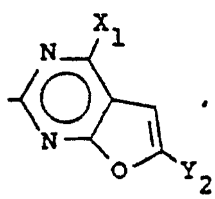 , 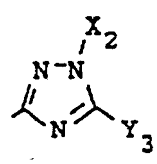 or 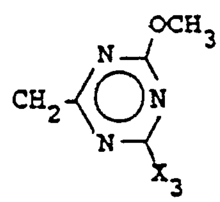 ;

A-4 A-5 A-6

X is $CH_3$, $OCH_3$, $OCH_2CH_3$, Cl, F, Br, $OCF_2H$, $CH_2F$ or $CF_3$;
Y is H, $CH_3$, $OCH_3$, $OCH_2CH_3$, $CH_2OCH_3$, $CH_2OCH_2CH_3$, $N(CH_3)_2$, $CH_2CH_3$, $CF_3$ $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

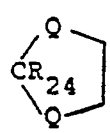 , 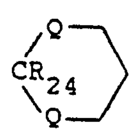 , 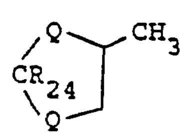

or $CR_{24}(QCH_2CH_3)_2$;
Q is O or S;
$R_{24}$ is H or $CH_3$;
Z is CH or N;
$Y_1$ is $CH_2$ or O;
$X_1$ is $CH_3$, $OCH_3$, $OCH_2CH_3$ or $OCF_2H$;
$Y_2$ is H or $CH_3$;
$X_2$ is $CH_3$, $CH_2CH_3$ or $CH_2CF_3$;

$Y_3$ is $OCH_3$, $OCH_2CH_3$, $SCH_3$, $CH_3$ or $CH_2CH_3$;

$X_3$ is $CH_3$ or $OCH_3$;

provided that

1) the total number of carbon atoms of $R_{20}$ and $R_{21}$ is less than or equal to four;

2) when m is 1, then $R_{13}$ is H;

3) when L is L—17, then $R_{17}$ and $R_{18}$ are not simultaneously H;

4) when X is Cl, F or Br, then Z is CH and Y is $OCH_3$, $OCH_2CH_3$, $N(CH_3)_2$ or $OCF_2H$.

2. A compound of Claim 1 where A is A—1.

3. A compound of Claim 2 where Y is $CH_3$, $OCH_3$, $OCH_2CH_3$, $CH_2OCH_3$, $OCH_2CF_3$ or $CH(OCH_3)_2$ and X is $CH_3$, $OCH_3$, Cl or $CF_3$.

4. A compound of Claim 3 where L is L—1, L—2, L—3, L—5, L—8, L—10, L—11, L—16 or L—17.

5. A compound of Claim 4 where L is L—1; $R_5$ is $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

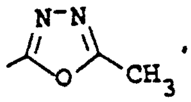, 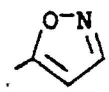, 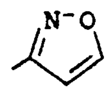 or 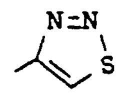 ;

$R_{22}$ is $C_1$—$C_3$ alkyl; $R_{23}$ is $CH_3$ and n is 2.

6. Compounds of Claim 4 where L is L—2 and $R_7$ is Cl, $CH_3$, $OCH_3$, $SCH_3$ or Br.

7. Compounds of Claim 4 where L is L—3 and $R_8$ is Cl, $SO_2CH_3$ or $SO_2N(CH_3)_2$.

8. Compounds of Claim 4 where L is L—5 and $R_9$ is $CO_2CH_3$ or $CO_2CH_2CH_3$.

9. The compound of Claim 1 which is 2-[[(4-methoxy-6-methylpyrimidin-2-yl)aminocarbonyl]aminosulfonyl]benzoic acid methyl ester, dimethylsulfonium inner salt.

10. The compound of Claim 1 which is 2-[[(4,6-dimethoxypyrimidin-2-yl)aminocarbonyl]-aminosulfonyl]benzoic acid methyl ester, dimethylsulfonium inner salt.

11. The compound of Claim 1 which is N-[(4,6-dimethylpyrimidin-2-yl)aminocarbonyl]-2-methylsulfonylbenzenesulfonamide, dimethylsulfonium inner salt.

12. The compound of Claim 1 which is 2-[[(4,6-dimethylpyrimidin-2-yl)aminocarbonyl]aminosulfonyl]benzoic acid methyl ester, dimethylsulfonium inner salt.

13. The compound of Claim 1 which is N-[(4,6-dimethoxypyrimidin-2-yl)aminocarbonyl]-2-dimethylaminosulfonylbenzenesulfonamide, dimethylsulfonium inner salt.

14. A compound having the formula:

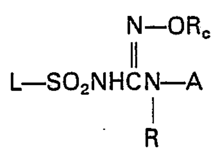 (II)

wherein

R is H or $CH_3$;

$R_c$ is H, $C(O)R_1$, $C(O)NR_2R_3$ or $CO_2R_4$;

$R_1$ is $C_1$—$C_3$ alkyl or $CF_3$;

$R_2$ is H, $CH_3$ or $C_2H_5$;

$R_3$ is $C_1$—$C_3$ alkyl;

$R_4$ is $C_1$—$C_3$ alkyl;

L is

L is 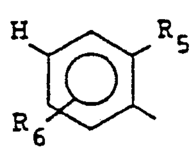, 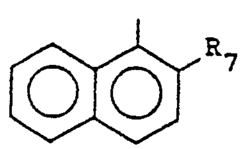, 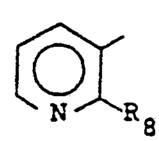,

L-1 L-2 L-3

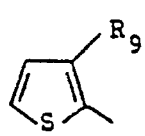, 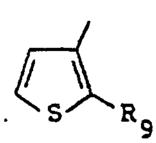, 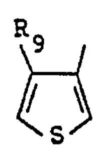.

L-4 L-5 L-6.

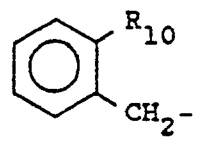 ,

L-7

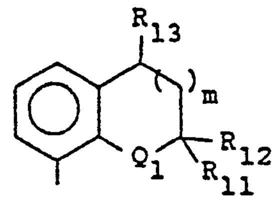 ,

L-8

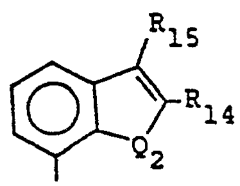 ,

L-9

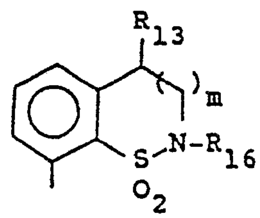 ,

L-10

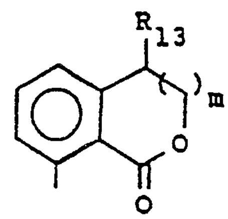 ,

L-11

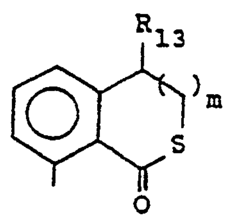 ,

L-12

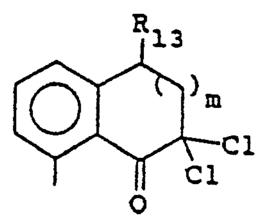 ,

L-13

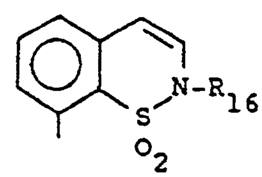 ,

L-14

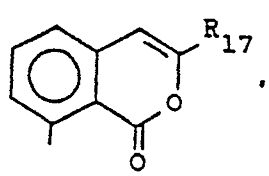 ,

L-15

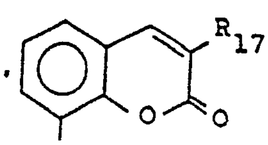

L-16

or

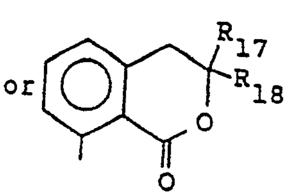 ;

L-17

$R_5$ is $C_1$—$C_4$ alkyl, $C_1$—$C_4$ alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

$OCH_2CH_3$, $C_6H_5$, 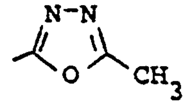 , 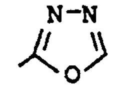 ,

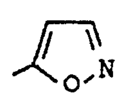 , 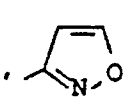 , 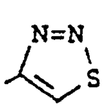 , 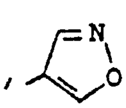 , 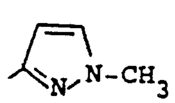 ,

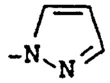 , 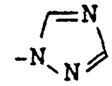 , 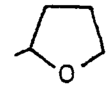 , 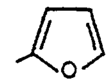 or 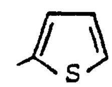 ;

$R_6$ is H, F, Cl, Br, $CF_3$, $CH_3$, $OCH_3$, $SCH_3$ or $OCF_2H$;
$R_7$ is H, $CH_3$, $OCH_3$, F, Cl, Br, $SO_2N(CH_3)_2$, $OSO_2CH_3$ or $S(O)_nCH_3$;
$R_8$ is $CH_3$, $CH_2CH_3$, $OCH_3$, $OCH_2CH_3$, F, Cl, Br, $SO_2NR_{20}R_{21}$, $SO_2N(OCH_3)CH_3$ or $S(O)_nR_{23}$;
$R_9$ is $C_1$—$C_3$ alkyl, F, Cl, Br, $NO_2$, $CO_2R_{19}$, $SO_2NR_{20}R_{21}$, $SO_2N(OCH_3)CH_3$ or $S(O)_nR_{23}$;
$R_{10}$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;
$R_{11}$ is H, $CH_3$ or $CH_2CH_3$;
$R_{12}$ is H, $CH_3$ or $CH_2CH_3$;
$R_{13}$ is H or $CH_3$;
$R_{14}$ is H or $CH_3$;
$R_{13}$ is H or $CH_3$;
$R_{16}$ is $CH_3$ or $CH_2CH_3$;
$R_{17}$ is H or $C_1$—$C_4$ alkyl;
$R_{18}$ is H or $CH_3$;
$R_{19}$ is $C_1$—$C_4$ alkyl, $CH_2CH_2OCH_3$, $CH_2CH_2Cl$ or $CH_2CH{=}CH_2$;
$R_{20}$ is $C_1$—$C_3$ alkyl;
$R_{21}$ is $C_1$—$C_3$ alkyl;
$R_{22}$ is $C_1$—$C_3$ alkyl or $N(CH_3)_2$;
$R_{23}$ is $C_1$—$C_3$ alkyl or $CH_2CH{=}CH_2$;
m is 0 or 1;
n is 0 or 2;
$Q_1$ is O, S, $SO_2$ or $NR_{17}$;
$Q_2$ is O, S or $NR_{17}$; and
W is O, S or $SO_2$;
A is

A is 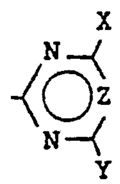 , 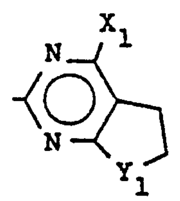 , 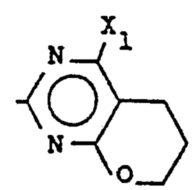 .

A-1 A-2 A-3

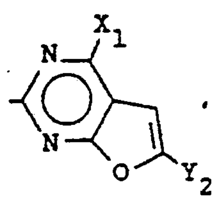 , 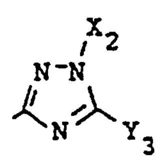 or 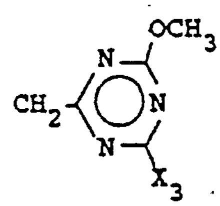 ;

A-4 A-5 A-6

X is $CH_3$, $OCH_3$, $OCH_2CH_3$, Cl, F, Br, $OCF_2H$, $CH_2F$ or $CF_3$;
Y is H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

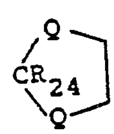 , 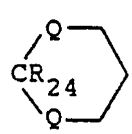 , 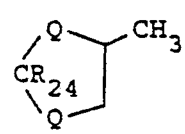

or $CR_{24}(QCH_2CH_3)_2$;
Q is O or S;
$R_{24}$ is H or $CH_3$;
Z is CH or N;
$Y_1$ is $CH_2$ or O;
$X_1$ is $CH_3$, $OCH_3$, $OCH_2CH_3$ or $OCF_2H$;
$Y_2$ is H or $CH_3$;
$X_2$ is $CH_3$, $CH_2CH_3$ or $CH_2CF_3$;

$Y_3$ is $OCH_3$, $OCH_2CH_3$, $SCH_3$, $CH_3$ or $CH_2CH_3$;
$X_3$ is $CH_3$ or $OCH_3$;
and their agriculturally suitable salts; provided that
1) the total number of carbon atoms of $R_{20}$ and $R_{21}$ is less than or equal to four;
2) when m is 1, then $R_{13}$ is H;
3) when L is L—17, then $R_{17}$ and $R_{18}$ are not simultaneously H; and
4) when X is Cl, F or Br, then Z is CH and Y is $OCH_3$, $OCH_2CH_3$, $N(CH_3)_2$ or $OCF_2H$.

15. A compound of Claim 14 where R is H, $R_c$ is H and A is A—1.

16. A compound of Claim 15 where Y is $CH_3$, $OCH_3$, $OCH_2CH_3$, $CH_2OCH_3$, $OCH_2CF_2$ or $CH(OCH_3)_2$ and X is $CH_3$, Cl or $CF_3$.

17. A compound of Claim 16 where L is L—1, L—2, L—3, L—5, L—8, L—10, L—11, L—16 or L—17.

18. A compound of Claim 17 where L is L—1, $R_5$ is $OCH_3$, $OCH_2CH_3$, Cl, $NO_2$ $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

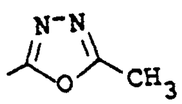, 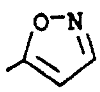, 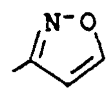 or 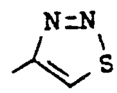 ;

$R_{22}$ is $C_1$—$C_3$ alkyl; $R_{23}$ is $CH_3$ and n is 2.

19. A compound of Claim 17 where L is L—2 and $R_7$ is Cl, $CH_3$, $OCH_3$, $SCH_3$ or Br.

20. A compound of Claim 17 where L is L—3 and $R_8$ is Cl, $SO_2CH_3$ or $SO_2N(CH_3)_2$.

21. A compound of Claim 17 where L is L—5 and $R_9$ is $CO_2CH_3$ or $CO_2CH_2CH_3$.

22. A compound of Claim 14 which is 2-[[1-(4,6-dimethylpyrimidin-2-yl)amino]-1-(hydroxyimino)methyl]aminosulfonyl]benzoic acid methyl ester.

23. A compound of Claim 14 which is 2-[[1-(4-methoxy-6-methylpyrimidin-2-yl)amino]-1-(hydroxyimino)methyl]aminosulfonyl]benzoic acid methyl ester.

24. A compound of Claim 14 which is N',N'-dimethyl-N-[[1-(4,6-dimethyl-1,3,5-triazin-2-yl)amino]-1-(hydroxyimino)methyl]-1,2-benzenedisulfonamide.

25. A compound of Claim 14 which is N',N'-dimethyl-N-[[1-(4-methoxy-6-methylpyrimidin-2-yl)amino]-1-(hydroxyimino)methyl]-1,2-benzenedisulfonamide.

26. A compound of claim 1 wherein:
L is L—1, L—2, L—3, L—4, L—5, L—8, L—10, L—11 or L—17;
$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, $C_3$—$C_4$ alkenyloxy, $CH_2CH_2OCH_3$, $CH_2CH_2OC_2H_5$,

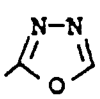, 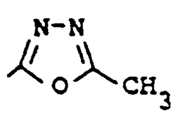, 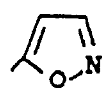, 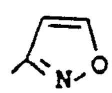 or 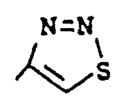 ;

$R_6$ is H, F, Cl, Br, $CF_3$, $CH_3$, or $OCH_3$;
$R_7$ is H, $CH_3$, $OCH_3$, Cl, $OSO_2CH_3$ or $S(O)_nCH_3$;
$R_8$ is $CH_3$, $OCH_3$, Cl, $SO_2NR_{20}R_{21}$ or $SO_2N(OCH_3)CH_3$;
$R_9$ is $CH_3$, Cl, Br, $NO_2$, $CO_2R_{19}$, $SO_2NR_{20}R_{21}$, $SO_2N(OCH_3)CH_3$ or $S(O)_nR_{13}$;
$R_{11}$ is H or $CH_3$;
$R_{12}$ is H or $CH_3$;
$R_{13}$ is H;
$R_{17}$ and $R_{18}$ are H;
$R_{22}$ is $C_1$—$C_3$ alkyl;
$Q_1$ is O, S or $SO_2$;
A is A—1;
X is $CH_3$ or $OCH_3$; or may be Cl when Y is $CH_3$ or $OCH_3$;
Y is H, $CH_3$, $OCH_3$, $OCH_2CH_3$, $CH_2OCH_3$, $CH_2CH_3$, $CF_3$, $OCH_2CF_3$, $CH(OCH_3)_2$;
and $R_a$, $R_b$, $R_{16}$, $R_{19}$, $R_{20}$, $R_{21}$, $R_{23}$, m, n and Z are as defined in claim 1.

27. A compound of claim 14 wherein:
R is H or $CH_3$;
$R_c$ is as defined in claim 14;
L is L—1;
$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, or $C_1$—$C_2$ alkyl substituted with $OCH_3$ or $OC_2H_5$;
$R_6$ is H, F, Cl, Br, $CF_3$, $CH_3$, $OCH_3$ or $SCH_3$;
$R_{19}$, $R_{20}$, $R_{21}$, $R_{23}$ and W are as defined in claim 14;
$R_{22}$ is $C_1$—$C_3$ alkyl;

A is A—1;
X is $CH_3$, $OCH_3$ or Cl;
Y is $CH_3$, $C_2H_5$, $OCH_3$, $OC_2H_5$, $CH_2OCH_3$, $CH(OCH_3)_2$ or

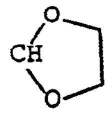

and Z is as defined in claim 14.

28. A composition suitable for controlling the growth of undesired vegetation or for regulating the growth of plants which comprises an effective amount of a herbicidal compound or plant growth regulant compound and at least one of the following: surfactant, solid or liquid diluent, characterized in that said herbicidal compound or plant growth regulant compound comprises a compound of any of claims 1 to 27.

29. A method for controlling the growth of undesired vegetation by applying to the locus to be protected an effective amount of a herbicidal compound characterized in that said herbicidal compound comprises a compound of any of claim 1 to 27.

30. A method for regulating the growth of plants by applying to the locus of said plants an effective but substantially non-phytotoxic amount of a plant growth regulant, characterized in that said plant regulant comprises a compound of any of claims 1 to 27.

31. A method for the preparation of a compound of claim 1 which comprises reacting a sulfonyl isocyanate of formula

$$LSO_2NCO \qquad (IV)$$

wherein L is as defined in claim 1, with an S,S-dialkyl-N-(heterocyclic)sulfilimine of formula

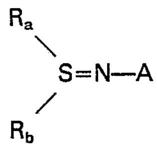

(V)

wherein $R_a$, $R_b$ and A are as defined in claim 1.

32. A method for the preparation of a compound of claim 14 which comprises reacting an isothiourea derivative of formula

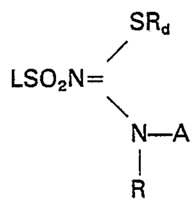

(III)

wherein L, R, $R_d$ and A are as defined in claim 26, with hydroxylamine, to obtain a compound of claim 14 wherein $R_c$ is H, and optionally reacting the product with $R_cCl$ or $R_3NCO$, wherein $R_c$ is $C(O)R_1$, $C(O)NR_2R_3$ or $CO_2R_4$ and $R_1$, $R_2$, $R_3$ and $R_4$ are as defined in claim 14.

**Claims for the Contracting State: AT**

1. A process for the preparation of a compound of the formula

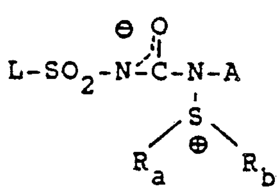

I

wherein
$R_a$ is $CH_3$ or $CH_2CH_3$;
$R_b$ is $CH_3$ or $CH_2CH_3$;
L is

L is 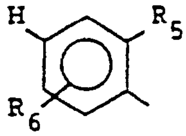 , 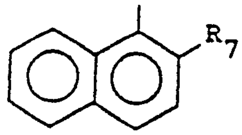 , 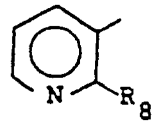 ,

L-1 L-2 L-3

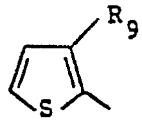 , 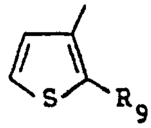 , 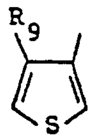 ,

L-4 L-5 L-6

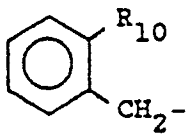 , 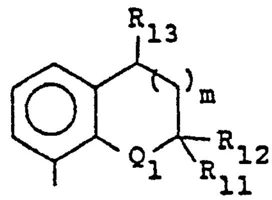 ,

L-7 L-8

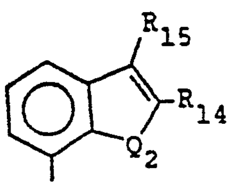 , 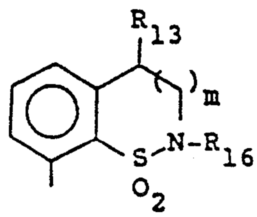 , 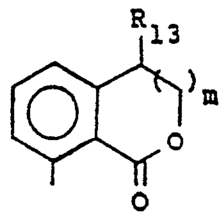 ,

L-9 L-10 L-11

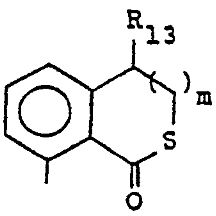 , 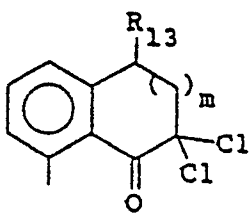 , 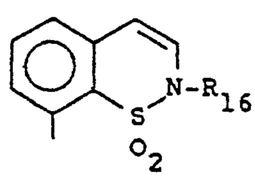 ,

L-12 L-13 L-14

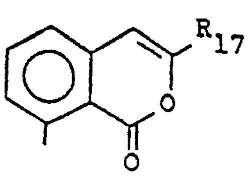 , 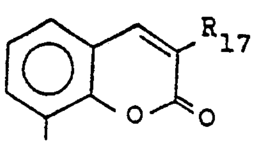 or 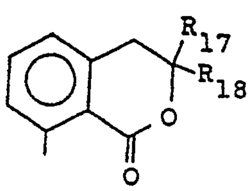 ;

L-15 L-16 L-17

$R_5$ is $C_1$—$C_4$ alkyl, $C_1$—$C_4$ alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

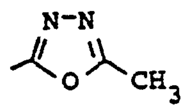 , 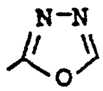 , 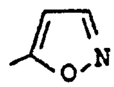 , 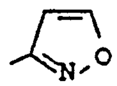 , 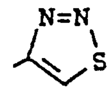 ,

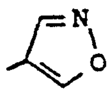 , 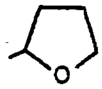 , 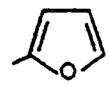 or 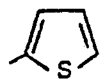 ;

$R_6$ is H, F, Cl, Br, $CF_3$, $CH_3$, $OCH_3$, $SCH_3$ or $OCF_2H$;
$R_7$ is H, $CH_3$, $OCH_3$, F, Cl, Br, $OSO_2CH_3$, $SO_2N(CH_3)_2$ or $S(O)_nCH_3$;
$R_8$ is $CH_3$, $CH_2CH_3$, $OCH_3$, $OCH_2CH_3$, F, Cl, Br, $SO_2NR_{20}R_{21}$, $SO_2N(OCH_3)CH_3$ or $S(O)_nR_{23}$;
$R_9$ is $C_1$—$C_3$ alkyl, F, Cl, Br, $NO_2$, $CO_2R_{19}$, $SO_2NR_{20}R_{21}$, $SO_2N(OCH_3)CH_3$ or $S(O)_nR_{23}$;
$R_{10}$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;
$R_{11}$ is H, $CH_3$ or $CH_2CH_3$;
$R_{12}$ is H, $CH_3$ or $CH_2CH_3$;
$R_{13}$ is H or $CH_3$;
$R_{14}$ is H or $CH_3$;
$R_{15}$ is H or $CH_3$;
$R_{16}$ is $CH_3$ or $CH_2CH_3$;
$R_{17}$ is H or $C_1$—$C_4$ alkyl;
$R_{18}$ is H or $CH_3$;
$R_{19}$ is $C_1$—$C_4$ alkyl, $CH_2CH_2OCH_3$, $CH_2CH_2Cl$ or $CH_2CH{=}CH_2$;
$R_{20}$ is $C_1$—$C_3$ alkyl;
$R_{21}$ is $C_1$—$C_3$ alkyl;
$R_{22}$ is $C_1$—$C_3$ alkyl or $N(CH_3)_2$;
$R_{23}$ is $C_1$—$C_3$ alkyl or $CH_2CH{=}CH_2$;
m is 0 or 1;
n is 0 or 2;
$Q_1$ is O, S, $SO_2$ or $NR_{17}$;
$Q_2$ is O, S or $NR_{17}$; and
W is O, S or $SO_2$;
A is

A is 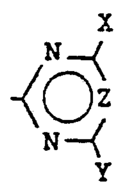 , 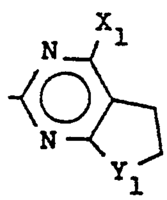 , 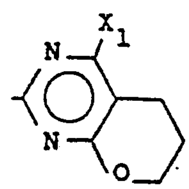 ,

A-1 A-2 A-3

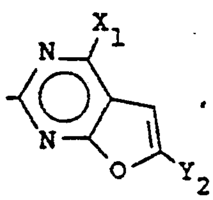 , 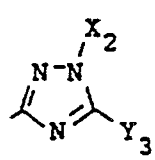 or 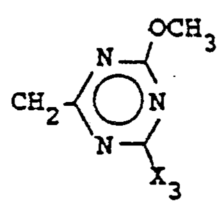 ;

A-4 A-5 A-6

X is $CH_3$, $OCH_3$, $OCH_2CH_3$, Cl, F, Br, $OCF_2H$, $CH_2F$ or $CF_3$;
Y is H, $CH_3$, $OCH_3$, $OCH_2CH_3$, $CH_2OCH_3$, $CH_2OCH_2CH_3$, $N(CH_3)_2$, $CH_2CH_3$, $CF_3$ $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

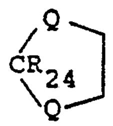 , 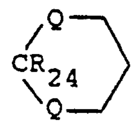 , 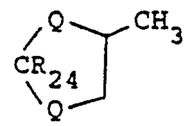

or $CR_{24}(QCH_2CH_3)_2$;

Q is O or S;

$R_{24}$ is H or $CH_3$;

Z is CH or N;

$Y_1$ is $CH_2$ or O;

$X_1$ is $CH_3$, $OCH_3$, $OCH_2CH_3$ or $OCF_2H$;

$Y_2$ is H or $CH_3$;

$X_2$ is $CH_3$, $CH_2CH_3$ or $CH_2CF_3$;

$Y_3$ is $OCH_3$, $OCH_2CH_3$, $SCH_3$, $CH_3$ or $CH_2CH_3$;

$X_3$ is $CH_3$ or $OCH_3$;

provided that

1) the total number of carbon atoms of $R_{20}$ and $R_{21}$ is less than or equal to four;

2) when m is 1, then $R_{13}$ is H;

3) when L is L—17, then $R_{17}$ and $R_{18}$ are not simultaneously H;

4) when X is Cl, F or Br, then Z is CH and Y is $OCH_3$, $OCH_2CH_3$, $N(CH_3)_2$ or $OCF_2H$, which comprises reacting a sulfonyl isocyanate of formula

$$LSO_2NCO \quad \text{(IV)}$$

wherein L is as defined above, with an S,S-dialkyl-N-(heterocyclic)sulfilimine of formula

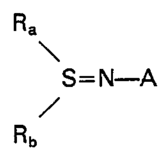 (V)

wherein $R_a$, $R_b$ and A are as defined above.

2. A process of claim 1 wherein:

L is L—1, L—2, L—3, L—4, L—5, L—8, L—10, L—11 or L—17;

$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, $C_3$—$C_4$ alkenyloxy, $CH_2CH_2OCH_3$, $CH_2CH_2OC_2H_5$,

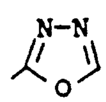 , 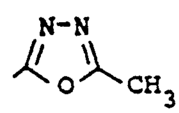 , 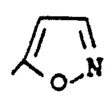 , 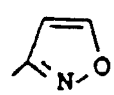 or 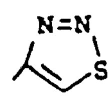 ;

$R_6$ is H, F, Cl, Br, $CF_3$, $CH_3$, or $OCH_3$;

$R_7$ is H, $CH_3$, $OCH_3$, Cl, $OSO_2CH_3$ or $S(O)_nCH_3$;

$R_8$ is $CH_3$, $OCH_3$, Cl, $SO_2NR_{20}R_{21}$ or $SO_2N(OCH_3)CH_3$;

$R_9$ is $CH_3$,. Cl, Br, $NO_2$, $CO_2R_{19}$, $SO_2NR_{20}R_{21}$, $SO_2N(OCH_3)CH_3$ or $S(O)_nR_{13}$;

$R_{11}$ is H or $CH_3$;

$R_{12}$ is H or $CH_3$;

$R_{13}$ is H;

$R_{17}$ and $R_{18}$ are H;

$R_{22}$ is $C_1$—$C_3$ alkyl;

$Q_1$ is O, S or $SO_2$;

A is A—1;

X is $CH_3$ or $OCH_3$; or may be Cl when Y is $CH_3$ or $OCH_3$;

Y is H, $CH_3$, $OCH_3$, $OCH_2CH_3$, $CH_2OCH_3$, $CH_2CH_3$, $CF_3$, $OCH_2CH_3$, $CH(OCH_3)_2$;

and $R_a$, $R_b$, $R_{16}$, $R_{19}$, $R_{20}$, $R_{21}$, $R_{23}$, m, n and Z are as defined in claim 1.

3. A process of Claim 1 wherein A is A—1.

4. A process of Claim 2 where Y is $CH_3$, $OCH_3$, $OCH_2CH_3$, $CH_2OCH_3$, $OCH_2CF_3$ or $CH(OCH_3)_2$;

X is $CH_3$, $OCH_3$, Cl or $CF_3$; and

L is L—1, L—2, L—3, L—5, L—8, L—10, L—11, L—16 or L—17.

5. A process of Claim 4 where L is L—1; $R_5$ is $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

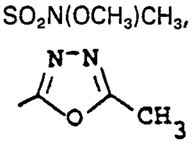 , 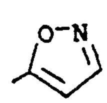 , 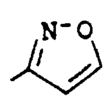 or 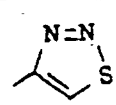 ;

$R_{22}$ is $C_1$—$C_3$ alkyl; $R_{23}$ is $CH_3$ and n is 2.

6. A process of Claim 4 where L is L—2 and $R_7$ is Cl, $CH_3$, $OCH_3$, $SCH_3$ or Br; or where L is L—3 and $R_8$ is Cl, $SO_2CH_3$ or $SO_2N(CH_3)_2$; or where L is L—5 and $R_9$ is $CO_2CH_3$ or $CO_2CH_2CH_3$.

7. The process of claim 1 or 2 wherein the product is a compound selected from 2-[[(4-methoxy-6-methylpyrimidin-2-yl)aminocarbonyl]aminosulfonyl]benzoic acid methyl ester, dimethylsulfonium inner salt;
2-[[(4,6-dimethoxypyrimidin-2-yl)aminocarbonyl]aminosulfonyl]benzoic acid methyl ester, dimethylsulfonium inner salt;
N-[(4,6-dimethylpyrimidin-2-yl)aminocarbonyl]-2-methylsulfonylbenzenesulfonamide, dimethylsulfonium inner salt;
2-[[(4,6-dimethylpyrimidin-2-yl)aminocarbonyl]aminosulfonyl]benzoic acid methyl ester, dimethylsulfonium inner salt; and
N-[(4,6-dimethoxypyrimidin-2-yl)aminocarbonyl]-2-dimethylaminosulfonylbenzenesulfonamide, dimethylsulfonium inner salt.

8. A process for the preparation of a compound having the formula:

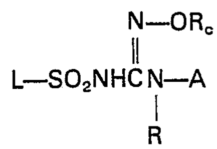 (II)

wherein
R is H or $CH_3$;
$R_c$ is H, $C(O)R_1$, $C(O)NR_2R_3$ or $CO_2R_4$;
$R_1$ is $C_1$—$C_3$ alkyl or $CF_3$;
$R_2$ is H, $CH_3$ or $C_2H_5$;
$R_3$ is $C_1$—$C_3$ alkyl;
$R_4$ is $C_1$—$C_3$ alkyl;
L is

L is 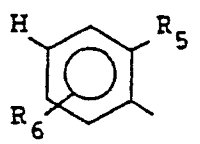 , 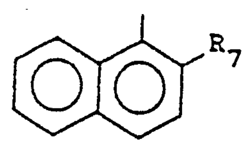 , 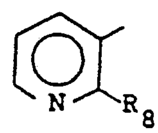 ,

L-1 L-2 L-3

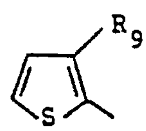 , 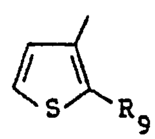 , 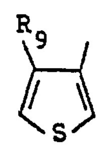 ,

L-4 L-5 L-6

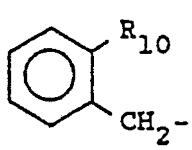 , 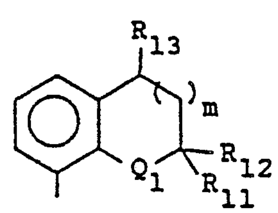 ,

L-7 L-8

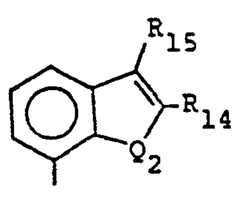, 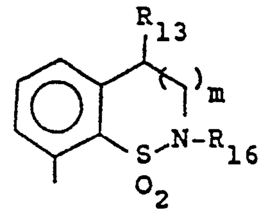, 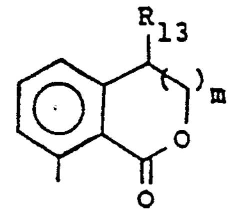,

L-9 L-10 L-11

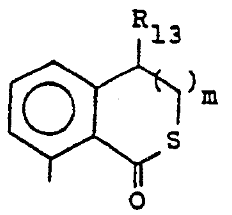, 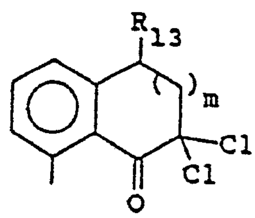, 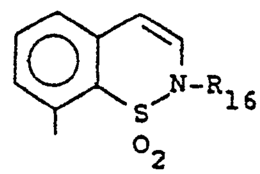,

L-12 L-13 L-14

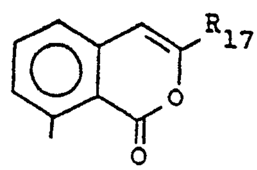, 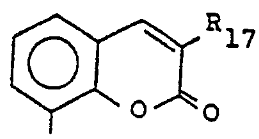 or 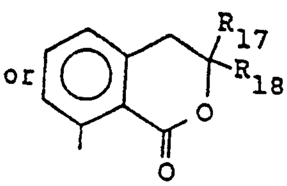 ;

L-15 L-16 L-17

$R_5$ is $C_1$—$C_4$ alkyl, $C_1$—$C_4$ alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ alkenyloxy, $C_3$—$C_4$ alkynyloxy, $C_1$—$C_2$ alkyl substituted with $OCH_3$ or $OCH_2CH_3$, $C_6H_5$,

$OCH_2CH_3$, $C_6H_5$, 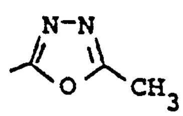, 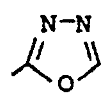,

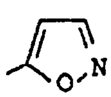, 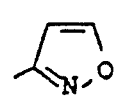, 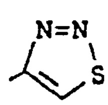, 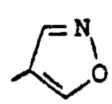, 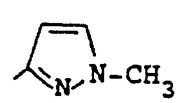,

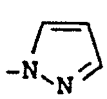, 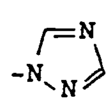, 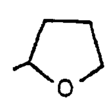, 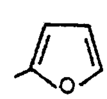 or 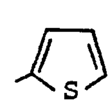;

$R_6$ is H, F, Cl, Br, $CF_3$, $CH_3$, $OCH_3$, $SCH_3$ or $OCF_2H$;
$R_7$ is H, $CH_3$, $OCH_3$, F, Cl, Br, $SO_2N(CH_3)_2$ $OSO_2CH_3$ or $S(O)_nCH_3$;
$R_8$ is $CH_3$, $CH_2CH_3$, $OCH_3$, $OCH_2CH_3$, F, Cl, Br, $SO_2NR_{20}R_{21}$, $SO_2N(OCH_3)CH_3$ or $S(O)_nR_{23}$;
$R_9$ is $C_1$—$C_3$ alkyl, F, Cl, Br, $NO_2$, $CO_2R_{19}$, $SO_2NR_{20}R_{21}$, $SO_2N(OCH_3)CH_3$ or $S(O)_nR_{23}$;
$R_{10}$ is Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ or $OCH_2CH_3$;
$R_{11}$ is H, $CH_3$ or $CH_2CH_3$;
$R_{12}$ is H, $CH_3$ or $CH_2CH_3$;

$R_{13}$ is H or $CH_3$;
$R_{14}$ is H or $CH_3$;
$R_{15}$ is H or $CH_3$;
$R_{16}$ is $CH_3$ or $CH_2CH_3$;
$R_{17}$ is H or $C_1$—$C_4$ alkyl;
$R_{18}$ is H or $CH_3$;
$R_{19}$ is $C_1$—$C_4$ alkyl, $CH_2CH_2OCH_3$, $CH_2CH_2Cl$ or $CH_2CH{=}CH_2$;
$R_{20}$ is $C_1$—$C_3$ alkyl;
$R_{21}$ is $C_1$—$C_3$ alkyl;
$R_{22}$ is $C_1$—$C_3$ alkyl or $N(CH_3)_2$;
$R_{23}$ is $C_1$—$C_3$ alkyl or $CH_2CH{=}CH_2$;
m is 0 or 1;
n is 0 or 2;
$Q_1$ is O, S, $SO_2$ or $NR_{17}$;
$Q_2$ is O, S or $NR_{17}$; and
W is O, S or $SO_2$;
A is

A is 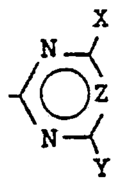 , 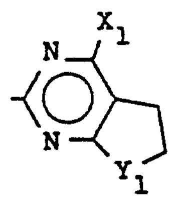 , 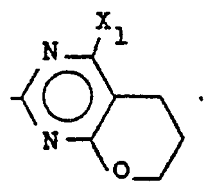 .

A-1 A-2 A-3

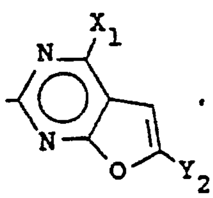 , 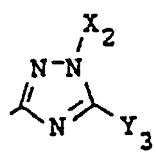 or 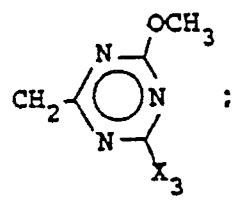 ;

A-4 A-5 A-6

X is $CH_3$, $OCH_3$, $OCH_2CH_3$, Cl, F, Br, $OCF_2H$, $CH_2F$ or $CF_3$;
Y is H, $CH_3$, $OCH_3$, $OCH_2CH_3$, $CH_2OCH_3$, $CH_2OCH_2CH_3$, $N(CH_3)_2$, $CH_2CH_3$, $CF_3$ $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

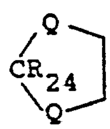 , 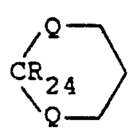 , 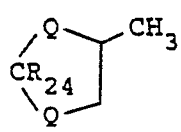

or $CR_{24}(QCH_2CH_3)_2$;
Q is O or S;
$R_{24}$ is H or $CH_3$;
Z is CH or N;
$Y_1$ is $CH_2$ or O;
$X_1$ is $CH_3$, $OCH_3$, $OCH_2CH_3$ or $OCF_2H$;
$Y_2$ is H or $CH_3$;
$X_2$ is $CH_3$, $CH_2CH_3$ or $CH_2CF_3$;
$Y_3$ is $OCH_3$, $OCH_2CH_3$, $SCH_3$, $CH_3$ or $CH_2CH_3$;
$X_3$ is $CH_3$ or $OCH_3$;
and their agriculturally suitable salts; provided that
1) the total number of carbon atoms of $R_{20}$ and $R_{21}$ is less than or equal to four;
2) when m is 1, then $R_{13}$ is H;
3) when L is L—17, then $R_{17}$ and $R_{18}$ are not simultaneously H; and
4) when X is Cl, F or Br, then Z is CH and Y is $OCH_3$, $OCH_2CH_3$, $N(CH_3)_2$ or $OCF_2H$.

which comprises reacting an isothiourea derivative of formula

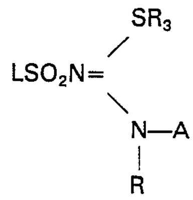

(III)

wherein L, R, $R_d$ and A are as defined above, with hydroxylamine, to obtain a compound of formula (II) wherein $R_c$ is H, and optionally reacting the product with $R_cCl$ or $R_3NCO$, wherein $R_c$ is $C(O)R_1$, $C(O)NR_2R_3$ or $CO_2R_4$ and $R_1$, $R_2$, $R_3$ and $R_4$ are as defined above.

9. A process of claim 8 wherein:
R is H or $CH_3$;
$R_c$ is as defined in claim 8;
L is L—1;
$R_5$ is $C_1$—$C_4$ alkyl, $C_1$—$C_4$ alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, or $C_1$—$C_2$ alkyl substituted with $OCH_3$ or $OC_2H_5$;
$R_6$ is H, F, Cl, Br, $CF_3$, $CH_3$, $OCH_3$ or $SCH_3$;
$R_{19}$, $R_{20}$, $R_{21}$, $R_{23}$ and W are as defined in claim 8;
$R_{22}$ is $C_1$—$C_3$ alkyl;
A is A—1;
X is $CH_3$, $OCH_3$ or Cl;
Y is $CH_3$, $C_2H_5$, $OCH_3$, $OC_2H_5$, $CH_2OCH_3$, $CH(OCH_3)_2$ or

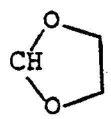

and Z is as defined in claim 8.

10. A process of claim 8 or 9 where R is H, $R_c$ is H and A is A—1.

11. A process of claim 10 where Y is $CH_3$, $OCH_3$, $OCH_2CH_3$, $CH_2OCH_3$, $OCH_2CF_3$ or $CH(OCH_3)_2$;
X is $CH_3$, $OCH_3$, Cl or $CF_3$; and
L is L—1, L—2, L—3, L—5, L—8, L—10, L—11, L—16 or L—17.

12. A process of Claim 11 where L is L—1; $R_5$ is $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

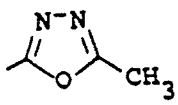 , 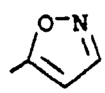 , 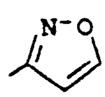 or 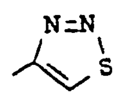 ;

$R_{22}$ is $C_1$—$C_3$ alkyl; $R_{23}$ is $CH_3$ and n is 2.

13. A process of claim 11 where L is L—2 and $R_7$ is Cl, $CH_3$, $OCH_3$, $SCH_3$ or Br; or
where L is L—3 and $R_8$ is Cl, $SO_2CH_3$ or $SO_2N(CH_3)_2$; or
where L is L—5 and $R_9$ is $CO_2CH_3$ or $CO_2CH_2CH_3$.

14. A process of Claim 8 or 9 wherein the product is a compound selected from
2-[[1-(4,6-dimethylpyrimidin-2-yl)amino]-1-(hydroxyimino)methyl]-aminosulfonyl]benzoic acid methyl ester;
2-[[1-(4-methoxy-6-methylpyrimidin-2-yl)amino]-1-(hydroxyimino)-methyl]aminosulfonyl]benzoic acid methyl ester;
N',N'-dimethyl-N-[[1-(4,6-dimethyl-1,3,5-triazin-2-yl)amino]-1-(hydroxyimino)methyl]-1,2-benzenedisulfonamide; and
N',N'-dimethyl-N-[[1-(4-methoxy-6-methylpyrimidin-2-yl)amino]-1-(hydroxyimino)methyl]-1,2-benzenedisulfonamide.

15. A method for controlling the growth of undesired vegetation by applying to the locus to be protected an effective amount of a herbicidal compound characterized in that said herbicidal compound comprises a compound of formulae (I) or (II) as defined in any of claims 1 to 14.

16. A method for regulating the growth of plants by applying to the locus of said plants an effective but substantially non-phytotoxic amount of a plant growth regulant, characterized in said plant growth regulant comprises a compound of formulae (I) or (II) as defined in any of claims 1 to 14.

**Patentansprüche für die Vertragsstaaten: BE CH DE FR GB IT LI LU NL SE**

1. Verbindung der Formel

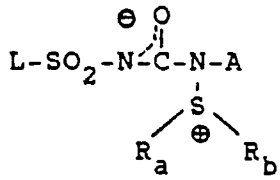

I

worin
$R_a$ $CH_3$ oder $CH_2CH_3$ ist;
$R_b$ $CH_3$ oder $CH_2CH_3$ ist;
L

L is 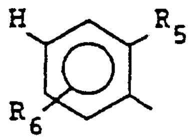 , 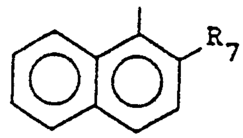 , 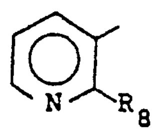 ,

L-1 L-2 L-3

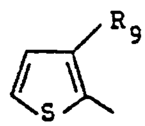 , 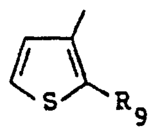 , 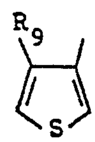 ,

L-4 L-5 L-6

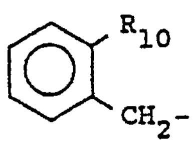 , 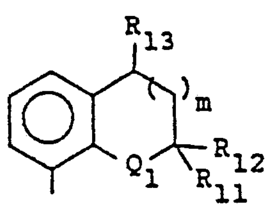 ,

L-7 L-8

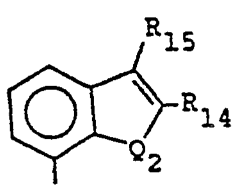 , 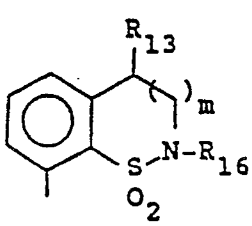 , 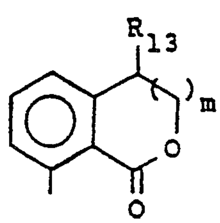 ,

L-9 L-10 L-11

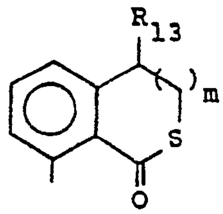, 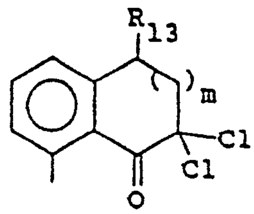, 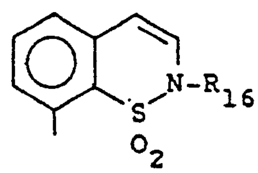,

L-12 L-13 L-14

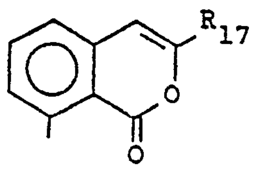, 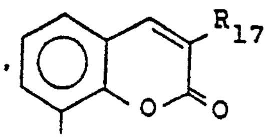 or 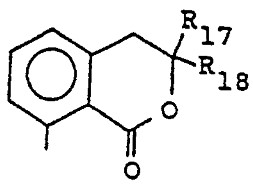;

L-15 L-16 L-17

$R_5$ $C_1$—$C_4$ Alkyl, $C_1$—$C_4$ Alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ Alkenyloxy, $C_3$—$C_4$ Alkinyloxy, mit $OCH_3$ oder $OCH_2CH_3$ substituiertes $C_1$—$C_2$ Alkyl, $C_6H_5$,

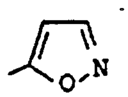, 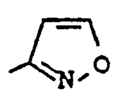, 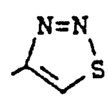, 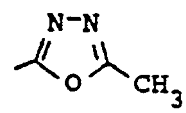, 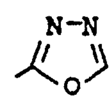,

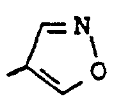, 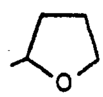, 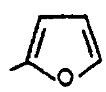 oder 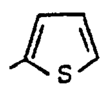 ist;

$R_6$ H, F, Cl, Br, $CF_3$, $CH_3$, $OCH_3$, $SCH_3$ oder $OCF_2H$ ist;
$R_7$ H, $CH_3$, $OCH_3$, F, Cl, Br, $OSO_2CH_3$, $SO_2N(CH_3)_2$ oder $S(O)_nCH_3$ ist;
$R_8$ $CH_3$, $CH_2CH_3$, $OCH_3$, $OCH_2CH_3$, F, Cl, Br, $SO_2NR_{20}R_{21}$, $SO_2N(OCH_3)CH_3$ oder $S(O)_nR_{23}$ ist;
$R_9$ $C_1$—$C_3$ Alkyl, F, Cl, Br, $NO_2$, $CO_2R_{19}$, $SO_2NR_{20}R_{21}$, $SO_2N(OCH_3)CH_3$ oder $S(O)_nR_{23}$ ist;
$R_{10}$ Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ $OCH_2CH_3$ ist;
$R_{11}$ H, $CH_3$ oder $CH_2CH_3$ ist;
$R_{12}$ H, $CH_3$ oder $CH_2CH_3$ ist;
$R_{13}$ H oder $CH_3$ ist;
$R_{14}$ H oder $CH_3$ ist;
$R_{15}$ H oder $CH_3$ ist;
$R_{16}$ $CH_3$ oder $CH_2CH_3$ ist;
$R_{17}$ H oder $C_1$—$C_4$ Alkyl ist;
$R_{18}$ H oder $CH_3$ ist;
$R_{19}$ $C_1$—$C_4$ Alkyl, $CH_2CH_2OCH_3$, $CH_2CH_2Cl$ oder $CH_2CH{=}CH_2$ ist;
$R_{20}$ $C_1$—$C_3$ Alkyl ist;
$R_{21}$ $C_1$—$C_3$ Alkyl ist;
$R_{22}$ $C_1$—$C_3$ Alkyl oder $N(CH_3)_2$ ist;
$R_{23}$ $C_1$—$C_3$ Alkyl oder $CH_2CH{=}CH_2$ ist;
m 0 oder 1 ist;
n 0 oder 2 ist;
$Q_1$ O, S, $SO_2$ oder $NR_{17}$ ist;
$Q_2$ O, S oder $NR_{17}$; und
W O, S oder $SO_2$ ist;

A 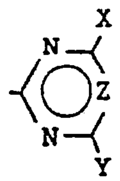 , 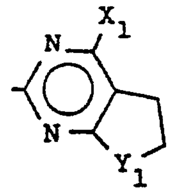 , 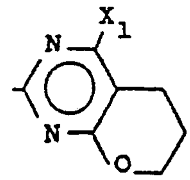 .

A-1 A-2 A-3

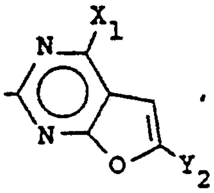 , 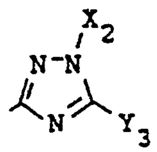 or 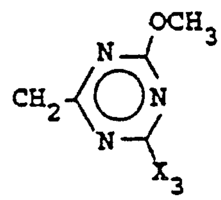 ;

A-4 A-5 A-6

X $CH_3$, $OCH_3$, $OCH_2CH_3$, Cl, F, Br, $OCF_2H$, $CH_2F$ oder $CF_3$ ist;

Y H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

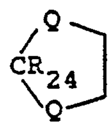 , 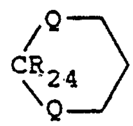 , 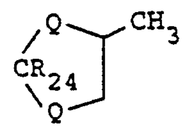

oder $CR_{24}(QCH_2CH_3)_2$ ist;

Q 0 oder S ist;

$R_{24}$ H oder $CH_3$ ist;

Z CH oder N ist;

$Y_1$ $CH_2$ oder O ist;

$X_1$ $CH_3$, $OCH_3$, $OCH_2CH_3$ oder $OCF_2H$ ist;

$Y_2$ H oder $CH_3$ ist;

$X_2$ $CH_3$, $CH_2CH_3$ oder $CH_2CF_3$ ist;

$Y_3$ $OCH_3$, $OCH_2CH_3$, $SCH_3$, $CH_3$ oder $CH_2CH_3$ ist;

$X_3$ $CH_3$ oder $OCH_3$ ist;

mit der Massgabe, dass

1) die Gesamtzahl der Kohlenstoffatome von $R_{20}$ und $R_{21}$ weniger als oder gleich vier ist;

2) $R_{13}$ H ist, wenn m 1 ist;

3) $R_{17}$ und $R_{18}$ nicht gleichzeitig H sind, wenn L L—17 ist;

4) wenn X Cl, F oder Br ist, dann ist Z $CH_3$ und Y ist $OCH_3$, $OCH_2CH_3$, $N(CH_3)_2$ oder $OCF_2H$.

2. Verbindung von Anspruch 1, worin A A—1 ist.

3. Verbindung von Anspruch 2, worin Y $CH_3$, $OCH_3$, $OCH_2CH_3$, $CH_2OCH_3$, $OCH_2CF_3$ oder $CH(OCH_3)_2$ und X $CH_3$, $OCH_3$, Cl oder $CF_3$ ist.

4. Verbindung von Anspruch 3, worin L L—1, L—2, L—3, L—5, L—8, L—10, L—11, L—16 oder L—17 ist.

5. Verbindung von Anspruch 4, worin L L—1; $R_5$ $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $SO_nR_{23}$, $OCH_2H$, $SCF_2H$, $OCH_2CH{=}CH_2$, $OCH_2C{\equiv}CH$,

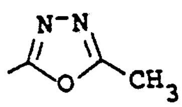 , 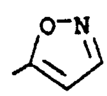 , 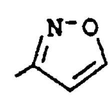 oder 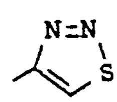 ;

$R_{22}$ $C_1$—$C_3$ Alkyl; $R_{23}$ $CH_3$ und n 2 ist.

6. Verbindung von Anspruch 4, worin L L—2 und $R_7$ Cl, $CH_3$, $OCH_3$, $SCH_3$ oder Br ist.

7. Verbindung von Anspruch 4, worin L L—3 und $R_8$ Cl, $SO_2CH_3$ oder $SO_2N(CH_3)_2$ ist.

8. Verbindung von Anspruch 4, worin L L—5 und $R_9$ $CO_2CH_3$ oder $CO_2CH_2CH_3$ ist.

9. Die Verbindung von Anspruch 1, nämlich Inneres Dimethylsulfoniumsalz von 2-[[(4-Methoxy-6-methylpyrimidin-2-yl)aminocarbonyl]aminosulfonyl]benzoesäuremethylester.

10. Die Verbindung von Anspruch 1, nämlich Inneres Dimethylsulfoniumsalz von 2-[[(4,6-Dimethoxypyrimidin-2-yl)aminocarbonyl]aminosulfonyl]-benzoesäuremethylester.

11. Die Verbindung von Anspruch 1, nämlich Inneres Dimethylsulfoniumsalz von N-[(4,6-Dimethylpyrimidin-2-yl)aminocarbonyl]-2-methylsulfonylbenzolsulfonamid.

12. Die Verbindung von Anspruch 1, nämlich Inneres Dimethylsulfoniumsalz von 2-[[(4,6-Dimethylpyrimidin-2-yl)aminocarbonyl]aminosulfonyl]-benzoesäuremethylester.

13. Die Verbindung von Anspruch 1, nämlich Inneres Dimethylsulfoniumsalz von N-[(4,6-Dimethoxypyrimidin-2-yl)aminocarbonyl]-2-dimethylaminosulfonylbenzolsulfonamid.

14. Verbindung der Formel

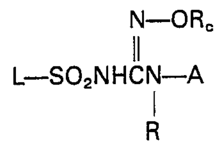

(II)

worin

R H oder $CH_3$ ist;
$R_c$ H, $C(O)R_1$, $C(O)NR_2R_3$ oder $CO_2R_4$ ist;
$R_1$ $C_1$—$C_3$ Alkyl oder $CF_3$ ist;
$R_2$ H, $CH_3$ oder $C_2H_5$ ist;
$R_3$ $C_1$—$C_3$ Alkyl;
$R_4$ $C_1$—$C_3$ Alkyl;

L

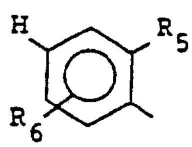

L-1

,

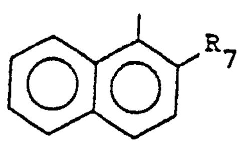

L-2

,

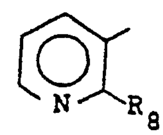

L-3

,

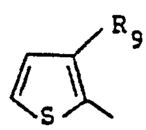

L-4

,

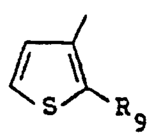

L-5

,

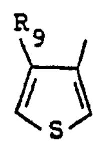

L-6

,

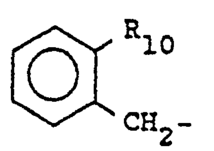

L-7

,

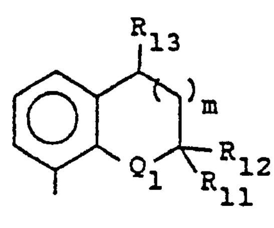

L-8

,

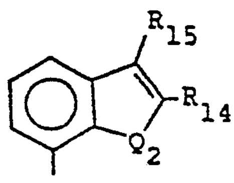

L-9

,

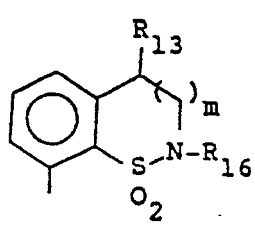

L-10

,

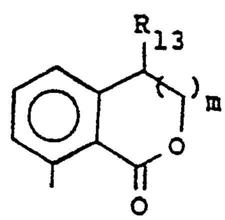

L-11

,

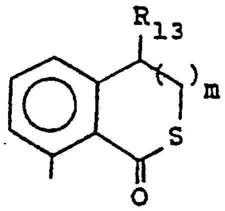 , 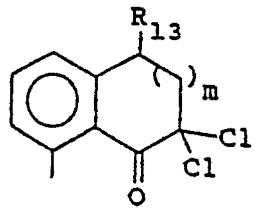 , 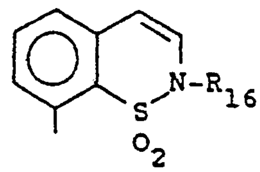 ,

L-12 L-13 L-14

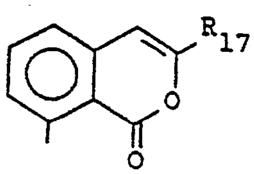 , 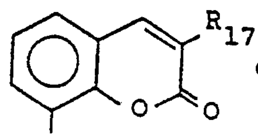 oder 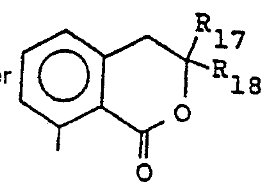 ;

L-15 L-16 L-17

$R_5$ $C_1$—$C_4$ Alkyl, $C_1$—$C_4$ Alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ Alkenyloxy, $C_3$—$C_4$ Alkinyloxy, mit $OCH_3$ oder $OCH_2CH_3$ substituiertes $C_1$—$C_2$ Alkyl, $C_6H_5$,

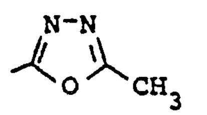 , 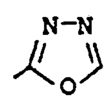 ,

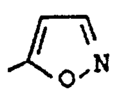 , 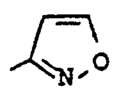 , 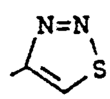 , 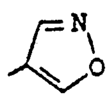 , 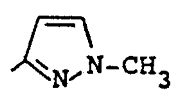 ,

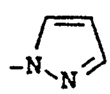 , 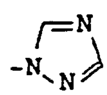 , 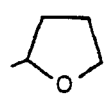 , 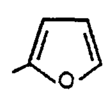 oder 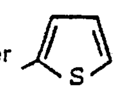 ;

$R_6$ H, F, Cl, Br, $CF_3$, $CH_3$, $OCH_3$, $SCH_3$ oder $OCF_2H$ ist;
$R_7$ H, $CH_3$, $OCH_3$, F, Cl, Br, $SO_2N(CH_3)_2$, $OSO_2CH_3$ oder $S(O)_nCH_3$ ist;
$R_8$ $CH_3$, $CH_2CH_3$, $OCH_3$, $OCH_2CH_3$, F, Cl, Br, $SO_2NR_{20}R_{21}$, $SO_2N(OCH_3)CH_3$ oder $S(O)_nR_{23}$ ist;
$R_9$ $C_1$—$C_3$ Alkyl, F, Cl, Br, $NO_2$, $CO_2R_{19}$, $SO_2NR_{20}R_{21}$, $SO_2N(OCH_3)CH_3$ oder $S(O)_nR_{23}$ ist;
$R_{10}$ Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ oder $OCH_2CH_3$ ist;
$R_{11}$ H, $CH_3$ oder $CH_2CH_3$ ist;
$R_{12}$ H, $CH_3$ oder $CH_2CH_3$ ist;
$R_{13}$ H oder $CH_3$ ist;
$R_{14}$ H oder $CH_3$ ist;
$R_{15}$ H oder $CH_3$ ist;
$R_{16}$ $CH_3$ oder $CH_2CH_3$ ist;
$R_{17}$ H oder $C_1$—$C_4$ Alkyl ist;
$R_{18}$ H oder $CH_3$ ist;
$R_{19}$ $C_1$—$C_4$ Alkyl, $CH_2CH_2OCH_3$, $CH_2CH_2Cl$ oder $CH_2CH{=}CH_2$ ist;
$R_{20}$ $C_1$—$C_3$ Alkyl ist;
$R_{21}$ $C_1$—$C_3$ Alkyl ist;
$R_{22}$ $C_1$—$C_3$ Alkyl oder $N(CH_3)_2$ ist;
$R_{23}$ $C_1$—$C_3$ Alkyl oder $CH_2CH{=}CH_2$ ist;
m 0 oder 1 ist;
n 0 oder 2 ist;

$Q_1$ O, S, $SO_2$ oder $NR_{17}$ ist;
$Q_2$ O, S, oder $NR_{17}$ ist; und
W O, S oder $SO_2$ ist;

A 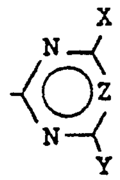 , 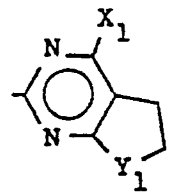 , 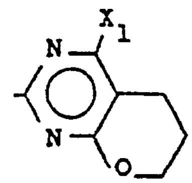 ,

A-1 A-2 A-3

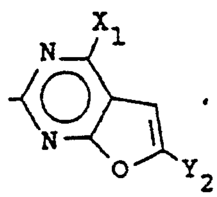 , 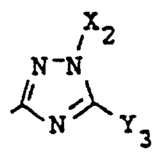 oder 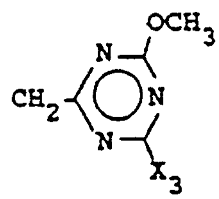 ;

A-4 A-5 A-6

X $CH_3$, $OCH_3$, $OCH_2CH_3$, Cl, F, Br, $OCF_2H$, $CH_2F$ oder $CF_3$ ist;
Y H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH=CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

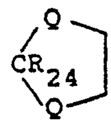 , 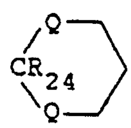 , 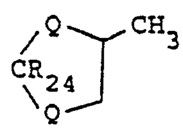

oder $CR_{24}(QCH_2CH_3)_2$ ist;
Q O oder S ist;
$R_{24}$ H oder $CH_3$ ist;
Z CH oder N ist;
$Y_1$ $CH_2$ oder O ist;
$X_1$ $CH_3$, $OCH_3$, $OCH_2CH_3$ oder $OCF_2H$ ist;
$Y_2$ H oder $CH_3$ ist;
$X_2$ $CH_3$, $CH_2CH_3$ oder $CH_2CF_3$ ist;
$Y_3$ $OCH_3$, $OCH_2CH_3$, $SCH_3$, $CH_3$ oder $CH_2CH_3$ ist;
$X_3$ $CH_3$ oder $OCH_3$ ist;
und ihre landwirtschaftlich geeigneten Salze;
mit er Massgabe, dass
1) die Gesamtzahl von Kohlenstoffatomen von $R_{20}$ und $R_{21}$ weniger als oder gleich vier ist;
2) $R_{13}$ H ist, wenn m 1 ist;
3) $R_{17}$ und $R_{18}$ nicht gleichzeitig H sind, wenn L L—17 ist; und
4) Wenn X Cl, F oder Br ist, dann ist Z CH und Y ist $OCH_3$, $OCH_2CH_3$, $N(CH_3)_2$ oder $OCF_2H$.

15. Verbindung von Anspruch 14, worin R H ist, $R_c$ H ist und A A—1 ist.

16. Verbindung von Anspruch 15, worin Y $CH_3$, $OCH_3$, $OCH_2CH_3$, $CH_2OCH_3$, $OCH_2CF_3$ oder $CH(OCH_3)_2$ und X $CH_3$, $OCH_3$, Cl oder $CF_3$ ist.

17. Verbindung von Anspruch 16, worin L L—1, L—2, L—3, L—5, L—8, L—10, L—11, L—16 oder L—17 ist.

18. Verbindung von Anspruch 17, worin L L—1 ist, $R_5$ $OCH_3$, $OCH_2CH_3$, Cl, NO, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH=CH_2$, $OCH_2C{\equiv}CH$,

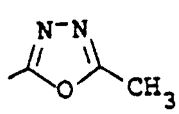 , 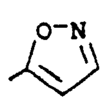 , 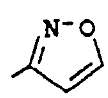 oder 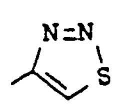 ist;

$R_{22}$ $C_1$—$C_3$ Alkyl; $R_{23}$ $CH_3$ und n 2 ist.

19. Verbindung von Anspruch 17, worin L L—2 und $R_7$ Cl, $CH_3$, $CH_3$, $OCH_3$, $SCH_3$ oder Br ist.

20. Verbindung von Anspruch 17, worin L L—3 und $R_8$ Cl, $SO_2CH_3$ oder $SO_2N(CH_3)_2$ ist.

21. Verbindung von Anspruch 17, worin L L—5 und $R_9$ $CO_2CH_3$ oder $CO_2CH_2CH_3$ ist.

22. Verbindung von Anspruch 14, nämlich 2-[[1-(4,6-Dimethylpyrimidin-2-yl)amino]-1-(hydroxyimino)-methyl]aminosulfonyl]benzoesäuremethylester.

23. Verbindung von Anspruch 14, nämlich 2-[[1-(4-Methoxy-6-methylpyrimidin-2-yl)amino]-1-(hydroxyimino)methyl]aminosulfonyl]benzoesäure.

24. Verbindung von Anspruch 14, nämlich N',N'-Dimethyl-N-[[1-(4,6-dimethyl-1,3,5-triazin-2-yl)amino]-1-(hydroxyimino)methyl]-1,2-benzoldisulfonamid.

25. Verbindung von Anspruch 14, nämlich N',N'-Dimethyl-N-[[1-(4-methoxy-6-methylpyrimidin-2-yl)-amino]-1-(hydroxyimino)methyl]-1,2-benzoldisulfonamid.

26. Verbindung von Anspruch 1, worin L L—1, L—2, L—3, L—4, L—5, L—8, L—10, L—11 oder L—17 ist;
$R_5$ $C_1$—$C_4$ Alkyl, $C_1$—$C_4$ Alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, $C_3$—$C_4$ Alkenyloxy, $CH_2CH_2OCH_3$, $CH_2CH_2OC_2H_5$,

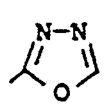 , 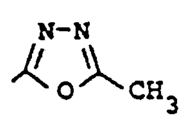 , 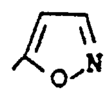 , 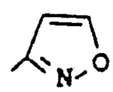 oder 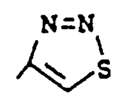 ist;

$R_6$ H, F, Cl, Br, $CF_3$, $CH_3$ oder $OCH_3$ ist;
$R_7$ H, $CH_3$, $OCH_3$, Cl, $OSO_2CH_3$ oder $S(O)_nCH_3$ ist;
$R_8$ $CH_3$, $OCH_3$, Cl, $SO_2NR_{20}R_{21}$ oder $SO_2N(OCH)CH_3$ ist;
$R_9$ $CH_3$, Cl, Br, $NO_2$, $CO_2R_{19}$, $SO_2NR_{20}R_{21}$, $SO_2N(OCH_3)CH_3$ oder $S(O)_nR_{13}$ ist;
$R_{11}$ H oder $CH_3$ ist;
$R_{12}$ H oder $CH_3$ ist;
$R_{13}$ H ist;
$R_{17}$ und $R_{18}$ H sind;
$R_{22}$ $C_1$—$C_3$ Alkyl ist;
$Q_1$ O, S oder $SO_2$ ist;
A A—1 ist;
X $CH_3$ oder $OCH_3$ ist; oder Cl sein kann, wenn Y $CH_3$ oder $OCH_3$ ist;
Y H, $CH_3$, $OCH_3$, $OCH_2OCH_3$, $CH_2CH_3$, $CF_3$, $—OCH_2CF_3$, oder $CH(OCH_3)_2$ ist;
und $R_a$, $R_b$, $R_{16}$, $R_{19}$, $R_{20}$, $R_{21}$, $R_{23}$, m, n und Z wie in Anspruch 1 definiert sind.

27. Verbindung von Anspruch 14, worin
R H oder $CH_3$ ist;
$R_c$ wie in Anspruch 14 definiert ist;
L L—1 ist;
$R_5$ $C_1$—$C_4$ Alkyl, $C_1$—$C_4$ Alkoxy, F, Cl, Br, $NO_2$, $CF_3$, $CO_2R_{19}$, $SO_2R_{20}R_{21}$, $SO_2N(OCH_3)CH_3$, $OSO_2R_{22}$, $S(O)_nR_{23}$, $WCF_3$, $WCHF_2$, oder mit $OCH_3$ oder $OC_2H_5$ substituiertes $C_1$—$C_2$ Alkyl ist;
$R_6$ H, F, Cl, Br, $CF_3$, $CH_3$, $OCH_3$ oder $SCH_3$ ist;
$R_{19}$, $R_{20}$, $R_{21}$, $R_{23}$ und W wie in Anspruch 14 definiert sind;
$R_{22}$ $C_1$—$C_3$ Alkyl ist;
A A—1 ist;
X $CH_3$, $OCH_3$ oder Cl ist;
Y $CH_3$, $C_2H_5$, $OCH_3$, $OC_2H_5$, $CH_2OCH_3$, $CH(OCH_3)_2$ oder

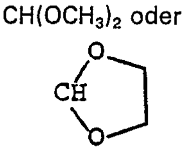

und Z wie in Anspruch 14 definiert ist.

28. Zusammensetzung geeignet zur Bekämpfung des Wachstums unerwünschter Vegetation oder zur Regulierung des Wachstums von Pflanzen, welche eine wirksame Menge einer herbiziden Verbindung oder eine das Wachstum von Pflanzen regulierende Verbindung und mindestens eines der folgenden: oberflachenaktives Mittel, festes oder flüssiges Verdünnungsmittel umfasst, dadurch gekennzeichnet, dass die herbizide Verbindung oder die das Wachstum von Pflanzen regulierende Verbindung eine Verbindung nach einem der Ansprüche 1 bis 27 enthält.

29. Verfahren zur Bekämpfung des Wachstums unerwünschter Vegetation durch Aufbringung einer wirksamen Menge einer herbizider Verbindung auf die zu schützende Stelle, dadurch gekennzeichnet, dass die herbizide Verbindung eine Verbindung nach einem der Ansprüche 1 bis 27 enthält.

30. Verfahren zur Regulierung des Wachstums von Pflanzen durch aufbringung einer wirksamen, aber im wesentlichen nicht-phytotoxischen Menge eines Pflanzenwachstumsreglers auf die schützende Stelle, dadurch gekennzeichnet, daß der Wachstumsregler eine Verbindung nach den Ansprüchen 1 bis 27 enthält.

31. Verfahren zur Herstellung einer Verbindung von Anspruch 1, bei dem ein Sulfonylisocyanat der Formel

$$LSO_2NCO \quad (IV)$$

worin L wie in Anspruch 1 definiert ist, mit einem S,S-Dialkyl-N-(heterocyclisch)sulfilimin der Formel

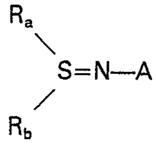

(V)

worin $R_a$, $R_b$ und A wie Anspruch 1 definiert sind, in Reaktion gebracht wird.

32. Verfahren zur herstellung einer Verbindung von Anspruch 14, bei dem ein Isothioharnstoffderivat der Formel

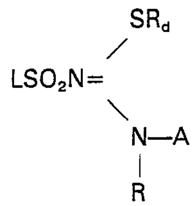

(III)

worin L, R, $R_d$ und A wie in Anspruch 26 definiert sind, mit Hydroxylamin in Reaktion gebracht wird, um eine Verbindung von Anspruch 14 zu erhalten, in den $R_c$ H ist, und gegebenenfalls das Produkt mit $R_cCl$ oder $R_3NCO$ in Reaktion gebracht wird, worin $R_c$ $C(O)R_1$, $C(O)NR_2R_3$ oder $CO_2R_4$ ist und $R_1$, $R_2$, $R_3$ und $R_4$ wie in Anspruch 14 definiert sind.

**Patentansprüche für den Vertragsstaat: AT**

1. Verfahren zur Herstellung einer Verbindung der Formel

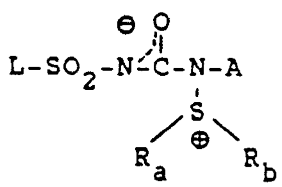

I

worin
$R_a$ $CH_3$ oder $CH_2CH_3$ ist;
$R_b$ $CH_3$ oder $CH_2CH_3$ ist;

L

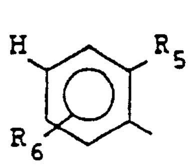

L-1

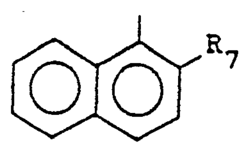

L-2

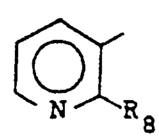

L-3

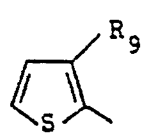

L-4

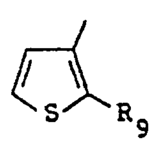

L-5

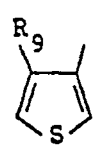

L-6

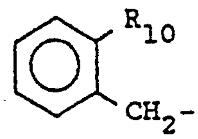 , 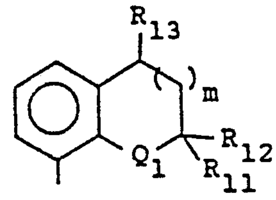 ,

L-7 L-8

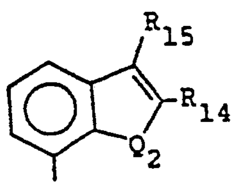 , 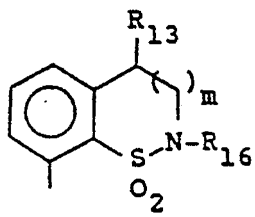 , 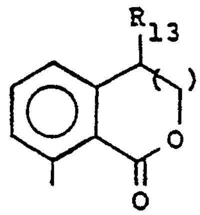 ,

L-9 L-10 L-11

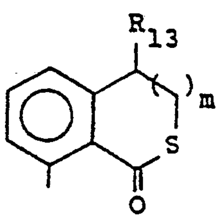 , 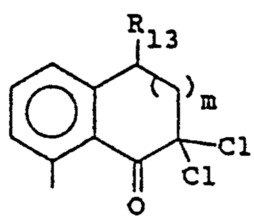 , 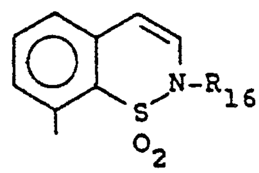 ,

L-12 L-13 L-14

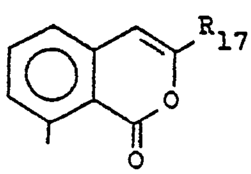 , 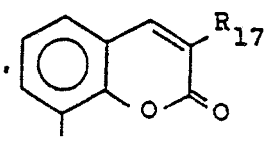 or 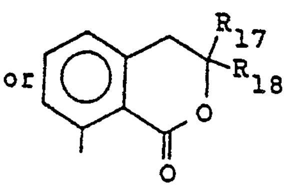 ;

L-15 L-16 L-17

$R_5$ $C_1$—$C_4$ Alkyl, $C_1$—$C_4$ Alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ Alkenyloxy, $C_3$—$C_4$ Alkinyloxy, mit $CCH_3$ oder $OCH_2CH_3$ substituiertes $C_1$ bis $C_2$ Alkyl, $C_6H_5$,

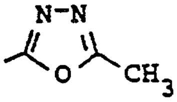 , 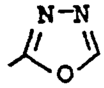 , 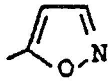 , 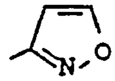 , 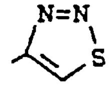 ,

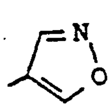 , 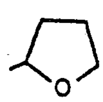 , 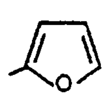 oder 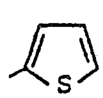 ist;

$R_6$ H, F, Cl, Br, $CF_3$, $CH_3$, $OCH_3$, $SCH_3$ oder $OCF_2H$ ist;
$R_7$ H, $CH_3$, $OCH_3$, F, Cl, Br, $OSO_2CH_3$, $SO_2N(CH_3)_2$ oder $S(O)_nCH_3$ ist;

$R_8$ $CH_3$, $CH_2CH_3$, $OCH_3$, $OCH_2CH_3$, F, Cl, Br, $SO_2NR_{20}R_{21}$, $SO_2N(OCH_3)CH_3$ oder $S(O)_nR_{23}$ ist;
$R_9$ $C_1$—$C_3$ Alkyl, F, Cl, Br, $NO_2$, $CO_2R_{19}$, $SO_2NR_{20}R_{21}$, $SO_2N(OCH_3)CH_3$ oder $S(O)_nR_{23}$ ist;
$R_{10}$ Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ oder $OCH_2CH_3$ ist;
$R_{11}$ H, $CH_3$ oder $CH_2CH_3$ ist;
$R_{12}$ H, $CH_3$ oder $CH_2CH_3$ ist;
$R_{13}$ H oder $CH_3$ ist;
$R_{14}$ H oder $CH_3$ ist;
$R_{15}$ H oder $CH_3$ ist;
$R_{16}$ $CH_3$ oder $CH_2CH_3$ ist;
$R_{17}$ H oder $C_1$—$C_4$ Alkyl ist;
$R_{18}$ H oder $CH_3$ ist;
$R_{19}$ $C_1$—$C_4$ Alkyl, $CH_2CH_2OCH_3$, $CH_2CH_2Cl$ oder $CH_2CH{=}CH_2$ ist;
$R_{20}$ $C_1$—$C_3$ Alkyl ist;
$R_{21}$ $C_1$—$C_3$ Alkyl ist;
$R_{22}$ $C_1$—$C_3$ Alkyl oder $N(CH_3)_2$ ist;
$R_{23}$ $C_1$—$C_3$ Alkyl oder $CH_2CH{=}CH_2$ ist;
m 0 oder 1 ist;
n 0 oder 2 ist;
$Q_1$ O, S, $SO_2$ oder $NR_{17}$ ist;
$Q_2$ O, S oder $NR_{17}$; und
W O, S oder $SO_2$ ist;

A 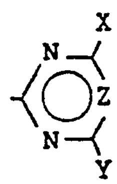 , 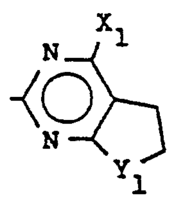 , 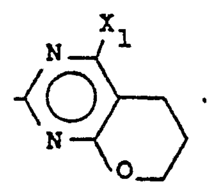 .

A-1 A-2 A-3

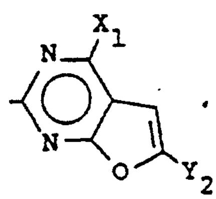 , 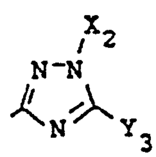 or 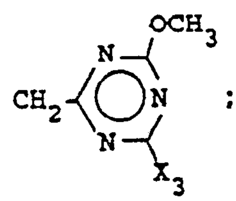 ;

A-4 A-5 A-6

X $CH_3$, $OCH_3$, $OCH_2CH_3$, Cl, F, Br, $OCF_2H$, $CH_2F$ oder $CF_3$ ist;
Y H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

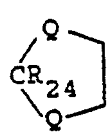 , 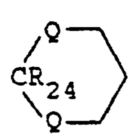 , 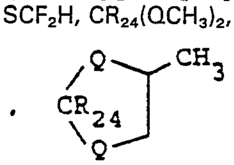

oder $CR_{24}(QCH_2CH_3)_2$ ist;
Q 0 oder S ist;
$R_{24}$ H oder $CH_3$ ist;
Z CH oder N ist;
$Y_1$ $CH_2$ oder O ist;
$X_1$ $CH_3$, $OCH_3$, $OCH_2CH_3$ oder $OCF_2H$ ist;
$Y_2$ H oder $CH_3$ ist;
$X_2$ $CH_3$, $CH_2CH_3$ oder $CH_2CF_3$ ist;
$Y_3$ $OCH_3$, $OCH_2CH_3$, $SCH_3$, $CH_3$ oder $CH_2CH_3$ ist;
$X_3$ $CH_3$ oder $OCH_3$ ist;
mit der Massgabe, dass

1) die Gesamtzahl der Kohlenstoffatome von $R_{20}$ und $R_{21}$ weniger als oder gleich vier ist;
2) $R_{13}$ H ist, wenn m 1 ist;
3) wenn L L—17 ist, dann sind $R_{17}$ und $R_{18}$ nicht gleichzeitig H;
4) wenn X Cl, F oder Br ist, dann ist Z $CH_3$ und Y ist $OCH_3$, $OCH_2CH_3$, $N(CH_3)_2$ oder $OCF_2H$, bei dem ein Sulfonylisocyanat der Formel

$$LSO_2NCO \qquad (IV)$$

worin L wie oben definiert ist, mit einem S,S-Dialkyl-N-(heterocyclisch)sulfilimin der Formel

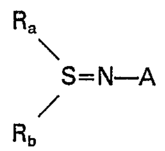
(V)

worin $R_a$, $R_b$ und A wie oben definiert sind, in Reaktion gebracht wird.

2. Verfahren von Anspruch 1, worin
L L—1, L—2, L—3, L—4, L—5, L—8, L—10, L—11, oder L—17 ist;
$R_5$ $C_1$—$C_4$ Alkyl, $C_1$—$C_4$ Alkoxy, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, $C_3$—$C_4$ Alkenyloxy, $CH_2CH_2OCH_3$, $CH_2CH_2OC_2H_5$,

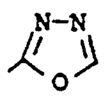 , 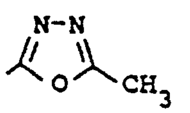 , 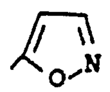 , 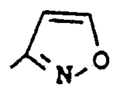 oder 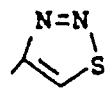 ist;

$R_6$ H, F, Cl, Br, $CF_3$, $CH_3$ oder $OCH_3$ ist;
$R_7$ H, $CH_3$, $OCH_3$, Cl, $OSO_2CH_3$ oder $S(O)_nCH_3$ ist;
$R_8$ $CH_3$, $OCH_3$, Cl, $SO_2NR_{20}R_{21}$ oder $SO_2N(OCH_3)CH_3$ ist;
$R_9$ $CH_3$, Cl, Br, $NO_2$, $CO_2R_{19}$, $SO_2NR_{20}R_{21}$, $SO_2N(OCH_3)CH_3$ oder $S(O)_nR_{13}$ ist;
$R_{11}$ H oder $CH_3$ ist;
$R_{12}$ H oder $CH_3$ ist;
$R_{13}$ H ist;
$R_{17}$ und $R_{18}$ H sind;
$R_{22}$ $C_1$—$C_3$ Alkyl ist;
$Q_1$ O, S oder $SO_2$ ist;
A A—1 ist;
X $CH_3$ oder $OCH_3$ ist; oder Cl sein kann, wenn Y $CH_3$ oder $OCH_3$ ist;
Y H, $CH_3$, $OCH_3$, $OCH_2CH_3$, $CH_2OCH_3$, $CH_2CH_3$, $CF_3$, —$OCH_2CF_3$ oder $CH(OCH_3)_2$ ist;
und $R_a$, $R_b$, $R_{16}$, $R_{19}$, $R_{20}$, $R_{21}$, $R_{23}$, m, n und Z wie in Anspruch 1 definiert sind.

3. Verfahren von Anspruch 1, worin A A—1 ist.

4. Verfahren von Anspruch 3, worin
Y $CH_3$, $OCH_3$, $OCH_2CH_3$, $CH_2OCH_3$, $OCH_2CF_3$ oder $CH(OCH_3)_2$ ist;
X $CH_3$, $OCH_3$, Cl oder $CF_3$ ist; und
L L—1, L—2, L—3, L—5, L—8, L—10, L—11, L—16 oder L—17 ist.

5. Verfahren von Anspruch 4, worin L L—1 ist; $R_5$ $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

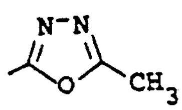 , 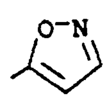 , 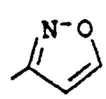 oder 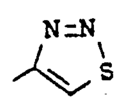 ist;

$R_{22}$ $C_1$—$C_3$ Alkyl ist;
$R_{23}$ $CH_3$ und
n 2 ist.

6. Verfahren von Anspruch 4, worin L L—2 und $R_7$ Cl, $CH_3$, $OCH_3$, $SCH_3$ oder Br ist; oder worin L L—3 ist und $R_8$ Cl, $SO_2CH_3$ oder $SO_2N(CH_3)_2$ ist; oder worin L L—5 und $R_9$ $CO_2CH_3$ oder $CO_2CH_2CH_3$ ist.

7. Verfahren von Anspruch 1 oder 2, worin das Produkt eine Verbindung ist ausgewählt aus

Inneres Dimethylsulfoniumsalz von 2-[[(4-Methoxy-6-methylpyrimidin-2-yl)aminocarbonyl]aminosulfonyl]benzoesäuremethylester;

Inneres Dimethylsulfoniumsalz von 2-[[(4,6-Dimethoxypyrimidin-2-yl)aminocarbonyl]aminosulfonyl]-benzoesäuremethylester;

Inneres Dimethylsulfoniumsalz von N-[(4,6-Dimethylpyrimidin-2-yl)aminocarbonyl]-2-methylsúlfonyl-benzolsulfonamid;

Inneres Dimethylsulfoniumsalz von 2-[[(4,6-Dimethylpyrimidin-2-yl)aminocarbonyl]aminosulfonyl]-benzoesäuremethylester;

Inneres Dimethylsulfoniumsalz von N-[(4,6-Dimethoxypyrimidin-2-yl)aminocarbonyl]-2-dimethylaminosulfonylbenzolsulfonamide;

8. Verfahren zur Herstellung einer Verbindung der Formel

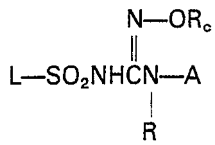

(II)

worin

R H oder $CH_3$ ist;
$R_c$ H, $C(O)R_1$, $C(O)NR_2R_3$ oder $CO_2R_4$ ist;
$R_1$ $C_1$—$C_3$ Alkyl oder $CF_3$ ist;
$R_2$ H, $CH_3$ oder $C_2H_5$ ist;
$R_3$ $C_1$—$C_3$ Alkyl;
$R_4$ $C_1$—$C_3$ Alkyl;

L 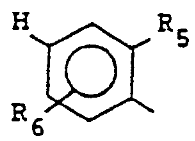 , 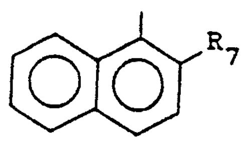 , 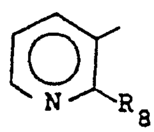 ,

L-1 L-2 L-3

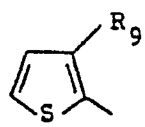 , 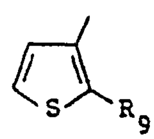 , 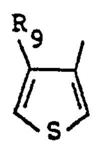 ,

L-4 L-5 L-6

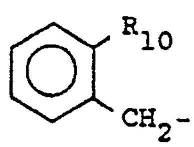 , 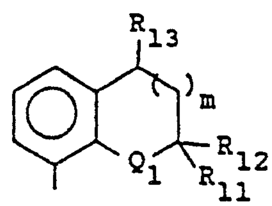 ,

L-7 L-8

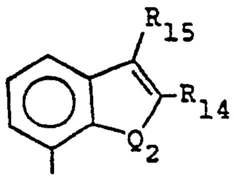 , 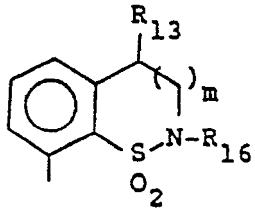 , 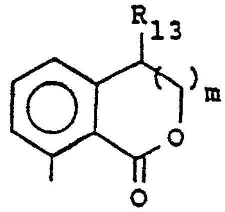 ,

L-9 L-10 L-11

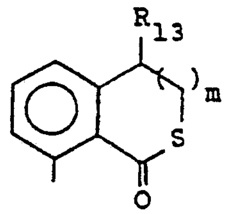 , 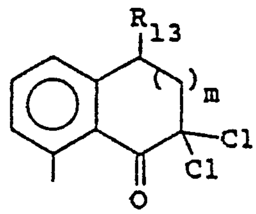 , 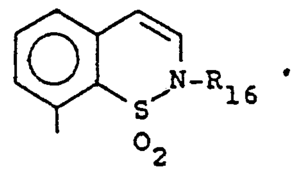 ,

L-12 L-13 L-14

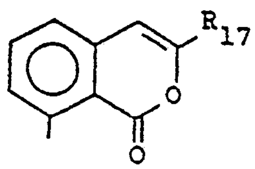 , 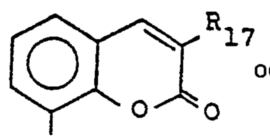 oder 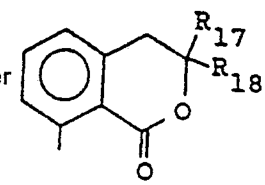 ist;

L-15 L-16 L-17

$R_5$ $C_1$—$C_4$ Alkyl, $C_1$—$C_4$ Alkoxy, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, $C_3$—$C_4$ Alkenyloxy, $C_3$—$C_4$ Alkinyloxy, mit $OCH_3$ oder $OCH_2CH_3$ substituiertes $C_1$—$C_2$ Alkyl, $C_6H_5$,

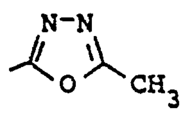 , 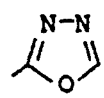 ,

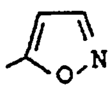 , 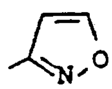 , 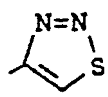 , 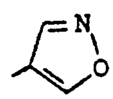 , 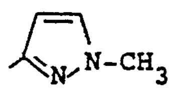 ,

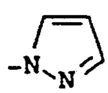 , 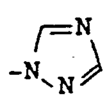 , 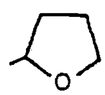 , 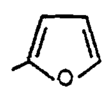 oder 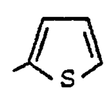 ist;

$R_6$ H, F, Cl, Br, $CF_3$, $CH_3$, $OCH_3$, $SCH_3$ oder $OCF_2H$ ist;
$R_7$ H, $CH_3$, $OCH_3$, F, Cl, Br, $SO_2N(CH_3)_2$, $OSO_2CH_3$ oder $S(O)_nCH_3$ ist;
$R_8$ $CH_3$, $CH_2CH_3$, $OCH_3$, $OCH_2CH_3$, F, Cl, Br, $SO_2NR_{20}R_{21}$, $SO_2N(OCH_3)CH_3$ oder $S(O)_nR_{23}$ ist;
$R_9$ $C_1$—$C_3$ alkyl, F, Cl, Br, $NO_2$, $CO_2R_{19}$, $SO_2NR_{20}R_{21}$, $SO_2N(OCH_3)CH_3$ oder $S(O)_nR_{23}$ ist;
$R_{10}$ Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ oder $OCH_2CH_3$ ist;
$R_{11}$ H, $CH_3$ oder $CH_2CH_3$ ist;
$R_{12}$ H, $CH_3$ oder $CH_2CH_3$ ist;
$R_{13}$ H oder $CH_3$ ist;
$R_{14}$ H oder $CH_3$ ist;
$R_{15}$ H oder $CH_3$ ist;
$R_{16}$ $CH_3$ oder $CH_2CH_3$ ist;
$R_{17}$ H oder $C_1$—$C_4$ Alkyl ist;
$R_{18}$ H oder $CH_3$ ist;
$R_{19}$ $C_1$—$C_4$ Alkyl, $CH_2CH_2OCH_3$, $CH_2CH_2Cl$ oder $CH_2CH=CH_2$ ist;
$R_{20}$ $C_1$—$C_3$ Alkyl ist;
$R_{21}$ $C_1$—$C_3$ Alkyl ist;
$R_{22}$ $C_1$—$C_3$ Alkyl oder $N(CH_3)_2$ ist;
$R_{23}$ $C_1$—$C_3$ Alkyl oder $CH_2CH=CH_2$ ist;
m 0 oder 1 ist;
n 0 oder 2 ist;
$Q_1$ O, S, $SO_2$ oder $NR_{17}$ ist;
$R_2$ O, S, oder $NR_{17}$ ist; und
W O, S oder $SO_2$ ist;

A 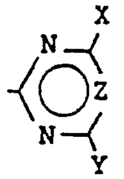 , 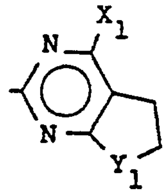 , 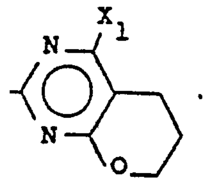 .

A-1 A-2 A-3

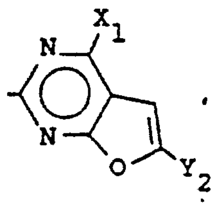 , 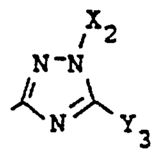 oder 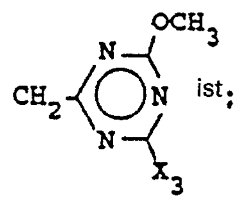 ist;

A-4 A-5 A-6

X $CH_3$, $OCH_3$, $OCH_2CH_3$, Cl, F, Br, $OCF_2H$, $CH_2F$ oder $CF_3$ ist;

Y H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

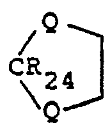 , 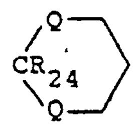 , 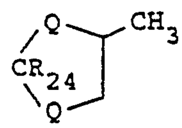

oder $CR_{24}(QCH_2CH_3)_2$ ist;

Q O oder S ist;

$R_{24}$ H oder $CH_3$ ist;

Z CH oder N ist;

$Y_1$ $CH_2$ oder O ist;

$X_1$ $CH_3$, $OCH_3$, $OCH_2CH_3$ oder $OCF_2H$ ist;

$Y_2$ H oder $CH_3$ ist;

$X_2$ $CH_3$, $CH_2CH_3$ oder $CH_2CF_3$ ist;

$Y_3$ $OCH_3$, $OCH_2CH_3$, $SCH_3$, $CH_3$ oder $CH_2CH_3$ ist;

$X_3$ $CH_3$ oder $OCH_3$ ist;

und ihre landwirtschaftlich geeigneten Salze, mit der Massgabe, dass

1) die Gesamtzahl von Kohlenstoffatomen von $R_{20}$ und $R_{21}$ weniger als oder gleich vier ist;

2) $R_{13}$ H ist, wenn m 1 ist;

3) wenn L L—17 ist, dann $R_{17}$ und $R_{18}$ nicht gleichzeitig H sind; und

4) wenn X Cl, F oder Br ist, dann Z CH ist und Y $OCH_3$, $OCH_2CH_3$, $N(CH_3)_2$ oder $OCF_2H$ ist;

wobei ein Isothioharnstoffderivat der Formel

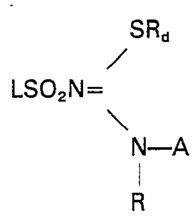 (III)

worin L, R, $R_d$ und A wie oben definiert sind, mit Hydroxylamin umgesetzt wird, um eine Verbindung der Formel (II) zu erhalten, worin $R_c$ H ist, und gegebenenfalls das Produkt mit $R_cCl$ oder $R_3NCO$ umgesetzt wird, worin $R_c$ $C(O)R_1$, $C(O)NR_2R_3$ oder $CO_2R_4$ ist und $R_1$, $R_2$, $R_3$ und $R_4$ wie oben definiert sind.

9. Verfahren von Anspruch 8, worin

R H oder $CH_3$ ist;

$R_c$ wie in Anspruch 8 definiert ist;

L L—1 ist;

$R_5$ $C_1$—$C_4$ Alkyl, $C_1$—$C_4$ Alkoxy, F, Cl, Br, $NO_2$, $CF_3$, $CO_2R_{19}$, $SO_2R_{20}R_{21}$, $SO_2N(OCH_3)CH_3$, $OSO_2R_{22}$, $S(O)_nR_{23}$, $WCF_3$, $WCHF_2$, oder mit $OCH_3$ oder $OC_2H_5$ substituiertes $C_1$—$C_2$ Alkyl ist;

$R_6$ H, F, Cl, Br, $CF_3$, $CH_3$, $OCH_3$ oder $SCH_3$ ist;
$R_{19}$, $R_{20}$, $R_{21}$, $R_{23}$ und W wie in Anspruch 14 definiert sind;
$R_{22}$ $C_1$—$C_3$ Alkyl ist;
A A—1 ist;
X $CH_3$, $OCH_3$ oder Cl ist;
Y $CH_3$, $C_2H_5$, $OCH_3$, $OC_2H_5$, $CH_2OCH_3$, $CH(OCH_3)_2$ oder

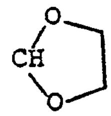 ist;

und Z wie in Anspruch 8 definiert ist.

10. Verfahren von Anspruch 8 oder 9, worin R H ist, $R_c$ H ist und A A—1 ist.

11. Verfahren von Anspruch 10, worin
Y $CH_3$, $OCH_3$, $OCH_2CH_3$, $CH_2OCH_3$, $OCH_2CF_3$ oder $CH(OCH_3)_2$ ist;
X $CH_3$, $OCH_3$, Cl oder $CF_3$ ist; und
L L—1, L—2, L—3, L—5, L—8, L—10, L—11, L—16 oder L—17 ist.

12. Verfahren von Anspruch 11 worin L L—1 ist, $R_5$ $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

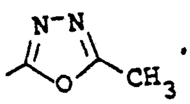, 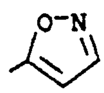, 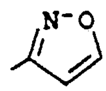 oder 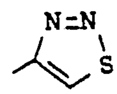 ist;

$R_{22}$ $C_1$—$C_3$ Alkyl ist, $R_{23}$ $CH_3$ ist und n 2 ist.

13. Verfahren von Anspruch 11, worin L L—2 ist und $R_7$, Cl, $CH_3$, $OCH_3$, $SCH_3$ oder Br ist; oder worin L L—3 ist und $R_8$ Cl, $SO_2CH_3$ oder $SO_2N(CH_3)_2$ ist; oder worin L L—5 ist und $R_9$ $CO_2CH_3$ oder $CO_2CH_2CH_3$ ist.

14. Verfahren von Anspruch 8 oder 9, worin das Produkt eine Verbindung ist, ausgewählt aus
2-[[1-(4,6-Dimethylpyrimidin-2-yl)amino]-1-(hydroxyimino)methyl]-aminosulfonyl]benzoesäuremethylester;
2-[[1-(4-Methoxy-6-methylpyrimidin-2-yl)amino]-1-(hydroxyimino)-methyl]aminosulfonyl]benzoesäuremethylester;
N',N'-Dimethyl-N-[[1-(4,6-dimethyl-1,3,5-triazin-2-yl)amino]-1-(hydroxyimino)methyl]-1,2-benzoldisulfonamid; und
N',N'-Dimethyl-N-[[1-(4-methoxy-6-methylpyrimidin-2-yl)amino]-1-(hydroxyimino)methyl]-1,2-benzoldisulfonamid.

15. Verfahren zur Bekämpfung des Wachstums unerwünschter Vegetation durch Aufbringung einer wirksamen Menge einer herbiziden Verbindung auf die zu schützende Stelle, dadurch gekennzeichnet, dass die herbizide Verbindung eine Verbindung der Formeln (I) oder (II), wie in einem der Ansprüche 1 bis 14 definiert, enthält.

16. Verfahren zur Regulierung des Wachstums von Pflanzen durch Aufbringung einer wirksamen, aber im wesentlichen nicht phytotoxischen Menge eines Pflanzenwachstumsreglers auf die zu schützende Stelle, dadurch gekennzeichnet, dass der Wachstumsregler eine Verbindung der Formeln (I) oder (II), wie in einem der Ansprüchhe 1 bis 14 definiert, enthält.

**Revendications pour les Etats contractants: BE CH DE FR GB IT LI LU NL SE**

1. Un composé de la formule:

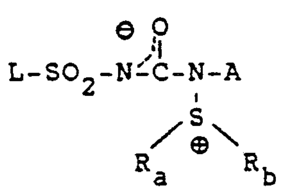 I

où
$R_a$ est $CH_3$ ou $CH_2CH_3$;
$R_b$ est $CH_3$ ou $CH_2CH_3$;

L est 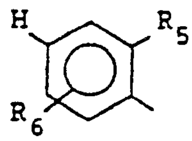 , 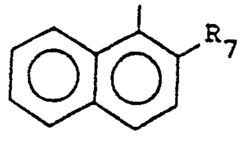 , 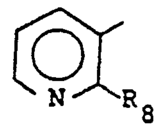 ,

L-1 L-2 L-3

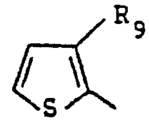 , 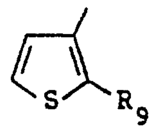 , 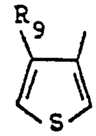 ,

L-4 L-5 L-6

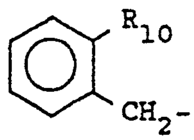 , 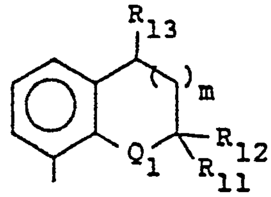 ,

L-7 L-8

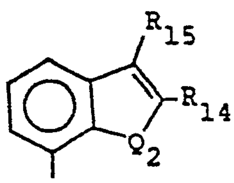 , 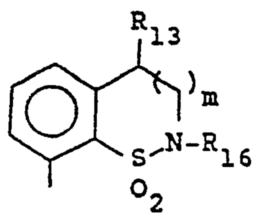 , 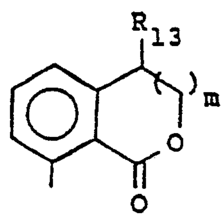 ,

L-9 L-10 L-11

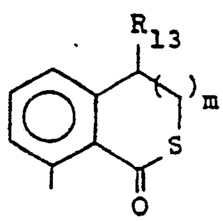 , 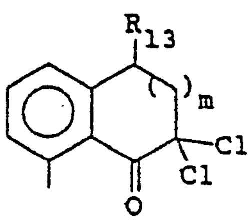 , 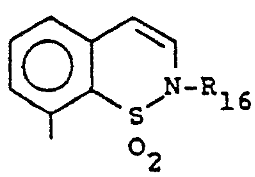 ,

L-12 L-13 L-14

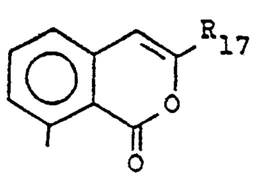 , 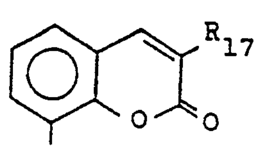 or 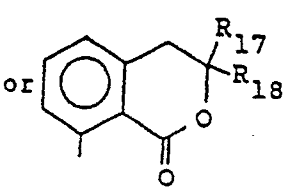 ;

L-15 L-16 L-17

$R_5$ est un radical alkyle en $C_1$—$C_4$, alcoxy en $C_1$—$C_4$, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, alcényloxy en $C_3$—$C_4$, alcynyloxy en $C_3$—$C_4$, alkyle en $C_1$—$C_2$ substitué par $OCH_3$ ou $OCH_2CH_3$, $C_6H_5$,

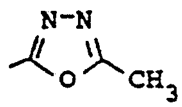 , 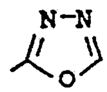 , 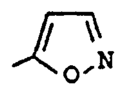 , 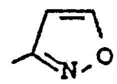 , 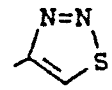 ,

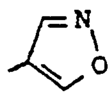 , 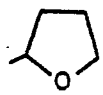 , 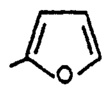 ou 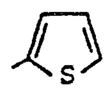 ;

$R_6$ est H, F, Cl, Br, $CF_3$, $CH_3$, $OCH_3$, $SCH_3$ ou $OCF_2H$;
$R_7$ est H, $CH_3$, $OCH_3$, F, Cl, Br, $OSO_2CH_3$, $SO_2N(OH_3)_2$ ou $S(O)_nCH_3$;
$R_8$ est $CH_3$, $CH_2CH_3$, $OCH_3$, $OCH_2CH_3$, F, Cl, Br, $SO_2NR_{20}R_{21}$, $SO_2N(OCH_3)CH_3$ ou $S(O)_nR_{23}$;
$R_9$ est un radical alkyle en $C_1$—$C_3$, F, Cl, Br, $NO_2$, $CO_2R_{19}$, $SO_2NR_{20}R_{21}$, $SO_2N(OCH_3)CH_3$ ou $S(O)_nR_{23}$;
$R_{10}$ est Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ ou $OCH_2CH_3$;
$R_{11}$ est H, $CH_3$ ou $CH_2CH_3$;
$R_{12}$ est H, $CH_3$ ou $CH_2CH_3$;
$R_{13}$ est H ou $CH_3$;
$R_{14}$ est H ou $CH_3$;
$R_{15}$ est H ou $CH_3$;
$R_{16}$ est $CH_3$ ou $CH_2CH_3$;
$R_{17}$ est H ou un radical alkyle en $C_1$—$C_4$;
$R_{18}$ est H ou $CH_3$;
$R_{19}$ est un radical alkyle en $C_1$—$C_4$, $CH_2CH_2OCH_3$, $CH_2CH_2Cl$ ou $CH_2CH=CH_2$;
$R_{20}$ est un radical alkyle en $C_1$—$C_3$;
$R_{21}$ est un radical alkyle en $C_1$—$C_3$;
$R_{22}$ est un radical alkyle en $C_1$—$C_3$ ou $N(CH_3)_2$;
$R_{23}$ est un radical alkyle en $C_1$—$C_3$ ou $CH_2CH=CH_2$;
m est 0 ou 1;
n est 0 ou 2;
$Q_1$ est O, S, $SO_2$ ou $NR_{17}$;
$Q_2$ est O, S ou $NR_{17}$; et
W est O, S ou $SO_2$;

A est 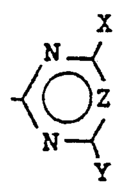 , 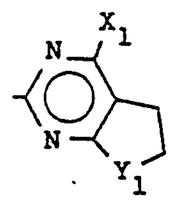 , 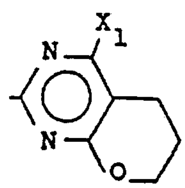 ,

A-1 A-2 A-3

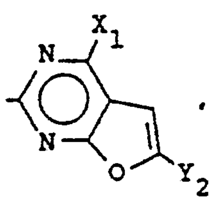 , 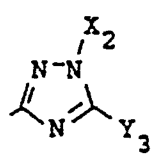 ou 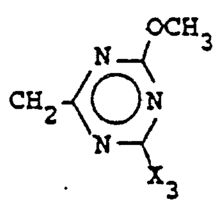 ;

A-4 A-5 A-6

X est $CH_3$, $OCH_3$, $OCH_2CH_3$, Cl, F, Br, $OCF_2H$, $CH_2F$ ou $CF_3$;
Y est H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH=CH_2$, $OCH_2C\equiv CH$, $OCH_2CF_3$, $OCH_2CH_2OCH_3$, $CH_2SCH_3$, $OCF_2H$, $SCF_2H$, $CR_{24}(QCH_3)_2$,

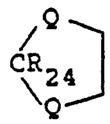, 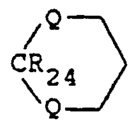, 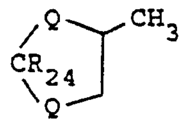

ou $CR_{24}(QCH_2CH_3)_2$;

Q est O ou S;

$R_{24}$ est H ou $CH_3$;

Z est CH ou N;

$Y_1$ est $CH_2$ ou O;

$X_1$ est $CH_3$, $OCH_3$, $OCH_2CH_3$ ou $OCF_2H$;

$Y_2$ est H ou $CH_3$;

$X_2$ est $CH_3$, $CH_2CH_3$ ou $CH_2CF_3$;

$Y_3$ est $OCH_3$, $OCH_2CH_3$, $SCH_3$, $CH_3$ ou $CH_2CH_3$;

$X_3$ est $CH_3$ ou $OCH_3$;

avec les conditions que

1) le nombre total d'atomes de carbone $R_{20}$ et $R_{21}$ est inférieur ou égal à quatre;

2) quand m est 1, alors $R_{13}$ est H;

3) quand L est L-17, alors $R_{17}$ et $R_{18}$ ne sont pas simultanément H;

4) quand X est Cl, F ou Br, alors Z est CH et Y est $OCH_3$, $OCH_2CH_3$, $N(CH_3)_2$ ou $OCF_2H$.

2. Un composé selon la revendication 1, dans lequel A est A-1.

3. Un composé selon la revendication 2, dans lequel Y est $CH_3$, $OCH_3$, $OCH_2CH_3$, $CH_2OCH_3$, $OCH_2CF_3$ ou $CH(OCH_3)_2$ et X est $CH_3$, $OCH_3$, Cl ou $CF_3$.

4. Un composé selon la revendication 3, dans lequel L est L-1, L-2, L-3, L-5, L-8, L-10, L-11, L-16 ou L-17.

5. Un composé selon la revendication 4, dans lequel L est L-1; $R_5$ est $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

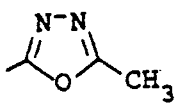, 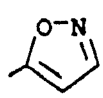, 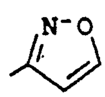 ou 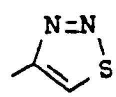 ;

$R_{22}$ est un radical alkyle en $C_1$—$C_3$; $R_{23}$ est $CH_3$ et n est 2.

6. Les composés selon la revendication 4, dans lesquels L est L-2 et $R_7$ est Cl, $CH_3$, $OCH_3$, $SCH_3$ ou Br.

7. Les composés selon la revendication 4, dans lesquels L est L-3 et $R_8$ est Cl, $SO_2CH_3$ ou $SO_2N(CH_3)_2$.

8. Les composés selon la revendication 4, dans lesquels L est L-5 et $R_9$ est $CO_2CH_3$ ou $CO_2CH_2CH_3$.

9. Le composé de la revendication 1, qui est le sel interne de diméthylsulfonium de l'ester méthylique de l'acide 2-[[(4-méthoxy-6-méthylpyrimidin-2-yl)aminocarbonyl]aminosulfonyl]benzoïque.

10. Le composé de la revendication 1, qui est le sel interne de diméthylsulfonium de l'ester méthylique de l'acide 2-[[(4,6-diméthoxypyrimidin-2-yl)-aminocarbonyl]aminosulfonyl]benzoïque.

11. Le composé de la revendication 1, qui est le sel interne de diméthylsulfonium du N-[(4,6-diméthylpyrimidin-2-yl)aminocarbonyl]-2-méthylsulfonylbenzènesulfonamide.

12. Le composé de la revendication 1, qui est le sel interne de diméthylsulfonium de l'ester méthylique de l'acide 2 - [[(4,6 - diméthylpyrimidin - 2 - yl)aminocarbonyl]aminosulfonyl]benzoïque.

13. Le composé de la revendication 1, qui est le sel interne de diméthylsulfonium du N-[(4,6-diméthoxy-pyrimidin-2-yl]aminocarbonyl]-2-diméthylaminosulfonylbenzènesulfonamide.

14. Un composé ayant la formule:

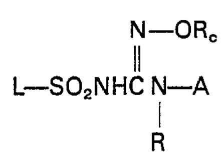 (II)

où

R est H ou $CH_3$;

$R_0$ est H, $C(O)R_1$, $C(O)NR_2R_3$ ou $CO_2R_4$;

$R_1$ est un radical alkyle en $C_1$—$C_3$ ou $CF_3$;

$R_2$ est H, $CH_3$ ou $C_2H_5$;

$R_3$ est un radical alkyle en $C_1$—$C_3$;

$R_4$ est un radical alkyle en $C_1$—$C_3$;

L est 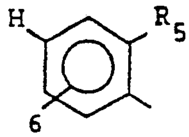 , 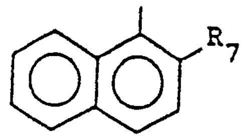 , 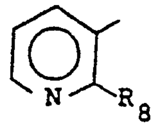 ,

L-1 L-2 L-3

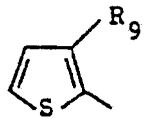 , 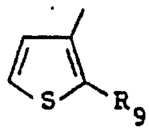 , 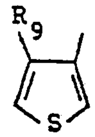 ,

L-4 L-5 L-6

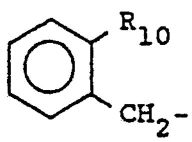 , 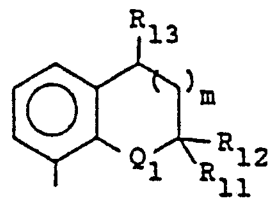 ,

L-7 L-8

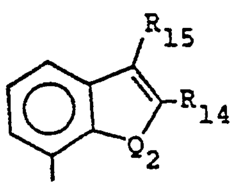 , 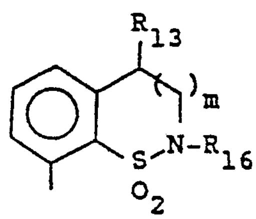 , 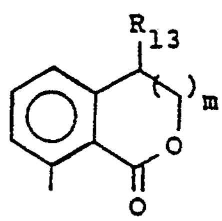 ,

L-9 L-10 L-11

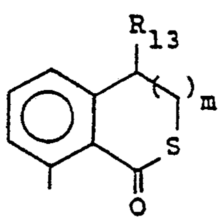 , 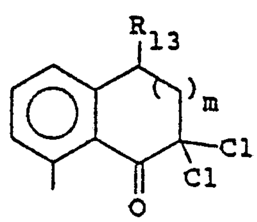 , 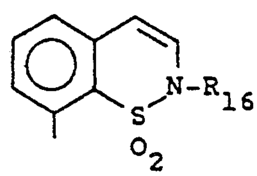 ,

L-12 L-13 L-14

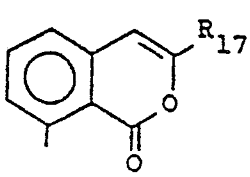 , 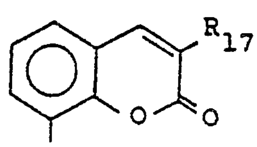 ou 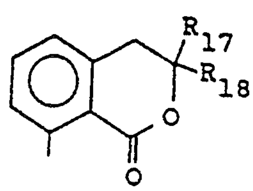 ;

L-15 L-16 L-17

$R_5$ est un radical alkyle en $C_1$—$C_4$, alcoxy en $C_1$—$C_4$, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, alcényloxy en $C_3$—$C_4$, alcynyloxy en $C_3$—$C_4$, alkyle en $C_1$—$C_2$ substitué par $OCH_3$ ou $OCH_2CH_3$, $C_6H_5$,

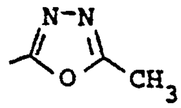 , 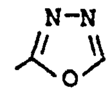 ,

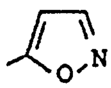 , 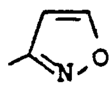 , 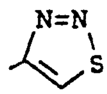 , 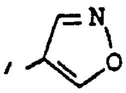 , 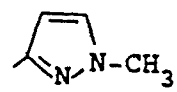 ,

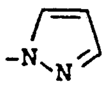 , 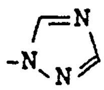 , 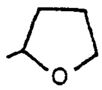 , 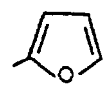 ou 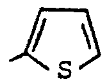 ;

$R_6$ est H, F, Cl, Br, $CF_3$, $CH_3$, $OCH_3$, $SCH_3$ ou $OCF_2H$;
$R_7$ est H, $CH_3$, $OCH_3$, F, Cl, Br, $SO_2N(CH_3)_2$, $OSO_2CH_3$ ou $S(O)_nCH_3$;
$R_8$ est $CH_3$, $CH_2CH_3$, $OCH_3$, $OCH_2CH_3$, F, Cl, Br, $SO_2NR_{20}R_{21}$, $SO_2N(OCH_3)CH_3$ ou $S(O)_nR_{23}$;
$R_9$ est un radical alkyle en $C_1$—$C_3$, F, Cl, Br, $NO_2$, $CO_2R_{19}$, $SO_2NR_{20}R_{21}$, $SO_2N(OCH_3)CH_3$ ou $S(O)_nR_{23}$;
$R_{10}$ est Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ ou $OCH_2CH_3$;
$R_{11}$ est H, $CH_3$ ou $CH_2CH_3$;
$R_{12}$ est H, $CH_3$ ou $CH_2CH_3$;
$R_{13}$ est H ou $CH_3$;
$R_{14}$ est H ou $CH_3$;
$R_{15}$ est H ou $CH_3$;
$R_{16}$ est $CH_3$ ou $CH_2CH_3$;
$R_{17}$ est H ou un radical alkyle en $C_1$—$C_4$;
$R_{18}$ est H ou $CH_3$;
$R_{19}$ est un radical alkyle en $C_1$—$C_4$, $CH_2CH_2OCH_3$, $CH_2CH_2Cl$ ou $CH_2CH=CH_2$;
$R_{20}$ est un radical alkyle en $C_1$—$C_3$;
$R_{21}$ est un radical alkyle en $C_1C_3$;
$R_{22}$ est un radical alkyle en $C_1$—$C_3$ ou $N(CH_3)_2$;
$R_{23}$ est un radical alkyle en $C_1$—$C_3$ ou $CH_2CH=CH_2$;
m est 0 ou 1;
n est 0 ou 2;
$Q_1$ est O, S, $SO_2$ ou $NR_{17}$;
$Q_2$ est O, S ou $NR_{17}$; et
W est O, S, ou $SO_2$;

A est 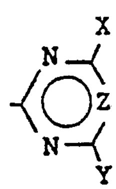 , 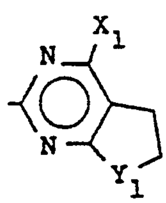 , 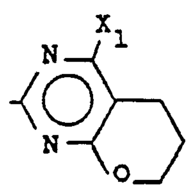 .

A-1 A-2 A-3

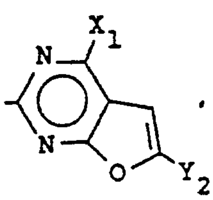 , 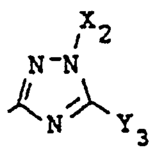 ou 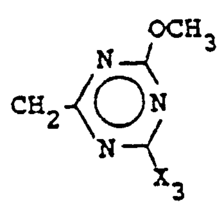 ;

A-4 A-5 A-6

X est $CH_3$, $OCH_3$, $OCH_2CH_3$, Cl, F, Br, $OCF_2H$, $CH_2F$ ou $CF_3$;

Y est H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

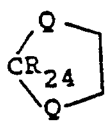 , 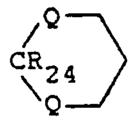 , 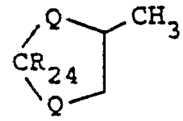

ou $CR_{24}(QCH_2CH_3)_2$;

Q est O ou S;

$R_{24}$ est H ou $CH_3$;

Z est CH ou N;

$Y_1$ est $CH_2$ ou O;

$X_1$ est $CH_3$, $OCH_3$, $OCH_2CH_3$ ou $OCF_2H$;

$Y_2$ est H ou $CH_3$;

$X_2$ est $CH_3$, $CH_2CH_3$ ou $CH_2CF_3$;

$Y_3$ est $OCH_3$, $OCH_2CH_3$, $SCH_3$, $CH_3$ ou $CH_2CH_3$;

$X_3$ est $CH_3$ ou $OCH_3$;

et leurs sels convenant pour l'agriculture; avec les conditions que:

1) le nombre total d'atomes de carbone de $R_{20}$ et $R_{21}$ est inférieur ou égal à quatre;

2) quand m est 1, alors $R_{13}$ est H;

3) quand L est L-17, alors $R_{17}$ et $R_{18}$ ne sont pas simultanément H; et

4) quand X est Cl, F ou Br, alors Z est CH et et Y est $OCH_3$, $OCH_2CH_3$, $N(CH_3)_2$ ou $OCF_2H$.

15. Un composé selon la revendication 14, dans lequel R est H, $R_0$ est H et A est A-1.

16. Un composé selon la revendication 15, dans lequel V est $CH_3$, $OCH_3$, $OCH_2CH_3$, $CH_2OCH_3$, $OCH_2CF_3$ ou $CH(OCH_3)_2$ et X est $CH_3$, $OCH_3$, Cl ou $CF_3$.

17. Un composé selon la revendication 16, dans lequel L est L-1, L-2, L-3, L-5, L-8, L-10, L-11, L-16 ou L-17.

18. Un composé selon la revendication 17, dans lequel est L-1, $R_5$ est $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH_2$, $OCH_2C{\equiv}CH$,

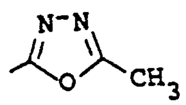 , 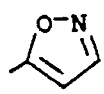 , 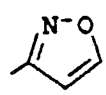 ou 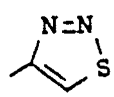 ;

$R_{22}$ est un radical alkyle en $C_1$—$C_3$; $R_{23}$ est $CH_3$ et n est 2.

19. Un composé selon la revendication 17, dans lequel L est L-2 et $R_7$ est Cl, $CH_3$, $OCH_3$, $SCH_3$ ou Br.

20. Un composé selon la revendication 17, dans lequel L est L-3 et $R_8$ est Cl, $SO_2CH_3$ ou $SO_2N(CH_3)_2$.

21. Un composé selon la revendication 17, dans lequel L est L-5 et $R_9$ est $CO_2CH_3$ ou $CO_2CH_2CH_3$.

22. Un composé selon la revendication 14, qui est l'ester méthylique de l'acide 2-[[1-(4,6-diméthylpyrimidin-2-yl)amino]-1-(hydroxyimino)méthyl]aminosulfonyl]benzoïque.

23. Un composé selon la revendication 14, qui est l'ester méthylique de l'acide 2-[[1-(4-méthoxy-6-méthylpyrimidin-2-yl)amino]-1-(hydroxyimino)méthyl]aminosulfonyl]benzoïque.

24. Un composé selon la revendication 14, qui est le N',N'-diméthyl-N-[[1-(4,6-diméthyl-1,3,5-triazin-2-yl)amino]-1-(hydroxyimino) méthyl]-1,2-benzènesulfonamide.

25. Un composé selon la revendication 14, qui est le N',N'-diméthyl-N-[[4-méthoxy-6-méthylpyrimidin-2-yl)amino]-1-(hydroxyimino)méthyl]-1,2-benzènedisulfonamide.

26. Un composé selon la revendication 1, dans lequel L est L-1, L-2, L-3, L-4, L-5, L-8, L-10, L-11 ou L-17;

$R_5$ est un radical alkyle en $C_1$—$C_4$, alcoxy en $C_1$—$C_4$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, alcényloxy en $C_3$—$C_4$, $CH_2CH_2OCH_3$, $CH_2CH_2OC_2H_5$,

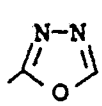 , 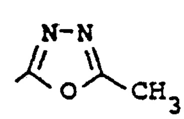 , 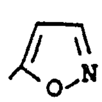 , 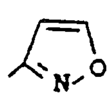 ou 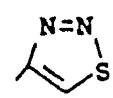 ;

$R_6$ est H, F, Cl, Br, $CF_3$, $CH_3$ ou $OCH_3$;

$R_7$ est H, $CH_3$, $OCH_3$, Cl, $OSO_2CH_3$ ou $S(O)_nCH_3$;

$R_8$ est $CH_3$, $OCH_3$, Cl, $SO_2NR_{20}R_{21}$ ou $SO_2N(OCH_3)CH_3$;

$R_9$ est $CH_3$, Cl, Br, $NO_2$, $CO_2R_{19}$, $SO_2NR_{20}R_{21}$, $SO_2N(OCH_3)CH_3$ ou $S(O)_nR_{13}$;

$R_{11}$ est H ou $CH_3$;

$R_{12}$ est H ou $CH_3$;
$R_{13}$ est H;
$R_{17}$ et $R_{18}$ sont H;
$R_{22}$ est un radical alkyle en $C_1—C_3$;
$Q_1$ est O, S ou $SO_2$;
A est A-1;
X est $CH_3$ ou $OCH_3$; ou peut être Cl quand Y est $CH_3$ ou $OCH_3$;
Y est H, $CH_3$, $OCH_3$, $OCH_2CH_3$, $CH_2OCH_3$, $CH_2CH_3$, $CF_3$, $OCH_2CF_3$, $CH(OCH_3)_2$;
et $R_a$, $R_b$, $R_{16}$, $R_{19}$, $R_{20}$, $R_{21}$, $R_{23}$, m, n et Z sont tels que définis à la revendication 1.

27. Un composé selon la revendication 14, dans lequel:
R est H ou $CH_3$;
$R_c$ est tel que défini à la revendication 14;
L est L-1;
$R_5$ est un radical alkyle en $C_1—C_4$, alcoxy en $C_1—C_4$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, ou alkyle en $C_1—C_2$ substitué par $OCH_3$ ou $OC_2H_5$;
$R_6$ est H, F, Cl, Br, $CF_3$, $CH_3$, $OCH_3$ ou $SCH_3$; $R_{19}$, $R_{20}$, $R_{21}$, $R_{23}$ et W sont tels que définis à la revendication 14;
$R_{22}$ est un radical alkyle en $C_1—c_3$;
A est A-1;
X est $CH_3$, $OCH_3$ ou Cl;
Y est $CH_3$, $C_2H_5$, $OCH_3$, $OC_2H_5$, $CH_2CH_3$, $CH(OCH_3)_2$ ou

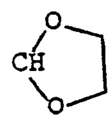

et Z est tel que défini à la revendication 14.

28. Une composition convenant pour lutter contre la croissance de végétation indésirable ou pour régler la croissance de plantes, qui comprend une quantité efficace d'un composé herbicide ou d'un composé régulateur de la croissance de plantes et au moins l'un des ingrédients suivants: un agent tensioactif, un diluant solide ou liquide, caractérisée en ce que ledit composé herbicide ou ledit composé régulateur de la croissance de plantes comprend un composé selon l'une quelcoque des revendications 1 à 27.

29. Un procédé pour lutter contre la croissance de végétation indésirable en appliquant à l'endroit à protéger une quantité efficace d'un composé herbicide, caractérisé en ce que ledit composé herbicide comprend un composé selon l'une quelconque des revendications 1 à 27.

30. Un procédé pour régler la croissance de plantes en appliquant à l'endroit desdites plantes une quantité efficace mais substantiellement non-phytotoxique d'un régulateur de la croissance de plantes, caractérisé en ce que ledit régulateur de la croissance de plantes comprend un composé selon l'une quelconque des revendications 1 à 27.

31. Un procédé de préparation d'un composé de la revendication 1, qui comprend la réaction d'un isocyanate de sulfonyle de la formule

$$LSO_2NCO \qquad (IV)$$

où L est tel que défini à la revendication 1, avec une S,S-dialkyle-N-(hétérocycle)sulfilimine de formule:

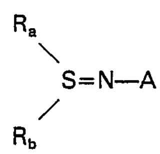

(V)

où $R_a$, $R_b$ et A sont tels que définis à la revendication 1.

32. Un procédé de préparation d'un composé de la revendication 14, qui comprend la réaction d'un dérivé d'isothiourée de formule:

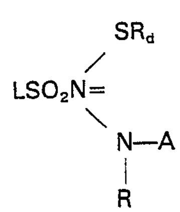

(III)

où L, R, $R_d$ et A sont tels que définis à la revendication 26, avec de l'hydroxylamine, pour obtenir un composé de la revendication 14 dans lequel $R_c$ est H, et, facultativement, la réaction du produit avec $R_cCl$ ou $R_3NCO$, où $R_c$ est $C(O)R_1$, $C(O)NR_2R_3$ ou $CO_2R_4$ et $R_1$, $R_2$, $R_3$ et $R_4$ sont tels que définis à la revendication 14.

**Revendications pour l'Etat contractant: AT**

1. Un procédé de préparation d'un composé

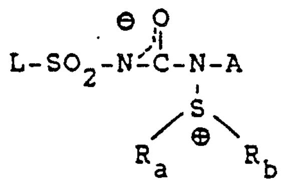

où

$R_a$ est $CH_3$ ou $CH_2CH_3$;
$R_b$ est $CH_3$ ou $CH_2CH_3$;

L est 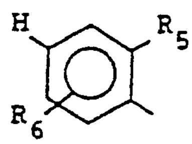, 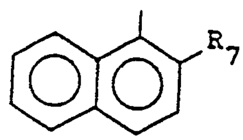, 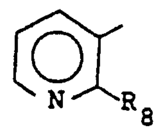,

L-1 L-2 L-3

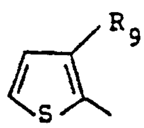, 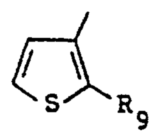, 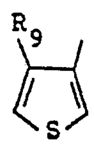,

L-4 L-5 L-6

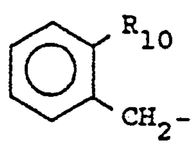, 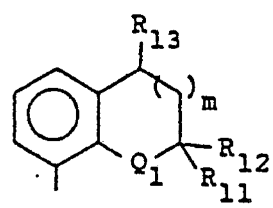,

L-7 L-8

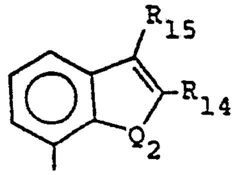, 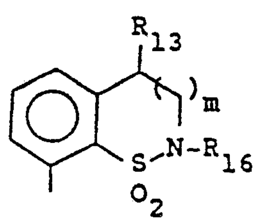, 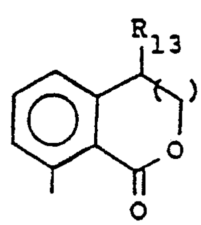,

L-9 L-10 L-11

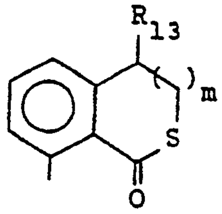, 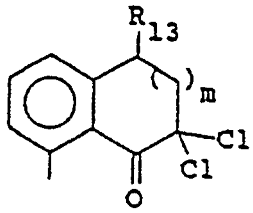, 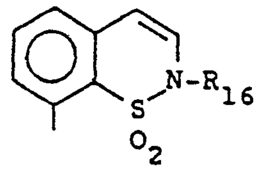,

L-12 L-13 L-14

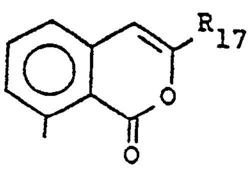, 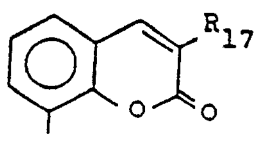 ou 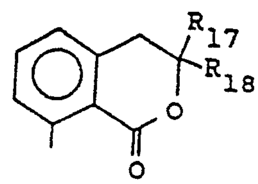 ;

L-15 L-16 L-17

$R_5$ est un radical alkyle en $C_1$—$C_4$, alcoxy en $C_1$—$C_4$, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2r_{22}$, $S(O)_nR_{23}$, $WCF_3$, $WCHF_2$, alcényloxy en $C_3$—$C_4$, alcynyloxy en $C_3$—$C_4$, alkyle en $C_1$—$C_2$ substitué par $OCH_3$ ou $OCH_2CH_3$, $C_6H_5$,

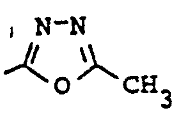, 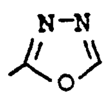, 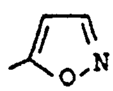, 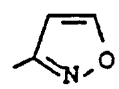, 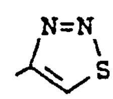,

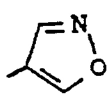, 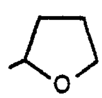, 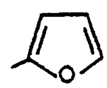 ou 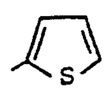 ;

$R_6$ est H, F, Cl, Br, $CF_3$, $CH_3$, $OCH_3$, $SCH_3$ ou $OCF_2H$;
$R_7$ est H, $CH_3$, $OCH_3$, F, Cl, Br, $OSO_2CH_3$, $SO_2N(CH_3)_2$ ou $S(O)_nCH_3$;
$R_8$ est $CH_3$, $CH_2CH_3$, $OCH_3$, $OCH_2CH_3$, F, Cl, Br, $SO_2NR_{20}R_{21}$, $SO_2N(OCH_3)CH_3$ ou $S(O)_nR_{23}$;
$R_9$ est un radical alkyle en $C_1$—$C_3$, F, Cl, Br, $NO_2$, $CO_2R_{19}$, $SO_2NR_{20}R_{21}$, $SO_2N(OCH_3)CH_3$ ou $S(O)_nR_{23}$;
$R_{10}$ est Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ ou $OCH_2CH_3$;
$R_{11}$ est H, $CH_3$ ou $CH_2CH_3$;
$R_{12}$ est H, $CH_3$ ou $CH_2CH_3$;
$R_{13}$ est H ou $CH_3$;
$R_{14}$ est H ou $CH_3$;
$R_{15}$ est H ou $CH_3$;
$R_{16}$ est $CH_3$ ou $CH_2CH_3$;
$R_{17}$ est H ou un radical alkyle en $C_1$—$C_4$;
$R_{18}$ est H ou $CH_3$;
$R_{19}$ est un radical alkyle en $C_1$—$C_4$, $CH_2CH_2OCH_3$, $CH_2CH_2Cl$ ou $CH_2CH{=}CH_2$;
$R_{20}$ est un radical alkyle en $C_1$—$C_3$;
$R_{21}$ est un radical alkyle en $C_1$—$C_3$;
$R_{22}$ est un radical alkyle en $C_1$—$C_3$ ou $N(CH_3)_2$;
$R_{23}$ est un radical alkyle en $C_1$—$C_3$ ou $CH_2CH{=}CH_2$;
m est 0 ou 1;
n est 0 ou 2;
$Q_1$ est O, S, $SO_2$ ou $NR_{17}$;
$Q_2$ est O, S ou $NR_{17}$; et
W est O, S ou $SO_2$;

A est 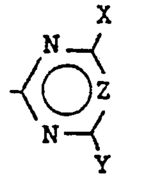 , 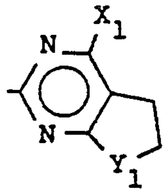 , 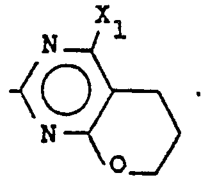 ,

A-1 A-2 A-3

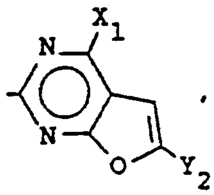 , 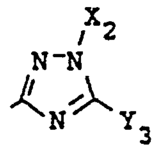 ou 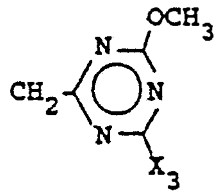 ;

A-4 A-5 A-6

X est $CH_3$, $OCH_3$, $OCH_2CH_3$, Cl, F, Br, $OCF_2H$, $CH_2F$ ou $CF_3$;

Y est H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

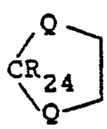 , 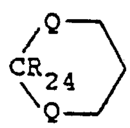 , 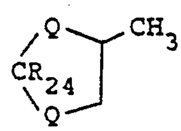

ou $CR_{24}(QCH_2CH_3)_2$;

Q est O ou S;

$R_{24}$ est H ou $CH_3$;

Z est CH ou N;

$Y_1$ est $CH_2$ ou O;

$X_1$ est $CH_3$, $OCH_3$, $OCH_2CH_3$ ou $OCF_2H$;

$Y_2$ est H ou $CH_3$;

$X_2$ est $CH_3$, $CH_2CH_3$ ou $CH_2CF_3$;

$Y_3$ est $OCH_3$, $OCH_2CH_3$, $SCH_3$, $CH_3$ ou $CH_2CH_3$;

$X_3$ est $CH_3$ ou $OCH_3$;

avec les conditions que

1) le nombre total d'atomes de carbone $R_{20}$ et $R_{21}$ est inférieur ou égal à quatre;

2) quand m est 1, alors $R_{13}$ est H;

3) quand L est L-17, alors $R_{17}$ et $R_{18}$ ne sont pas simultanément H;

4) quand X est Cl, F ou Br, alors Z est CH et Y est $OCH_3$, $OCH_2CH_3$, $N(CH_3)_2$ ou $OCF_2H$,

qui comprend la réaction d'un isocyanate de sulfonyle de la formule:

$$LSO_2NCO \qquad (IV)$$

où L est tel que défini ci-dessus, avec une S,S-dialkyle-N-(hétérocycle)sulfilimine de formule

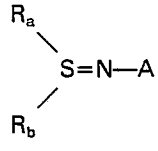 (V)

où $R_a$, $R_b$ et A sont tels que définis ci-dessus.

2. Un procédé selon la revendication 1, dans lequel

L est L-1, L-2, L-3, L-4, L-5, L-8, L-10, L-11 ou L-17;

$R_5$ est un radical alkyle en $C_1$—$C_4$, alcoxy en $C_1$—$C_4$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $OCF_3$, $OCHF_2$, alcényloxy en $C_3$—$C_4$, $CH_2CH_2OCH_3$, $CH_2CH_2OC_2H_5$,

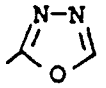 , 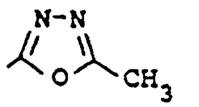 , 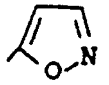 , 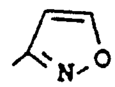 ou 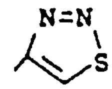 ;

$R_6$ est H, F, Cl, Br, $CF_3$, $CH_3$ ou $OCH_3$;
$R_7$ est H, $CH_3$, $OCH_3$, Cl, $OSO_2CH_3$ ou $S(O)_nCH_3$;
$R_8$ est $CH_3$, $OCH_3$, Cl, $SO_2NR_{20}R_{21}$ ou $SO_2N(OCH_3)CH_3$;
$R_9$ est $CH_3$, Cl, Br, $NO_2$, $CO_2R_{19}$, $SO_2NR_{20}R_{21}$, $SO_2N(OCH_3)CH_3$ ou $S(O)_nR_{13}$;
$R_{11}$ est H ou $CH_3$;
$R_{12}$ est H ou $CH_3$;
$R_{13}$ est H;
$R_{17}$ et $R_{18}$ sont H;
$R_{22}$ est un radical alkyle en $C_1$—$C_3$;
$Q_1$ est O, S ou $SO_2$;
A est A-1;
X est $CH_3$ ou $OCH_3$; ou peut être Cl quand Y est $CH_3$ ou $OCH_3$;
Y est H, $CH_3$, $OCH_3$, $OCH_2CH_3$, $CH_2OCH_3$, $CH_2CH_3$, $CF_3$, $OCH_2CF_3$, $CH(OCH_3)_2$;
et $R_a$, $R_b$, $R_{16}$, $R_{19}$, $R_{20}$, $R_{21}$, $R_{23}$, m, n et Z sont tels que définis à la revendication 1.

3. Un procédé selon la revendication 1, dans lequel A est A-1.

4. Un procédé selon la revendication 3, dans lequel V est $CH_3$, $OCH_3$, $OCH_2CH_3$, $CH_2OCH_3$, $OCH_2CF_3$ ou $CH(OCH_3)_2$ et X est $CH_3$, $OCH_3$, Cl ou $CF_3$; et L est L-1, L-2, L-3, L-5, L-8, L-10, L-11, L-16 ou L-17.

5. Un procédé selon la revendication 4, dans lequel L est L-1, $R_5$ est $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_3$, $SCF_2H$, $OCH_2CH{=}CH_2$, $OCH_2C{\equiv}CH$,

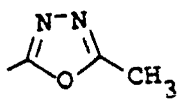 , 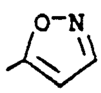 , 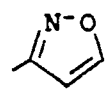 ou 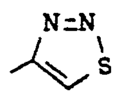 ;

$R_{22}$ est un radical alkyle en $C_1$—$C_3$; $R_{23}$ est $CH_3$ et n est 2.

6. Un procédé selon la revendication 4, dans lequel L est L-2 et $R_7$ est Cl, $CH_3$, $OCH_3$, $SCH_3$ ou Br; ou dans lequel L est L-3 et $R_8$ est Cl, $SO_2CH_3$ ou $SO_2N(CH_3)_2$; ou dans lequel L est L-5 et $R_9$ est $CO_2CH_3$ ou $CO_2CH_2CH_3$.

7. Un procédé selon la revendication 1 ou 2, dans lequel le produit est un composé choisi parmi, le sel interne de diméthylsulfonium de l'ester méthylique de l'acide 2-[[(4-méthoxy-6-méthylpyrimidin-2-yl)-aminocarbonyl]aminosulfonyl]benzoïque;
le sel interne de diméthylsulfonium de l'ester méthylique de l'acide 2-[[(4,6-diméthoxypyrimidin-2-yl)aminocarbonyl]aminosulfonyl]benzoïque;
le sel interne de diméthylsulfonium du N-[(4,6-diméthylpyrimidin-2-yl)aminocarbonyl]-2-méthylsulfonylbenzènesulfonamide;
le sel interne de diméthylsulfonium de l'ester méthylique de l'acide 2-[[(4,6-diméthylpyrimidin-2-yl)aminocarbonyl]aminosulfonyl]benzoïque; et
le sel interne de diméthylsulfonium du N-[(4,6-diméthoxypyrimidin-2-yl)aminocarbonyl]-2-diméthylaminosulfonylbenzenesulfonamide.

8. Un procédé de préparation d'un composé

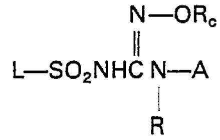 (II)

où
R est H ou $CH_3$;
$R_0$ est H, $C(O)R_1$, $C(O)NR_2R_3$ ou $CO_2R_4$;
$R_1$ est un radical alkyle en $C_1$—$C_3$ ou $CF_5$;
$R_2$ est H, $CH_3$ ou $C_2H_5$;
$R_3$ est un radical alkyle en $C_1$—$C_3$;
$R_4$ est un radical alkyle en $C_1$—$C_3$;

L est 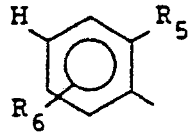 , 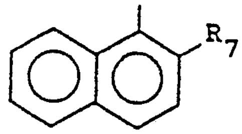 , 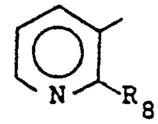 ,

L-1 L-2 L-3

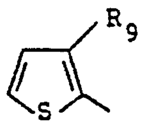 , 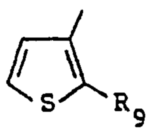 , 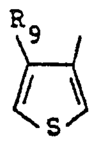 ,

L-4 L-5 L-6

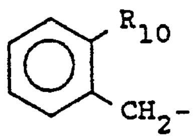 , 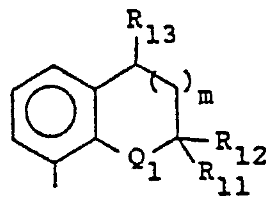 ,

L-7 L-8

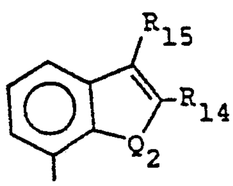 , 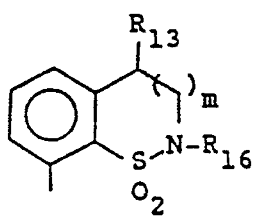 , 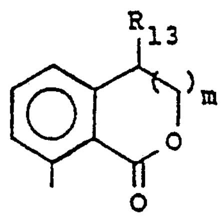 ,

L-9 L-10 L-11

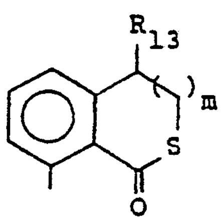 , 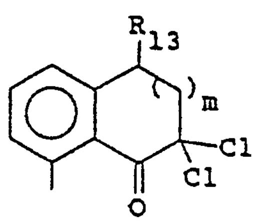 , 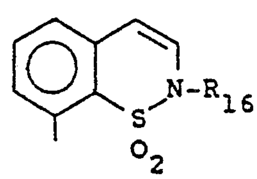 ,

L-12 L-13 L-14

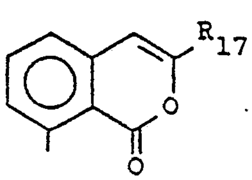 , 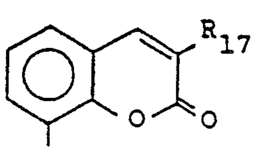 ou 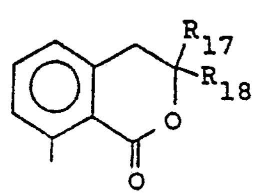 ;

L-15 L-16 L-17

$R_5$ est un radical alkyle en $C_1$—$C_4$, alcoxy en $C_1$—$C_4$, $OCH_2CH_2OCH_3$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, alcényloxy en $C_3$—$C_4$, alcynyloxy en $C_3$—$C_4$, alkyle en $C_1$—$C_2$ substitué par $OCH_3$ ou $OCH_2CH_3$, $C_6H_5$,

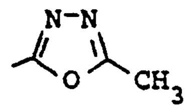, 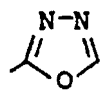,

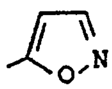, 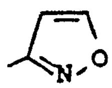, 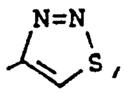, 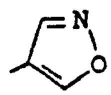, 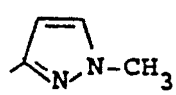,

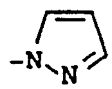, 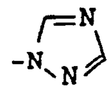, 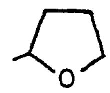, 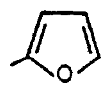 ou 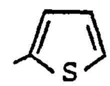;

$R_6$ est H, F, Cl, Br, $CF_3$, $CH_3$, $OCH_3$, $SCH_3$ ou $OCF_2H$;
$R_7$ est H, $CH_3$, $OCH_3$, F, Cl, Br, $SO_3N(CH_3)_2$, $OSO_2CH_3$ ou $S(O)_nCH_3$;
$R_8$ est $CH_3$, $CH_2CH_3$, $OCH_3$, $OCH_2CH_3$, F, Cl, Br, $SO_2NR_{20}R_{21}$, $SO_2N(OCH_3)CH_3$ ou $S(O)_nR_{23}$;
$R_9$ est un radical alkyle en $C_1$—$C_3$, F, Cl, Br, $NO_2$, $CO_2R_{19}$, $SO_2NR_{20}R_{21}$, $SO_2N(OCH_3)CH_3$ ou $S(O)_nR_{23}$;
$R_{10}$ est Cl, $NO_2$, $CO_2CH_3$, $CO_2CH_2CH_3$, $SO_2N(CH_3)_2$, $OSO_2CH_3$, $SO_2CH_3$, $SO_2CH_2CH_3$, $OCH_3$ ou $OCH_2CH_3$;
$R_{11}$ est H, $CH_3$ ou $CH_2CH_3$;
$R_{12}$ est H, $CH_3$ ou $CH_2CH_3$;
$R_{13}$ est H ou $CH_3$;
$R_{14}$ est H ou $CH_3$;
$R_{15}$ est H ou $CH_3$;
$R_{16}$ est $CH_3$ ou $CH_2CH_3$;
$R_{17}$ est H ou un radical alkyle en $C_1$—$C_4$;
$R_{18}$ est H ou $CH_3$;
$R_{19}$ est un radical alkyle en $C_1$—$C_4$, $CH_2CH_2OCH_3$, $CH_2CH_2Cl$ ou $CH_2CH{=}CH_2$;
$R_{20}$ est un radical alkyle en $C_1$—$C_3$;
$R_{21}$ est un radical alkyle en $C_1$—$C_3$;
$R_{22}$ est un radical alkyle en $C_1$—$C_3$ ou $N(CH_3)_2$;
$R_{23}$ est un radical alkyle en $C_1$—$C_3$ ou $CH_2CH{=}CH_2$;
m est 0 ou 1;
n est 0 ou 2;
$Q_1$ est O, S, $SO_2$ ou $NR_{17}$;
$Q_2$ est O, S ou $NR_{17}$; et
W est O, S, ou $SO_2$;

A est 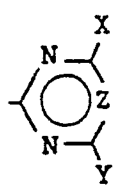, 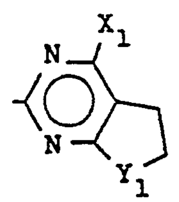, 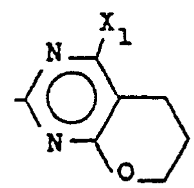.

A-1 A-2 A-3

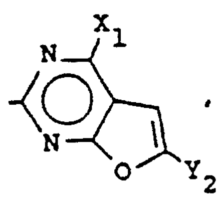, 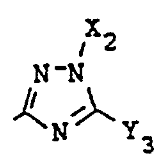 ou 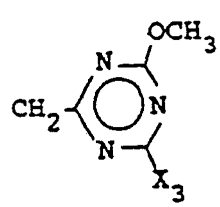;

A-4 A-5 A-6

X est $CH_3$, $OCH_3$, $OCH_2CH_3$, Cl, F, Br, $OCF_2H$, $CH_2F$ ou $CF_3$;

Y est H, $CH_3$, $OCH_3$, $OCH_2CH_3$, $CH_2OCH_3$, $CH_2OCH_2CH_3$, $N(CH_3)_2$, $CH_2CH_3$, $CF_3$, $SCH_3$, $OCH_2CH{=}CH_2$, $OCH_2C{\equiv}CH$, $OCH_2CF_3$, $OCH_2CH_2OCH_3$, $CH_2SCH_3$, $OCF_2H$, $SCF_2H$, $CR_{24}(QCH_3)_2$,

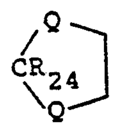 , 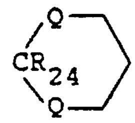 , 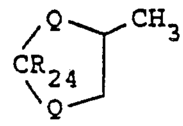

ou $CR_{24}(QCH_2CH_3)_2$;

Q est O ou S;

$R_{24}$ est H ou $CH_3$;

Z est CH ou N;

$Y_1$ est $CH_2$ ou O;

$X_1$ est $CH_3$, $OCH_3$, $OCH_2CH_3$ ou $OCF_2H$;

$Y_2$ est H ou $CH_3$;

$X_2$ est $CH_3$, $CH_2CH_3$ ou $CH_2CF_3$;

$Y_3$ est $OCH_3$, $OCH_2CH_3$, $SCH_3$, $CH_3$ ou $CH_2CH_3$;

$X_3$ est $CH_3$ ou $OCH_3$;

et leurs sels convenant pour l'agriculture; avec les conditions que:

1) le nombre total d'atomes de carbone de $R_{20}$ et $R_{21}$ est inférieur ou égal à quatre;

2) quand m est 1, alors $R_{13}$ est H;

3) quand L est L-17, alors $R_{17}$ et $R_{18}$ ne sont pas simultanément H; et

4) quand X est Cl, F ou Br, alors Z est CH et Y est $OCH_2$, $OCH_2CH_3$, $N(CH_3)_2$ ou $OCF_2H$, qui comprend la réaction d'un dérivé d'isothiourée de formule

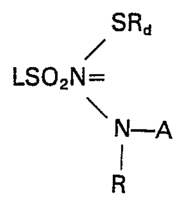

(III)

où L, R, $R_d$ et A sont tels que définis ci-dessus, avec de l'hydroxylamine, pour obtenir un composé de formule (II) dans lequel $R_c$ est H, et, facultativement, la réaction du produit avec $R_cCl$ ou $R_3NCO$, où $R_c$ est $C(O)R_1$, $C(O)NR_2R_3$ ou $CO_2R_4$ et $R_1$, $R_2$, $R_3$ et $R_4$ sont tels que définis ci-dessus.

9. Un procédé selon la revendication 8, dans lequel

R est H ou $CH_3$;

$R_c$ est tel que défini à la revendication 8;

L est L-1;

$R_5$ est un radical alkyle en $C_1$—$C_4$, alcoxy en $C_1$—$C_4$, F, Cl, Br, $NO_2$, $CF_3$, $CO_2R_{19}$, $SO_2NR_{20}R_{21}$, $SO_2N(OCH_3)CH_3$, $OSO_2R_{22}$, $S(O)_nR_{23}$, $WCF_3$, $WCHF_2$, ou alkyle en $C_1$—$C_2$ substitué par $OCH_3$ ou $OC_2H_5$;

$R_6$ est H, F, Cl, Br, $CF_3$, $CH_3$, $OCH_3$ ou $SCH_3$; $R_{19}$, $R_{20}$, $R_{21}$, $R_{23}$ et W sont tels que définis à la revendication 8;

$R_{22}$ est un radical alkyle en $C_1$—$C_3$;

A est A-1;

X est $CH_3$, $OCH_3$ ou Cl;

Y est $CH_3$, $C_2H_5$, $OCH_3$, $OC_2H_5$, $CH_2OCH_3$, $CH(OCH_3)_2$ ou

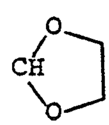

et Z est tel que défini à la revendication 8.

10. Un procédé selon la revendication 8 ou 9, dans lequel R est H, $R_c$ est H et A est A-1.

11. Un procédé selon la revendication 10, dans lequel V est $CH_3$, $OCH_3$, $OCH_2CH_3$, $CH_2OCH_3$, $OCH_2CF_3$ ou $CH(OCH_3)_2$ et X est $CH_3$, $OCH_3$, Cl ou $CF_3$; et L est L-1, L-2, L-3, L-5, L-8, L-10, L-11, L-16 ou L-17.

12. Un procédé selon la revendication 11, dans lequel L est L-1, $R_5$ est $OCH_3$, $OCH_2CH_3$, Cl, $NO_2$, $CF_3$, $CO_2CH_3$, $CO_2CH_2CH_3$, $SO_2N(CH_3)_2$, $SO_2N(OCH_3)CH_3$, $OSO_2R_{22}$, $S(O)_nR_{23}$, $OCF_2H$, $SCF_2H$, $OCH_2CH{=}CH$, $OCH_2C{\equiv}CH$,

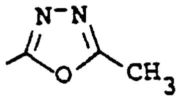, 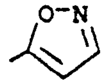, 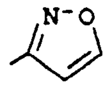 ou 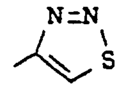 ;

$R_{22}$ est un radical alkyle en $C_1$—$C_3$; $R_{23}$ est $CH_3$ et n est 2.

13. Un procédé selon la revendication 11, dans lequel L est L-2 et $R_7$ est Cl, $CH_3$, $OCH_3$, $SCH_3$ ou Br; ou dans lequel L est L-3 et $R_8$ est Cl, $SO_2CH_3$ ou $SO_2N(CH_3)_2$; ou dans lequel L est L-5 et $R_9$ est $CO_2CH_3$ ou $CO_2CH_2CH_3$.

14. Un procédé selon la revendication 8 ou 9, dans lequel le produit est un composé choisi parmi l'ester méthylique de l'acide 2-[[1-(4,6-diméthylpyrimidin-2-yl)amino]-1-(hydroxyimino)méthyl]aminosulfonyl]benzoïque;
l'ester méthylique de l'acide 2-[[1-(4-méthoxy-6-méthylpyrimidin-2-yl)amino]-1-(hydroxyimino)méthyl]-aminosulfonyl]benzoïque;
le N',N'-diméthyl-N-[[1-(4,6-diméthyl-1,3,5-triazin-2-yl)amino]-1-(hydroxyimino)méthyl]-1,2-benzènedisulfonamide;
le N',N'-diméthyl-N-[[1-(4-méthoxy-6-méthylpyrimidin-2-yl)amino]-1-(hydroxyimino)méthyl]-1,2-benzènesulfonamide.

15. Un procédé pour lutter contre la croissance de végétation indésirable en appliquant à l'endroit à protéger une quantité efficace d'un composé herbicide, caractérisé en ce que ledit composé herbicide comprend un composé de formule (I) ou (II) tel que défini à l'une quelconque des revendications 1 à 14.

16. Un procédé pour régler la croissance de plantes en appliquant à l'endroit desdites plantes une quantité efficace mais substantiellement non-phytotoxique d'un régulateur de la croissance de plantes, caractérisé en ce que ledit régulateur de la croissance de plantes comprend un composé de formule (I) ou (II) tel que défini à l'une quelconque des revendications 1 à 14.